(19) Europäisches Patentamt
European Patent Office
Office européen des brevets

(11) EP 0 652 262 B1

(12) EUROPÄISCHE PATENTSCHRIFT

(45) Veröffentlichungstag und Bekanntmachung des Hinweises auf die Patenterteilung:
**12.07.2000 Patentblatt 2000/28**

(51) Int. Cl.[7]: **C09B 62/002**, C07C 317/08, C07C 315/02, D06P 1/38

(21) Anmeldenummer: **94116949.2**

(22) Anmeldetag: **26.10.1994**

(54) **Reaktivfarbstoffe, deren Herstellung und Verwendung**

Reactive dyestuffs, their preparation and use

Colorants réactifs, leur procédé de préparation et leur utilisation

(84) Benannte Vertragsstaaten:
**CH DE FR GB LI**

(30) Priorität: **08.11.1993 DE 4338117**

(43) Veröffentlichungstag der Anmeldung:
**10.05.1995 Patentblatt 1995/19**

(73) Patentinhaber: **BAYER AG**
**51368 Leverkusen (DE)**

(72) Erfinder:
- **Kunder, Klaus, Dr.**
**D-53819 Neunkirchen-Seelscheid (DE)**
- **Herd, Karl Josef, Dr.**
**D-51519 Odenthal (DE)**

(56) Entgegenhaltungen:
**EP-A- 0 074 928**
**WO-A-90/09983**
**WO-A-91/13866**
**DE-A- 2 614 550**
**US-A- 4 912 244**

Bemerkungen:
Die Akte enthält technische Angaben, die nach dem Eingang der Anmeldung eingereicht wurden und die nicht in dieser Patentschrift enthalten sind.

EP 0 652 262 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

## Beschreibung

Die Erfindung betrifft neue bi- und polyfunktionelle Reaktivfarbstoffe, ihre Herstellung und Verwendung.

Bifunktionelle Reaktivfarbstoffe sind zwar beispielsweise aus der DE-A-2 614 550, EP-A-70 807, EP-A-70 806 und EP-A-74 928 und tri- und tetrafunktionelle Reaktivfarbstoffe beispielsweise aus EP-A-395 951 bekannt, jedoch weisen die bekannten Reaktivfarbstoffe noch diverse anwendungstechnische Nachteile, beispielsweise eine zu geringe Fixierausbeute, auf.

Die vorliegende Erfindung betrifft neue Reaktivfarbstoffe der Formel

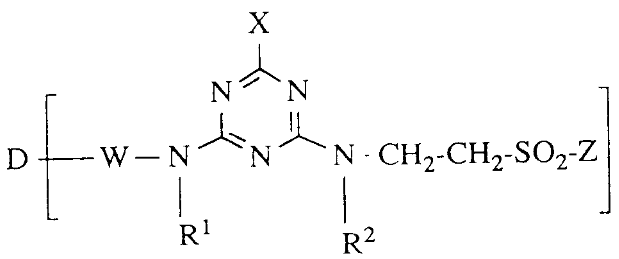

(I),

worin

D Rest eines organischen Farbstoffes aus der Monoazo-, Polyazo-, Metallkomplexazo-, Anthrachinon-, Phthalocyanin-, Formazan-, Azomethin-, Dioxazin-, Phenazin-, Stilben-, Triphenylmethan-, Xanthen-, Thioxanthon- oder der Nitroarylreihe ist,
W direkte Bindung oder Brückenglied bedeutet, insbesondere für eine direkte Bindung steht,
$R^1$ H oder $C_1$-$C_4$-Alkyl bedeutet, das beispielsweise durch OR, $OSO_3H$, $SO_3H$, COOH oder Halogen substituiert sein kann,
R für H, $CH_3$ oder $C_2H_5$ steht,
$R^2$ $C_1$-$C_4$-Alkyl bedeutet, insbesondere Methyl,
Z für $-CH_2-CH_2-OSO_3H$ oder $-CH=CH_2$,
X für F, Cl oder Br steht, und
n 1 oder 2 bedeutet.

Für die in der vorliegenden Anmeldung genannten Alkyl-, Aryl-, Aralkyl-, Hetaryl-, Alkoxy-, Halogen- und Acylaminoreste sowie für die Brückenglieder gilt folgendes:

Unter Alkylgruppen werden insbesondere solche mit 1 bis 4 C-Atomen verstanden, die gegebenenfalls Substituenten aufweisen können, beispielsweise Halogen wie Cl oder Br, OH, CN, $CO_2H$, $SO_3H$ oder $OSO_3H$.

Unter Alkoxyresten werden insbesondere solche mit 1 bis 4 C-Atomen verstanden.

Unter Halogen wird insbesondere Chlor oder Fluor verstanden.

Unter Acylaminoresten werden insbesondere solche mit 1 bis 4 C-Atomen verstanden, wie Formylamino, Acetylamino, Propionylamino, n-Butyrylamino.

Geeignete Brückenglieder W sind beispielsweise:

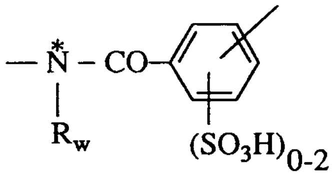 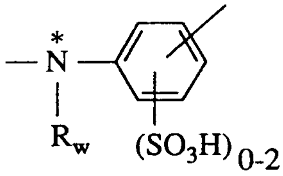

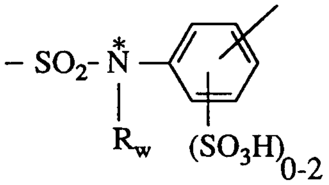 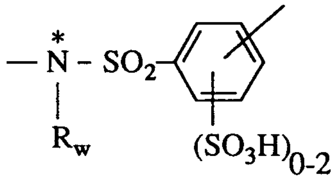

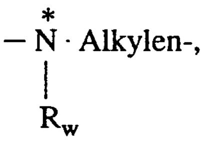 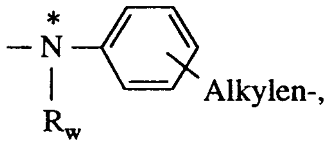 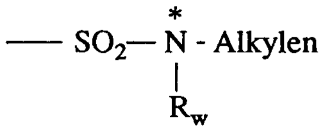

-$\overset{*}{O}$-Alkylen-, -Alkylen-, -$\overset{*}{A}$lkylen-CO-, -$\overset{*}{A}$lkylen-$SO_2$-,
wobei $R_w$ für Wasserstoff oder Alkyl steht, und Alkylen einen Alkylenrest mit 1 bis 6 C-Atomen bedeutet, wobei * das Atom bzw. die Gruppe kennzeichnet, welches mit dem Chromophor D verbunden ist.

Als Alkylenreste seien aufgeführt: $-CH_2-$, $-CH_2-CH_2-$,

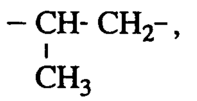

$-(CH_2)_3-$, $-(CH_2)_4-$, $-(CH_2)_6-$.

Die vorliegende Erfindung betrifft weiterhin die Herstellung der Reaktivfarbstoffe der Formel (I):

a) entweder durch Kondensation von Farbstoffen der Formel

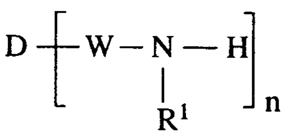 (II),

worin

D, W, $R^1$ und n die oben angegebene Bedeutung haben,
mit n-Molen Trihalogentriazinen der Formel

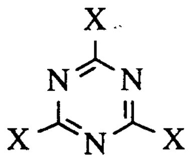

(III)

zu Verbindungen der Formel

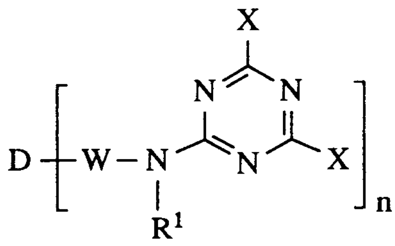

(IV)

und weitere Kondensation der Verbindungen der Formel (IV) mit n-Molen der Komponenten der Formel

$$R^2\text{-NH-}CH_2CH_2\text{-}SO_2\text{-Z} \qquad (V),$$

worin
$R^2$ und Z die oben angegebene Bedeutung haben,

oder
b) in umgekehrter Reihenfolge durch Kondensation von Trihalogentriazinen der Formel (III) mit den Komponenten der Formel (V) zu den Primärkondensationsprodukten

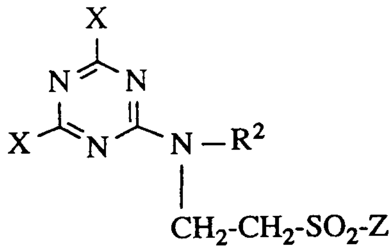

(VI),

worin

$R^2$ und Z die oben angegebene Bedeutung haben,
und weitere Kondensation von n-Molen der Verbindungen der Formel (VI) mit den Farbstoffen der Formel (II),

oder
c) durch Kondensation geeigneter Vorprodukte mit den Trihalogentriazinen (III) und den Komponenten der Formel (V) bzw. durch Kondensation geeigneter Vorprodukte mit den Primärkondensationsprodukten der Formel (VI) und anschließender Farbstoffsynthese.

Die Erfindung betrifft weiterhin ein Verfahren zur Herstellung von Verbindungen der Formel (V), dadurch gekennzeichnet, daß Verbindungen der Formel

$$R^2\text{-NH-}C_2H_4\text{-S-}C_2H_4\text{-OH},$$

die durch Umsetzung von 3-Alkyl-2-oxo-oxazolidinonen der allgemeinen Formel

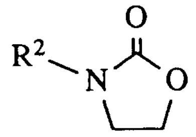

mit 2-Mercaptoethanol erhalten werden durch Oxidation, insbesondere mit $H_2O_2$, in Verbindungen der Formel

$$R^2\text{-NH-}C_2H_4\text{-}SO_2\text{-}C_2H_4\text{-OH}$$

überführt werden und dann in üblicher Weise zu Verbindungen der allgemeinen Formel (V) umgesetzt werden.

Die Kondensationen der Ausgangskomponenten mit den Trihalogentriazinen erfolgen unabhängig von der Reihenfolge in wäßrigen oder organisch-wäßrigen Medien in Anwesenheit von säurebindenden Mitteln. In Abhängigkeit von der Natur der Ausgangskomponenten erfolgt dabei die erste Stufe der Kondensation in pH-Bereichen von 2 bis 8, vorzugsweise 3 bis 7, und bei Temperaturen von 0 bis 40°C, vorzugsweise 0 bis 25°C. Der Austausch des zweiten Halogenatoms des Triazins vollzieht sich im pH-Bereich von 4 bis 10, vorzugsweise 5 bis 9, und im Temperaturbereich von 0 bis 60°C, vorzugsweise 0 bis 30°C.

Säurebindende Mittel sind beispielsweise Carbonate, Hydroxide oder Phosphate wie Natriumcarbonat, Natriumhydrogencarbonat, verdünnte Natronlauge, Di- oder Trinatriumphosphat oder Natriumfluorid.

Soll die Kondensation bzw. die Farbstoffsynthese direkt zu einer Farbstofflösung bzw. zu einer flüssigen Farbstoffpräparation führen, kann die Verwendung von Lithiumcarbonaten oder Lithiumhydroxid vorteilhaft sein, gegebenenfalls zusammen mit Lösungsvermittlern und/oder stabilisierenden Puffersystemen. Andere Umwandlungsreaktionen der Farbstoffe oder deren Vorprodukte wie Metallisierungsreaktionen, Sulfierungen oder Einführung von Acylaminogruppierungen können im allgemeinen in beliebigen Stufen der Farbstoffsynthesen vorgenommen werden.

Besonders wertvolle Farbstoffe aus der Anthrachinon-, Phthalocyanin-, Formazan-, Azomethin-, Dioxazin-, Phenazin-, Stilben-, Triphenylmethan-, Xanthen-, Thioxanthon-oder Nitroarylreihe sind wasserlösliche Formazan-, Anthrachinon- und Phthalocyaninfarbstoffe und insbesondere solche, die Sulfonsäure- und/oder Carbonsäure-gruppen aufweisen. Die Farbstoffe können sowohl metallfrei als auch metallhaltig sein, wobei unter den Metallkomplexen die Kupfer-, Nickel-, Chrom- und Kobalt-Komplexe bevorzugtes Interesse besitzen.

Geeignete Farbstoffreste D dieser Klasse beziehungsweise die den Farbstoffen der Formel (I) zugrundeliegenden aminogruppenhaltigen Farbstoffe sind in der Literatur in sehr großer Zahl beschrieben. Beispielhaft seien hier erwähnt:

EP-A 54 515, EP-A 69 703, EP-A 70 807, DE- 3 222 726, DE-A 2 650 555, DE-A 3 023 855, DE-A 2 847 938, DE-A 2 817 780, GB-A 2 057 479, DE-A 2 916 715, DE-A 2 814 206, DE-A 3 019 936, EP-A 45 488 sowie Venkataraman: The Chemistry of Synthetic Dyes, BD. VI, Kapitel II, S. 211-325, New York, London; 1972.

Dabei kommt insbesondere als Rest eines organischen Farbstoffs dieser Klassse ein solcher in Frage, der eine oder mehrere wasserlöslichmachende Gruppen, insbesondere Sulfogruppen aber keine weiteren faserreaktiven Reste enthält.

Bevorzugte Farbstoffe sind die der folgenden Formel (1) bis (8), worin generell B' einen Rest der Formel

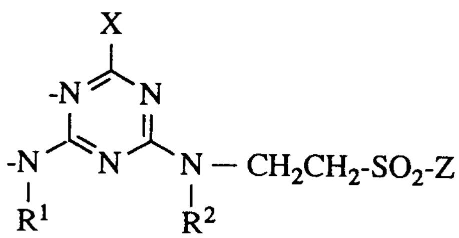

(VIb)

beinhaltet und $R^1$, X, $R^2$ und Z die oben angegebene Bedeutung haben

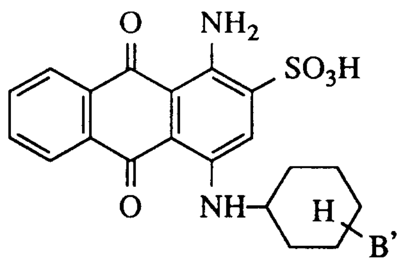

(1)

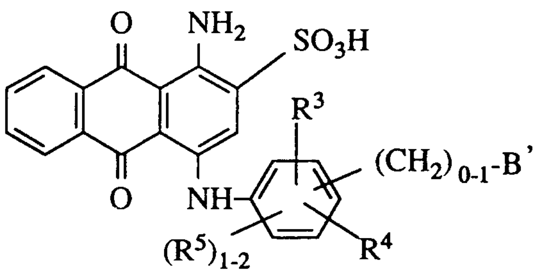

(2)

worin

$R^3$ = H, Methyl, Methoxy, Chlor
$R^4$ = H, $SO_3H$ und
$R^5$ = H, Methyl oder Ethyl

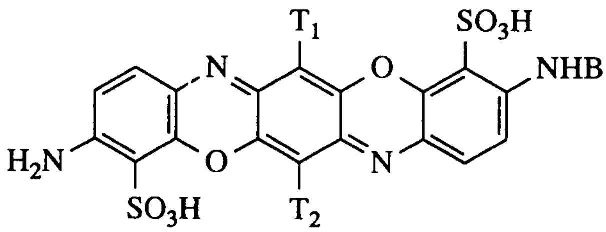

(3a)

worin

B einen Rest der Formel

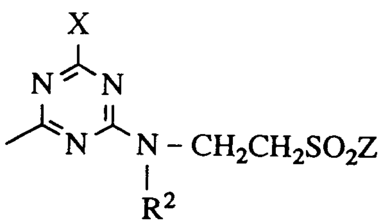

(VIa)

bedeutet, in welchem die Substituenten X, $R^2$ und Z die oben angegebene Bedeutung haben, und

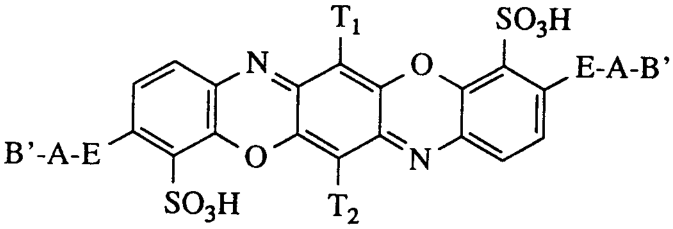

(3b)

worin

A für ein gegebenenfalls substituiertes Phenylen oder ein gegebenenfalls substituiertes aromatisch-aliphatisches Brückenglied oder für ein gegebenenenfalls durch Heteroatome wie $NR^6$, O oder S enthaltende Gruppierungen unterbrochenes, geradkettiges oder verzweigtes $C_1$-$C_6$-Alkylen, das mit $C_1$-$C_6$-Alkoxy, $OSO_3H$, $SO_3H$, COOR oder Halogen substituiert sein kann, steht
und wobei innerhalb eines Brückengliedes A die Gruppe $NR^6$ mit der Gruppe $NR^1$ auch einen heterocyclischen aliphatischen Ring bilden kann, insbesondere

E =

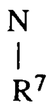

,

O und

$T_1$, $T_2$ = unabhängig voneinander H, Cl, Br, $C_1$-$C_2$-Alkyl, $OCH_3$, $OC_2H_5$, Acylamino, $C_1$-$C_2$-Alkoxycarbonyl ist,

$R^6$, $R^7$ = unabhängig voneinander H, $C_1$-$C_4$-Alkyl, das durch OR, $OSO_3H$, $SO_3H$, COOR oder Halogen substituiert sein kann,

R = H, $CH_3$ oder $C_2H_5$.

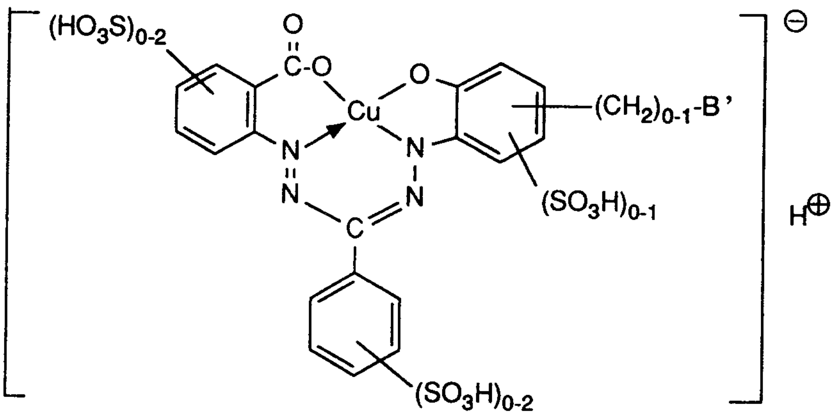

(4)

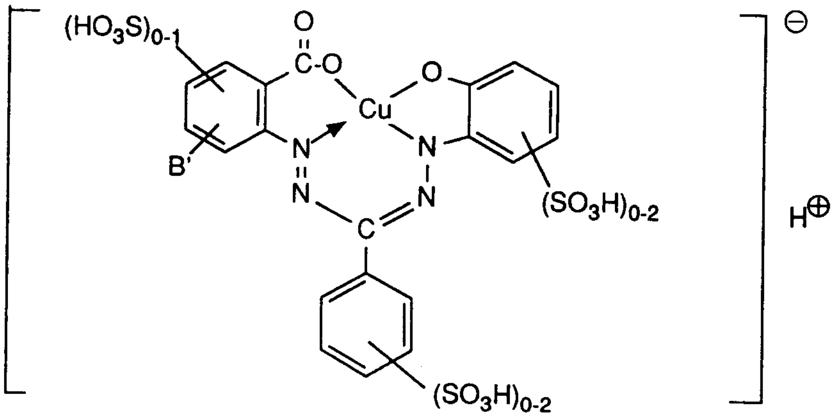

(5)

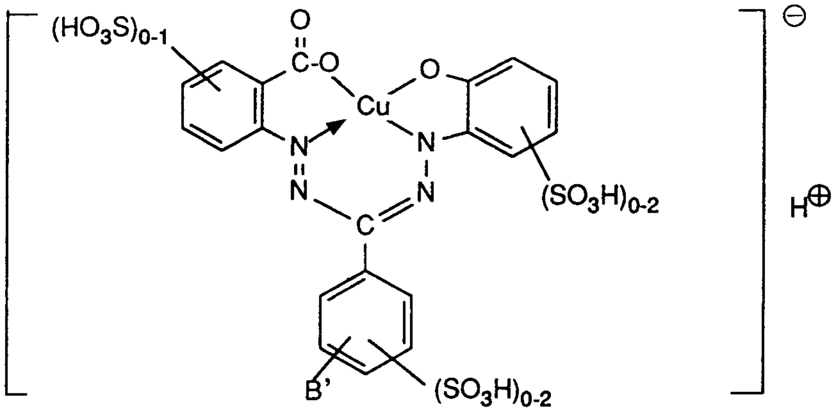

(6)

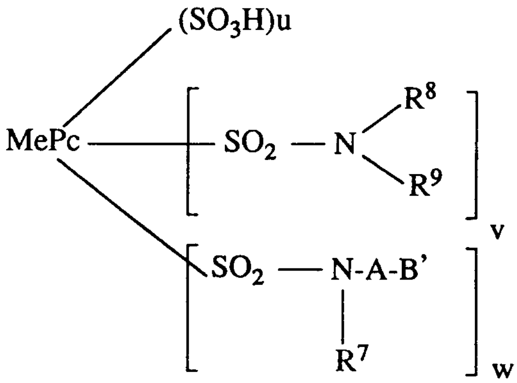

(7)

worin

Me = Cu, Ni,
Pc = Rest eines Phthalocyanins,
u+v+w = 3,4 - 4,0 , mit der Maßgabe, daß
u = 0,8 - 2,0,
v = 0 - 1,0,
w = 1,0 - 3,0 ist und

A die oben angegebene Bedeutung hat,
$R^7$ die oben angegebene Bedeutung hat,
$R^8$ und $R^9$ = H, $C_1$-$C_2$-Alkyl, das gegebenenfalls substituiert durch OH, $OSO_3H$, $SO_3H$ oder COOH ist.

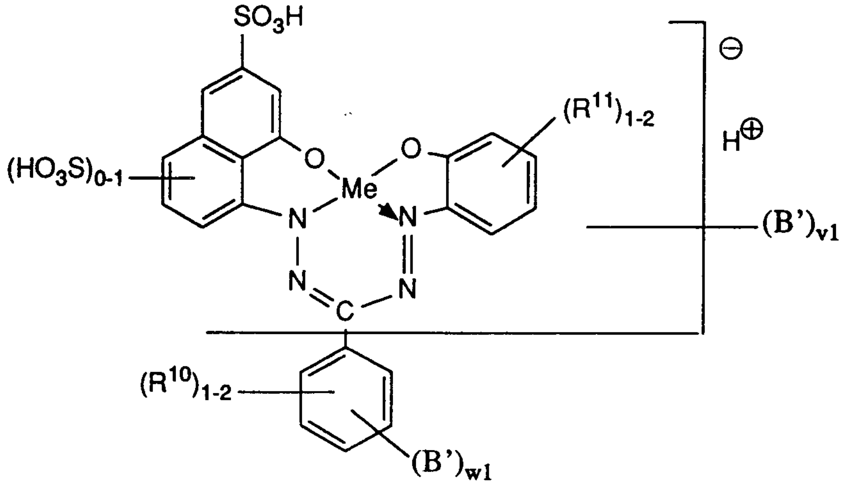

(8)

worin

v1, w1 = 0 oder 1, wobei w1 ungleich v1 ist,

$R^{10}$, $R^{11}$ unabhängig voneinander H, $C_1$-$C_4$-Alkyl, $C_1$-$C_4$-Alkoxy, OH, Halogen, COOH, $NO_2$, $SO_3H$, Sulfon-amido, $C_1$-$C_4$-Allkylcarbonylamino, gegebenenfalls substituiertes Phenylcarbonylamino, $C_1$-$C_4$-Alkylsulfonylamino, gegebenenfalls substituiertes Phenylsulfonylamino sind.

Weitere besonders wertvolle Farbstoffe dieser Reihe sind wasserlösliche Azofarbstoffe, und insbesondere solche, die Sulfonsäure- und/oder Carbonsäuregruppen aufweisen. Die Farbstoffe können sowohl metallfrei als auch metallhaltig sein, wobei unter den Metallkomplexen die Kupfer-, Nickel-, Chrom- und Kobalt-Komplexe bevorzugtes Interesse besitzen.

Geeignete Farbstoffreste D beziehungsweise die den Farbstoffen der Formel (I) zugrundeliegenden aminogruppenhaltigen Farbstoffe dieser Klasse sind in der Literatur in sehr großer Zahl beschrieben. Beispielhaft seien hier erwähnt:

EP-A 54 515, EP-A 69 703, EP-A 70 807, EP-A 497 174, DE- 3 222 726, DE-A 2 650 555, DE-A 3 023 855, DE-A 2 847 938, DE-A 2 817 780, GB-A 2 057 479, DE-A 2 916 715, DE-A 2 814 206, DE-A 3 019 936, EP-A 45 488 sowie Venkataraman: The Chemistry of Synthetic Dyes, BD. VI, Kapitel II, S. 211-325, New York, London; 1972.

Bevorzugte Reste eines organischen Azofarbstoffes entsprechen beispielsweise folgenden Gruppen

Di-N=N-(M-N=N)$_{0\ \text{oder}\ 1}$ K-
-Di-N=N-(M-N=N)$_{0\ \text{oder}\ 1}$ K
-Di-N=N-(M-N=N)$_{0\ \text{oder}\ 1}$ K-

Di für den Rest einer gegebenenfalls durch in der Azochemie übliche Substituenten, insbesondere Hydroxy-, Methyl-, Ethyl-, Methoxy- oder Ethoxygruppen, gegebenenfalls substituierte Alkanoylaminogruppen mit 2-4 C-Atomen, gegebenenfalls substituierte Benzoylaminogruppen oder Halogenatome sowie $SO_2$-Z substituierte Diazokomponente der Benzol- oder Naphthalinreihe steht,

K für den Rest einer gegebenenfalls durch in der Azochemie übliche Substituenten, insbesondere Hydroxy-, Amino-, Methyl-, Ethyl-, Methoxy- oder Ethoxygruppen, gegebenenfalls substituierte Alkanoylaminogruppen mit 2-4 C-Atomen, gegebenenfalls substituierte Benzoylaminogruppen oder Halogenatome substituierte Kupplungskomponente der Benzol-, Napthalin- oder Ketomethylenreihe steht,

M für den Rest einer gegebenenfalls durch in der Azochemie übliche Substituenten, insbesondere Hydroxy-, Methyl-, Ethyl-, Methoxy- oder Ethoxygruppen, gegebenenfalls substituierte Alkanoylaminogruppen mit 2-4 C-Atomen, gegebenenfalls substituierte Benzoylaminogruppen oder Halogenatome substituierte Mittelkomponente der Benzol- oder Naphthalinreihe steht, und

Di, M und K zusammen mindestens zwei Sulfonsäuregruppen, vorzugsweise drei bis vier Sulfonsäuregruppen, enthalten.

Wichtige Azofarbstoffe sind beispielsweise solche der Benzol-azo-naphthalinreihe, der Bis-(Benzolazo)-naphthalinreihe, der Benzol-azo-5-pyrazolonreihe, der Benzol-azo-benzolreihe, der Naphtalin-azo-benzolreihe, der Benzol-azo-aminonaphthalinreihe, der Naphthalin-azo-naphthalinreihe, der Naphthalin-azo-pyrazolon-5-Reihe, der Benzol-azo-pyridonreihe, der Benzol-azo-aminopyridinreihe, der Naphthalin-azo-pyridonreihe, der Naphthalin-azo-amino-pyridinreihe, und der Stilben-azo-benzolreihe, wobei auch hier die sulfonsäuregruppenhaltigen Farbstoffe bevorzugt sind. Im Falle von Metallkomplexazofarbstoffen befinden sich die metallkomplexgebundenen Gruppen vorzugsweise in den o-Stellungen zur Azogruppe, z.B. in Form von o,o'-Dihydroxy-, o-Hydroxy-o'-carboxy-, o-Carboxy-o'-amino- und o-Hydroxy-o'-amino-azogruppierungen.

Bevorzugte Farbstoffe sind die der folgenden Formeln (9) bis (50), worin generell B einen Rest der Formel

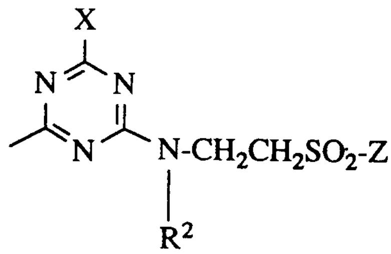

(VIa)

beinhaltet und X, $R^2$ und Z die unter Formel (I) angegebene Bedeutung haben:

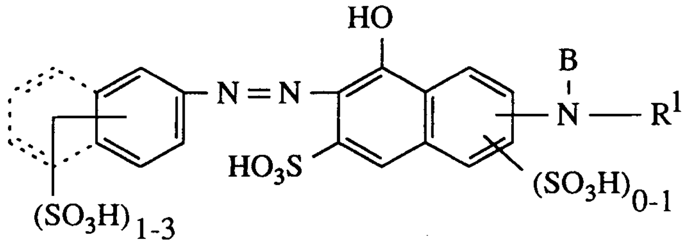

(9)

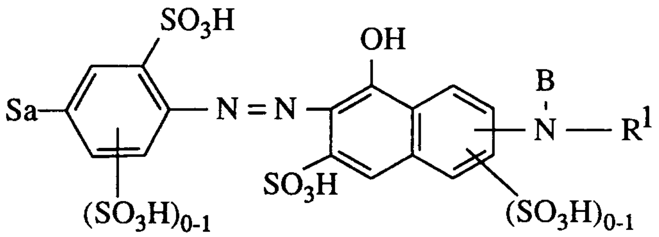

(10)

mit Sa = $OCH_3$ oder $OC_2H_5$

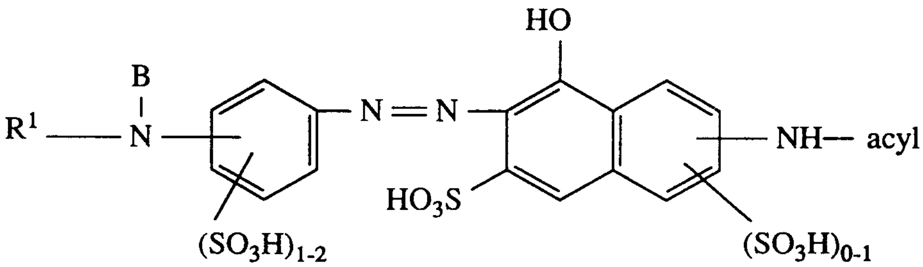

(11)

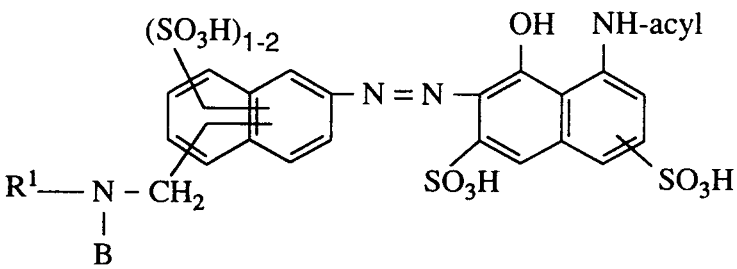

(12)

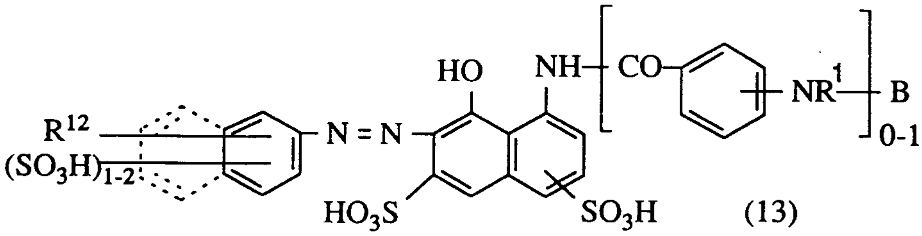

(13)

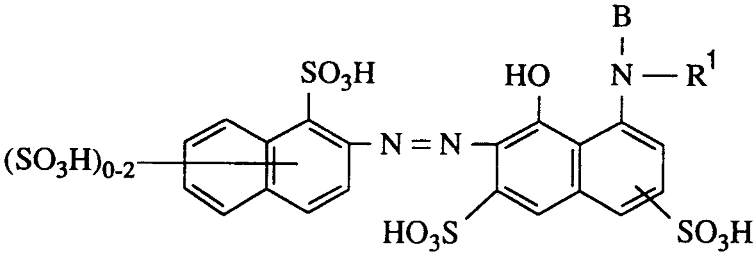

(14)

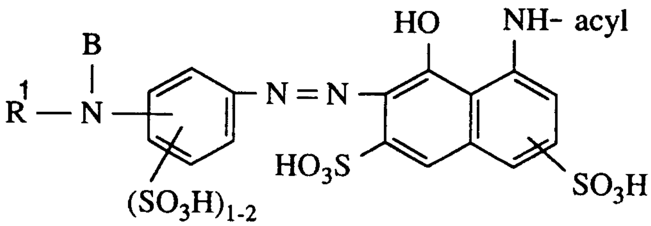

(15)

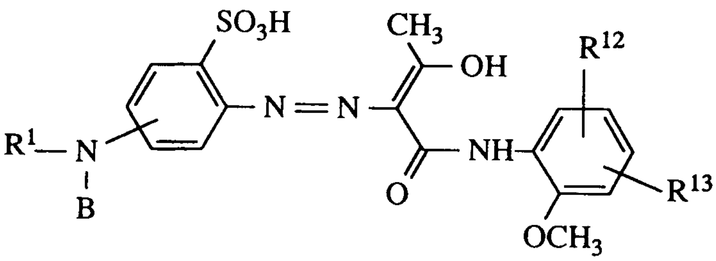

(16)

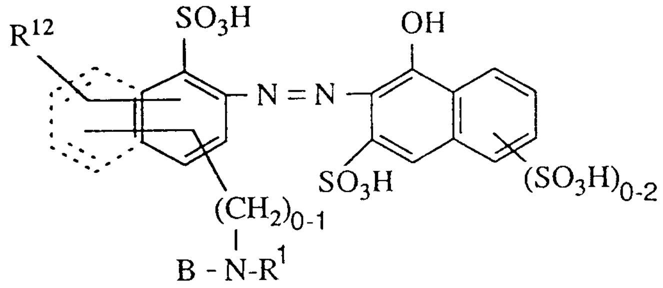

(17)

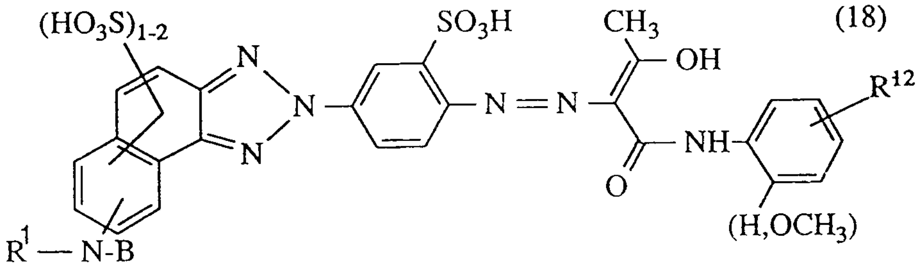

(18)

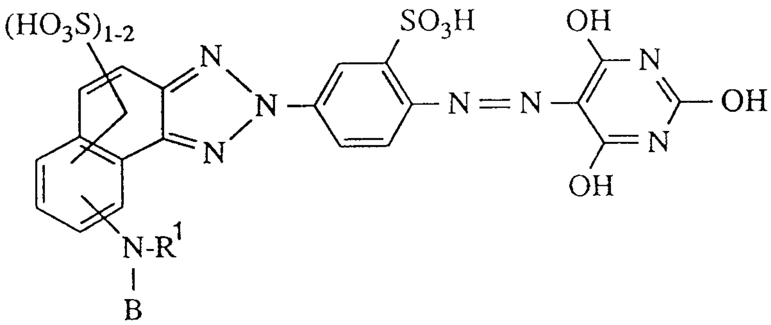

(19)

Metallkomplexe und Farbstoffe der Formeln (20) bis (50):

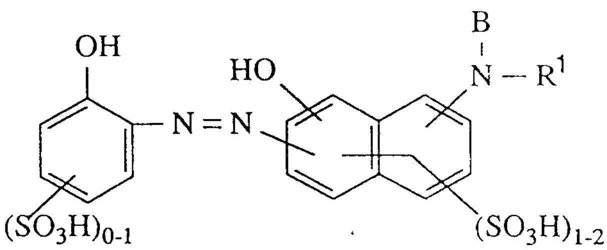

(20)

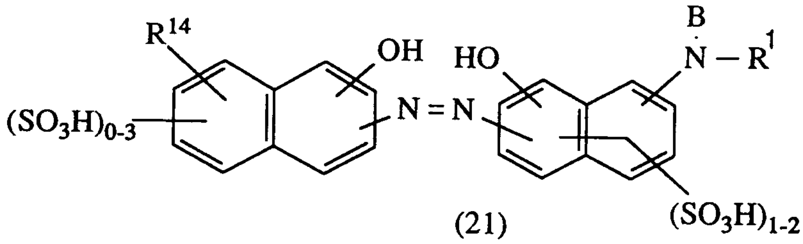

(21)

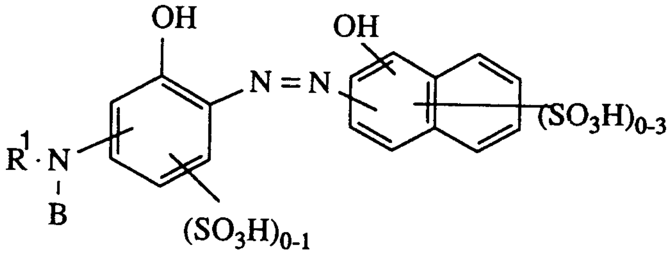

(22)

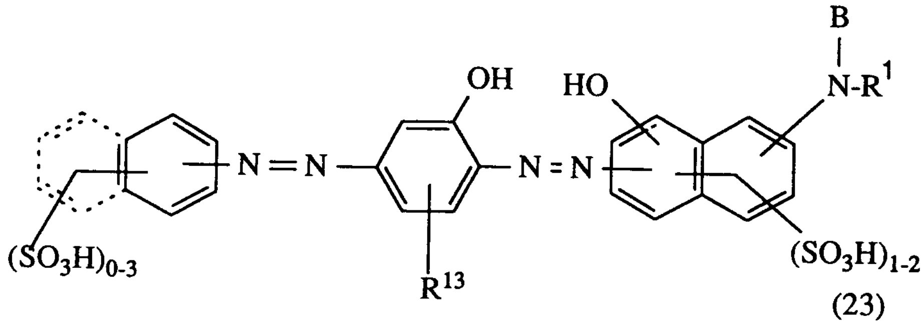

(23)

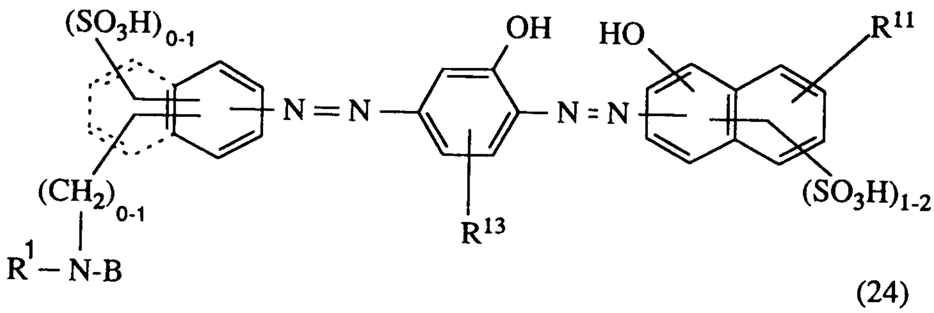

(24)

Als Metallatom sind Cu (1:1-Komplex) oder Cr und Co (1:2-Komplex) bevorzugt. Cr- und Co-Komplexe können die Azoverbindung der oben angegebenen Formel einmal oder zweimal enthalten, d.h., sie können symmetrisch oder mit beliebigen anderen Ligandengruppen unsymmetrisch aufgebaut sein.

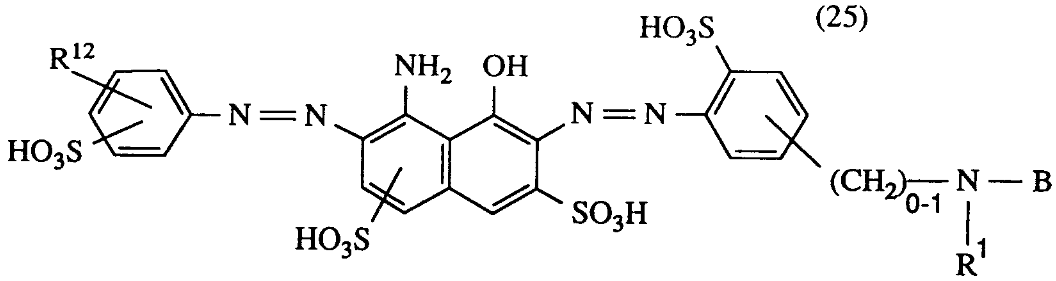

(25)

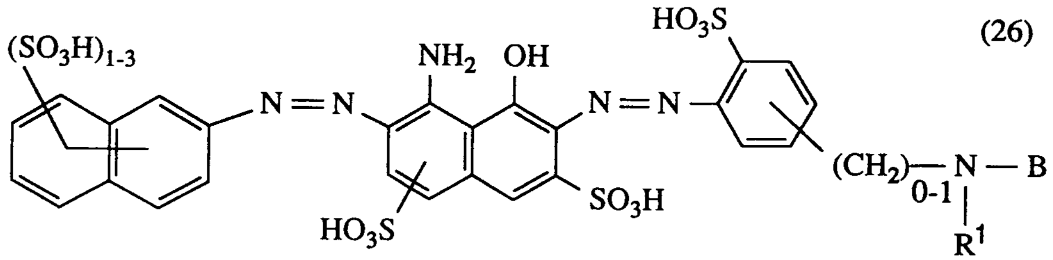

(26)

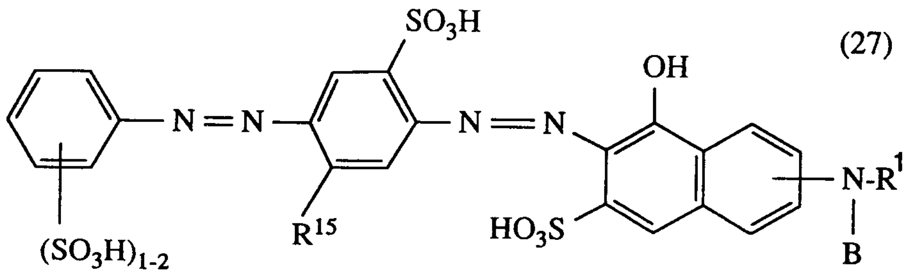

(27)

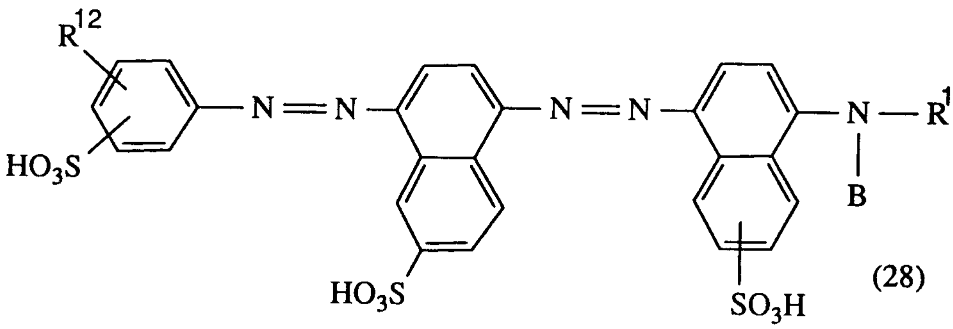

(28)

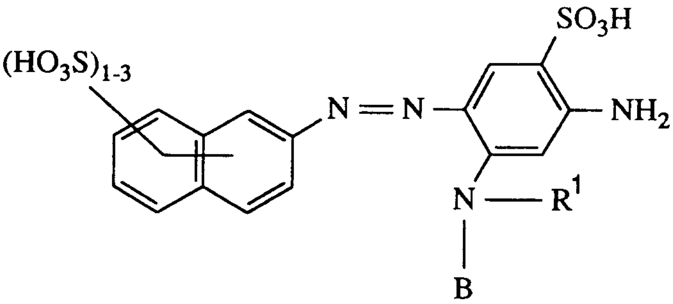

(29)

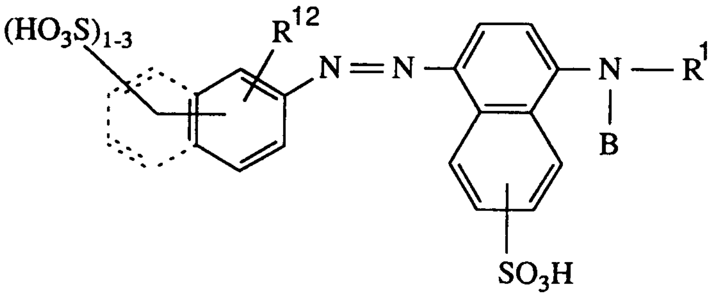

(30)

(31)

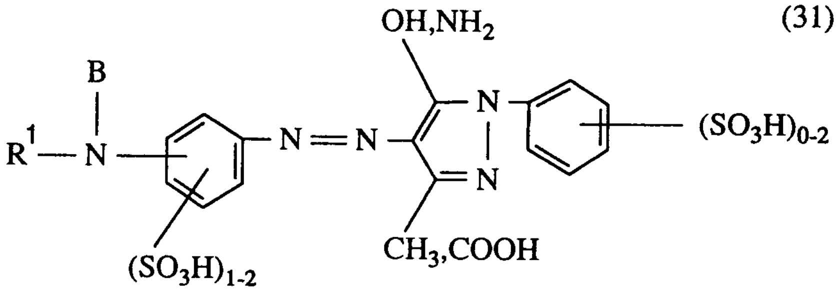

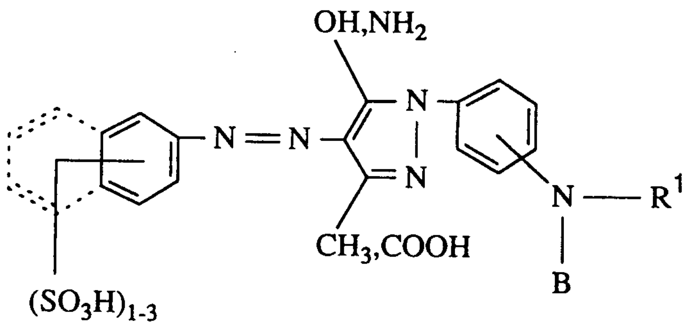

(32)

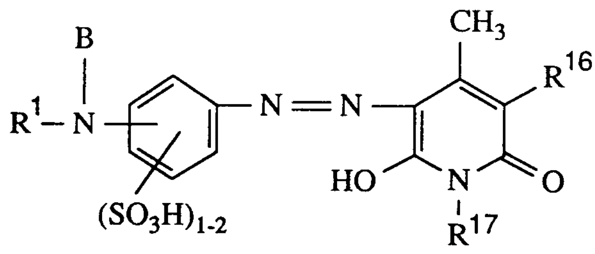

(33)

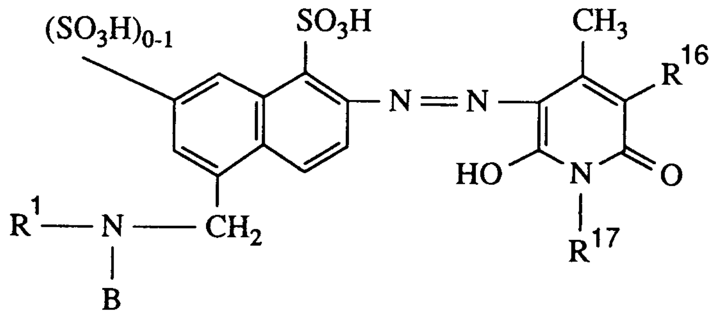

(34)

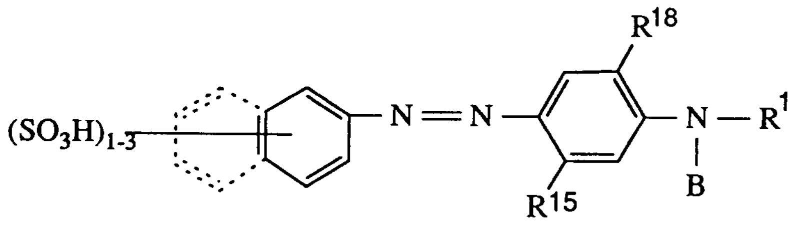

(35)

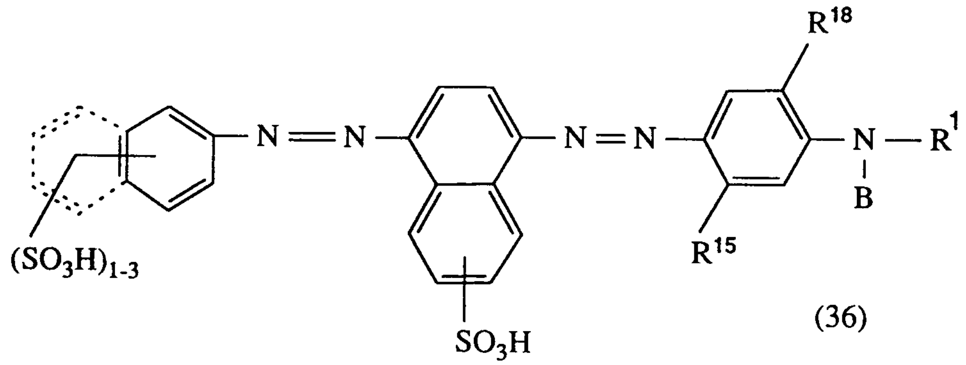

(36)

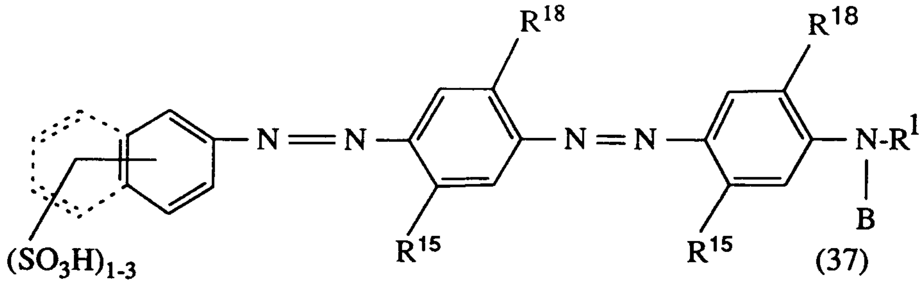

(37)

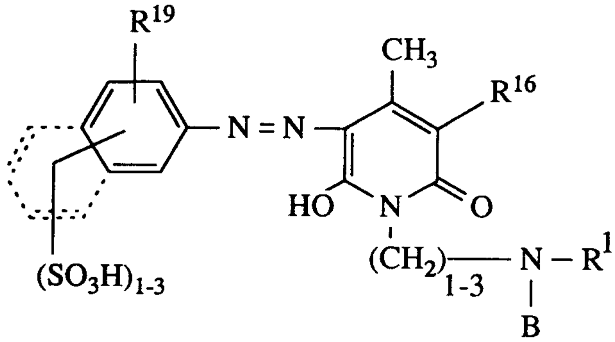

(38)

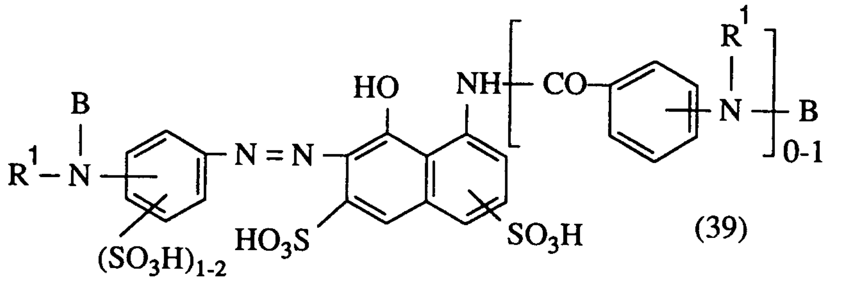

(39)

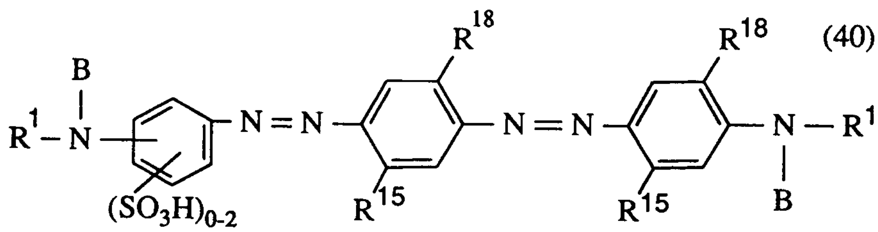

(40)

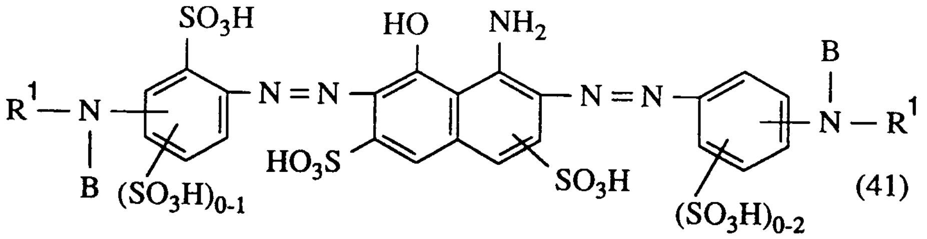

(41)

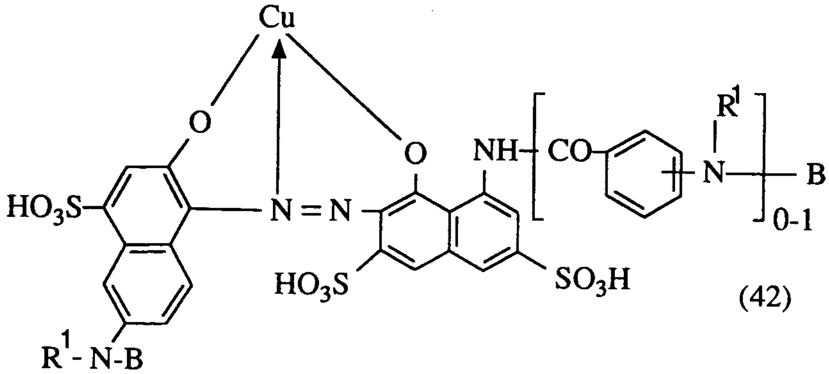

(42)

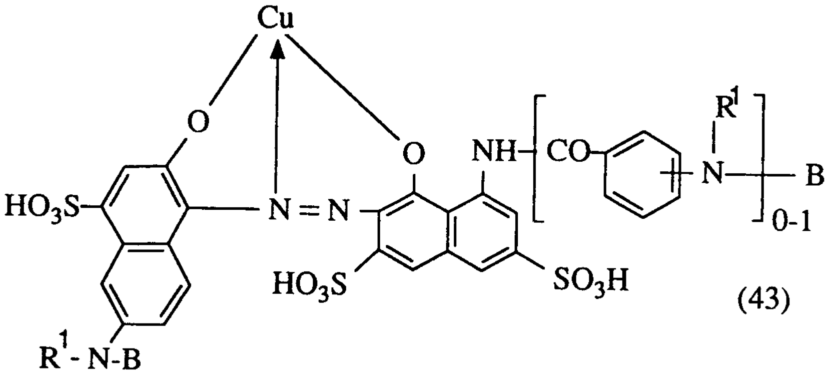

(43)

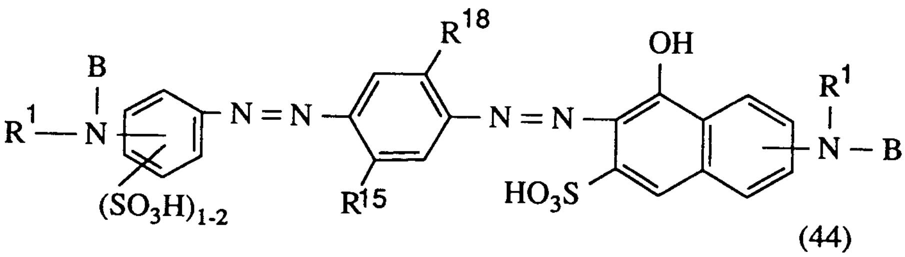

(44)

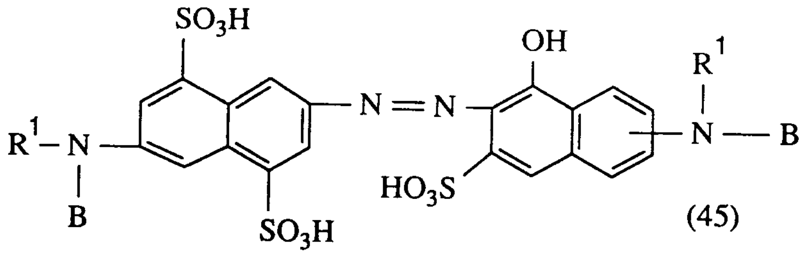

(45)

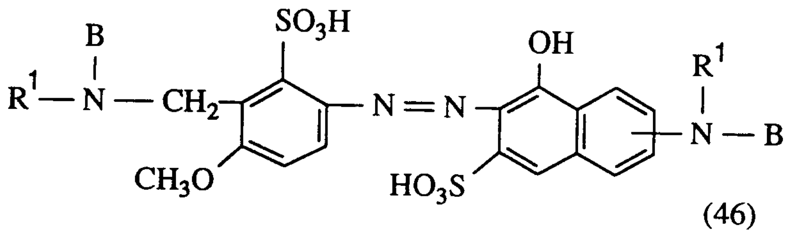

(46)

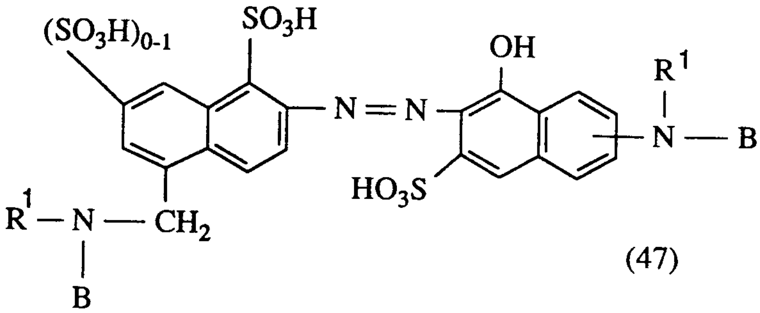

(47)

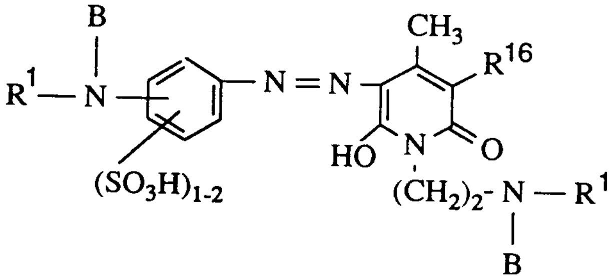

(48)

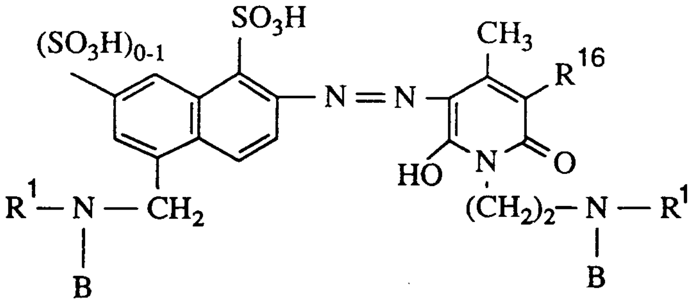

(49)

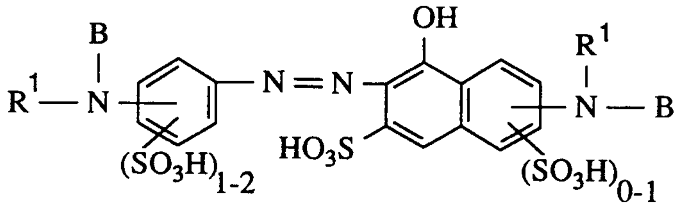

(50),

worin
Acyl z.B. Acetyl oder gegebenenfalls substituiertes Benzoyl ist,

- $R^{17}$ = H, $C_1$-$C_2$-Alkyl, gegebenenfalls substituiert durch $SO_3H$, $NH_2$,
- $R^1$ = H, $CH_3$ oder $C_2H_5$,
- $R^{19}$ = H oder Sulfo,
- $R^{13}$ = H, $CH_3$, $OCH_3$ oder Cl,
- $R^{12}$ = H, $C_1$-$C_4$-Alkyl, $C_1$-$C_4$-Alkoxy, Cl, Br, COOH, $SO_3H$,
- $R^{14}$ = H, OH, $NH_2$, $NHCOCH_3$, NHCOPh, Cl, $C_1$-$C_4$-Alkoxy oder $C_1$-$C_4$-Alkyl ist,
- $R^{16}$ = H, $SO_3H$, $CH_2SO_3H$, Cl, $C_1$-$C_4$-Alkylsulfonyl, CN, Carbonamid, insbesondere $CONH_2$,
- $R^{15}$ = H, $C_1$-$C_4$-Alkyl, $C_1$-$C_4$-Alkoxy, Cl, Br, Acylamino, insbesondere $C_1$-$C_4$-Alkylcarbonylamino oder Arylcarbonylamino wie gegebenenfalls substituiertes Phenylcarbonylamino, $C_1$-$C_4$-Alkylsulfonylamino, Aminocarbonylamino, $C_1$-$C_4$-Alkylsulfonylamino, Arylsulfonylamino,
- $R^{18}$= H, $C_1$-$C_4$-Alkyl, $C_1$-$C_4$-Alkoxy, OH, $SO_3H$.

Die durch Strichelungen angedeuteten kondensierten Ringe stehen für alternativ mögliche Naphthalinsysteme.

Bevorzugt sind weiterhin Reaktivfarbstoffe der Formel (I), worin

der Rest D, vorzugsweise ein Rest eines organischen Farbstoffes aus der Monoazo- oder Polyazoreihe, ein- oder zweimal mit der Gruppe

$$Z'\text{-}O_2S\text{-}(CH_2)_{0\text{-}1}\text{—}$$

substituiert ist und
worin

Z' -CH=$CH_2$, -$CH_2$-$CH_2$-$OSO_3H$, -$CH_2$-$CH_2$-Cl, -$CH_2$-$CH_2$-Br, -$CH_2$-$CH_2$-$S_2O_3H$, -$CH_2$-$CH_2$-O-CO-$CH_3$, -$CH_2$-$CH_2$-$OPO_3H_2$ oder -$CH_2$-$CH_2$-OH bedeutet.

Besonders bevorzugt sind Reaktivfarbstoffe der Formel (I), worin

der Rest D, vorzugsweise ein Rest eines organischen Farbstoffes aus der Monoazo- oder Polyazoreihe, ein- oder zweimal mit der Gruppe

$$Z'-O_2S-(CH_2)_{0-1}-$$

substituiert ist,
worin

Z' die obige Bedeutung hat und n = 1 ist.

Besonders bevorzugt sind weiterhin Reaktivfarbstoffe der Formel (I), in der

n = 1,
W = direkte Bindung und
D ein Rest der allgemeinen Formel (IX) ist,

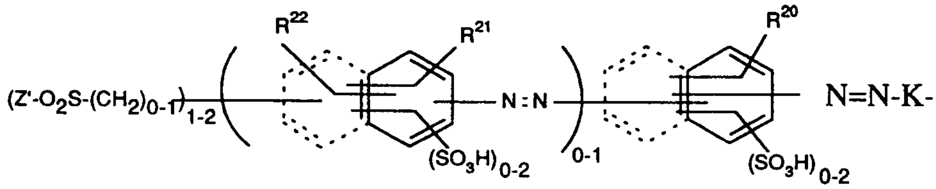

(IX)

worin

$R^{20}$ H, $C_1$-$C_4$-Alkyl, $C_1$-$C_4$-Alkoxy, Cl, Br, Acylamino, insbesondere $C_1$-$C_4$-Alkylcarbonylamino oder Arylcarbonylamino, wie gegebenefalls substituiertes Phenylcarbonylamino, $C_1$-$C_4$-Alkylsulfonylamino, Aminocarbonylamino, $C_1$-$C_4$-Alkylsulfonylamino, Arylsulfonylamino,
$R^{21}$ H, $C_1$-$C_4$-Alkyl, Cl, Br, $C_1$-$C_4$-Alkoxy oder COOH,
$R^{22}$ H, $C_1$-$C_4$-Alkyl, $C_1$-$C_4$-Alkoxy, $SO_3H$, Cl oder Br,
K ein bivalenter Rest der allgemeinen Formeln (Xa) - (Xd)

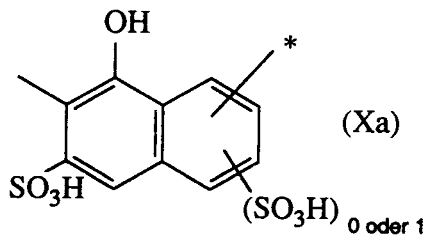

(Xa) ,

oder

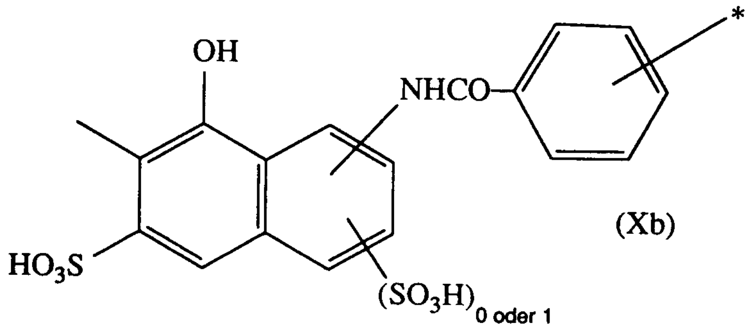

(Xb)

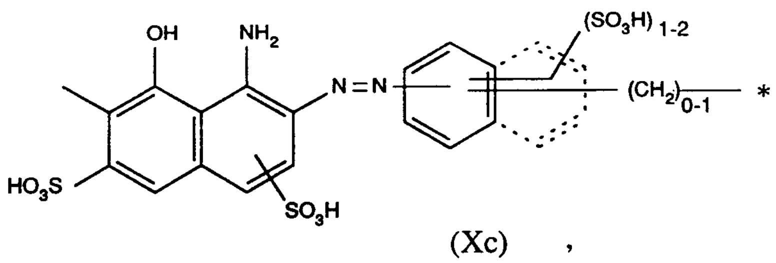

(Xc) ,

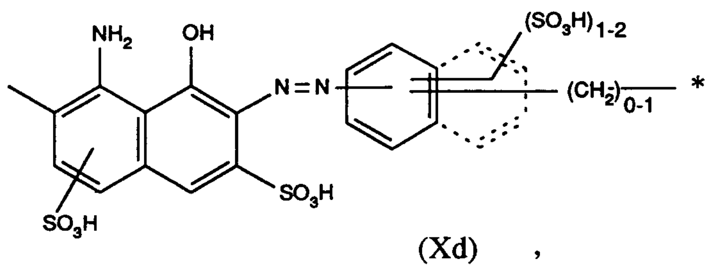

(Xd) ,

bedeutet,
wobei die mit * gekennzeichneten Bindungen an die Gruppe $-NR^1$-B gebunden sind. B hat die Bedeutung von Formel (VIa).

Besonders bevorzugt bedeutet Z' $-CH=CH_2$ oder $-C_2H_4-OSO_3H$.

Geeignete den Resten D der Formel (IX) zugrundeliegende Diazokomponenten für die Farbstoffe der Formeln (I) sind z.B.:

Anilin-4-β-sulfatoethylsulfon,
Anilin-4-β-thiosulfatoethylsulfon,
Anilin-4-vinylsulfon,
Anilin-3-β-sulfatoethylsulfon,
Anilin-3-vinylsulfon,
2-Methoxy-anilin-5-β-sulfatoethylsulfon,
2-Methoxy-anilin-5-β-thiosulfatoethylsulfon,
2-Methoxyanilin-5-vinylsulfon,
4-Methoxy-anilin-3-β-sulfatoethylsulfon,
4-Methoxy-anilin-3-β-vinylsulfon,
2,5-Dimethoxyanilin-4-β-sulfatoethylsulfon,
2,5-Dimethoxy-anilin-4-vinylsulfon,
2-Methoxy-5-methyl-anilin-4-β-sulfatoethylsulfon,
Anilin-2-β-sulfatoethylsulfon,
3-(3- oder 4-Aminobenzoyl)-aminophenyl-β-sulfatoethylsulfon,
2-Methoxy-5-methyl-anilin-4-vinylsulfon,
6-Carboxy-anilin-3-β-sulfatoethylsulfon,
6-Carboxyanilin-3-vinylsulfon,
2-Sulfoanilin-4-β-sulfatoethylsulfon,
2-Sulfoanilin-4-vinylsulfon,
2,4-Disulfoanilin-5-vinylsulfon,
2-Naphthylamin-8-β-sulfatoethylsulfon,
2-Naphthylamin-6-β-sulfatoethylsulfon,
1-Sulfo-2-naphthylamin-6-β-sulfatoethylsulfon,
1-Naphthylamin-4-β-sulfatoethylsulfon,
1-Sulfo-2-naphthylamin-5-β-sulfatoethylsulfon,
6-Sulfo-2-naphthylamin-8-β-sulfatoethylsulfon,
2-Amino-3-sulfo-naphthalin-6,8-bis-(β-sulfatoethylsulfon),
1-Naphthylamin-5-β-sulfatoethylsulfon,
2-Naphthylamin-5-β-sulfatoethylsulfon,
2-Naphthylamin-8-β-sulfatoethylsulfon,
8-Sulfo-2-naphthylamin-6-β-sulfatoethylsulfon,
4-Aminobenzyl-β-sulfatoethylsulfon,
3-Aminobenzyl-β-sulfatoethylsulfon,
4-Aminobenzylvinylsulfon,
3-Aminobenzylvinylsulfon,
3-Amino-4-sulfobenzyl-β-sulfatoethylsulfon,
4-Amino-3-sulfobenzylvinylsulfon,
2'-(β-Sulfatoethylsulfonyl)-3-sulfo-4-aminoazobenzol,
3'-(β-Sulfatoethylsulfonyl)-3-sulfo-4-aminoazobenzol,
4'-Methoxy-3'-(β-sulfatoethylsulfonyl)-3-sulfo-4-aminoazobenzol,
4'-Vinylsulfonyl-2',3-disulfo-4-aminoazobenzol,
2'-(β-Sulfatoethylsulfonyl)-6-methyl-3-sulfo-4-aminoazobenzol,
3'-(β-Sulfatoethylsulfonyl)-6-methyl-3-sulfo-4-aminoazobenzol,
4'-(β-Sulfatoethylsulfonyl)-6-methyl-3-sulfo-4-aminoazobenzol,
4'-(β-Sulfatoethylsulfonyl)-2,6-dimethyl-3-sulfo-4-aminoazobenzol,
3'-(β-Sulfatoethylsulfonyl)-6-methoxy-3-sulfo-4-aminoazobenzol,
3',4'-Bis-(β-sulfatoethylsulfonyl)-6-methoxy-3-sulfo-4-aminoazobenzol,
4'-(β-Sulfatoethylsulfonyl)-6-methoxy-3-sulfo-4-aminoazobenzol,
4'-(β-Sulfatoethylsulfonyl)-2-methyl-5-methoxy-3-sulfo-4-aminoazobenzol,
4'-(β-Sulfatoethylsulfonyl)-2,5-dimethoxy-3-sulfo-4-aminoazobenzol,
3'-(β-Sulfatoethylsulfonyl)-2,5-dimethoxy-3-sulfo-4-aminoazobenzol,
2-(4'-Amino-3'-sulfophenylazo)-6-(β-sulfatoethylsulfonyl)-naphthalin,
2-(4'-Amino-6'-methyl-3'-sulfophenylazo)-1-sulfo-6-(β-sulfatoethylsulfonyl)-naphthalin,
2-(4'-Amino-6'-methyl-3'-sulfophenylazo)-6-(β-sulfatoethylsulfonyl)-naphthalin,
2-(4'-Amino-3'-sulfophenylazo)-8-sulfo-6-(β-sulfatoethylsulfonyl)-naphthalin,
2-(4'-Amino-6'-methyl-3'-sulfophenylazo)-1,7-disulfo-5-(β-sulfatoethylsulfonyl)-naphthalin.

Geeignete, den Resten K zugrundeliegende Kupplungskomponenten H-K-NHR[1] sind beispielsweise:

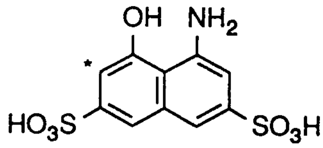
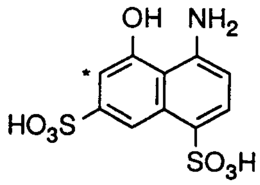
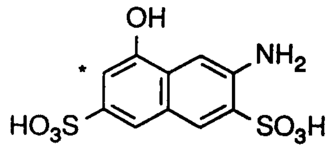
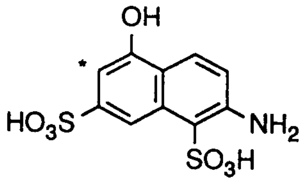
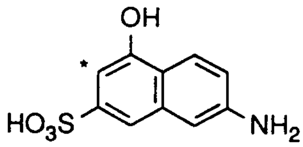
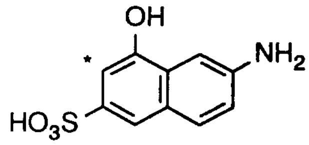

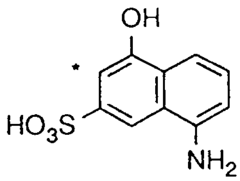
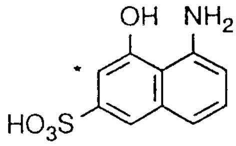
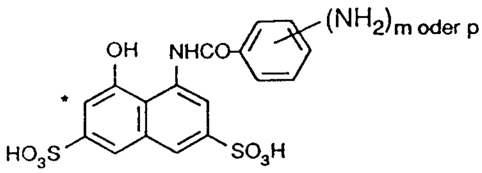

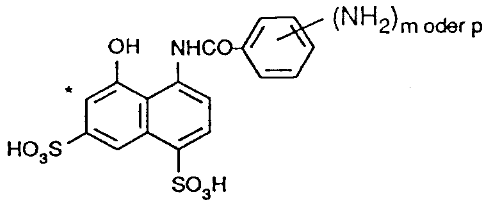
bzw.

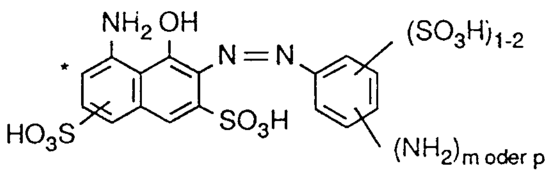
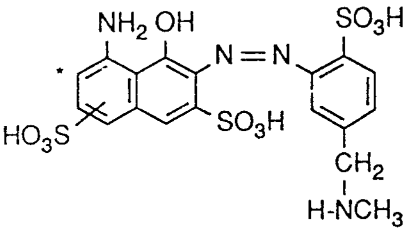

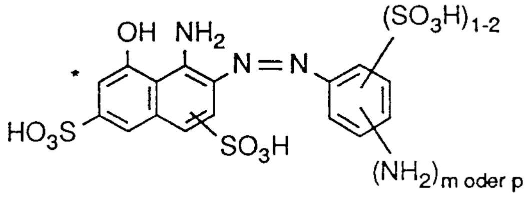
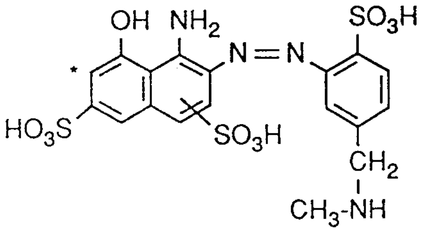

oder
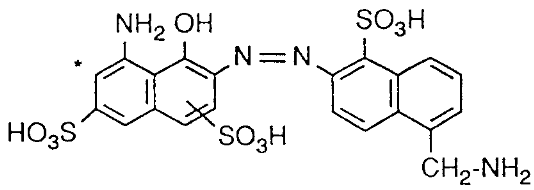
,

wobei * die Kupplungsstelle markiert und m bzw. p für die meta- bzw. para-Position der Aminogruppe zur Azogruppe steht.

Bevorzugte Farbstoffe im Sinne der Formel (I) mit einem Farbstoffrest D der Formel (IX) sind die der folgenden Formeln (51) bis (58), worin generell B einen Rest der Formel

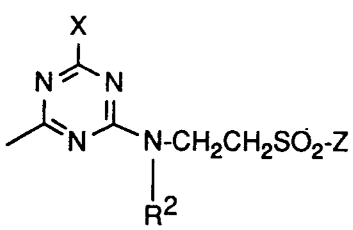
(VIa)

beinhaltet und X, $R^2$ und Z die oben angegebene Bedeutung haben:

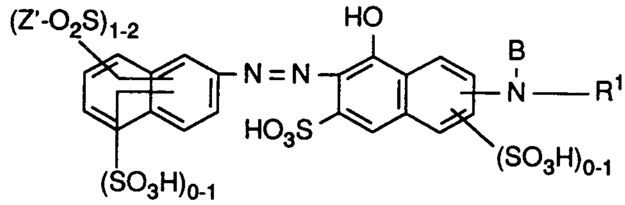

(51),

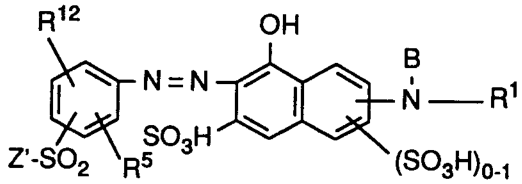

(52),

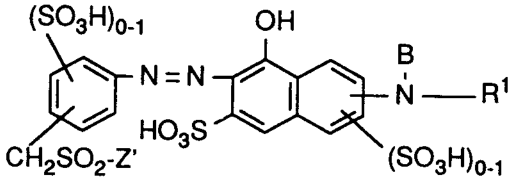

(53),

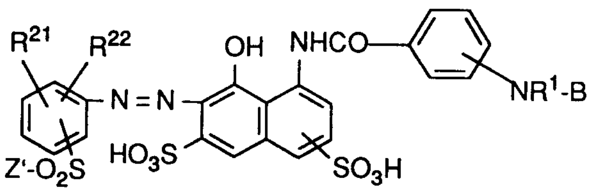

(54),

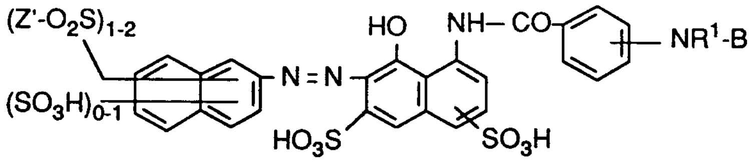

(55),

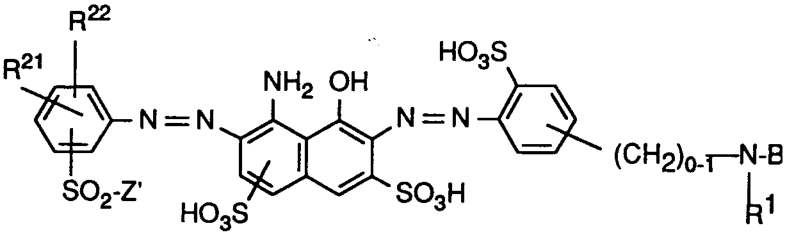

(56),

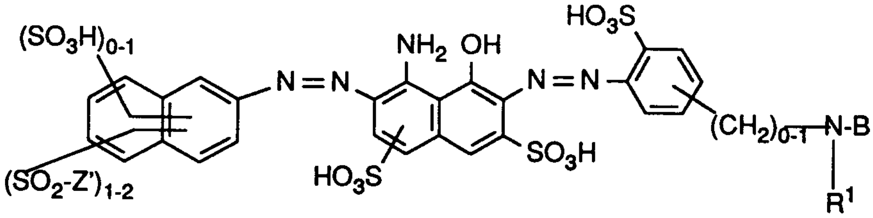

(57),

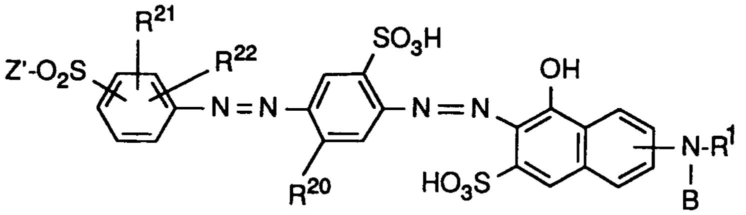

(58),

worin

$R^1$ = H, $CH_3$ oder $C_2H_5$,
$R^{20}$= H, $C_1$-$C_4$-Alkyl, $C_1$-$C_4$-Alkoxy, Cl, Br, Acylamino, insbesondere $C_1$-$C_4$-Alkylcarbonylamino oder Arylcarbonylamino wie gegebenenfalls substituiertes Phenylcarbonylamino, $C_1$-$C_4$-Alkylsulfonylamino, Aminocarbonylamino, $C_1$-$C_4$-Alkylsulfonylamino, Arylsulfonylamino,
$R^{21}$= H, $C_1$-$C_4$-Alkyl, $C_1$-$C_4$-Alkoxy, Cl, Br oder COOH,
$R^{22}$= H, $C_1$-$C_4$-Alkyl, $C_1$-$C_4$-Alkoxy, Cl, Br, $SO_3H$.

Ebenfalls besonders wertvolle Farbstoffe dieser Reihe sind Reaktivfarbstoffe der Formel

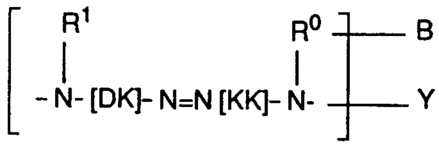 (XI),

DK = Rest einer Diazokomponente der Benzol- oder Naphthalinreihe,

KK = Rest einer Kupplungskomponente der Formel

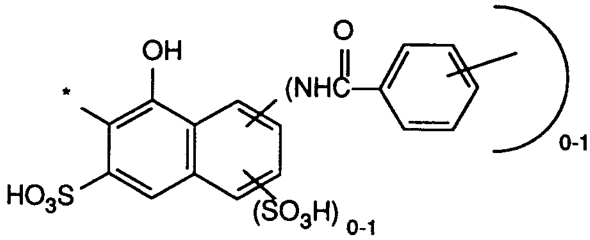 (XII)

wobei der Rest der Formel XII und die Azo-Gruppe über die mit * markierte Bindung miteinander verknüpft sind,

B = Rest der Formel

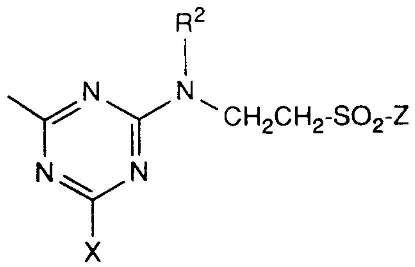 (VIa)

Y = heterocyclischer faserteaktiver Rest, der von B verschieden ist,

$R^0$, $R^1$ = unabhängig voneinander H, gegebenenfalls mit Substituenten wie beispielsweise OH, COOH, $SO_3H$ oder $OSO_3H$ substituiertes $C_1$-$C_6$-Alkyl,

und $R^2$, X und Z die obige Bedeutung haben.

Geeignete faserreaktive Reste Y, d.h. solche die mit den OH- oder NH-Gruppen der Faser unter Färbebedingungen unter Ausbildung kovalenter Bindungen reagieren, sind vorzugsweise solche, die mindestens einen reaktiven Substituenten an einen 5- oder 6-gliedrigen aromatisch-heterocyclischen Ring gebunden enthalten, beispielsweise an einen Monoazin-, Diazin- oder Triazinring, insbesondere einen Pyridin-, Pyrimidin-, Pyridazin-, Thiazin-, Oxazin- oder asymmetrischen oder symmetrischen Triazinring, oder an ein derartiges Ringsystem, welches einen oder mehrere ankondensierte aromatisch-carbocyclische Ringe aufweist, beispielsweise ein Chinolin-, Phthalazin-, Cinnolin-, Chinazolin-, Chinoxalin-, Acridin-, Phenazin- oder Phenanthridin-Ring-System, und die an kein weiteres Chromophor gebunden sind.

Unter den reaktiven Substituenten am Heterocyclus sind beispielsweise zu erwähnten Halogen (Cl, Br oder F), Ammonium einschließlich Hydrazinium, Pyridinium, Picolinium, Carboxypyridinium, Sulfonium, Sulfonyl, Azido-($N_3$), Rhodanido, Thiolether, Oxyether, Sulfinsäure und Sulfonsäure.

Im einzelnen sind beispielsweise folgende Reste für Y zu nennen:

2,4-Difluortriazinyl-6-, 2,4-Dichlortriazinyl-6, Mono-halogen-sym.-triazinylreste, insbesondere Monochlor- und Monofluortriazinylreste, die durch Alkyl, Aryl, Amino, Monoalkylamino, Dialkylamino, Aralkylamino, Arylamino, Morpholino, Piperidino, Pyrrolidino, Piperazino, Alkoxy, Aryloxy, Alkylthio, Arylthio substituiert sind, wobei Alkyl vorzugsweise gegebenenfalls substituiertes $C_1$-$C_4$-Alkyl, Aralkyl vorzugsweise gegebenenfalls substituiertes Phenyl-$C_1$-$C_4$-Alkyl und Aryl vorzugsweise gegebenenfalls substituiertes Phenyl oder Naphthyl bedeutet und wobei bevorzugte Substituenten für Alkyl, Halogen, Hydroxy, Cyan, Dialkylamino, Morpholino, $C_1$-$C_4$-Alkoxy, Carboxy, Sulfo oder Sulfato sind und für Phenyl und Napthyl, Sulfo, $C_1$-$C_4$-Alkyl, $C_1$-$C_4$-Alkoxy, Carboxy, Halogen, Acylamino, Hydroxy und Amino. Weiter zu nennen sind 2-Amino-4-fluor-triazinyl-6, 2-Methylamino-4-fluortriazinyl-6, 2-Ethylamino-4-fluortriazinyl-6, 2-Isopropylamino-4-fluor-triazinyl-6, 2-Dimethylamino-4-fluortriazinyl-6, 2-Diethylamino-4-fluor-triazinyl-6, 2-β-Methoxy-ethylamino-4-fluor-triazinyl-6, 2-β-Hydroxyethylamino-4-fluor-triazinyl-6, 2-Di-(β-hydroxy-ethylamino)-4-fluor-triazinyl-6, 2-Carboxymethylamino-4-fluor-triazinyl-6, 2-Di-(carboxymethylamino)-4-fluor-triazinyl-6, 2-Sulfomethyl-methylamino-4-fluor-triazinyl-6, 2-β-Cyanethylamino-4-fluor-triazinyl-6, 2-Benzylamino-4-fluor-triazinyl-6, 2-β-Phenylethylamino-4-fluor-triazinyl-6, 2-Benzyl-methylamino-4-fluor-triazinyl-6, 2-(4'-Sulfobenzyl)amino-4-fluor-triazinyl-6, 2-Cyclohexylamino-4-fluor-triazinyl-6, 2-(o-, m-, p-Methylphenyl)-amino-4-fluor-triazinyl-6, 2-(o-, m-, p-Sulfophenyl)-amino-4-fluor-triazinyl-6, 2-(2', 5'-Disulfophenyl)-amino-4-fluor-triazinyl-6, 2-(o-, m-, p-Chlorphenyl)-amino-4-fluor-triazinyl-6, 2-(o-, m-, p-Methoxyphenyl)-amino-4-fluor-triazinyl-6, 2-(2'-Methyl-4'-sulfophenyl)-amino-4-fluor-triazinyl-6, 2-(2'-Methyl-5'-sulfophenyl)-amino-4-fluor-triazinyl-6, 2-(2'-Chlor-4'-sulfophenyl)-amino-4-fluor-triazinyl-6, 2-(2'-Chlor-5'-sulfophenyl)-amino-4-fluor-triazinyl-6, 2-(2'-Methoxy-4'-sulfophenyl)-amino-4-fluor-triazinyl-6, 2-(o-, m-, p-Carboxyphenyl-)-amino-4-fluor-triazinyl-6, 2-(2'-,4'-Disulfophenyl-)-amino-4-fluor-triazinyl-6, 2-(3'-,5'-Disulfophenyl-)-amino-4-fluor-triazinyl-6, 2-(2' -Carboxy-4'-sulfophenyl-)-amino-4-fluor-triazinyl-6, 2-(2' -Carboxy-5'-sulfophenyl-)-amino-4-fluor-triazinyl-6, 2-(6'-Sulfonaphthyl-(2'))-amino-4-fluor-triazinyl-6, 2-(4' ,8'-Disulfonaphthyl-(2'))-amino-4-fluor-triazinyl-6, 2-(6' ,8'--Disulfonaphthyl-(2'))-amino-4-fluor-triazinyl-6, 2-(N-Methyl-N-phenyl)-amino-4-fluor-triazinyl-6, 2-(N-Ethyl-N-phenyl)-amino-4-fluor-triazinyl-6, 2-(N-β-Hydroxyethyl-N-phenyl)-amino-4-fluor-triazinyl-6, 2-(N-iso-propyl-N-phenyl)-amino-4-fluor-triazinyl-6, 2-Morpholino-4-fluor-triazinyl-6, 2-Piperidino-4-fluor-triazinyl-6, 2-(4',6',8'-Trisulfonaphthyl-(2'))-amino-4-fluor-triazinyl-6, 2-(3',6',8'-Trisulfo naphthyl-(2'))-amino-4-fluor-triazinyl-6, 2-(3',6'-Disulfonaphthyl-(1'))-amino-4-fluor-triazinyl-6, N-Methyl-N-(2-methyl-amino-4-chlortriazinyl-6)-carbamyl, N-Methyl-N-(2-dimethylamino-4-chlortriazinyl-6)-carbamyl, N-Methyl- bzw. N-Ethyl-N-(2,4-dichlortriazinyl-6)-aminoacetyl, 2-Methyloxy-4-fluor-triazinyl-6, 2-Ethoxy-4-fluor-triazinyl-6, 2-Phenoxy-4-fluor-triazinyl-6, 2-(o-, m- oder p-Methyl oder -Methoxy-phenoxy)-4-fluor-triazinyl-6, 2-β-Hydroxy-ethylmercapto-4-fluor-triazinyl-6, 2-Phenylmercapto-4-fluor-thazinyl-6, 2-(4'-Methylphenyl)-mercapto-4-fluortriazinyl-6, 2-(2',4'-Dinitrophenyl)-mercapto-4-fluor-triazinyl-6, 2-Methyl-4-fluor-triazinyl-6, 2-Phenyl-4-fluor-triazinyl-6 sowie die entsprechenden 4-Chlor- bzw. 4-Brom-triazinyl-Reste und die entsprechenden durch Halogenaustausch mit tertiären Basen wie Trimethylamin, Triethylamin, Dimethyl-β-hydroxyethyl-amin, Triethanolamin, N,N-Dimethylhydrazin, Pyridin, α-, β- oder γ-Picolin, Nicotinsäure oder Isonicotinsäure, Sulfinaten insbesondere Benzolsulfinsäure oder Hydrogensulfit erhältlichen Reste sowie Di- oder Trihalogen-pyrimidinreste, wie 2,4-Dichlorpyrimidinyl-6, 2,4,5-Trichlor-pyrimidinyl-6, 4,5-Dichlorpyrimidinyl-6, 2,4-Difluorpyrimidinyl-6-, 4,5-Difluor-5-chlorpyrimidyl, sowie 2,3-Dichlorchinoxalin-5-carbonyl und 2,3-Dichlorchinoxalin-6-carbonyl.

Die Reaktivfarbstoffe der Formel (XI) besitzen zwei mögliche Formen

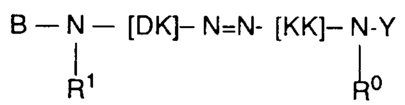

(XIa),

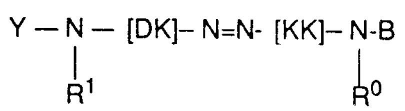

(XIb).

Bevorzugt sind Reaktivfarbstoffe der Formel (XI), worin

KK einen bivalenten Rest einer Kupplungskomponente der allgemeinen Formel

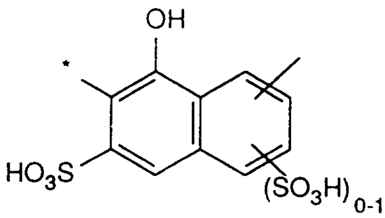

(XIIa)

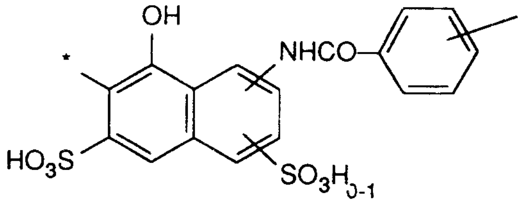

(XIIb)

bedeutet, wobei der Rest (XIIa) bzw. (XIIb) und die Azogruppe über die mit * markierte Bindung miteinander verknüpft sind,

DK einen Rest einer Diazokomponente der allgemeinen Formel

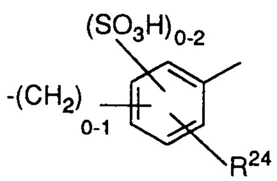

(XIIIa)

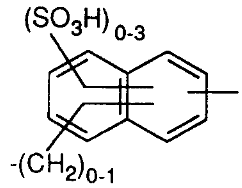

(XIIIb)

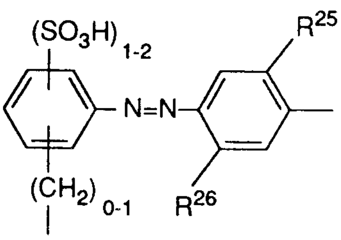

(XIIIc)

oder

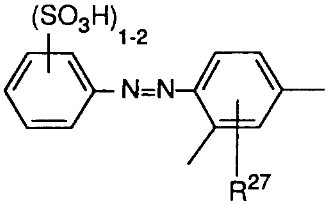

(XIIId)

bedeutet, und

$R^{25}$ = H, $C_1$-$C_4$-Alkyl, $C_1$-$C_4$-Alkoxy, OH oder $SO_3H$,

$R^{26}$ = H, $C_1$-$C_4$-Alkyl, $C_1$-$C_4$-Alkoxy, Acylamino, insbesonders $C_1$-$C_4$-Alkylcarbonylamino, $C_1$-$C_4$-Alkylsulfonylamino oder Aminocarbonylamino,

$R^{24}$ und $R^{27}$ = unabhängig H, $CH_3$, $C_2H_5$ oder $OCH_3$

und B, R, Y und $R^1$ die oben angegebenen Bedeutungen besitzen.

Weiterhin bevorzugt sind erfindungsgemäße Reaktivfarbstoffe der Formel (XI) bzw. (XIa)-(XIb), worin Y ein faserreaktiver fluorhaltiger Pyrimidinyl-6-Rest oder ein Rest der Formel

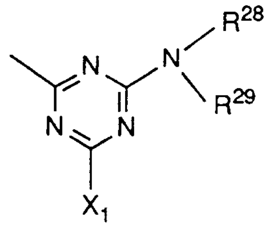

(XIV)

bedeutet, worin

$R^{28}$ und $R^{29}$ unabhängig voneinander H, gegebenenfalls durch Substituenten wie beispielsweise Halogen, Cyano, $C_1$-$C_4$-Alkoxy, Hydroxy, Phenyl, Carboxy, Sulfo oder Sulfato substituiertes $C_1$-$C_4$-Alkyl und $C_5$-$C_6$-Cycloalkyl, beispielsweise Benzyl, Phenethyl oder Cyclohexyl, wie Halogen, Nitro, Cyano, Trifluormethyl, Sulfamoyl, Carbamoyl, $C_1$-$C_4$-Alkyl, $C_1$-$C_4$-Alkoxy, $C_1$-$C_4$-Alkanoylamino, Benzoylamino, Ureido, Hydroxy, Carboxy, Sulfomethyl oder Sulfo substituiertes $C_6$-$C_{10}$-Aryl, inbesondere Phenyl oder Naphthyl bedeuten,

oder worin $R^{28}$ und$^{29}$ zusammen mit dem Aminostickstoffatom einen Morphotino-, Piperidino- oder Piperazinorest bilden, und worin $X_1$ = Cl, F oder ein gegebenenfalls mit beispielsweise COOH oder $SO_3H$ substituierter Pyridiniumrest ist.

Als besonders bevorzugt gelten Reaktivfarbstoffe der Formeln (61)- (72)

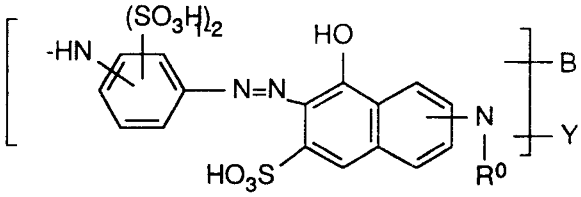

(61)

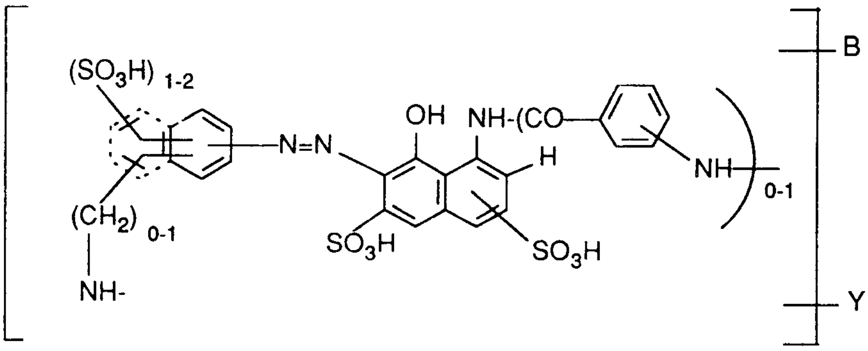

(62)

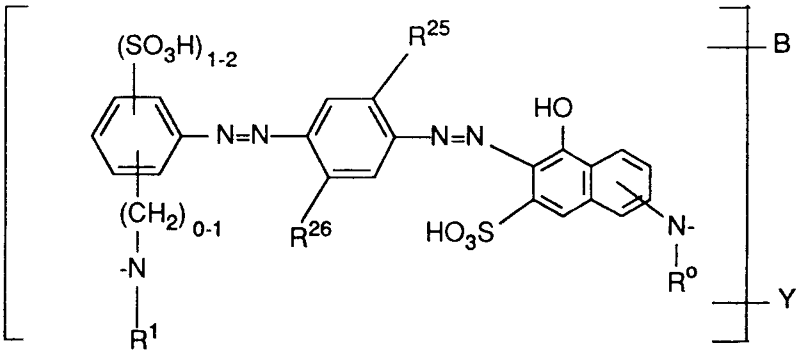

(63)

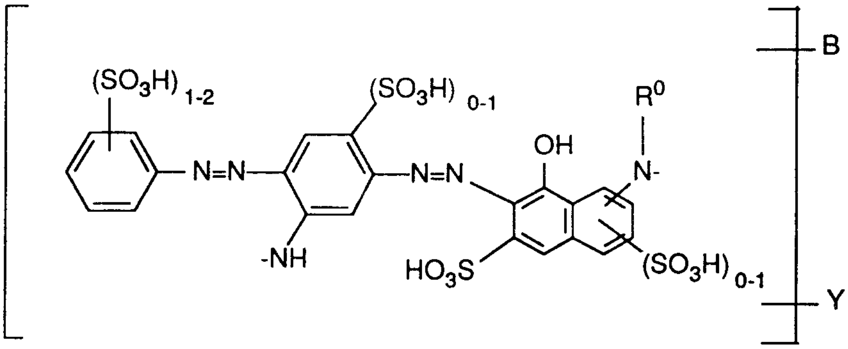

(64)

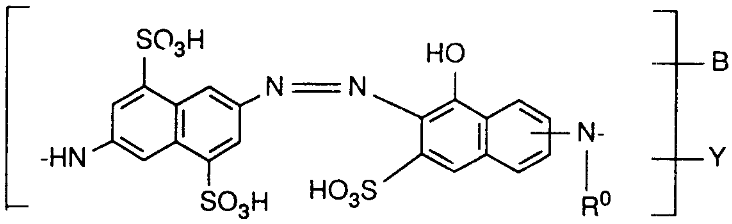

(65)

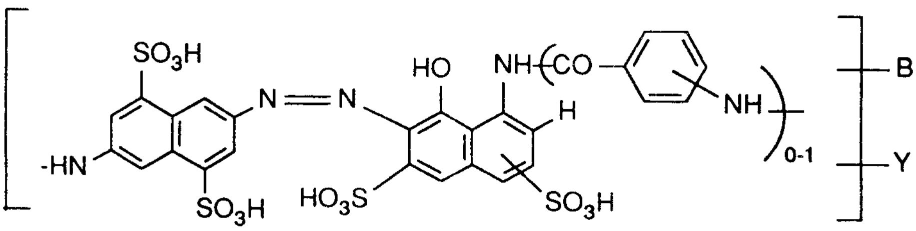

(66)

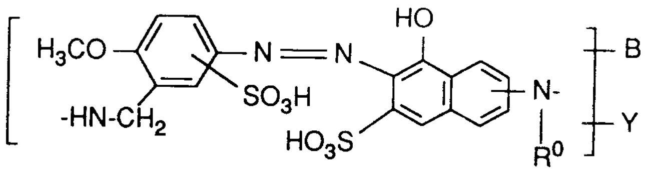
(67)

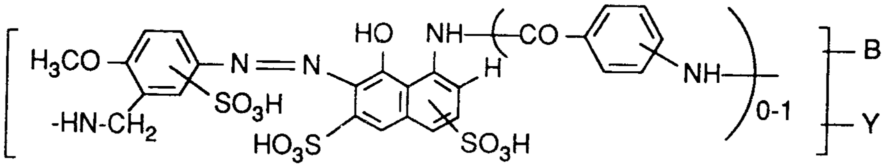
(68)

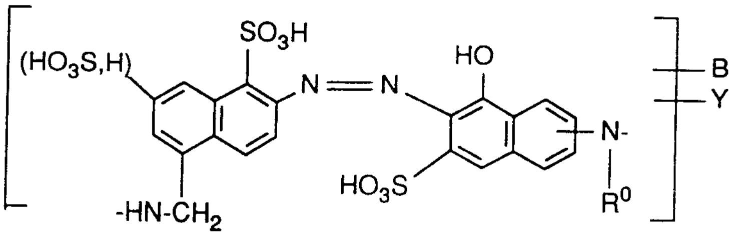
(69)

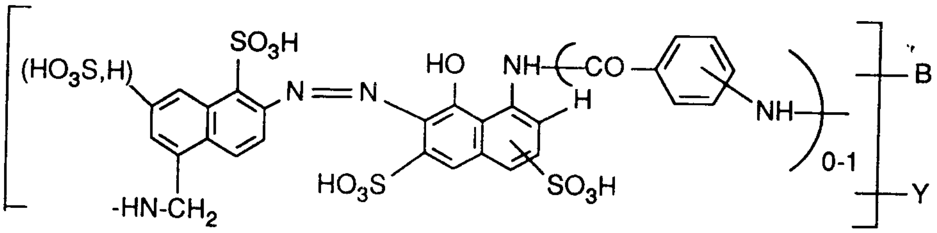
(70)

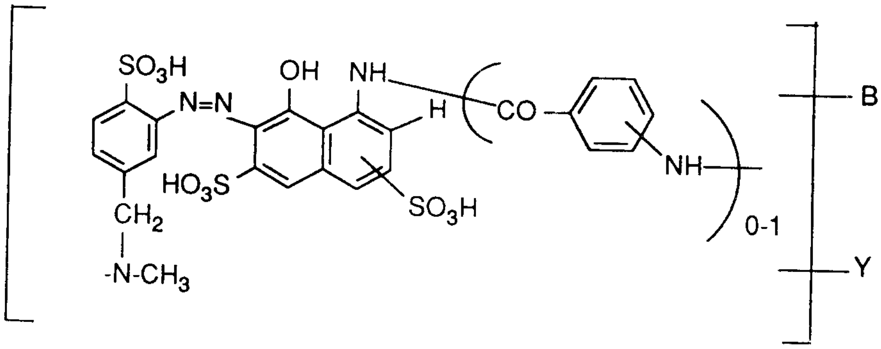
(71)

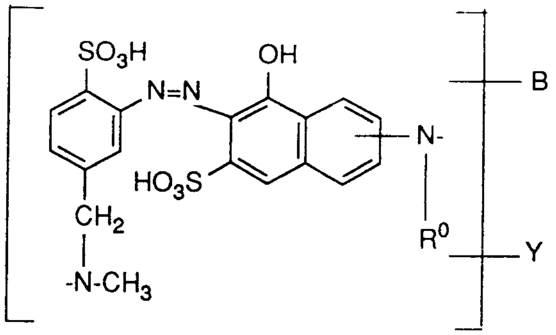

(72)

worin

| | |
|---|---|
| $R^0$ = | H, $CH_3$ oder Ethyl und |
| $R^1$, $R^{25}$, $R^{26}$ | , B und Y die obengenannte Bedeutung besitzen. |

Besonders bevorzugt sind vor allem Reaktivfarbstoffe der Formel (61) bis (72), worin Y ein mit $-NR^{28}R^{29}$ substituierter Fluor-s-triazin-Rest ist, wobei $-NR^4R^5$ vorzugsweise steht für: $-NH_2$, Morpholino, N-β-Hydroxyethylamino, N,N-Di-β-hydroxyethylamino, β-Sulfoethylamino, Phenylamino, das gegebenenfalls am Phenylkern substituiert ist durch Chlor, Methyl, Ethyl, Methoxy, Ethoxy, Acetylamino, Hydroxy, Carboxy, Sulfomethyl oder Sulfo, N-$C_{1-4}$-Alkyl-phenylamino, das gegebenenfalls am Phenylkern substituiert ist durch Chlor, Methyl oder Ethyl, N-(Sulfo-$C_{1-4}$-alkyl)-phenylamino, das gegebenenfalls am Phenylkern substituiert ist durch Chlor, Methyl oder Ethyl, N-(Hydroxy-$C_{1-4}$-alkyl-)-phenylamino oder Sulfonaphthylamino ist.

Weiterer Gegenstand der Erfindung ist ein Verfahren zur Herstellung von Reaktivfarbstoffen der Formel (XI), dadurch gekennzeichnet, daß man Farbstoffe der Formel

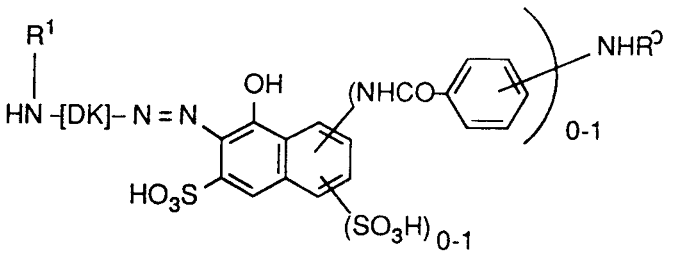

(XV)

in beliebiger Reihenfolge mit je einem Mol-Äquivalent der Reaktivkomponente der Formel

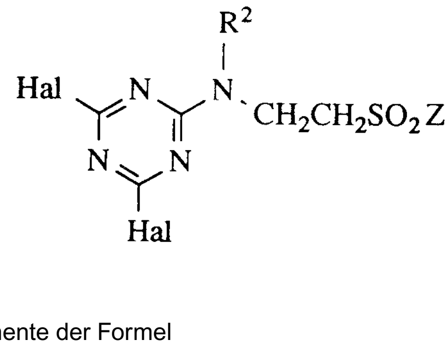

(XVI)

und der Reaktivkomponente der Formel

$$Y\text{-}Hal \qquad (VIII)$$

umsetzt,

oder daß man die entsprechenden Farbstoffvorprodukte der Formeln

$$R^1\text{-NH-[DK]-NH}_2 \text{ und H-[KK]-NHR}$$

jeweils mit je einem Mol-Äquivalent der Reaktivkomponente der Formel (VII) oder (VIII) umsetzt und durch Diazotierung und Kupplung und gegebenenfalls weiteren Umwandlungsreaktionen in die Reaktivfarbstoffe der Formel (I) überführt, wobei die Bedeutung der Reste R, $R^1$, $R^2$, Hal, B, p und M der oben angegebenen entspricht.

Bei der Herstellung der bevorzugten Azofarbstoffe besitzen die Diazokomponente und die Kupplungskomponente zusammen zwei Aminogruppen -N($R^0$)H und -N($R^1$)H, und gegebenenfalls weitere acylierbare Aminogruppen. Gegebenenfalls verwendet man entsprechende Acetylamino- oder Nitroverbindungen, worin die Acetylamino- bzw. Nitrogruppe vor der Kondensation mit einem Halogentriazin, Halogenpyrimidin oder dergleichen durch Verseifen bzw. Reduktion in die $NH_2$-Gruppe übergeführt wird. Die Einführung der Reaktivreste B und Y erfolgt durch Kondensation von Farbstoffen oder Farbstoffvorprodukten, welche acylierbare Aminogruppen enthalten, mit faserreaktiven halogenierten Acylierungsmitteln.

Da die einzelnen oben angegebenen Verfahrensschritte in unterschiedlicher Reihenfolge ausgeführt werden können, sind verschiedene Verfahrensvarianten möglich. Im allgemeinen führt man die Umsetzungen schrittweise nacheinander aus, wobei die Reihenfolge der einfachen Reaktionen zwischen den einzelnen Reaktionskomponenten vorteilhafterweise sich nach den besonderen Bedingungen richtet.

Da unter bestimmten Voraussetzungen Hydrolyse eines Halogentriazin- oder Halogenpyrimidinrestes etc. eintritt, muß ein Zwischenprodukt, welches Acetylaminogruppen enthalt, zwecks Abspaltung der Acetylgruppen verseift werden, bevor mit einem Aminodifluortriazin oder Trifluorthazin etc. kondensiert wird. Als weitere Umwandlungsreaktion kommt z.B. die nachträgliche Umsetzung eines Dihalogentriazinylrestes mit einem Amin in Betracht. Welche Reaktion bei der Herstellung eines sekundären Kondensationsproduktes aus Amin $HNR^{25}R^{29}$, 2,5,6-Trihalogen-s-triazin und Diaminobenzolsulfonsäure zweckmäßigerweise zuerst ausgeführt wird, die des Trihalogentriazins mit dem Amin oder mit der Diaminobenzolsulfonsäure, ist von Fall zu Fall verschieden und richtet sich vor allem nach der Löslichkeit der beteiligten Aminoverbindungen und der Basiszität der zu acylierenden Aminogruppen. Die wichtigsten Verfahrensvarianten sind in den Ausführungsbeispielen dargestellt.

Geeignete Ausgangsverbindungen für die Herstellung von Mono- oder Polyazofarbstoffen (XI) sind beispielsweise:

<u>Diazokomponenten</u> ($R^1$-NH-[DK]-$NH_2$)

1,3-Diaminobenzol, 1,4-Diaminobenzol, 1,3-Diamino-4-methylbenzol, 1,3-Diamino-4-ethylbenzol, 1,3-Diamino-4-methoxybenzol, 1,3-Diamino-4-ethoxybenzol, 1,4-Diamino-2-methylbenzol, 1,4-Diamino-2-methoxybenzol, 1,4-Diamino-2-ethoxybenzol, 1,4-Diamino-2,5-dimethylbenzol, 1,4-Diamino-2,5-diethylbenzol, 1,4-Diamino-2-methyl-5-methoxybenzol, 1,4-Diamino-2,5-dimethoxybenzol, 1,4-Diamino-2,5-diethoxybenzol, 2,6-Diamino-naphthalin, 1,3-Diamino-2,4,6-trimethylbenzol, 1,4-Diamino-2,3,5,6-tetramethylbenzol, 1,3-Diamino-4-nitrobenzol, 4,4'-Diaminostilben, 4,4'-Diaminodiphenylmethan, 2,6-Diaminonaphthalin-4,8-disulfonsäure, 1,4-Diaminobenzol-2-sulfonsäure, 1,4-Diaminobenzol-2,5-disulfonsäure, 1,4-Diaminobenzol-2,6-disulfonsäure, 1,3-Diaminobenzol-4-sulfonsäure, 1,3-Diaminobenzol-4,6-disulfonsäure,1,4-Diamino-2-methylbenzol-5-sulfonsäure, 1,5-Diamino-6-methylbenzol-3-sulfonsäure, 1,3-Diamino-6-methylbenzol-4-sulfonsäure, 3-(3'- bzw. 4'-Aminobenzoylamino)-1-aminobenzol-6-sulfonsäure, 1-(4'-Aminobenzoylamino)-4-aminobenzol-2,5-disulfonsäure, 1,4-Diaminobenzol-2-carbonsäure, 1,3-Diaminobenzol-4-carbonsäure, 1,2-Diaminobenzol-4-carbonsäure, 1,3-Diaminobenzol-5-carbonsäure, 1,4-Diaminobenzol-2-methylbenzol, 4,4'-Diaminodiphenyloxid, 4,4'-Diaminodiphenylharnstoff-2,2'-disulfonsäure, 4,4'-Diaminodiphenyloxyethan-2,2'-disulfonsäure, 4,4'-Diaminostilben-2,2'-disulfonsäure, 4,4'-Diaminodiphenylethan-2,2'-disulfonsäure, 2-Amino-5-aminomethylnaphthalin-1-sulfonsäure, 2-Amino-5-aminomethylnaphthalin-1,7-disulfonsäure, 1-Amino-4-methoxy-5-aminomethylbenzol-6-sulfonsäure, 1-Amino-3-(N-methyl)-aminomethylbenzol-6-sulfonsäure, 1-Amino-4-(N-methyl)-aminomethylbenzol-3-sulfonsäure, 1-Amino-4-aminomethylbenzol--3-sulfonsäure, 1,3-Diaminobenzol-4-(azo-phenyl-4'-sulfonsäure), 1,3-Diaminobenzol-4-(azo-phenyl-2',4'-disulfonsäure), 1,3-Diaminobenzol-6-sulfonsäure-4-(azophenyl-4'-sulfonsäure), 1,3-Diaminobenzol-6-sulfonsäure-4-(azophenyl-3', 6'-disulfonsäure).

Wenn als Diazokomponente statt eines Diamins eine Aminoacetyl-Verbindung eingesetzt werden soll, aus welcher nachträglich die Acetylgruppe durch Verseifen wieder abgespalten wird, wie dies oben in den Erläuterungen der Verfahrensvarianten beschrieben ist, kommen die Monoacetylverbindungen der obengenannten Diazokomponenten in Frage, z.B. 1-Acetylamino-3-aminobenzol-4-sulfonsäure oder 1-Acetylamino-4-aminobenzol-3-sulfonsäure.

<u>Kupplungskomponenten</u>(H-[KK]-$NR^0$H)

1-Amino-8-hydroxynaphthalin-6-sulfonsäure, 1-Amino-8-hydroxynaphthälin-2,4-disulfonsäure, 2-Hydroxy-3-aminonaphthalin-5,7-disulfonsäure, 1-Amino-8-hydroxynaphthalin-2,4,6-trisulfonsäure, 1-Hydroxy-8-acetyl-aminonaphthalin-3-sulfonsäure, 2-Amino-5-hydroxynaphthalin-7-sulfonsäure, 2-Methyl- bzw. 2-Ethylamino-5-hydroxynaphthalin-7-sulfonsäure, 2-(N-Acetyl-N-methylamino)-5-hydroxynaphthalin-7-sulfonsäure, 2-Acetylamino-5-hydroxynaphthalin-7-sulfonsäure, 2-Amino-5-hydroxynaphthalin-1,7-disulfonsäure, 2-Amino-8-hydroxynaphthalin-6-sulfonsäure, 2-(N-Acetyl-N-methylamino)-8-hydroxynaphthalin-6-sulfonsäure, 2-Acetylamino-8-hydroxynaphthalin-6-sulfonsäure, 2-Amino-8-hydroxynaphthalin-3,6-disulfonsäure, 2-Acetylamino-8-hydroxynaphthalin-3,6-disulfonsäure, 1-Amino-5-hydroxynaphthalin-7-sulfonsäure, 1-Amino-8-hydroxynaphthalin-3,6- bzw. -4,6-disulfonsäure, 1-Acetylamino-8-hydroxynaphthalin-3,6- bzw. -4,6-disulfonsäure, 1-(4'-Aminobenzoylamino)-8-hydroxynaphthalin-3,6- bzw. -4,6-disulfonsäure, 1-(4'-Nitrobenzoylamino)-8-hydroxynaphthalin-3,6- bzw. -4,6-disulfonsäure, 1-(3'-Aminobenzoylamino)-8-hydroxynaphthalin-3,6-bzw. -4,6-disulfonsäure, 1-(3'-Nitrobenzoylamino)-8-hydroxynaphthalin-3,6- bzw. -4,6-disulfonsäure, 1-Amino-8-hydroxynaphthalin-4-sulfonsäure.

Die Diazotierung der Diazokomponenten bzw. der eine diazotierbare Aminogruppe enthaltenden Zwischenprodukte erfolgt in der Regel durch Einwirkung salpetriger Säure in wäßrig-mineralsauerer Lösung bei tiefer Temperatur. Die Kupplung auf die Kupplungskomponente erfolgt bei schwach sauren, neutralen bis schwach alkalischen pH-Werten.

Die Kondensation der Reaktivkomponenten mit den Diazokomponenten und den Kupplungskomponenten und mit den Aminen bzw. mit acylierbaren Monoazo- oder Diazo-Zwischenprodukten bzw. mit den aminogruppenhaltigen Farbstoffen erfolgt vorzugsweise in wäßriger Lösung oder Suspension, bei niedriger Temperatur und bei schwach saurem, neutralem bis schwach alkalischem pH-Wert. Vorteilhaft wird der bei der Kondensation freiwerdende Halogenwasserstoff laufend durch Zugabe wäßriger Alkalihydroxide, -carbonate oder Hydrogencarbonate neutralisiert.

Die angegebenen Formeln sind die der freien Säuren. Bei der Herstellung werden im allgemeinen die Salze erhalten, insbesondere die Alkalisalze wie Natrium-, Kalium- oder Lithiumsalze. Die durch Quarternierung mit Pyridinen entstehende Ladung wird, in Abhängigkeit der Isolierungsbedingungen, durch ein Gegenion z.B. Chlorid, Fluorid oder Sulfat ausgeglichen; oder die Farbstoffe bilden innere Salze mit Sulfo-oder Carboxylgruppen. Die Farbstoffe können auch als konzentrierte Lösungen eingesetzt werden.

Die Reaktivfarbstoffe der Formel (I) eignen sich zum Färben und Bedrucken von natürlichen oder synthetischen hydroxy- oder amidgruppenhaltigen Materialien wie Seide, Leder, Wolle, synthetischen Polyamidfasern, insbesondere aber cellulosehaltiger Materialien faseriger Struktur wie Leinen, Zellstoff, regenerierte Zellulose und vor allem Baumwolle. Sie eignen sich sowohl für das Ausziehverfahren als auch zum Färben nach den üblichen Foulardfärbeverfahren, wonach die Ware mit wäßrigen und gegebenenfalls auch salzhaltigen Farbstofflösungen imprägniert wird, und die Farbstoffe nach einer Alkalibehandlung oder in Gegenwart von Alkali, gegebenenfalls unter Wärmeeinwirkung fixiert werden.

Die Reaktivfarbstoffe der Formel (I) zeichnen sich durch hohe Reaktivität und ausgezeichnetes Fixiervermögen aus. Aufgrund ihrer Trifunkfionalität ergeben sich auch aus langer Flotte hohe Fixierausbeuten. Sie sind charakterisiert durch relative Unabhängikeit der Ergiebigkeit von der Färbetemperatur und können daher nach dem Ausziehverfahren bei niedrigen bis mittleren Färbetemperaturen eingesetzt werden. Beim Klotz-Dämpf-Verfahren erfordern sie nur kurze Dämpfzeiten. Sie ergeben Färbungen hoher Farbstärke mit guten Licht- und Naßechtheiten.

Erfindungsgemäß sind weiterhin Textilprodukte, enthaltend mit Farbstoffen der Formel (I) gefärbte hydroxyl-oder amidgruppenhaltige Materialien.

Die $\lambda_{max}$-Werte der Farbstoffe aus den nachstehenden Beispielen sind, sofern nicht anders angegeben, in Wasser gemessen worden.

<u>Beispiel 1</u>

A) 0,1 Mol 1-Amino-8-hydroxy-3,6-naphthalin-disulfonsäure (H-Säure) wurden in 150 Teilen Eis und 150 Teilen Wasser suspendiert und mit Lithiumhydroxidlösung bei pH 6 gelöst. Man gab 0,11 Mol 2,4,6-Trichlor-1,3,5-triazin zu, ließ den pH-Wert auf 4 abfallen und hielt ihn bis zur Beendigung der Kondensation mit Lithiumcarbonat.

B) Die obige Lösung wurde bei pH 8 und 20°C in 10 Minuten in eine Lösung von 0,11 Mol der Verbindung der Formel

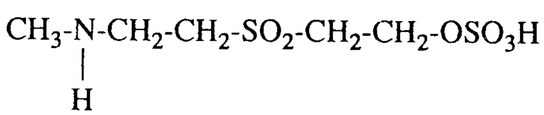 (Va)

in 50 Teilen Wasser getropft, wobei der pH-Wert mit Lithiumhydroxidlösung konstant gehalten wurde.

C) 0,1 Mol 2-Amino-1-naphthalinsulfonsäure wurden in der üblichen Weise diazotiert und bei pH 7,5-8,5 auf das H-Säure-Kondensationsprodukt gekuppelt. Der Farbstoff wurde durch Zugabe von Kaliumchlorid ausgesalzen und isoliert. Nach Trocknung erhielt man ca. 35 g eines salzhaltigen Farbstoffpulvers. Diesem Farbstoff kommt folgende Struktur

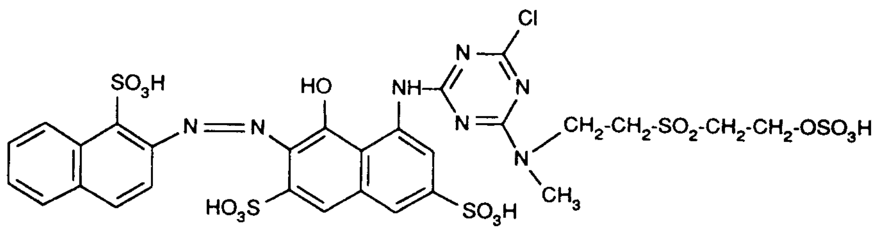

zu und färbt Baumwolle nach den für Reaktivfarbstoffe üblichen Färbe- oder Druckverfahren in roten Farbtönen.

$\lambda_{max}$ = 522 nm, 545 nm (Wasser)

Beispiel 2

A) 0,1 Mol 1-Amino-5-hydroxy-2-naphthalin-sulfonsäure (I-Säure) wurden in 300 Teilen Eis und 300 Teilen Wasser suspendiert und mit Lithiumhydroxidlösung bei pH 6 gelöst. Innerhalb von 10 Minuten tropfte man bei pH 4,5 0,15 Mol 2,4,6-Trifluor-1,3,5-triazin zu, wobei der pH-Wert mit Lithiumcarbonat konstant gehalten wurde.

B) Die obige Suspension wurde bei pH 8 und 20°C in 10 Minuten in eine Lösung von 0,12 Mol der Verbindung der Formel Va in 50 Teilen Wasser getropft, wobei der pH-Wert mit Lithiumhydroxidlösung konstant gehalten wurde.

C) 0,1 Mol 2-Amino-1,5-naphthahndisulfonsäure wurden in 200 Teilen Wasser und 100 Teilen Eis bei 0°C suspendiert. Man gab 28 Teile konzentrierte Salzsäure zu. Innerhalb von 15 Minuten tropfte man eine Lösung von 7 Teilen Natriumnitrit in 70 Teilen Wasser zu. Nach 30-minütigem Nachrühren war die Diazotierung beendet. Es resultierte eine hellgelbe Suspension. Der Nitritüberschuß wurde mit Amidosulfonsäure zerstört.

Diese Suspension wurde nun über einen Zeitraum von 15-20 Minuten zu 0,1 Mol des I-Säure-Kondensationsproduktes zudosiert. Gekuppelt wurde bei 20°C und einem pH-Wert von 7-8. Der Farbstoff wurde durch Zugabe von Ethanol ausgefällt und isoliert. Nach Trocknung erhielt man ca. 40 g eines Farbstoffpulvers. Die Struktur des Farbstoffs entspricht der Formel

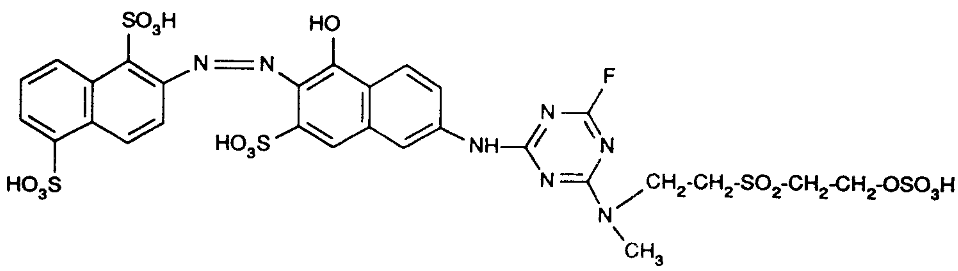

und er färbt Baumwolle nach den für Reaktivfarbstoffe üblichen Färbe- und Druckverfahren orange.

$\lambda_{max}$ = 484 nm

Weitere Reaktivfarbstoffe erhielt man durch Kondensation der folgenden Komponenten:

| Nr. | Diazokomponente | 1-Amino-8-hydroxy-naphthalin-disulfos. | Trihalogen-triazin | Komponente der Formel (V) | Farbton | $\lambda_{max}$ (nm) |
|---|---|---|---|---|---|---|
| 3 | 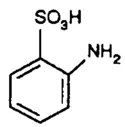 | 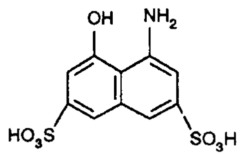 | 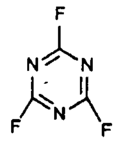 | $HN-CH_2-CH_2-SO_2-CH_2-CH_2-OSO_3H$<br>\|<br>$CH_3$ | rot | 513, 531 |
| 4 | 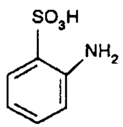 | 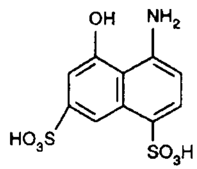 | 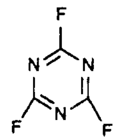 | . | rot | |
| 5 | 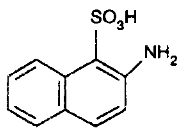 | 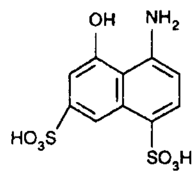 | 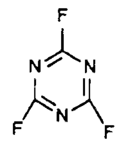 | . | blausti-chig rot | |
| 6 | 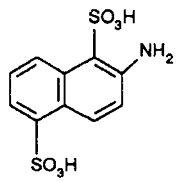 | 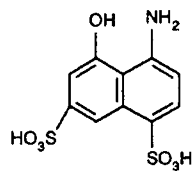 | 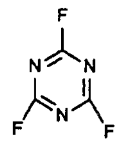 | . | rot | |

| Nr. | Diazokomponente | 1-Amino-8-hydroxy-naphthalin-disulfos. | Trihalogen-triazin | Komponente der Formel (V) | Farbton | $\lambda_{max}$ (nm) |
|---|---|---|---|---|---|---|
| 7 | 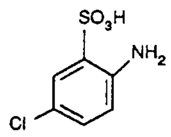 | 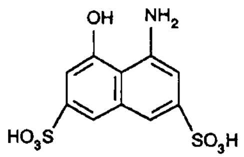 | 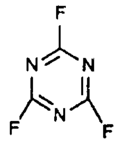 | $\underset{CH_3}{HN}-CH_2-CH_2-SO_2-CH_2-CH_2-OSO_3H$ | rot | |
| 8 | 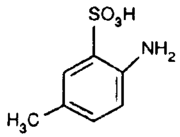 | 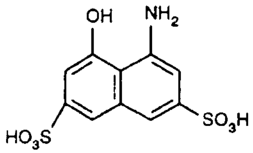 | 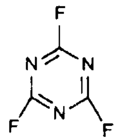 | " | rot | 518 |
| 9 | 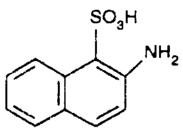 | 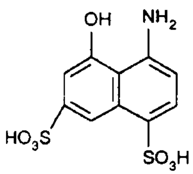 | 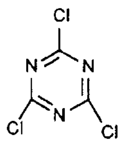 | " | blaustichig rot | |
| 10 | 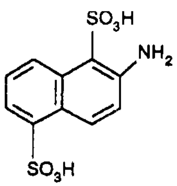 | 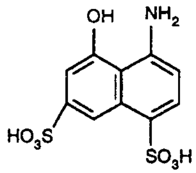 | 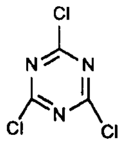 | " | rot | |

| Nr. | Diazokomponente | 1-Amino-8-hydroxy-naphthalin-disulfos. | Trihalogen-triazin | Komponente der Formel (V) | Farbton | $\lambda_{max}$ (nm) |
|---|---|---|---|---|---|---|
| 11 | 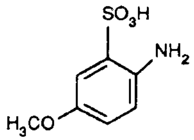 | 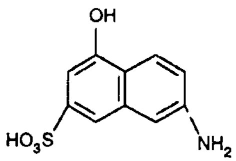 | 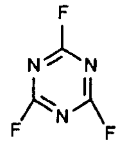 | $HN(CH_3)-CH_2-CH_2-SO_2-CH_2-CH_2-OSO_3H$ | gelbstichig | 501 |
| 12 | 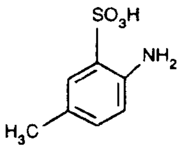 | 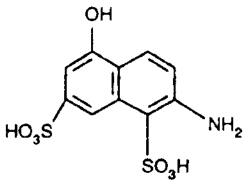 | 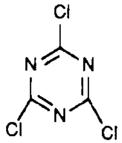 | . | orange | |
| 13 | 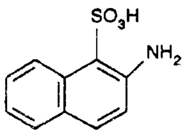 | 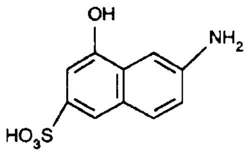 | 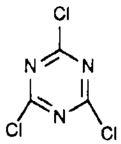 | . | rot | |
| 14 | 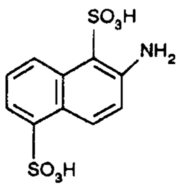 | 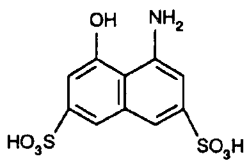 | 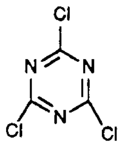 | . | rot | 518, 541 |

| Nr. | Diazokomponente | 1-Amino-8-hydroxy-naphthalin-disulfos. | Trihalogen-triazin | Komponente der Formel (V) | Farbton |
|---|---|---|---|---|---|
| 15 | 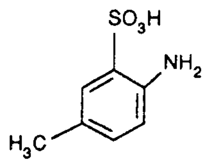 | 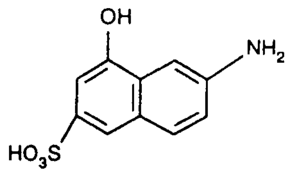 | 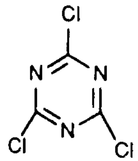 | $\underset{\mid \; CH_3}{HN}-CH_2-CH_2-SO_2-CH_2-CH_2-OSO_3H$ | orange |
| 16 | 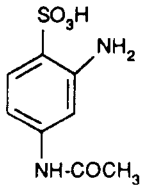 | 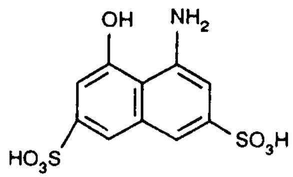 | 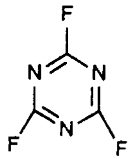 | • | rot |
| 17 | 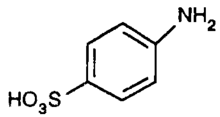 | 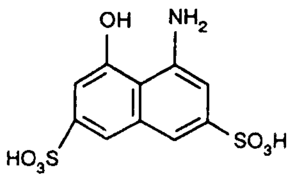 | 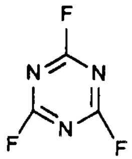 | • | rot |
| 18 | 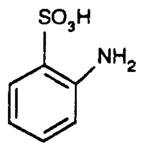 | 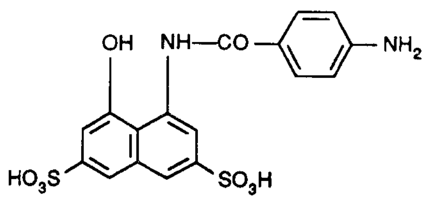 | 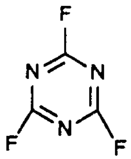 | • | rot |

Beispiel 19

0,1 Mol der Monoazoverbindung mit der Formel

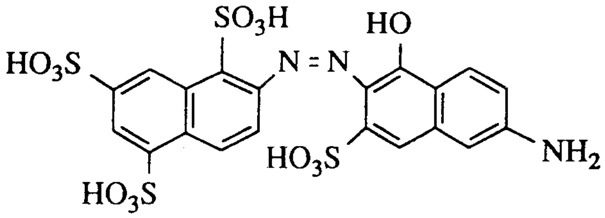

wurden in 500 Teilen Wasser und 200 Teilen Eis mit Lithiumhydroxidlösung bei pH 6 gelöst. Innerhalb 10 Minuten tropfte man bei pH 4,5 0,15 Mol 2,4,6-Trifluor-1,3,5-triazin zu, wobei der pH-Wert mit Lithiumcarbonat konstant gehalten wurde.

Die obige Lösung wurde bei pH 8 und 20°C in 10 Minuten in eine Lösung von 0,12 Mol der Verbindung der Formel (Va) in 50 Teilen Wasser getropft, wobei der pH-Wert mit Lithiumhydroxidlösung konstant gehalten wurde.

Der Farbstoff wurde durch Zugabe von Kaliumchlorid ausgesalzen, isoliert und getrocknet. Man erhielt ca. 72 g eines salzhaltigen Farbstoffes, dem die Struktur

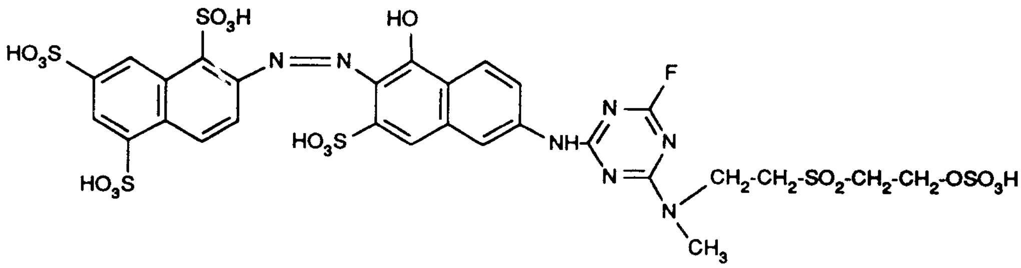

zukommt und der Baumwolle nach den für Reaktivfarbstoffe üblichen Färbe- oder Druckverfahren in orangen Farbtönen färbt.

$\lambda_{max}$ = 483 nm

Weitere Reaktivfarbstoffe wurden durch Kondensation von Trifluortriazin und den folgenden Komponenten analog Beispiel 19 erhalten.

| Nr. | Aminoazofarbstoff | Komponente der Formel (V) | Farbton | $\lambda_{max}$ |
|---|---|---|---|---|
| 20 | 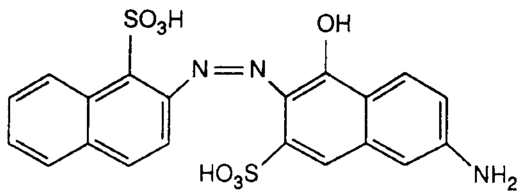 | $HN-CH_2-CH_2-SO_2-CH_2-CH_2OSO_3H$<br>\|<br>$CH_3$ | orange | |
| 21 | 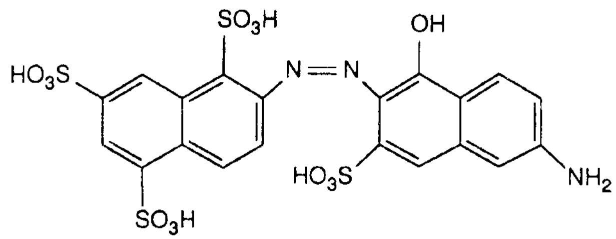 | " | orange | |
| 22 | 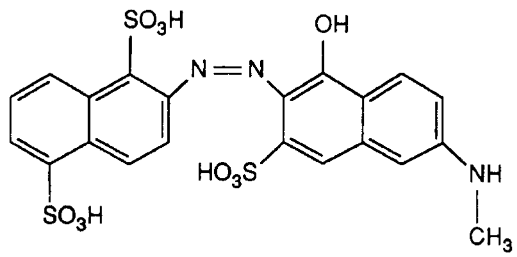 | " | orange | |

| Nr. | Aminoazofarbstoff | Komponente der Formel (V) | Farbton | $\lambda_{max}$ (nm) |
|---|---|---|---|---|
| 23 | 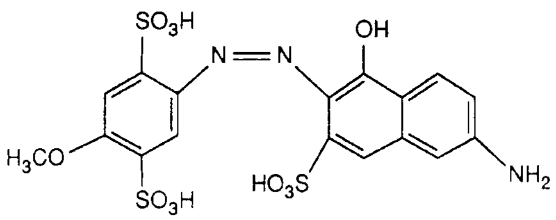 | $HN(CH_3)CH_2CH_2\text{-}SO_2\text{-}CH_2\text{-}CH_2OSO_3H$ | orange | 497 |
| 24 | 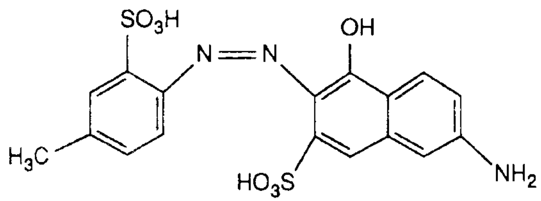 | " | orange | 488 |
| 25 | 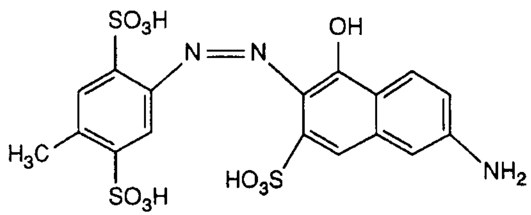 | " | orange | 485 |

| Nr. | Aminoazofarbstoff | Komponente der Formel (V) | Farbton | $\lambda_{max}$ (nm) |
|---|---|---|---|---|
| 26 | 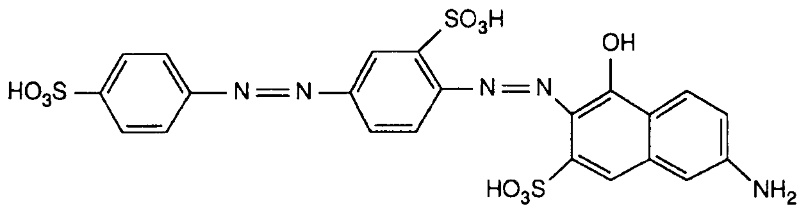 | $HNCH_2CH_2-SO_2CH_2CH_2OSO_3H$<br>\|<br>$CH_3$ | gelbstichig rot | 512 |
| 27 | 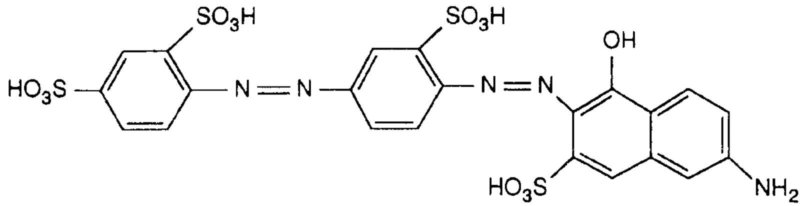 | " | gelbstichig rot | |
| 28 | 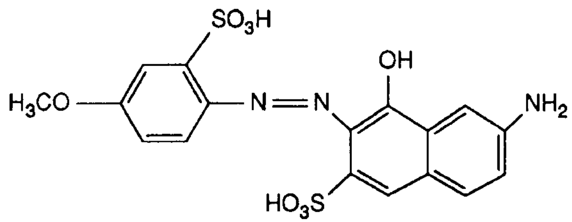 | " | gelbstichig rot | 513 |

| Nr. | Aminoazofarbstoff | Komponente der Formel (V) | Farbton |
|---|---|---|---|
| 29 | 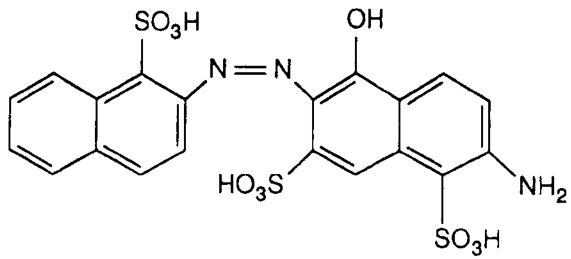 | $HNCH_2CH_2\text{-}SO_2\text{-}CH_2\text{-}CH_2OSO_3H$<br>$\vert$<br>$CH_3$ | orange |
| 30 | 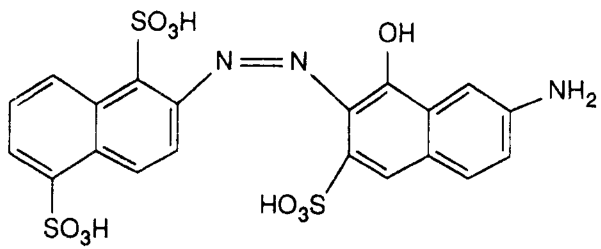 | " | scharlach |
| 31 | 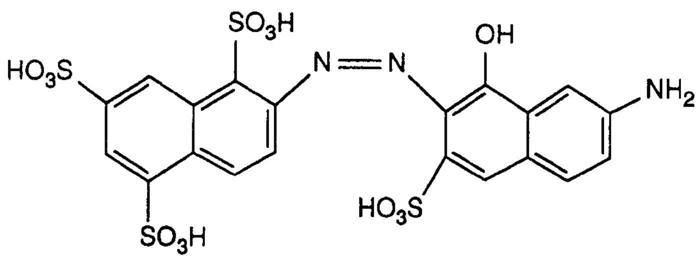 | " | scharlach |

Beispiel 32

0,1 Mol des Monoazofarbstoffes der Formel

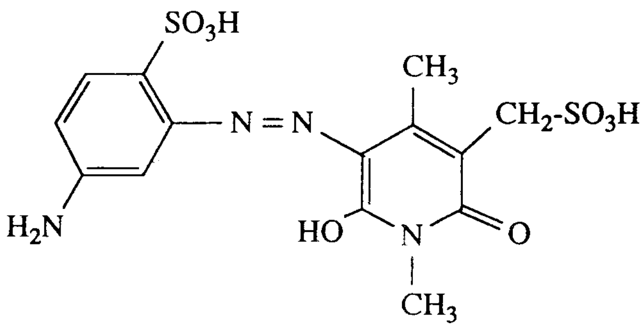

wurden analog Beispiel 19 mit 2,4,6-Trifluor-1,3,5-triazin und der Verbindung der Formel (Va) umgesetzt.

Man erhielt ca. 105 g eines salzhaltigen Farbstoffes, dem die Struktur

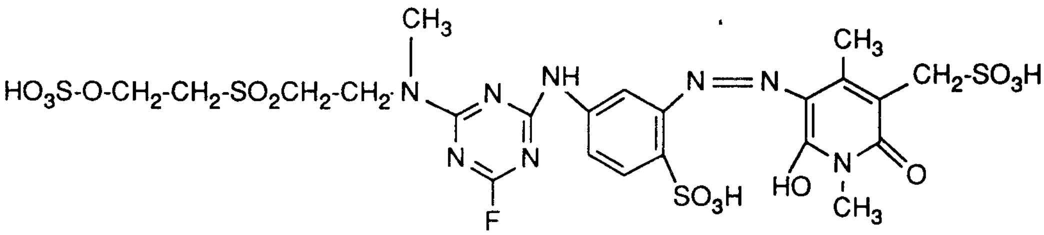

zukommt und Baumwolle nach dem für Reaktivfarbstoffe üblichen Färbe- oder Druckverfahren in gelben Farbtönen färbt.

$\lambda_{max}$ = 421 nm

Weitere gelbe Reaktivfarbstoffe erhielt man durch Kondensation der folgenden Komponenten:

| Nr. | Azokomponente | Trihalogen-triazin | Komponente der Formel (V) | Farbton |
|---|---|---|---|---|
| 33 | 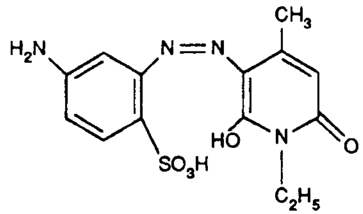 | 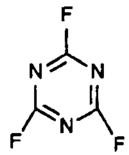 | $HN-CH_2-CH_2-SO_2-CH_2-CH_2-OSO_3H$<br>$CH_3$ | grünstichig gelb |
| 34 | 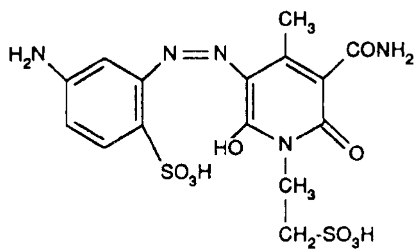 | 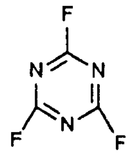 | " | grünstichig gelb |
| 35 | 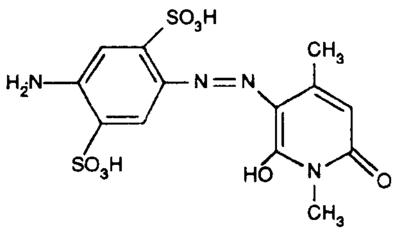 | 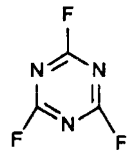 | " | gelb |
| 36 | 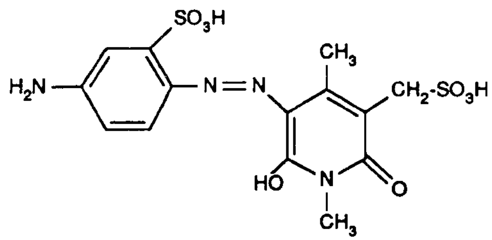 | 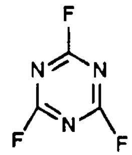 | " | gelb |

| Nr. | Azokomponente | Trihalogen-triazin | Komponente der Formel (V) | Farbton | $\lambda_{max}$ (nm) |
|---|---|---|---|---|---|
| 37 | 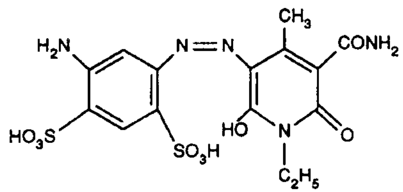 | 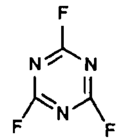 | $HN(CH_3)-CH_2-CH_2-SO_2-CH_2-CH_2OSO_3H$ | grünstichig gelb | |
| 38 | 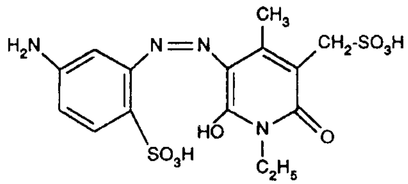 | 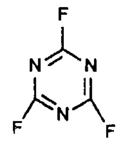 | " | grünstichig gelb | 422 |
| 39 | 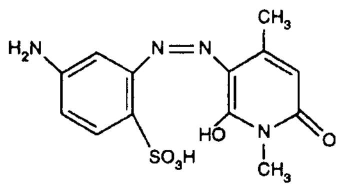 | 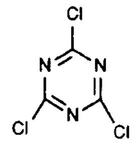 | " | grünstichig gelb | |
| 40 | 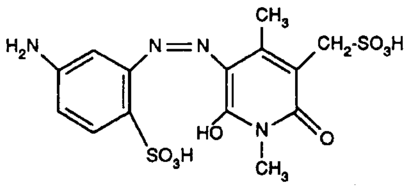 | 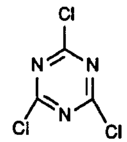 | " | grünstichig gelb | 421 |

| Nr. | Azokomponente | Trihalogen-triazin | Komponente der Formel (V) | Farbton | $\lambda_{max}$ (nm) |
|---|---|---|---|---|---|
| 41 | 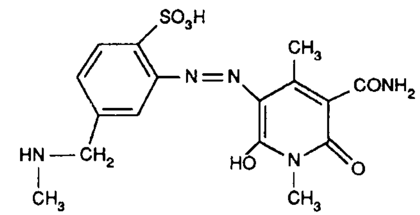 | 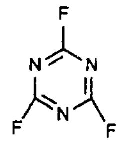 | $HN(CH_3)CH_2CH_2SO_2CH_2CH_2OSO_3H$ | grünstichig gelb | |
| 42 | 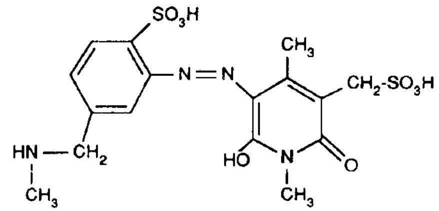 | " | " | grünstichig gelb | |
| 43 | 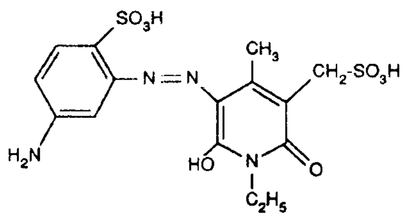 | 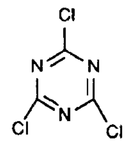 | " | grünstichig gelb | 421 |
| 44 | 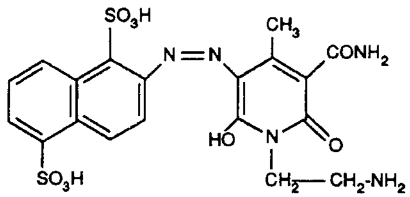 | " | " | gelb | |

Beispiel 45

0,1 Mol des Monoazofarbstoffes der Formel

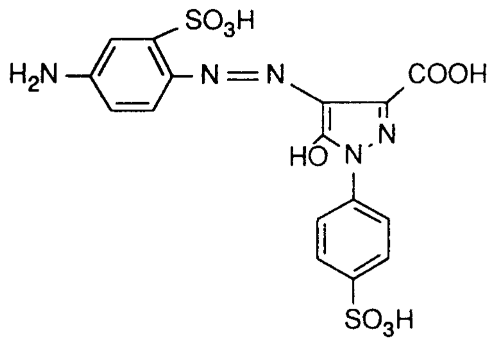

wurden analog Beispiel 19 mit 2,4,6-Trifluor-1,3,5-triazin und der Verbindung der Formel (Va) umgesetzt.

Man erhielt ca. 105 g eines salzhaltigen Farbstoffpulvers, dem die Struktur

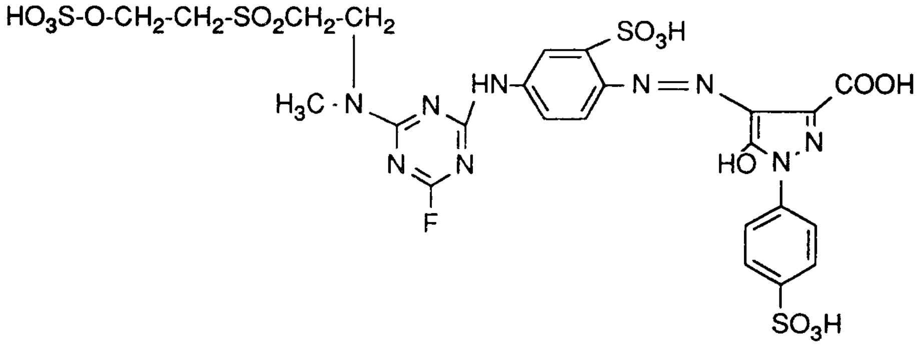

zukommt und Baumwolle nach dem für Reaktivfarbstoffe üblichen Färbe- oder Druckverfahren in gelben Farbtönen färbt.

Weitere gelbe Farbstoffe mit ähnlichen Eigenschaften erhielt man, wenn man die folgenden Aminophenylazopyrazolone mit Cyanurfluorid und der angegebenen Verbindung der Formel (V) kondensierte.

| Nr. | Aminophenylazopyrazolon | Komponente der Formel (V) |
|---|---|---|
| 46 | 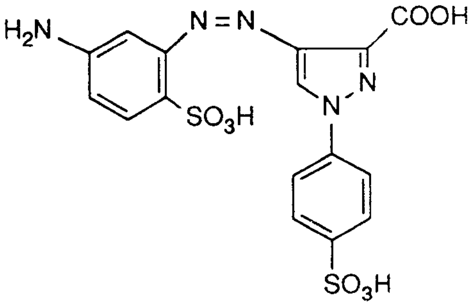 | $HN-CH_2-CH_2-SO_2-CH_2-CH_2OSO_3H$<br>I<br>$CH_3$ |
| 47 | 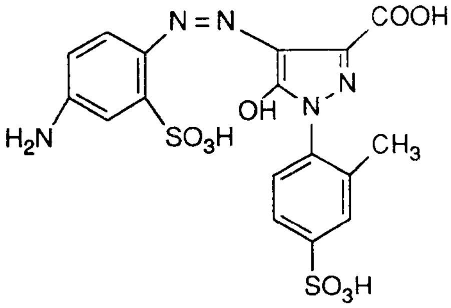 | " |
| 48 | 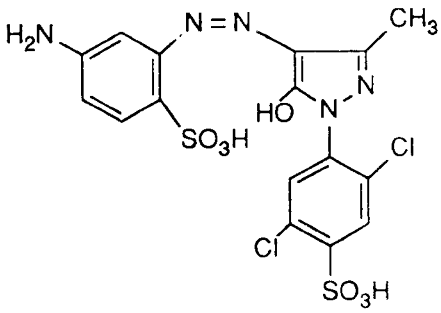 | " |

| Nr. | Aminophenylazopyrazolon | Komponente der Formel (V) |
|---|---|---|
| 49 | 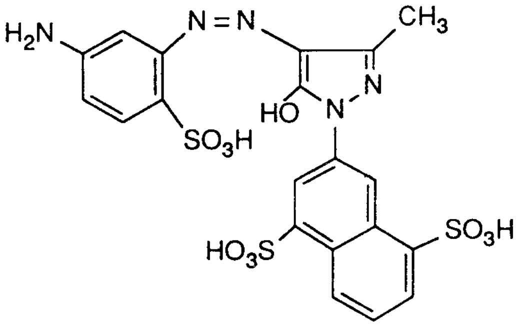 | $HN-CH_2-CH_2-SO_2-CH_2-CH_2OSO_3H$<br>\|<br>$CH_3$ |
| 50 | 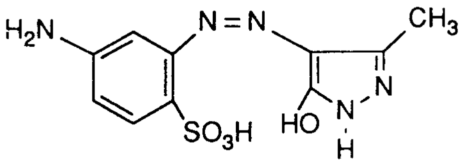 | " |
| 51 | 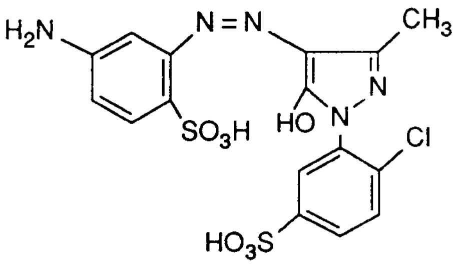 | " |
| 52 | 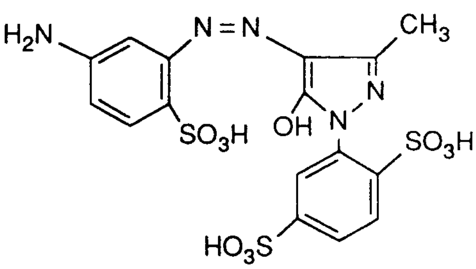 | " |

Beispiel 53

0,1 Mol des Monoazofarbstoffes der Formel

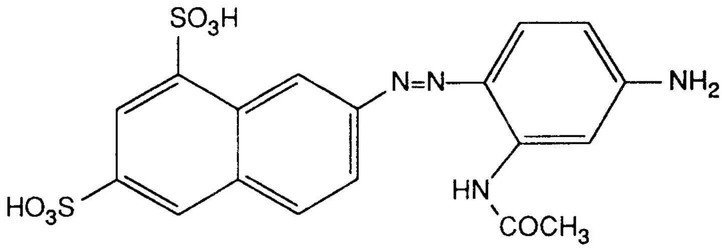

wurden analog Beispiel 19 mit 2,4,6-Trifluor-1,3,5-triazin und der Verbindung der Formel (Va) umgesetzt.

Die erhaltene Lösung wurde nach Zusatz von 1,5 g Phosphat-Puffer von pH 6 im Vakuum bei 35 bis 40°C eingedampft oder sprühgetrocknet. Der erhaltene Farbstoff der Formel

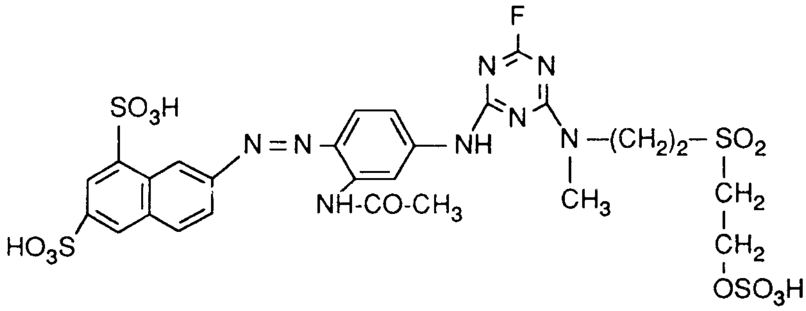

färbt Baumwolle aus langer Flotte in goldgelben Tönen.

$\lambda_{max}$ = 389 nm

Weitere rotstichig gelb färbende Farbstoffe erhielt man durch Kondensation folgender p-Aminoazoverbindungen mit Cyanurfluorid oder Cyanurchlorid und Verbindungen der Formel (V).

| Nr. | Aminoazofarbstoff | Trihalogen-triazin | Komponente der Formel (V) | Farbton | $\lambda_{max}$(nm) |
|---|---|---|---|---|---|
| 54 | 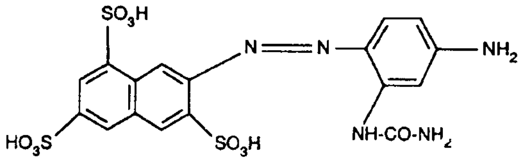 | 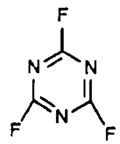 | $HNCH_2CH_2\text{-}SO_2\text{-}CH_2\text{-}CH_2OSO_3H$<br>$CH_3$ | rotstichig gelb | 419 |
| 55 | 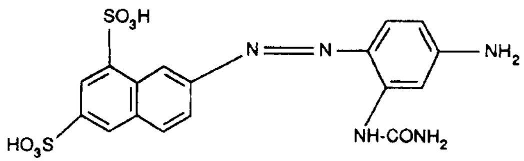 | 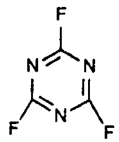 | " | rotstichig gelb | 399 |
| 56 | 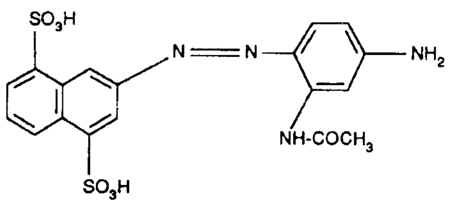 | 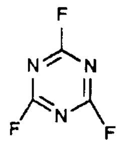 | " | rotstichig gelb | 389 |
| 57 | 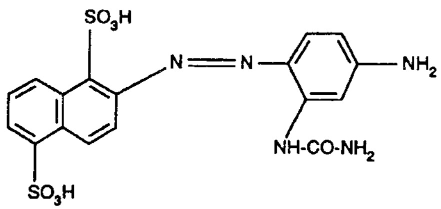 | 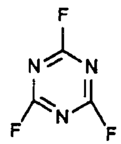 | " | rotstichig gelb | 395 |

| Nr. | Aminoazofarbstoff | Trihalogen-triazin | Komponente der Formel (V) | Farbton | $\lambda_{max}$(nm) |
|---|---|---|---|---|---|
| 58 | 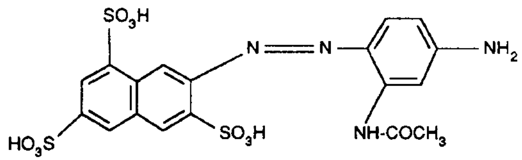 | 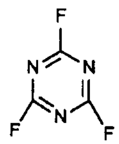 | $HNCH_2CH_2-SO_2-CH_2-CH_2OSO_3H$ (N–$CH_3$) | rotstichig gelb | 401 |
| 59 | 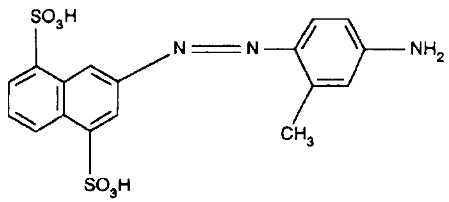 | 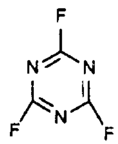 | " | rotstichig gelb | |
| 60 | 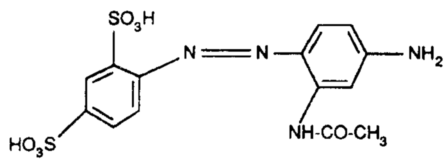 | 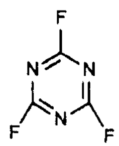 | " | rotstichig gelb | |
| 61 | 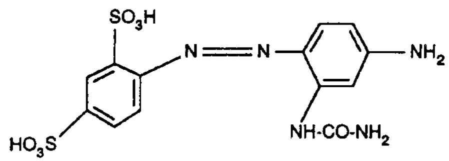 | 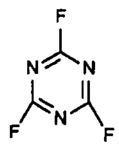 | " | rotstichig gelb | 401 |

| Nr. | Aminoazofarbstoffe | Trihalogen-triazin | Komponente der Formel (V) | Farbton | $\lambda_{max}$(nm) |
|---|---|---|---|---|---|
| 62 | 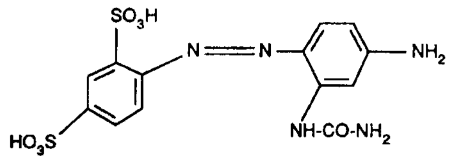 | 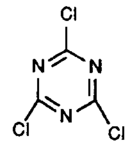 | $HNCH_2CH_2\text{-}SO_2\text{-}CH_2\text{-}CH_2OSO_3H$<br>I<br>$CH_3$ | rotstichig gelb | 401 |
| 63 | 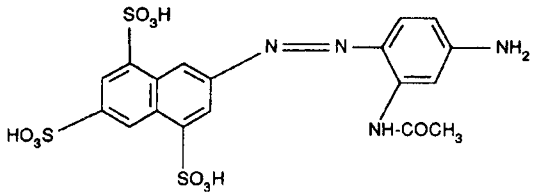 | 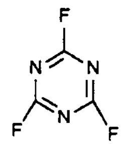 | " | rotstichig gelb | |
| 64 | 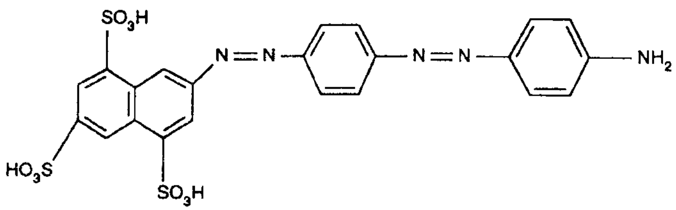 | 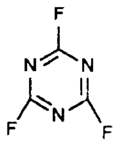 | " | orange | |

| Nr. | Aminoazofarbstoffe | Trihalogen-triazin | Komponente der Formel (V) | Farbton | $\lambda_{max}$(nm) |
|---|---|---|---|---|---|
| 65 | 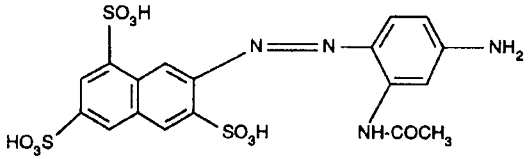 | 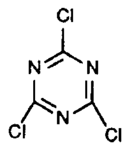 | $HNCH_2CH_2-SO_2-CH_2CH_2OSO_3H$ (N-$CH_3$) | rotstichig gelb | 402 |
| 66 | 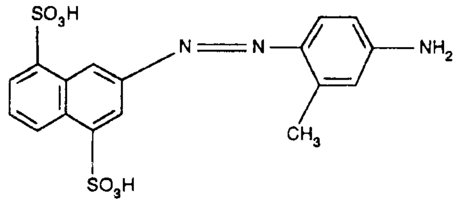 | 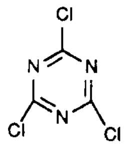 | " | rotstichig gelb | |
| 67 | 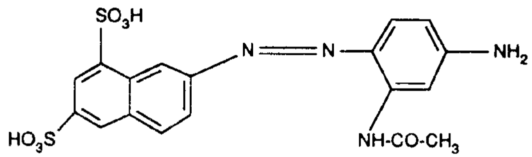 | 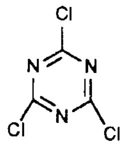 | " | rotstichig gelb | 389 |
| 68 | 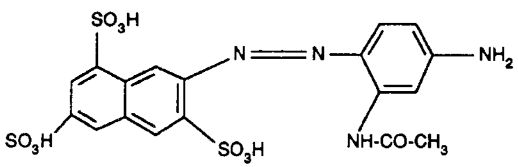 | 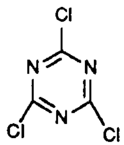 | " | rotstichig gelb | |

Beispiel 69

0,1 Mol des Monoazofarbstoffes der Formel

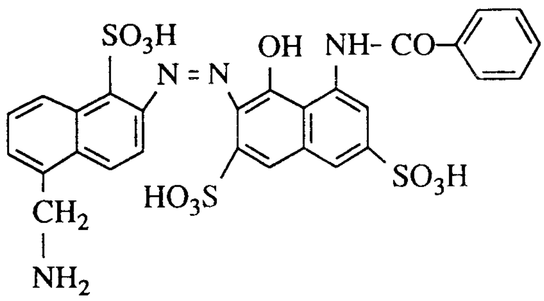

wurden analog Beispiel 19 mit 2,4,6-Trifluor-1,3,5-triazin und der Verbindung der Formel (Va) umgesetzt. Nach beendeter Kondensation wurde der Farbstoff durch Aussalzen und Absaugen isoliert und nach Pufferung auf einen pH-Wert von 6,5 im Vakuum bei 45°C getrocknet. Er entspricht der Formel

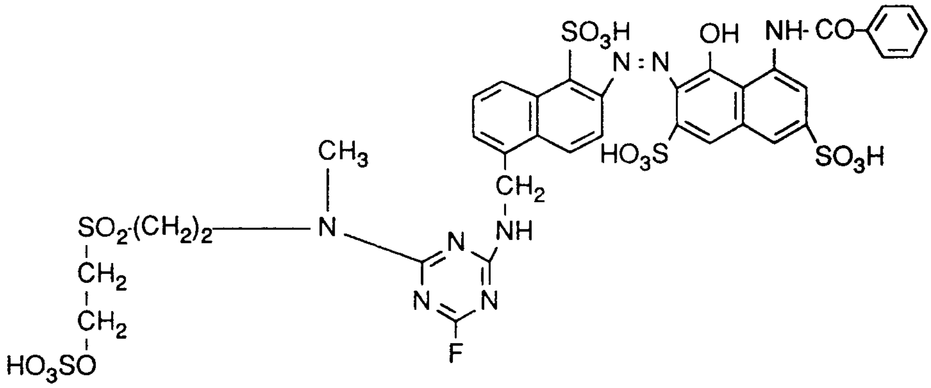

und färbt Baumwolle aus langer Flotte in blaustichig roten Tönen mit guter Fixierausbeute.

$\lambda_{max}$ = 520 nm, 544 nm

Ähnliche rote Farbstoffe erhielt man durch Umsetzung folgender Komponenten:

| Nr. | Diaminobenzolsulfonsäure | Trihalogentriazin | Kupplungskomponente | Komponente der Formel (V) | Farbton | $\lambda_{max}$(nm) |
|---|---|---|---|---|---|---|
| 70 | 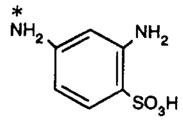 | 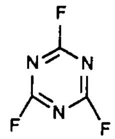 | 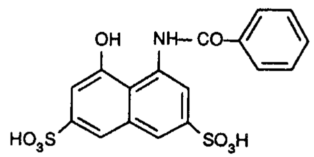 | 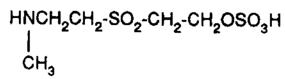 | rot | 514 |
| 71 | 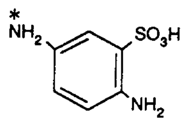 | 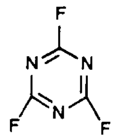 | 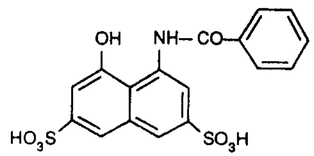 | " | blaust. rot | |
| 72 | 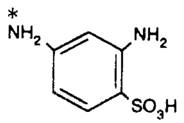 | 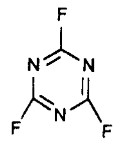 | 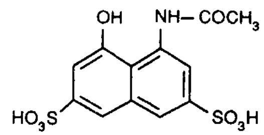 | " | rot | |
| 73 | 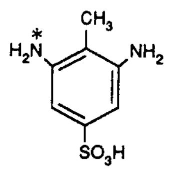 | 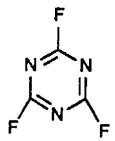 | 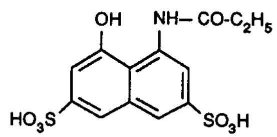 | " | rot | |

| Nr. | Azokomponente | Trihalogen-triazin | Komponente der Formel (V) | Farbton |
|---|---|---|---|---|
| 74 | 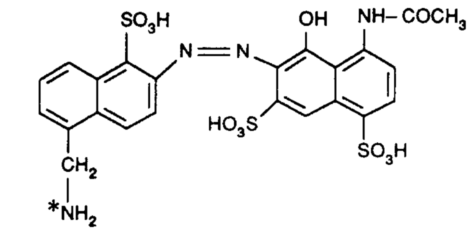 | 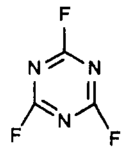 | 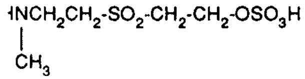 | rot |
| 75 | 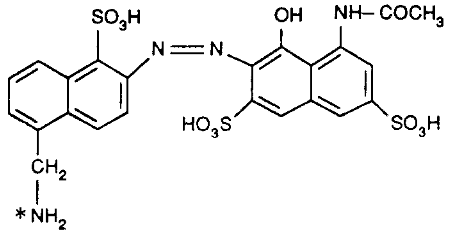 | 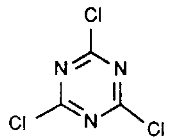 | " | blaust. rot |
| 76 | 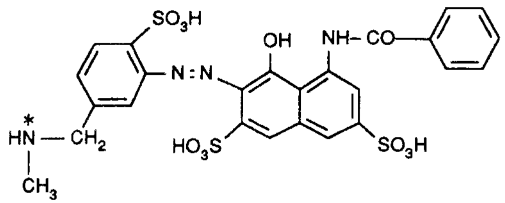 | 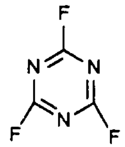 | " | rot |
| 77 | 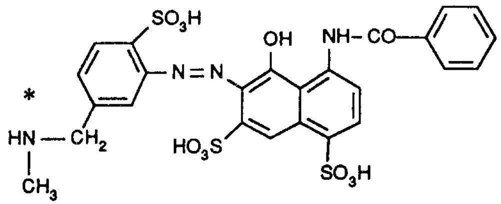 | 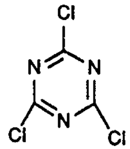 | " | gelbst. rot |

| Nr. | Diaminobenzol-sulfonsäure | Trihalogen-triazin | Kupplungskomponente | Komponente der Formel (V) | Farbton | $\lambda_{max}$(nm) |
|---|---|---|---|---|---|---|
| 80 | 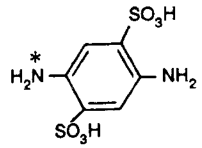 | 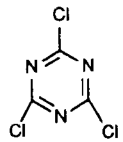 | 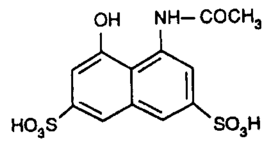 | $HNCH_2CH_2$-$SO_2$-$CH_2$-$CH_2OSO_3H$ (N-$CH_3$) | blaust. rot | |
| 81 | 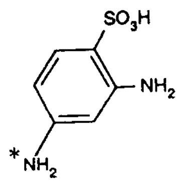 | 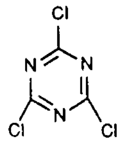 | 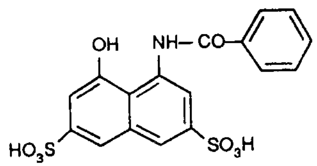 | " | rot | 515 |
| 82 | 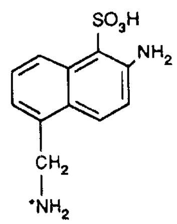 | 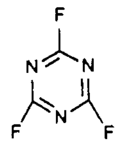 | 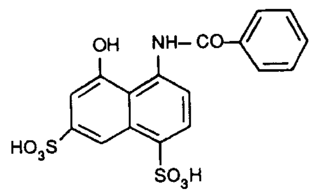 | " | rot | 515, 536 |

Wobei * das Atom kennzeichnet, welches mit dem Triazinring verbunden ist.

Beispiel 83

36,7 g der auf bekanntem Weg hergestellten Amino-disazoverbindung der Formel

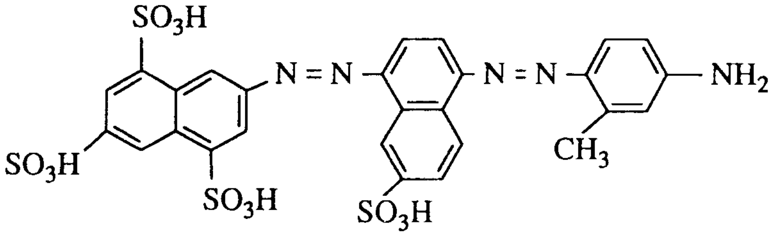

wurden analog Beispiel 19 mit 2,4,6-Trifluor-1,3,5-triazin und der Verbindung der Formel (Va) umgesetzt.

Der erhaltene Farbstoff der Formel

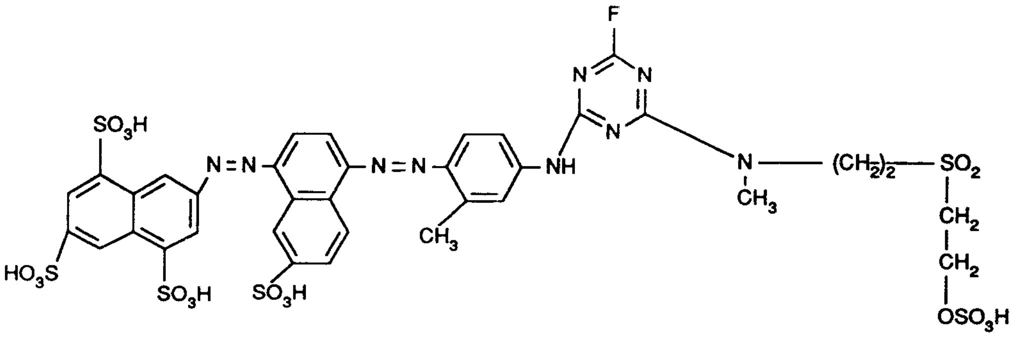

wurde mit Natriumchlorid ausgesalzen, abgesaugt und nach Pufferung bei pH 6 im Vakuum bei 45°C getrocknet. Er färbt Baumwolle nach den für Reaktivfarbstoffe bekannten Verfahren ergiebig in braunen Tönen.

$\lambda_{max}$ = 463 nm

Beispiel 83a

Setzte man anstelle der Aminodisazoverbindung des Beispiels 83 die Verbindung der Formel

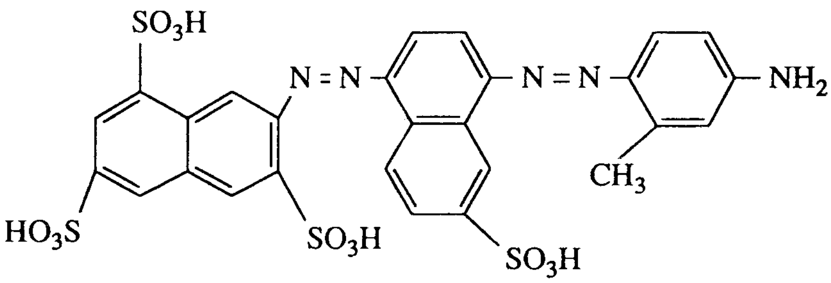

ein, so erhielt man einen Farbstoff der Formel

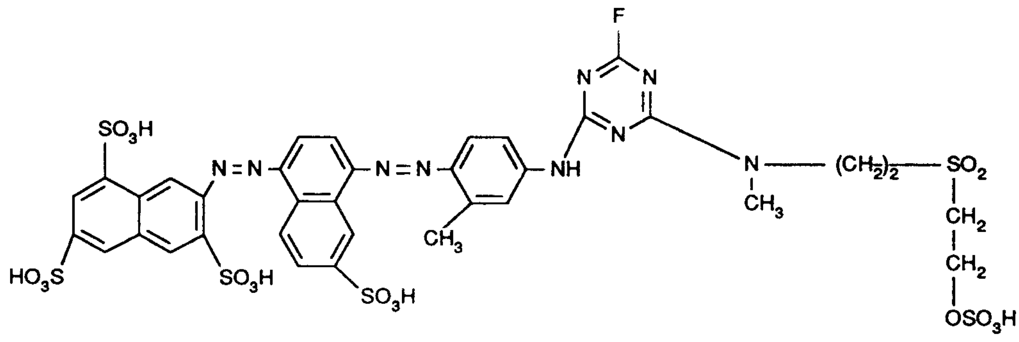

der Baumwolle ebenfalls ergiebig in braunen Tönen färbt.

Weitere braune Reaktivfarbstoffe erhielt man durch Kondensation der folgenden Komponenten:

| Nr. | Aminodisazoverbindung | Trihalogen-triazin | Komponente der Formel (V) | Farbton |
|---|---|---|---|---|
| 84 | 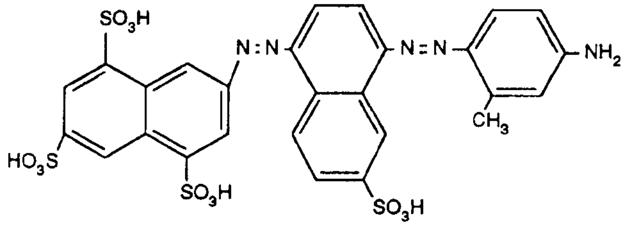 | 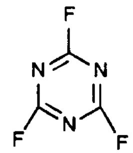 | 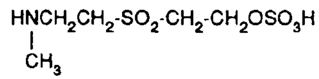 | braun |
| 85 | 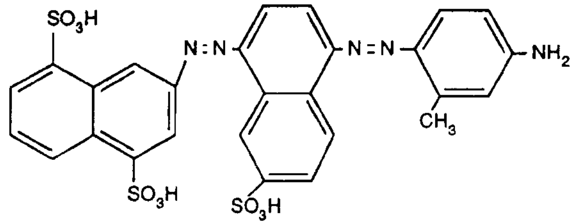 | 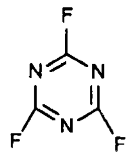 | " | braun |
| 86 | 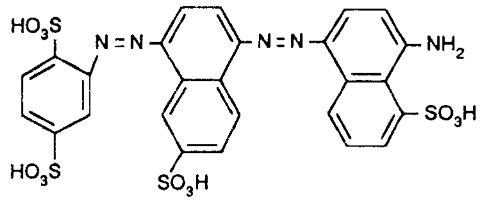 | 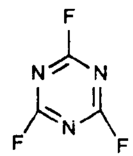 | " | braun |
| 87 | 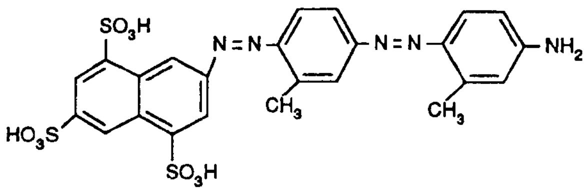 | 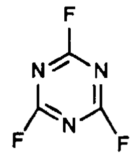 | " | orangebraun |

Beispiel 88

50,3 g Aminoazoverbindung (0,1 Mol) der Formel

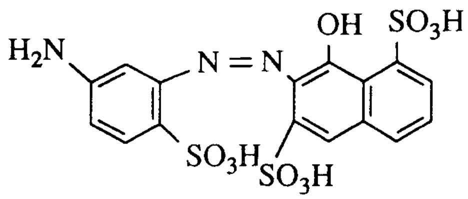

wurden analog Beispiel 19 mit 2,4,6-Trifluor-1,3,5-triazin und der Verbindung der Formel (Va) umgesetzt. Danach wurde der Farbstoff der Formel

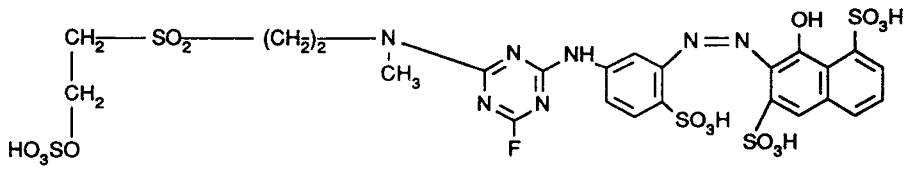

durch Aussalzen und Absaugen isoliert. Nach schonender Trocknung erhielt man ein Pulver, das Baumwolle nach den üblichen Methoden ergiebig in Scharlachtönen färbt.

Weitere Reaktivfarbstoffe auf der Basis von Aminoazonaphtholverbindungen erhielt man durch Kondensation nachstehender Komponenten.

| Nr. | Aminoazonaphtholkomponente | Trihalogen-triazin | Komponente der Formel (V) | Farbton |
|---|---|---|---|---|
| 89 | 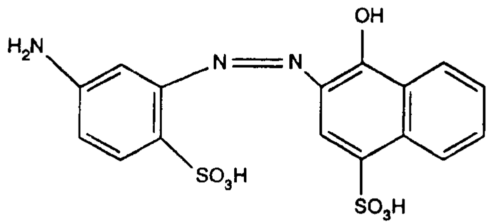 | 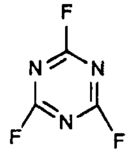 | 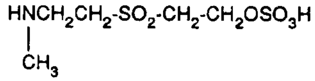 | orange |
| 90 | 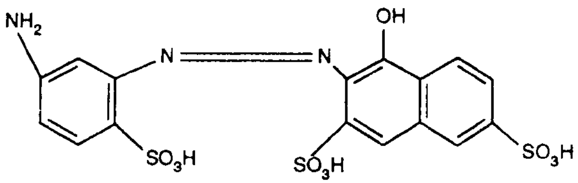 | 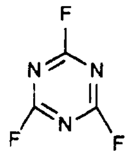 | " | orange |
| 91 | 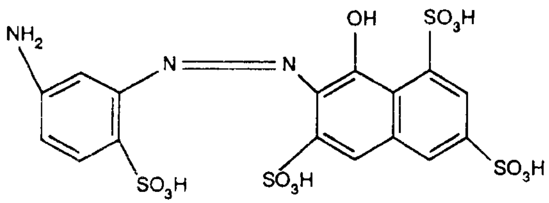 | 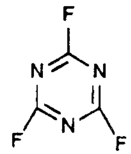 | " | gelbstichig rot |
| 92 | 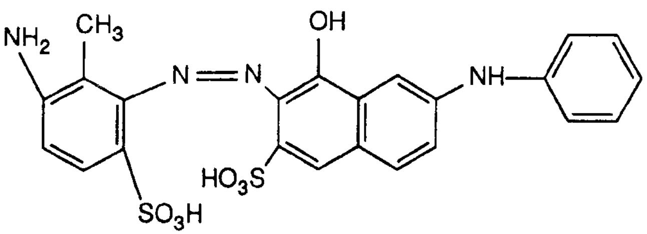 | 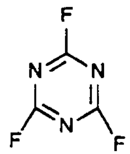 | " | |

Beispiel 93

0,1 Mol der bekannten Verbindung

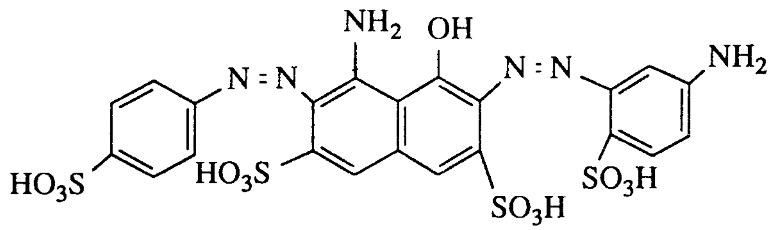

wurden analog Beispiel 19 mit 2,4,6-Trifluor-1,3,5-triazin und der Verbindung der Formel (Va) umgesetzt.

Nach abgeschlossener Kondensation wurde der Farbstoff nach Pufferung auf pH 6 entweder direkt durch Sprühtrocknung oder durch Aussalzen, Absaugen und Vakuumtrocknung bei 40°C isoliert. Der Farbstoff hat die Formel

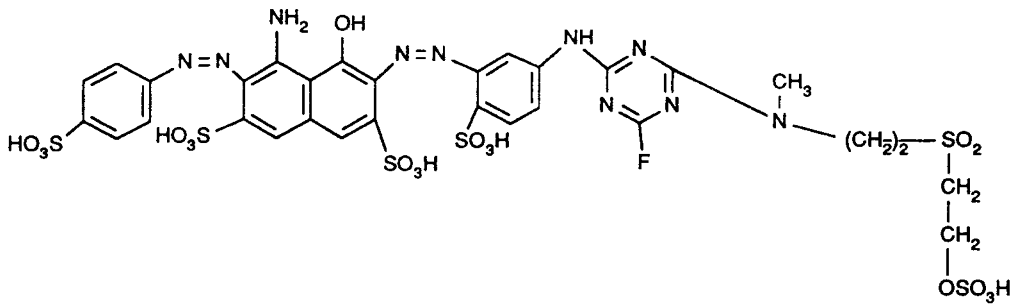

und färbt Baumwolle nach den für Reaktivfarbstoffe üblichen Färbetechniken sehr ergiebig in marineblauen Tönen.

Weitere ähnliche Cellulosefasern marineblau bis schwarz färbende Reaktivfarbstoffe wurden erhalten, wenn man die in nachfolgender Zusammenstellung angegebenen Aminodisazokomponenten der allgemeinen Formel

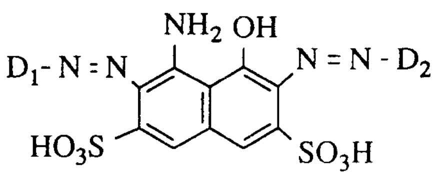

mit den Trihalogentriazinen und Verbindungen der Formel (V) kondensierte.

| Nr. | Aminodisazokomponente D1 | D2 | Trihalogen-triazin | Komponente der Formel (V) | Farbton |
|---|---|---|---|---|---|
| 94 | 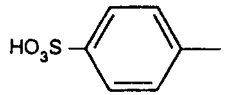 | 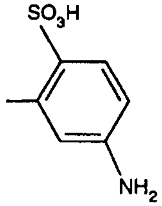 | 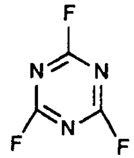 | 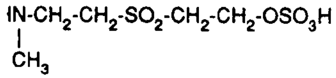 | marine |
| 95 | 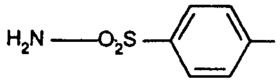 | 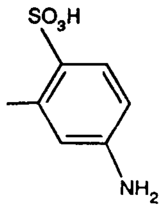 | 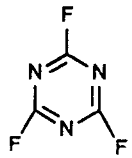 | " | marine |
| 96 | 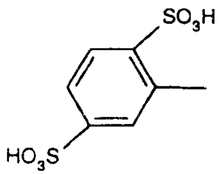 | 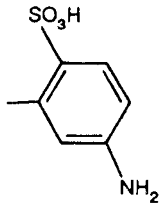 | 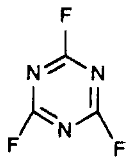 | " | marine |
| 97 | 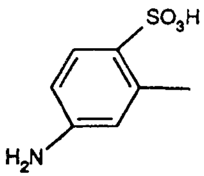 | 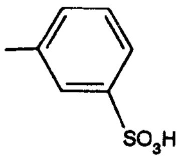 | 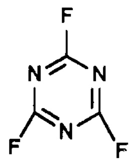 | " | marine |

| Nr. | Aminodisazokomponente D1 | D2 | Trihalogen-triazin | Komponente der Formel (V) | Farbton |
|---|---|---|---|---|---|
| 98 | 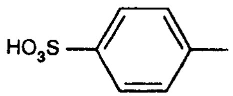 | 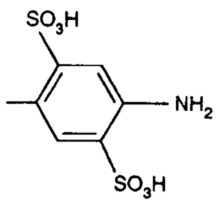 | 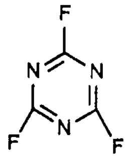 | 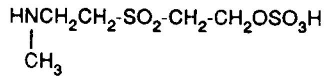 | schwarz |
| 99 | 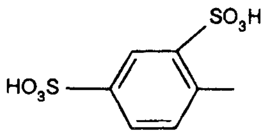 | 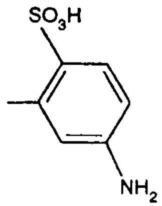 | 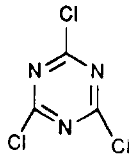 | " | marine |
| 100 | 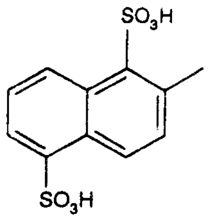 | 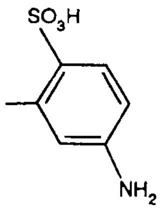 | 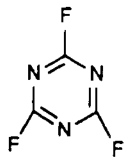 | " | schwarz |

Beispiel 101

0,1 Mol der Kupferkomplexverbindung der Formel

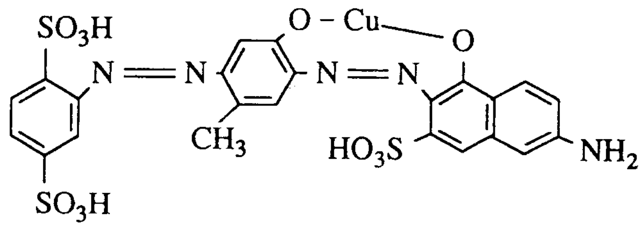

wurden analog Beispiel 19 mit 2,4,6-Trifluor-1,3,5-triazin und der Verbindung der Formel (Va) umgesetzt.

Nach beendeter Umsetzung wurde der Farbstoff der Formel

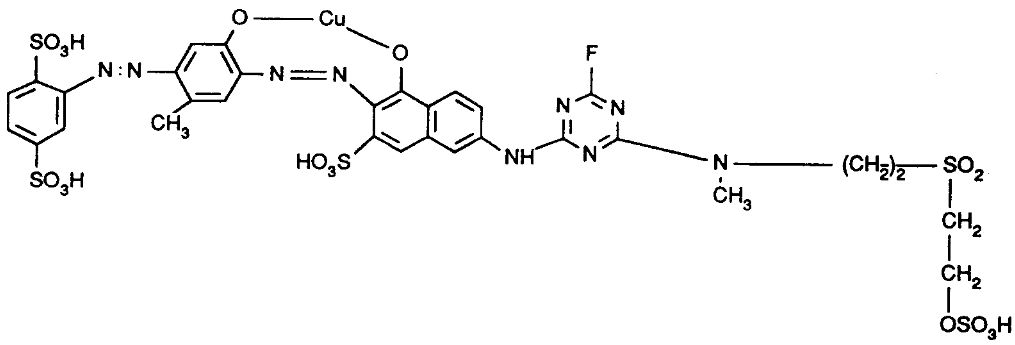

ausgesalzen, isoliert und nach Pufferung auf pH 6 im Vakuum bei 45°C getrocknet.

Das Produkt färbt Cellulosefasern nach den für Reaktivfarbstoffe üblichen Farbtechniken mit sehr guten Fixierausbeute in marineblauen Tönen.

Weitere Reaktivfarbstoffe, die Baumwolle nach den üblichen Färbetechniken sehr ergiebig färben, erhielt man, wenn man die in nachfolgender Zusammenstellung angegebenen, bekannten Kupferkomplexverbindungen nach den in Beispiel 101 dargelegten Verfahrensweisen mit den Trihalogentriazinen und den Komponenten der Formel (V) kondensierte.

| Nr. | Kupferkomplexverbindung | Trihalogen-triazin | Komponente der Formel (V) | Farbton |
|---|---|---|---|---|
| 102 | 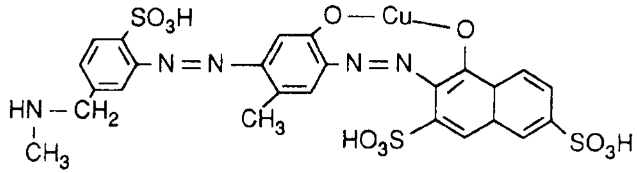 | 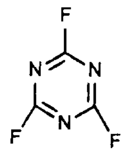 | 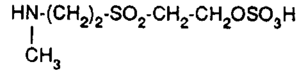 | marineblau |
| 103 | 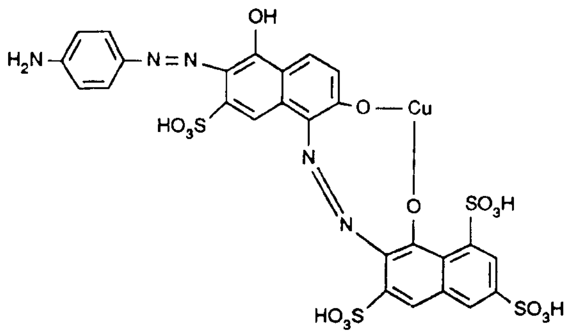 | 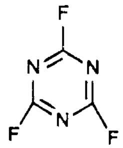 | " | marineblau |
| 104 | 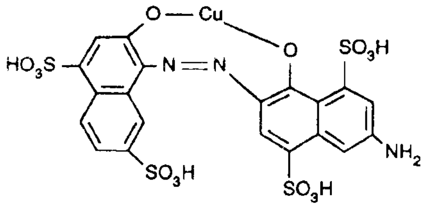 | 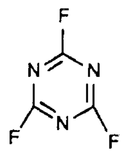 | " | rotstichig marineblau |
| 105 | 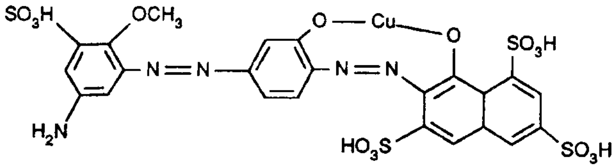 | 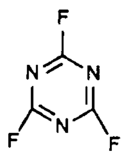 | " | marineblau |

| Nr. | Kupferkomplexverbindung | Trihalogen-triazin | Komponente der Formel (V) | Farbton |
|---|---|---|---|---|
| 106 | 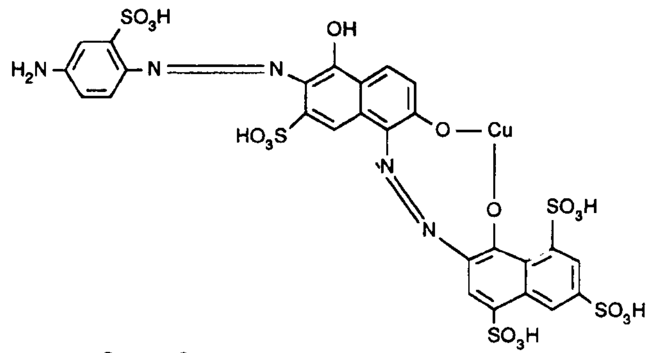 | 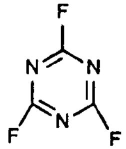 | 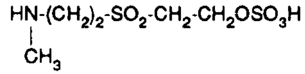 | marineblau |
| 107 | 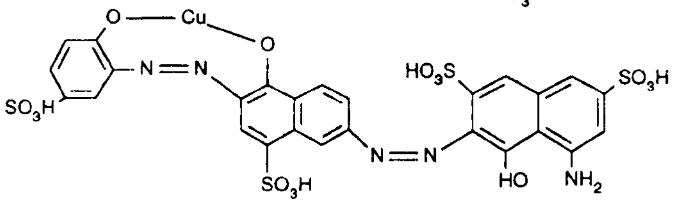 | 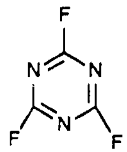 | " | dunkelblau |
| 108 | 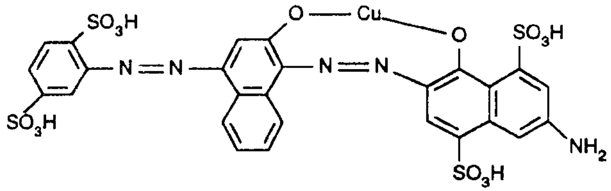 | 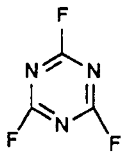 | " | grün |
| 109 | 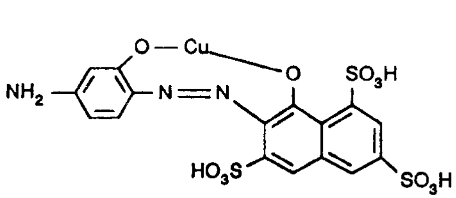 | 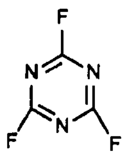 | " | rotviolett |

| Nr. | Kupferkomplexverbindung | Trihalogen-triazin | Komponente der Formel (V) | Farbton | $\lambda_{max}$(nm) |
|---|---|---|---|---|---|
| 110 | 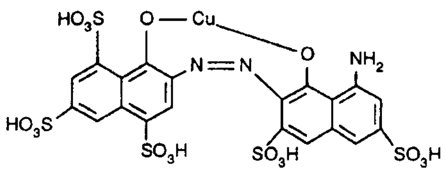 | 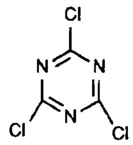 | $HN-(CH_2)_2-SO_2-CH_2-CH_2OSO_3H$<br>$\mid$<br>$CH_3$ | marineblau | 574 |
| 111 | 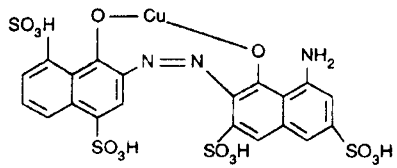 | 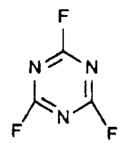 | " | marineblau | 568 |
| 112 | 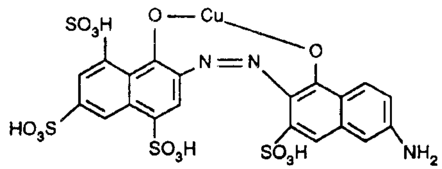 | 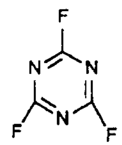 | " | blauviolett | |

Weitere interessante Farbstoffe z.B. aus der ortho-Disazometallkomplexreihe sind:

Beispiel 113

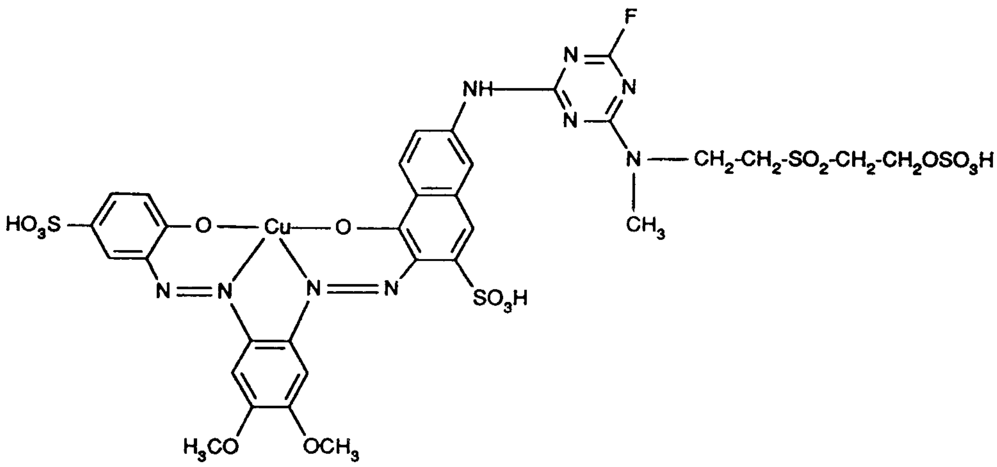

der Baumwolle in oliven Farbtönen färbt, oder aus der ortho-Aminoazometallkomplexreihe z.B. der Farbstoff der Formel

$\lambda_{max}$ = 468 nm, 590 nm

Beispiel 114

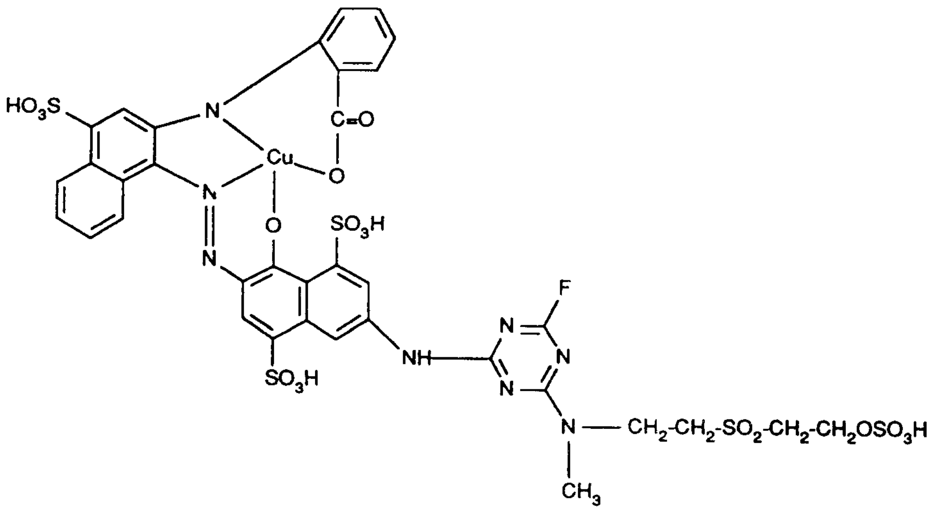

der Baumwolle in grünen Tönen färbt.

Beispiel 115

0,1 Mol 1-Amino-8-hydroxy-3,6-naphthalindisulfonsäure wurden wie Beispiel 2 A) mit 2,4,6-Trifluor-1,3,5-triazin und analog Beispiel 2 B) mit der Verbindung der Formel (Va) umgesetzt.

0,1 Mol 1-Sulfo-2-naphthylamin-6-β-sulfatoethylsulfon wurden in der üblichen Weise diazotiert und bei pH 7,5-8,5 auf das H-Säure-Kondensationsprodukt gekuppelt. Der Farbstoff wurde durch Zugabe von Kaliumchlorid ausgesalzen und isoliert. Nach Trocknung erhielt man ca. 35 g des salzhaltigen Farbstoffes der Formel

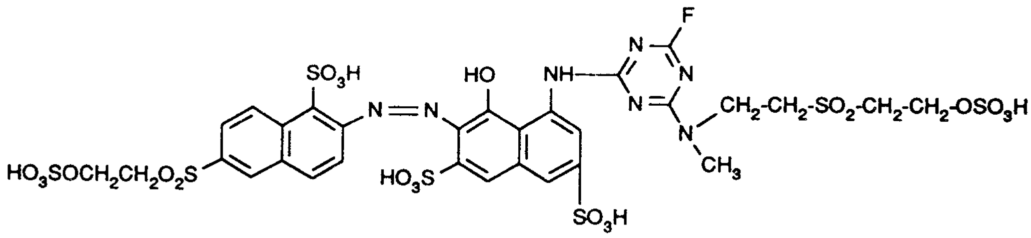

der Baumwolle nach den für Reaktivfarbstoffe üblichen Färbe- oder Druckverfahren in roten Farbtönen färbt.

$\lambda_{max}$ = 519 nm, 541 nm

Beispiel 116

0,1 Mol 4-(β-Sulfatoethylsulfonyl)anilin wurden in 200 Teilen Wasser und 100 Teilen Eis bei 0°C suspendiert Man gab 28 Teile konzentrierte Salzsäure zu. Innerhalb von 15 Minuten tropfte man eine Lösung von 7 Teilen Natriumnitrit in 70 Teilen Wasser zu. Nach 30-minütigem Nachrühren war die Diazotierung beendet. Es resultierte eine hellgelbe Suspension. Der Nitritüberschuß wurde mit Amidosulfonsäure zerstört.

Diese Suspension wurde nun über einen Zeitraum von 15-20 Minuten zu 0,1 Mol des H-Säure-Kondensationsproduktes aus Beispiel 115 zudosiert. Gekuppelt wurde bei 20°C und einem pH-Wert von 7-8. Der Farbstoff wurde durch Zugabe von Ethanol ausgefallt und isoliert. Nach Trocknung erhielt man ca. 40 g eines Farbstoffpulvers, dem die Struktur

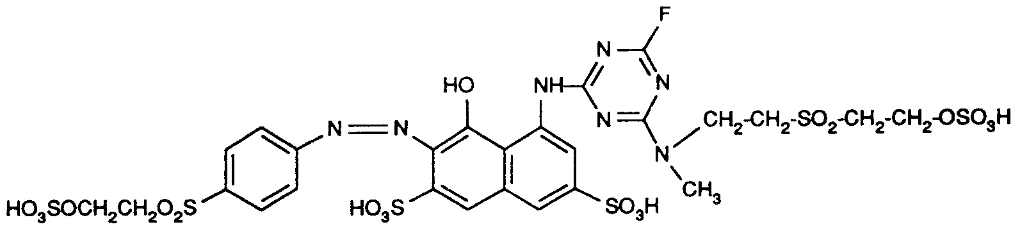

zukommt und das Baumwolle nach den für Reaktivfarbstoffe üblichen Farbe- und Druckverfahren in roten Farbtönen färbt.

$\lambda_{max}$ = 515 nm

Weitere rote Reaktivfarbstoffe erhielt man durch Kondensation der folgenden Komponenten:

| Nr. | Diazokomponente | 1-Amino-8-hydroxy-naphthalin-disulfos. | Trihalogen-triazin | Komponente der Formel (V) | Farbton | $\lambda_{max}$ (nm) |
|---|---|---|---|---|---|---|
| 117 | 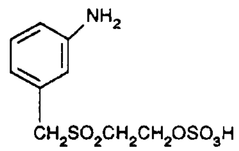 | 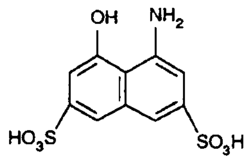 | 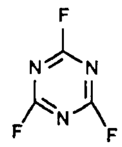 | $HN-CH_2-CH_2-SO_2-CH_2-CH_2-OSO_3H$ <br> $\vert$ <br> $CH_3$ | rot | |
| 118 | 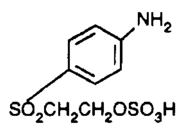 | 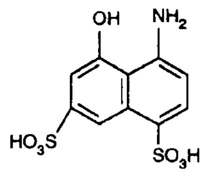 | 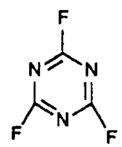 | . | rot | |
| 119 | 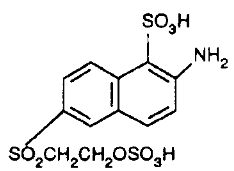 | 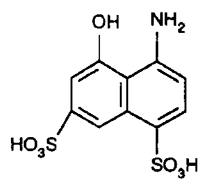 | 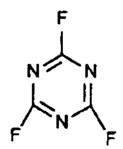 | . | rot | 519, 534 |
| 120 | 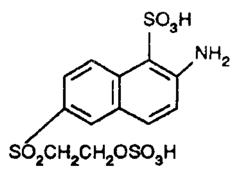 | 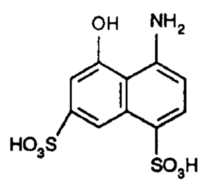 | 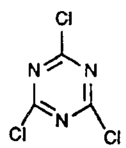 | . | rot | 512 <br> 534 |

| Nr. | Diazokomponente | 1-Amino-8-hydroxy-naphthalin-disulfos. | Trihalogen-triazin | Komponente der Formel (V) | Farbton | $\lambda_{max}$ (nm) |
|---|---|---|---|---|---|---|
| 121 | 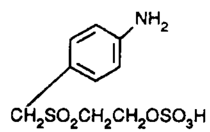 | 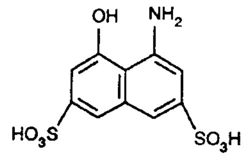 | 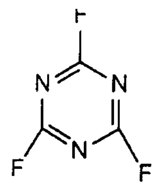 | $HN-CH_2-CH_2-SO_2-CH_2-CH_2-OSO_3H$<br>$\vert$<br>$CH_3$ | rot | |
| 122 | 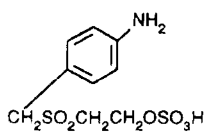 | 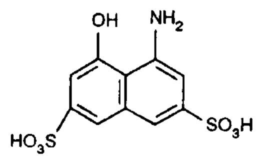 | 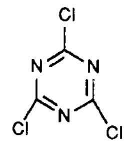 | . | rot | |
| 123 | 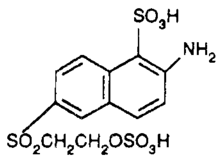 | 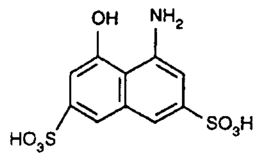 | 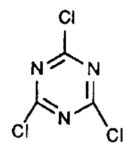 | . | rot | 518, 540 |
| 124 | 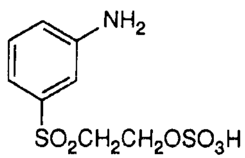 | 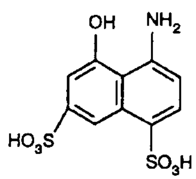 | 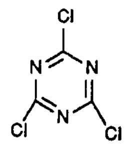 | . | rot | |

| Nr. | Diazokomponente | 1-Amino-8-hydroxy-naphthalin-disulfos. | Trihalogen-triazin | Komponente der Formel (V) | Farbton | $\lambda_{max}$(nm) |
|---|---|---|---|---|---|---|
| 125 | 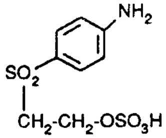 | 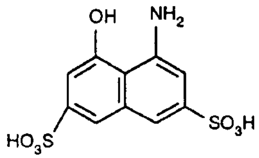 | 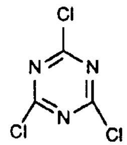 | 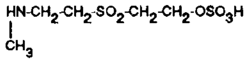 | rot | 517 |
| 126 | 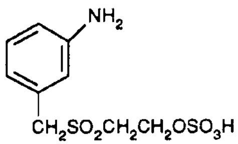 | 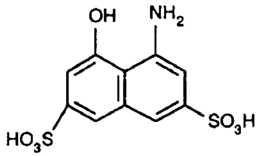 | 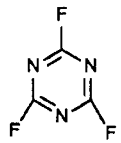 | " | rot | |
| 127 | 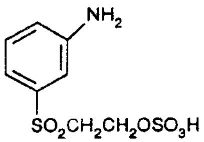 | 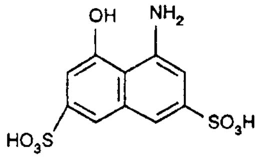 | 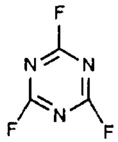 | " | rot | 513 |
| 128 | 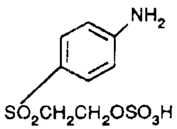 | 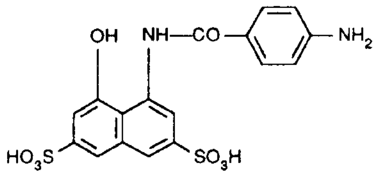 | 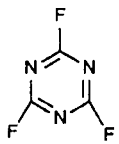 | " | rot | |
| 129 | 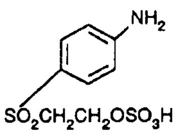 | 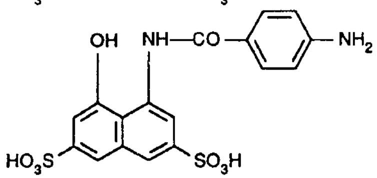 | 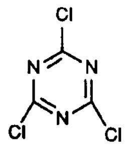 | " | rot | |

Beispiel 130

0,1 Mol der Monoazoverbindung mit der Formel

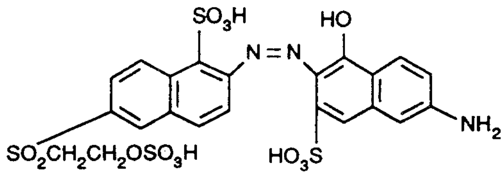

wurden analog Beispiel 19 mit 2,4,6-Trifluor-1,3,5-triazin und der Verbindung der Formel Va umgesetzt.

Der Farbstoff wurde durch Zugabe von Kaliumchlorid ausgesalzen, isoliert und getrocknet. Man erhielt ca. 82 g eines salzhaltigen Farbstoffes der Formel, dem die Struktur

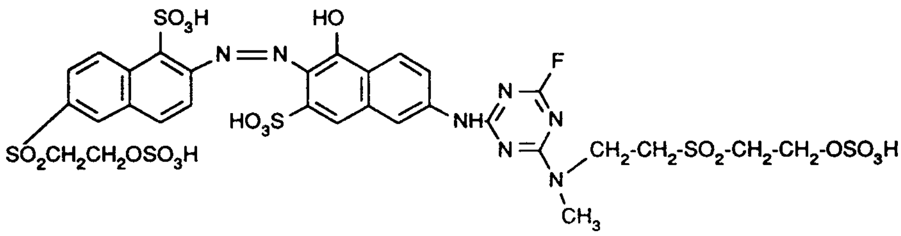

der Baumwolle nach den für Reaktivfarbstoffe üblichen Färbe- oder Druckverfahren in orangen Farbtönen färbt.

$\lambda_{max}$ = 492 nm

Beispiel 131

0,1 Mol (4-Aminophenyl-β-sulfatoethyl)sulfon wurden, wie in Beispiel 116 beschrieben, diazotiert und mit dem in Beispiel 2 A) und B) beschriebenen I-Säure-Kondensationsprodukt gekuppelt.

Der Farbstoff der Formel

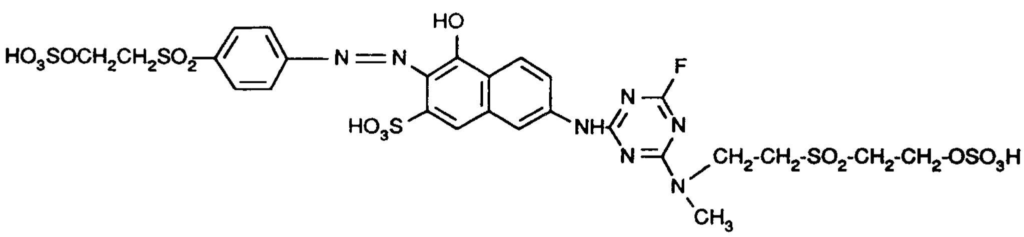

wurde durch Zugabe von NaCl ausgesalzen und isoliert. Nach Trocknung erhielt man ca. 105 g eines Farbstoffpulvers, der Baumwolle nach den für Reaktivfarbstoffe üblichen Färbe- oder Druckverfahren in Scharlachtönen färbt.

$\lambda_{max}$ = 479 nm

Weitere Reaktivfarbstoffe wurden durch Kondensation der folgenden Komponenten analog Beispiel 130 oder 131 erhalten.

| Nr. | Aminoazofarbstoff | Komponente der Formel (V) | Halogen-triazin | Farbton | $\lambda_{max}$(nm) |
|---|---|---|---|---|---|
| 132 | 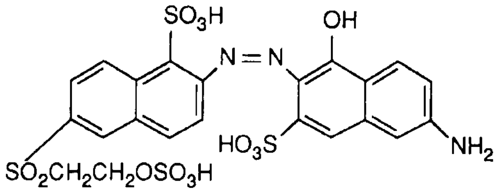 | 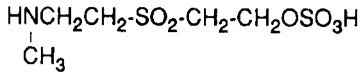 | 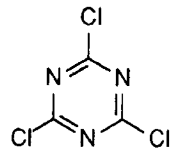 | orange | 491 |
| 133 | 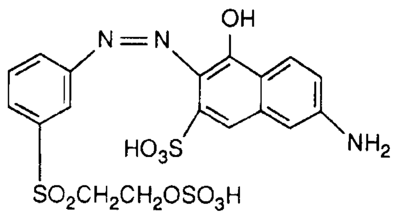 | " | 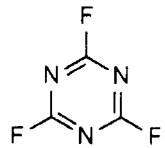 | orange | 478 |
| 134 | 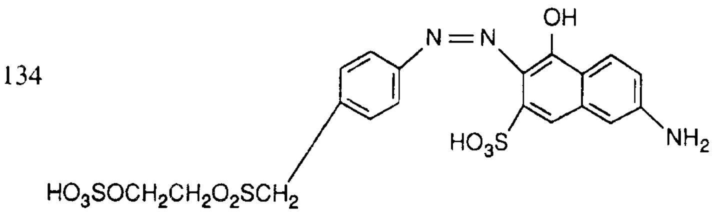 | " | " | orange | |
| 135 | 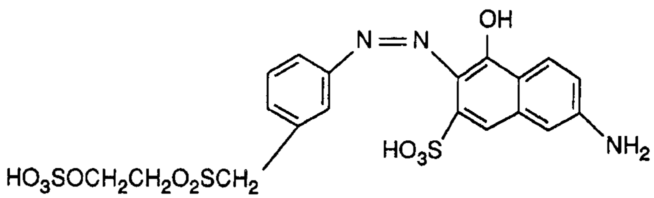 | " | " | orange | |

| Nr. | Aminoazofarbstoff | Komponente der Formel (V) | Halogen-triazin | Farbton | $\lambda_{max}$(nm) |
|---|---|---|---|---|---|
| 136 | 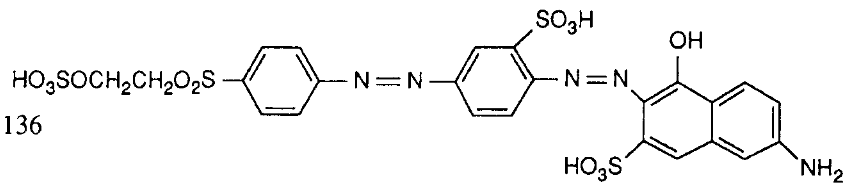 | 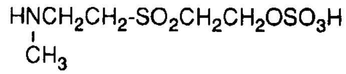 | 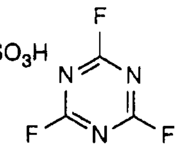 | gelbstichig rot | |
| 137 | 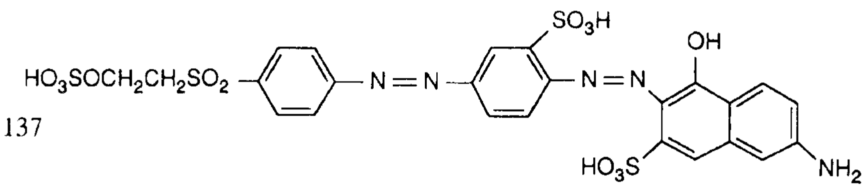 | " | 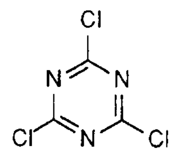 | gelbstichig rot | |
| 138 | 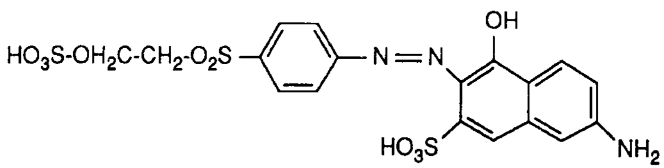 | " | " | orange | 478 |

Beispiel 139

0,1 Mol der bekannten Verbindung

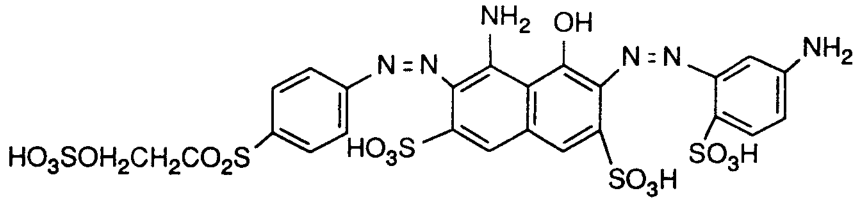

wurden analog Beispiel 19 mit 2,4,6-Trifluor-1,3,5-triazin und der Verbindung der Formel (Va) umgesetzt.

Nach abgeschlossener Kondensation wurde der Farbstoff nach Pufferung auf pH 6 entweder direkt durch Sprühtrocknung oder durch Aussalzen, Absaugen und Vakuumtrocknung bei 40°C isoliert. Der Farbstoff hat die Formel

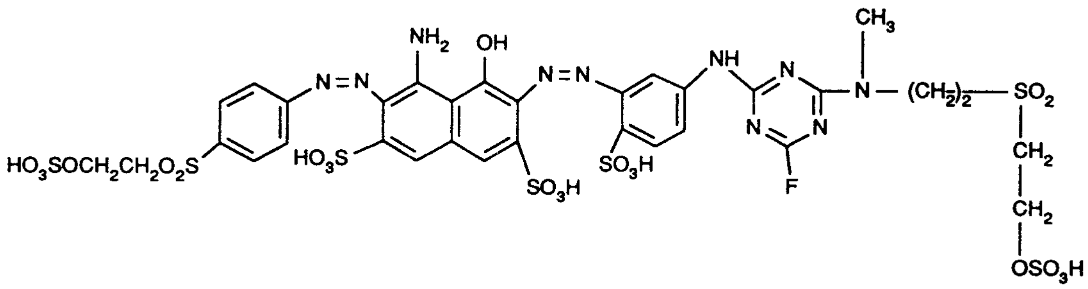

und färbt Baumwolle nach den für Reaktivfarbstoffe üblichen Färbetechniken sehr ergiebig in marineblauen Tönen.

$\lambda_{max}$ = 612 nm

Weitere ähnliche Cellulosefasern marineblau bis schwarz färbende Reaktivfarbstoffe wurden erhalten, wenn man die in nachfolgender Zusammenstellung angegebenen Aminodisazokomponenten der allgemeinen Formel

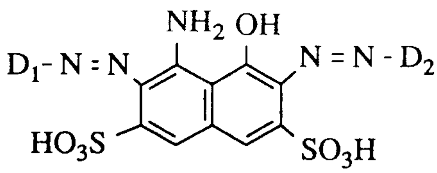

mit den Trihalogentriazinen und Verbindungen der Formel (V) kondensierte.

| Nr. | Aminodisazokomponente D1 | D2 | Trihalogen-triazin | Komponente der Formel (V) | Farbton | $\lambda_{max}$(nm) |
|---|---|---|---|---|---|---|
| 140 | 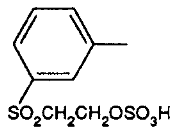 | 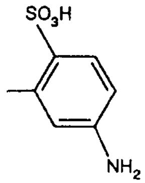 | 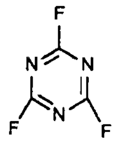 | 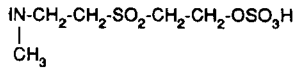 | marine | |
| 141 | 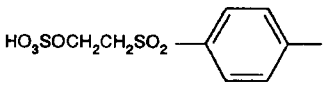 | 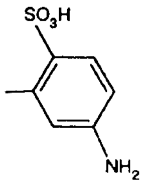 | 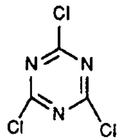 | " | marine | 613 |
| 142 | 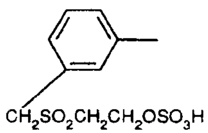 | 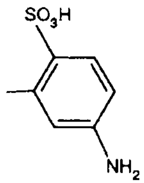 | 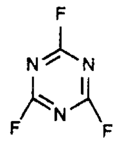 | " | marine | |
| 143 | 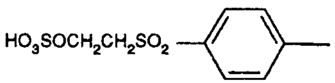 | 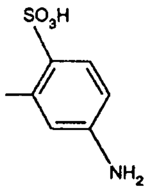 | 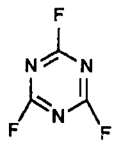 | " | marine | |

| Nr. | Aminodisazokomponente D1 | D2 | Trihalogen-triazin | Komponente der Formel (V) | Farbton |
|---|---|---|---|---|---|
| 144 | 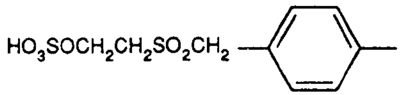 | 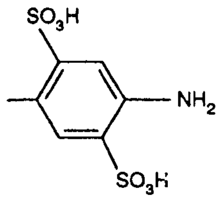 | 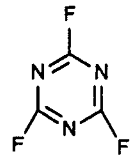 | 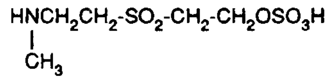 | schwarz |
| 145 | 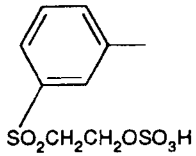 | 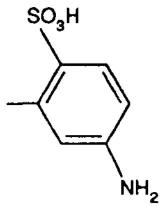 | 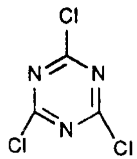 | " | marine |
| 146 | 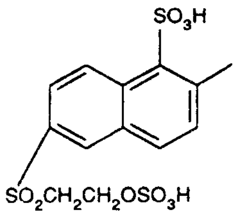 | 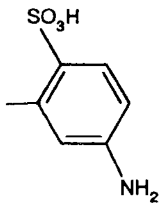 | 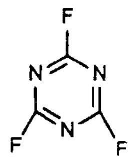 | " | schwarz |

Beispiel 147

A) 0,1 Mol 1-Amino-8-hydroxynaphthalin-3,6-disulfonsäure wurden in 250 ml Wasser bei pH 6,5 gelöst und mit 250 g Eis auf 0°C abgekühlt. 0,1 Mol 2,4,6-Trifluor-1,3,5-triazin (Cyanurfluorid) wurden zugegeben und der pH mit Soda-Lösung zwischen 3,5 und 4 gehalten. Nach 5 Minuten setzte man 0,1 Mol Morpholin zu und stellte den pH mit Soda-Lösung auf 7 ein. Die Temperatur stieg dabei auf ca. 10°C an.

B) 0,1 Mol 2,4-Diaminobenzolsulfonsäure wurden in 100 ml Wasser und 100 g Eis verrührt und neutral gelöst. 0,1 Mol 2,4,6-Trichlor-1,3,5-triazin wurden zugesetzt und bei pH 4-5 ankondensiert; der pH-Wert wurde mit Soda-Lösung gehalten. Nach Beendigung der ersten Kondensation wurden 0,1 Mol der Verbindung der Formel (Va) zugegeben. Die zweite Kondensation wurde bei 30°C und pH 8 durchgeführt. Man erhielt ein Produkt der Formel

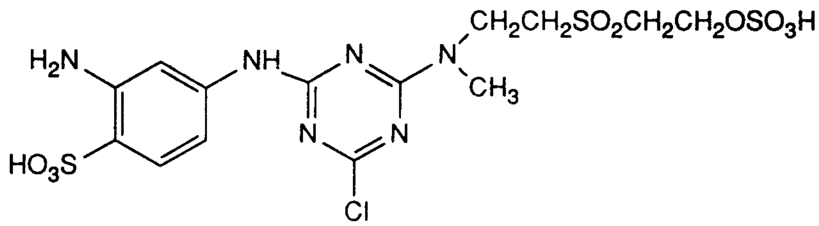

Diese Diazokomponente wurde bei 0°C und pH 3-3,5 diazotiert und zur Lösung der oben beschriebenen Kupplungskomponente gegeben, wobei mit Soda-Lösung ein pH von 6-7 eingestellt und gehalten wurde.

Nach beendeter Kupplung wurde das Produkt mit NaCl ausgesalzen, isoliert und getrocknet. Man erhielt 81 g salzhaltigen Farbstoff der Formel

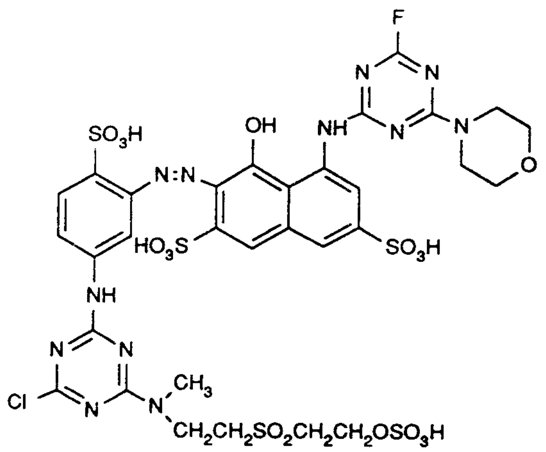

als Pulver, der Baumwolle in Klaren roten Tönen färbt.

$\lambda_{max}$ = 514 nm, 533 nm

Die Farbstoffe der folgenden Beispiele wurden analog hergestellt:

| B | Diazokomponente | Kupplungskomponente | Y | Beispiel |
|---|---|---|---|---|
| 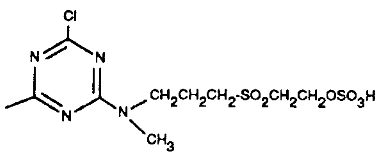 | 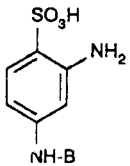 | 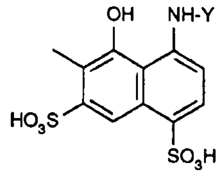 | 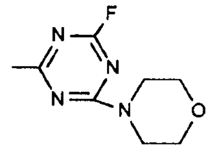 | 148 |
| 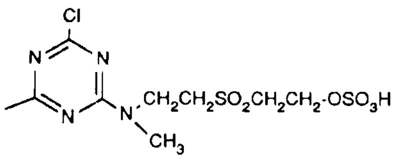 | · | 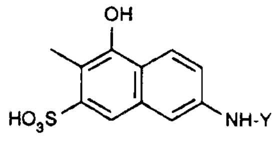 | 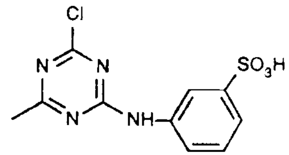 | 149 |
| 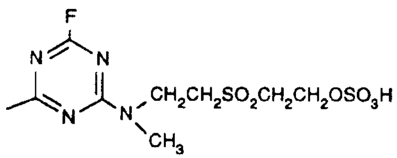 | 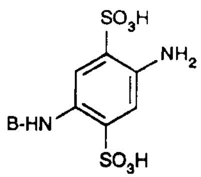 | 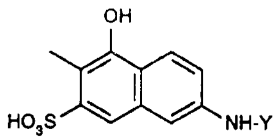 | 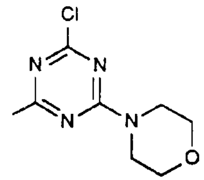 | 150 |

| B | Diazokomponente | Kupplungskomponente | Y | Beispiel |
|---|---|---|---|---|
| 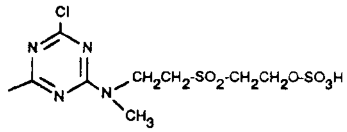 | 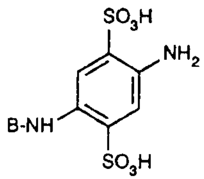 | 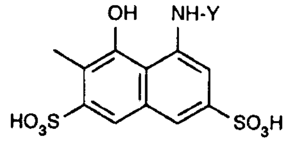 | 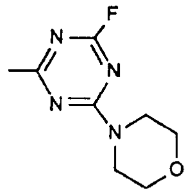 | 151 |
| 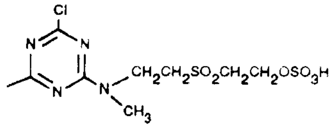 | 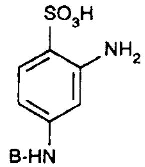 | 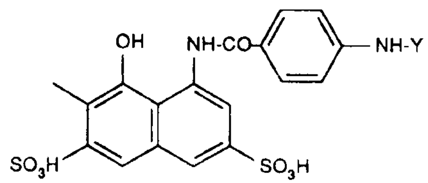 | 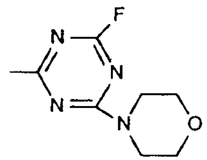 | 152 |
| | 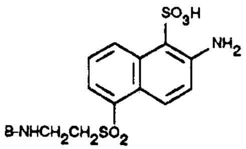 | " | 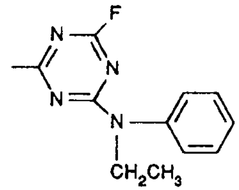 | 153 |

| B | Diazokomponente | Kupplungskomponente | Y | Beispiel |
|---|---|---|---|---|
| 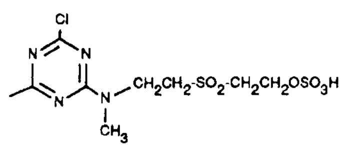 | 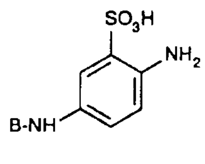 | 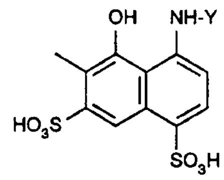 | 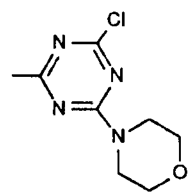 | 154 |
| 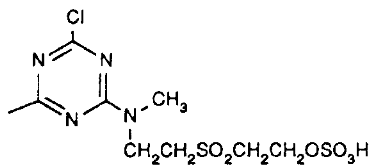 | 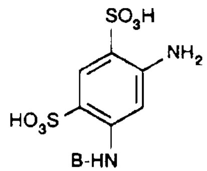 | " | 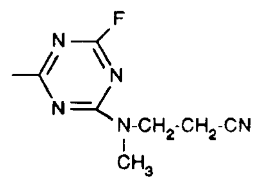 | 155 |
| " | " | 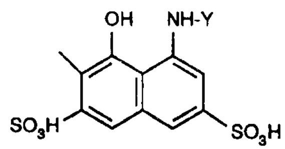 | 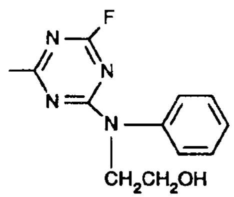 | 156 |

| B | Diazokomponente | Kupplungskomponente | Y | Beispiel |
|---|---|---|---|---|
| 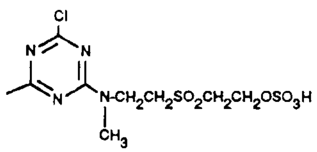 | 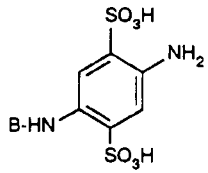 | 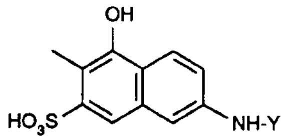 | 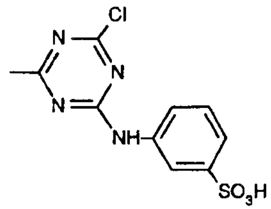 | 157 |
| | 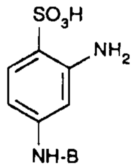 | 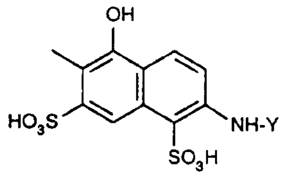 | 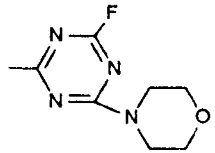 | 158 |
| 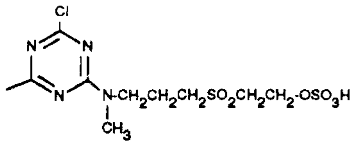 | 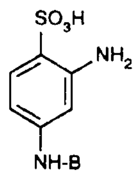 | 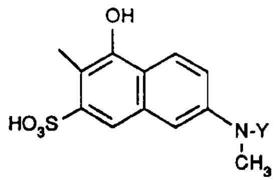 | 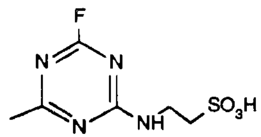 | 159 |

Beispiel 160

0,2 Mol 1-Amino-8-hydroxynaphthalin-3,6-disulfonsäure wurden in 450 ml Wasser mit Natronlauge bei pH 8-9 gelöst und mit 0,22 Mol 2,4,6-Trifluorpyrimidin versetzt. Die Kondensation erfolgte bei 35-40°C, wobei der pH-Wert mit Soda-Lösung gehalten wurde.

0,2 Mol der in Beispiel 147 B) beschriebenen Diazokomponente wurde diazotiert und mit der oben beschriebenen Kupplungskomponente gekuppelt. Nach Aussalzen, Isolieren und Trocknen erhielt man den Farbstoff der Formel

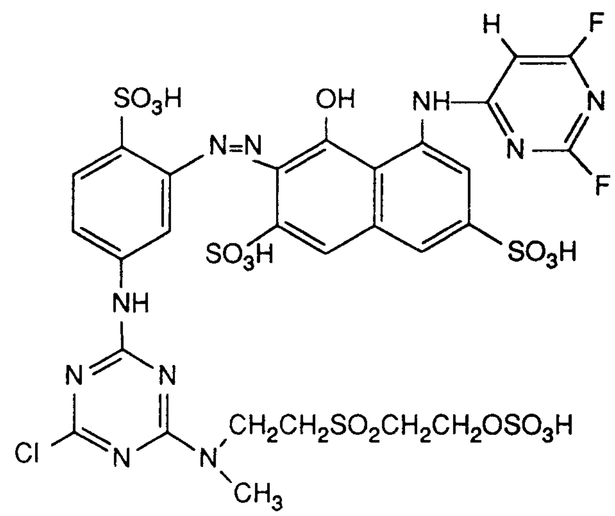

der Baumwolle in roten Tönen färbt.

Beispiel 161

0,2 Mol 8-(4'-Amino-benzoylamino)-1-naphthol-3,6-disulfonsäure wurden in 800 ml Wasser mit Soda-Lösung (20 g/100 ml) bei einem pH-Wert von 7 gelöst. Mit 10 %iger HCl-Lösung wurde pH 4,5 eingestellt. Man gab 0,2 Mol 2,4,6-Trifluorpyrimidin zu und heizte auf 30°C auf. Mit Soda-Lösung wurde ein pH-Wert von 4,5-6 gehalten. Die Umsetzung ist war 4 Stunden beendet.

Man gab nun 0,2 Mol des Diazoniumsalzes aus Beispiel 147 zu und hält gleichzeitig durch Zutropfen von Soda-Lösung einen pH-Wert von 7,5-8. Nach beendeter Kupplung salzte man mit NaCl aus, isolierte und trocknete. Der Farbstoff der Formel

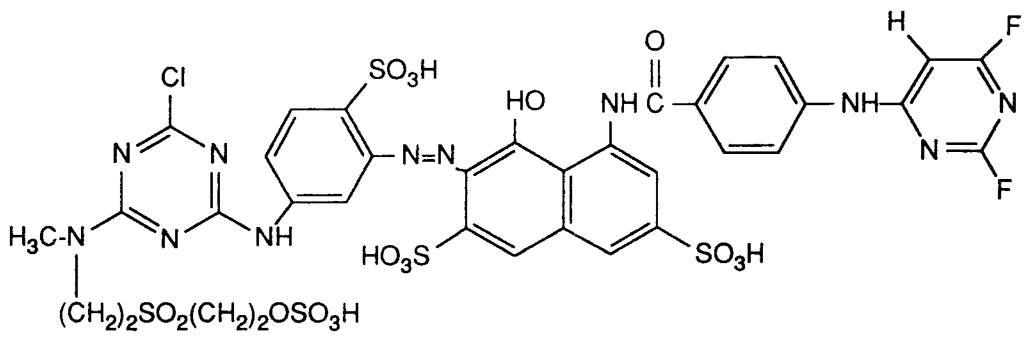

färbt Baumwolle in roten Tönen.

$\lambda_{max}$ = 520 nm, 537 nm

Die Farbstoffe der Beispiele 163-174 wurden analog der Beispiele 160 und 161 hergestellt:

| Beispiel | Y | Kupplungskomponente | Diazokomponente | B |
|---|---|---|---|---|
| 163 | 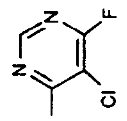 | 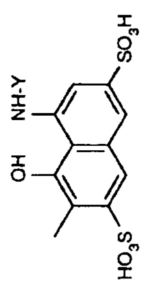 | 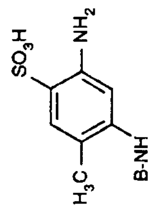 | 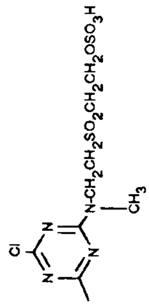 |
| 164 | 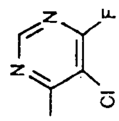 | 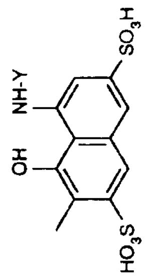 | 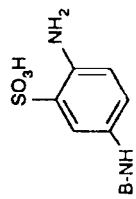 | " |
| 165 | " | " | 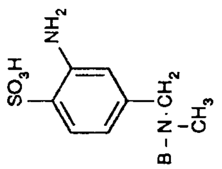 | 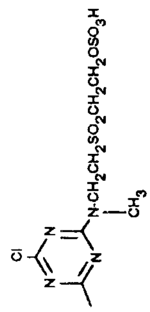 |

| B | Diazokomponente | Kupplungskomponente | Y | Beispiel | $\lambda_{max}$(nm) |
|---|---|---|---|---|---|
| 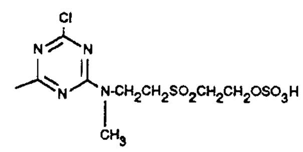 | 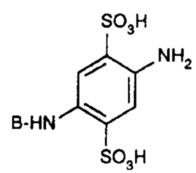 | 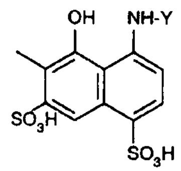 | 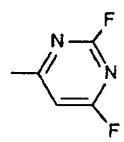 | 166 | |
| 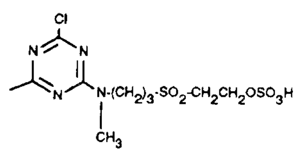 | 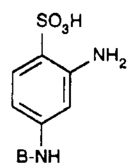 | 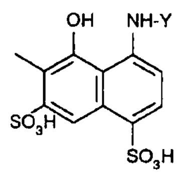 | 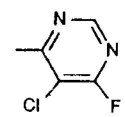 | 167 | 522 |
| 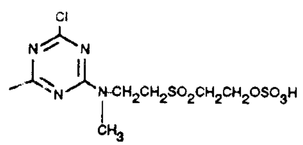 | 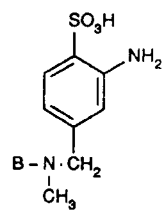 | 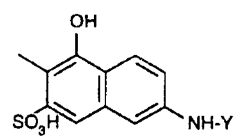 | 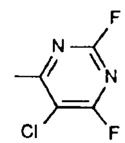 | 168 | |

| B | Diazokomponente | Kupplungskomponente | Y | Beispiel |
|---|---|---|---|---|
| 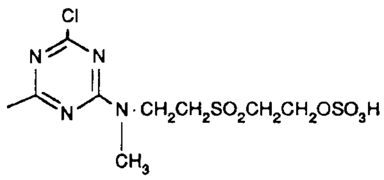 | 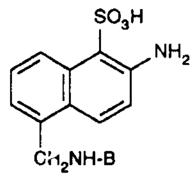 | 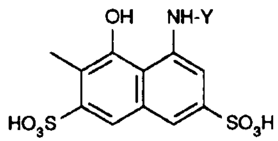 | 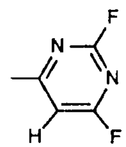 | 169 |
| 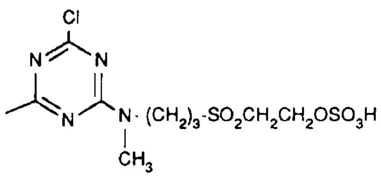 | 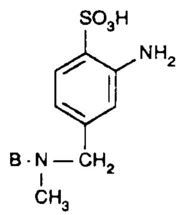 | 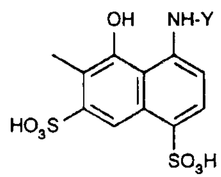 | 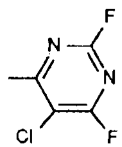 | 170 |
| 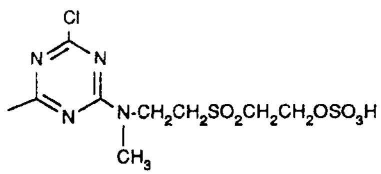 | 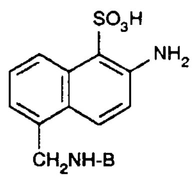 | " | 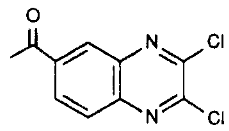 | 171 |

| B | Diazokomponente | Kupplungskomponente | Y | Beispiel |
|---|---|---|---|---|
| 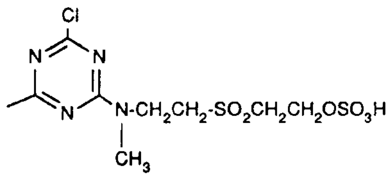 | 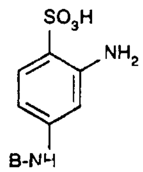 | 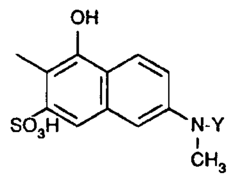 | 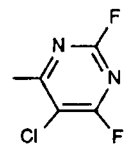 | 172 |
| " | 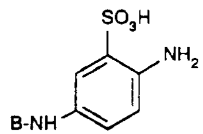 | 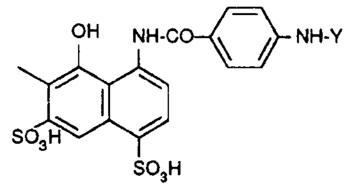 | 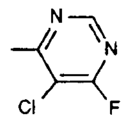 | 173 |
| " | 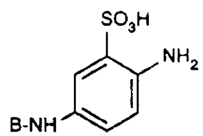 | 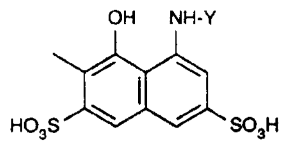 | 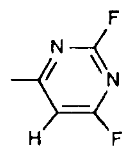 | 174 |

Beispiel 175

0,22 Mol N-Ethylanilin wurden in 200 ml Wasser mit Salzsäure bei pH 5 gelöst. Man gab 200 g Eis zu und streute 0,24 Mol Cyanurchlorid ein. Mit Soda-Lösung erhielt man einen pH-Wert von 5-6. Nach ca. 1 Stunde bei O°C war die Kondensation beendet. 0,2 Mol 2,4-Diamino-benzolsulfonsäure wurden in 250 ml Wasser durch Zugabe von konz. Natronlauge gelöst und zu 1 Stufe der Kondensation zugesetzt. Mit Soda-Lösung wurde ein pH-Wert von 6-7 gehalten. Man heizte auf 25-30°C auf. Nach beendeter Kondensation kühlte man auf 0°C ab. Es wurden 56 ml 30 %ige Salzsäure zugegeben. Man tropfte 47 ml Natriumnitritlösung (30 g/100 ml) zu und rührte 1 Stunde bei 0°C. Natriumnitrit wurde mit Amidosulfonsäure vernichtet und die so erhaltene Diazotierung zur Kupplungskomponente aus Beispiel 1 gegeben. Mit Soda-Lösung (20 g/100 ml) wurde ein pH-Wert von 7-8 gehalten. T = 10-15°C. Nach beendeter Kupplung salzte man mit NaCl aus, isolierte und trocknete. Der erhaltene Farbstofff der Formel

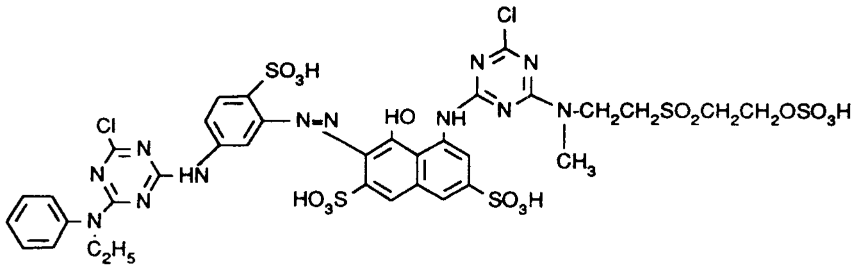

färbt Baumwolle in roten Tönen.

$\lambda_{max}$ = 515 nm, 533 nm

Die Farbstoffe der Beispiele 176-178 wurden analog Beispiel 175 hergestellt:

| Beispiel | Diazokomponente | Kupplungskomponente | Y | B |
|---|---|---|---|---|
| 176 | 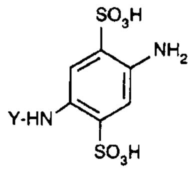 | 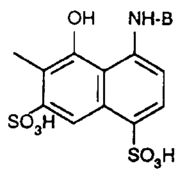 | 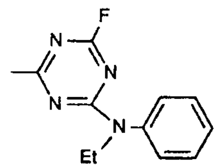 | 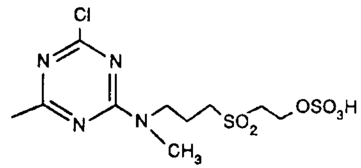 |
| 177 | 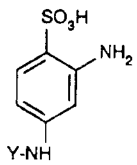 | 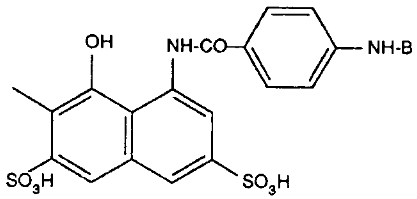 | 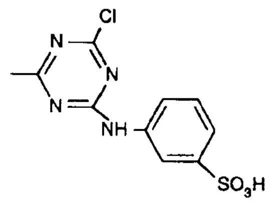 | " |
| 178 | 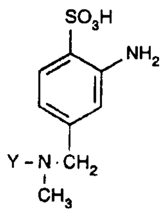 | . | 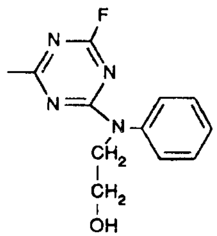 | " |

Beispiel 179

0,2 Mol 6-Fluor-5-chlor-4-(3'-amino-4'-sulfophenyl)-amino-pyrimidin (aus 2,4-Diaminobenzolsulfonsäure und 4,6-Difluor-5-chlorpyrimidin hergestellt) wurden in Wasser suspendiert und mit 65 ml 30 %iger Salzsäure und 300 g Eis versetzt. Danach wurden 46 ml 30%ige Natriumnitritlösung zugegeben und 1 Stunde bei 0°C gerührt. Überschüssiges Natriumnitrit wurde mit Amidosulfonsäure vernichtet und die so erhaltene Diazotierung zur Lösung der Kupplungskomponente aus Beispiel gegeben. Mit Sodalösung wurde ein pH-Wert von 7-8 gehalten. T = 10-15°C. Nach beendeter Kupplung salzte man mit NaCl aus, isolierte und trocknete. Der erhaltene Farbstoff der Formel

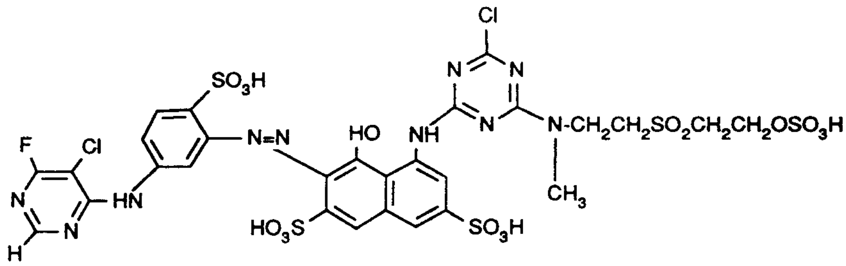

färbt Baumwolle in roten Tönen.

Die Farbstoffe der Beispiele 180-182 wurden analog Beispiel 179 hergestellt:

| Beispiel | Diazokomponente | Kupplungskomponente | Y | B |
|---|---|---|---|---|
| 180 | 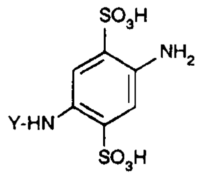 | 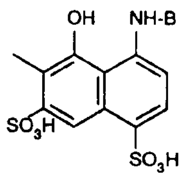 | 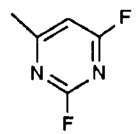 | 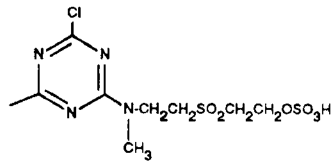 |
| 181 | 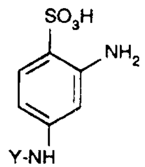 | 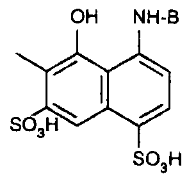 | " | 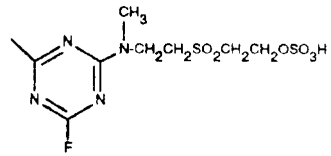 |
| 182 | 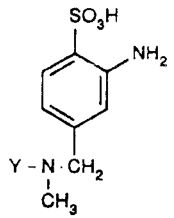 | 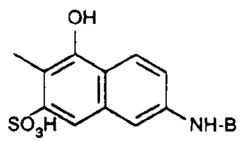 | 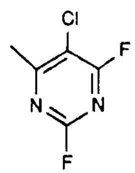 | " |

Beispiel 183

0,1 Mol des Kupferkomplexes von N-(2-Carboxy-5-sulfophenyl)-N'-(2'-hydroxy-3'-amino-5'-sulfophenyl)-ms-phenyl - formazan-di-natriumsalz wurden in 600 ml Wasser gelöst. Nach Abkühlung auf 0°C ließ man 0,12 Mol 2,4,6-Trifluor-1,3,5-triazin zutropfen und hielt den pH-Wert durch Zugabe von Soda-Lösung auf 7,0. Nach Zugabe von 0,11 Mol der Verbindung der Formel (Va) wurde der pH-Wert mit Soda-Lösung auf 8 gehalten und ließ die Temperatur allmählich auf 20°C ansteigen. Nach beendeter Kondensation wurde der Farbstoff ausgesalzen, isoliert und nach Pufferung bei pH 6 bei 45°C im Vakuum getrocknet.

Der Farbstofff entspricht der Formel

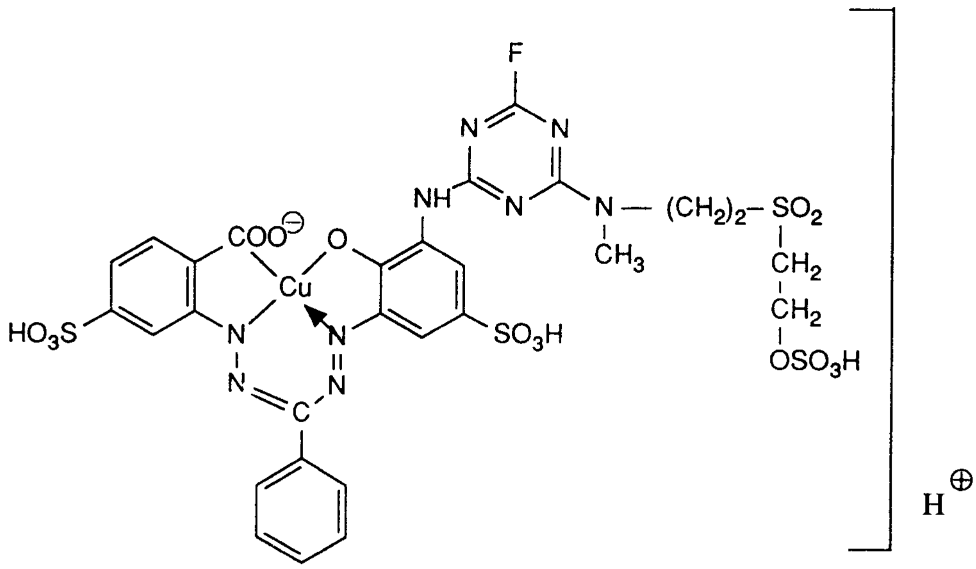

und färbt Baumwolle aus langer Flotte mit sehr guten Fixierausbeuten in blauen Tönen.

$\lambda_{max}$ = 609 nm

Weitere blaue Formazanfarbstoffe erhielt man durch Kondensation der folgenden Komponenten:

| Nr. | Aminoformazan | Trihalogen-triazin | Komponente der Formel (V) | Farbton |
|---|---|---|---|---|
| 184 | N-(2-Carboxy-5-sulfophenyl)-N'-(2'-hydroxy-5'-amino-3'-sulfophenyl)-ms-(2"-sulfophenyl)-formazan, Cu-Komplex | 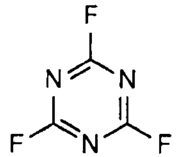 | $HN-(CH_2)_2-SO_2-CH_2-CH_2-OSO_3H$<br>$CH_3$ | blau |
| 185 | N-(2-Hydroxy-3-amino-5-sulfophenyl)-N'-(2'-hydroxy-4'-sulfophenyl)-ms-(2"-sulfophenyl)-formazan, Cu-Komplex | 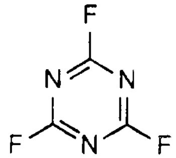 | " | marineblau |
| 186 | N-(2-Carboxy-5-sulfophenyl)-N'-2-hydroxy-5'-amino-3'-sulfophenyl)-ms-(4"-sulfophenyl)-formazan, Cu-Komplex | 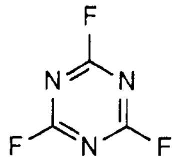 | " | blau |
| 187 | N-(2-Carboxy-4-sulfophenyl)-N'-(2'-hydroxy-3'-amino-5'-sulfophenyl)-ms-phenyl-formazan, Cu-Komplex | 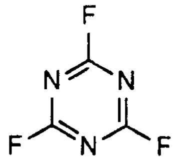 | " | blau |

| Nr. | Aminoformazan | Trihalogen-triazin | Komponente der Formel (V) | Farbton | $\lambda_{max}$(nm) |
|---|---|---|---|---|---|
| 188 | N-(2-Carboxy-5-sulfophenyl)-N'-(2'-hydroxy-3'-amino-5'-sulfophenyl)-ms-phenyl-formazan, Cu-Komplex | 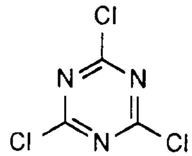 | 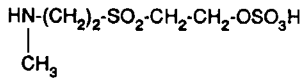 | blau | 610 |
| 189 | N-(2-Carboxy-4-sulfophenyl)-N'-(2'-hydroxy-3'-amino-5'-sulfo-phenyl)-ms-phenyl-formazan, Cu-Komplex | 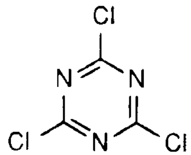 | " | blau | |
| 190 | N-(2-Hydroxy-3-amino-5-sulfo-phenyl)-N'-(2'-carboxy-4-sulfo-phenyl)-ms-(2"-chlor-5"-sulfophenyl)-formazan, Cu-Komplex | 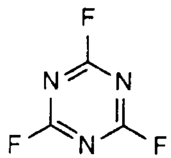 | 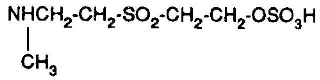 | blau | |
| 191 | N-(2-Hydroxy-5-amino-3-sulfo-phenyl)-N'-(2',5'-disulfophenyl)-ms-phenyl-formazan, Cu-Komplex | 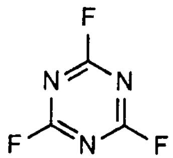 | " | blau | |

| Nr. | Aminoformazan | Trihalogen-triazin | Komponente der Formel (V) | Farbton |
|---|---|---|---|---|
| 192 | N-(2-Carboxy-5-sulfophenyl)-N'-(2'-hydroxy-3'-amino-5'-sulfophenyl)-ms-(2"-sulfophenyl)-formazan, Cu-Komplex | 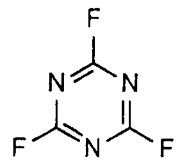 | $NHCH_2-CH_2-SO_2-CH_2CH_2OSO_3H$<br>$CH_3$ | blau |
| 193 | N-(2-Carboxy-5-sulfophenyl)-N'-(2'-hydroxy-3',5'-disulfophenyl)-ms-(3"-amino-phenyl)-formazan, Cu-Komplex | 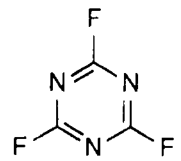 | " | grün-stichig blau |
| 194 | N-(2-Carboxy-5-sulfophenyl)-N'-(2'-hydroxy-3'-amino-5'-sulfophenyl)-ms-(2"-sulfophenyl)-formazan, Cu-Komplex | 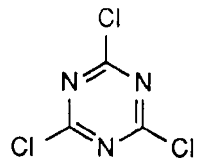 | " | blau |

Beispiel 195

0,1 Mol des Anthrachinons der Formel

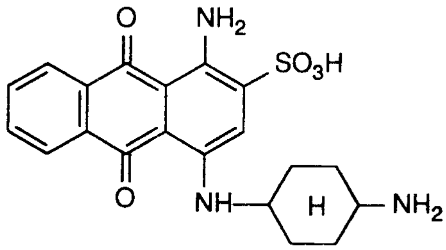

wurden in ca. 1 000 Teilen Wasser mit Kaliumhydroxydlösung bei einem pH-Wert von 12 bis 13 gelöst. Die Farbstofflösung wurde geklärt und mit 200 Teilen Hexahydro-2H-azepin-2-on versetzt. Bei 0°C wurden 0,11 Mol Cyanurchlorid ankondensiert. Die zweite Kondensation wurde nach Zugabe von 0,1 Mol der Verbindung der Formel (Va) bei pH 8 und 30°C durchgeführt.

Der Farbstoff wurde mit Ethanol ausgefällt, isoliert und getrocknet. Man erhielt ca. 200 g eines Reaktivfarbstoffes der Formel

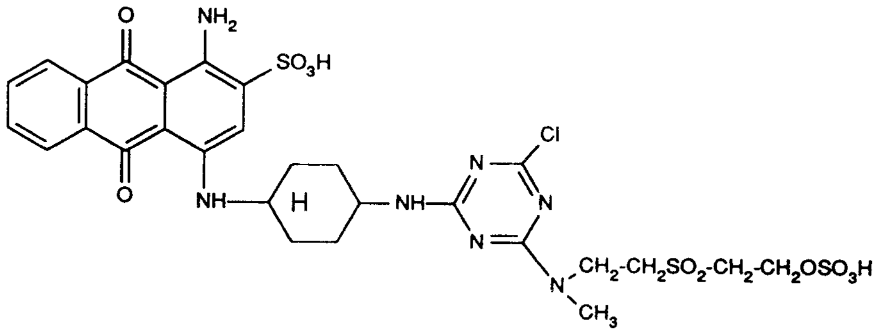

,

welcher Baumwolle nach den für Reaktivfarbstoffe üblichen Färbe- oder Druckverfahren in blauen Farbtönen färbt.

$\lambda_{max}$ = 593 nm, 640 nm

Weitere blaue Anthrachinonfarbstoffe mit ähnlichen Eigenschaften wurden erhalten, in dem man die nachfolgend aufgeführten Anthrachinonkomponenten der allgemeinen Formel

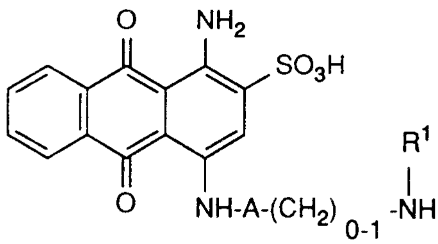

mit den Trihalogentriazinen und der Komponente der Formel (Va) nach einer der oben dargelegten Methoden kondensierte.

| Nr. | $-NH-A-(CH_2)_{0-1}-\overset{R^1}{\overset{\vert}{N}}H$ | Trihalogen-triazin | Komponente der Formel (V) | Farbton | $\lambda_{max}$(nm) |
|---|---|---|---|---|---|
| 196 | 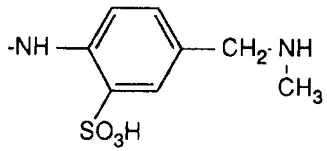 | 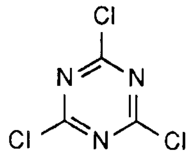 | $HN(CH_3)-(CH_2)_2-SO_2-CH_2-CH_2-OSO_3H$ | blau | 599 |
| 197 | 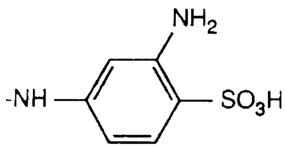 | 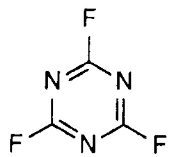 | " | blau | 595 |
| 198 | 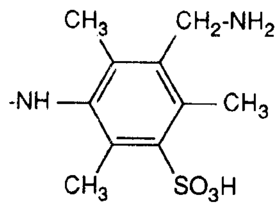 | 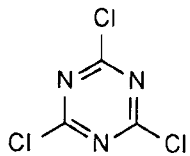 | " | rotstichig blau | |
| 199 | 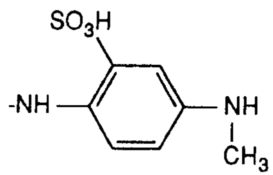 | 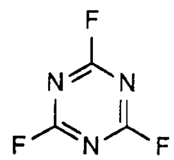 | " | blau | 610 |

| Nr. | $R^1$ <br> $-NH-A-(CH_2)_{0-1}-NH$ | Trihalogen-triazin | Komponente der Formel (V) | Farbton | $\lambda_{max}$(nm) |
|---|---|---|---|---|---|
| 200 | 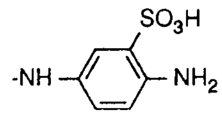 | 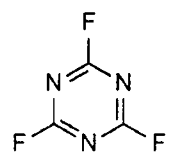 | $HN-(CH_2)_2-SO_2-CH_2-CH_2-OSO_3H$ <br> $CH_3$ | grünstichig blau | 605 |
| 201 | 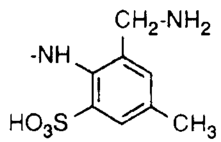 | 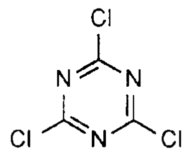 | " | rotstichig blau | 584, 618 |
| 202 | 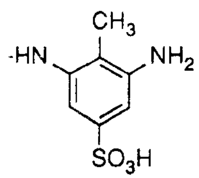 | 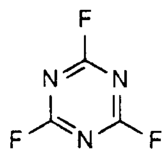 | " | rotstichig blau | 591, 619 |
| 203 | 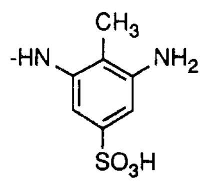 | 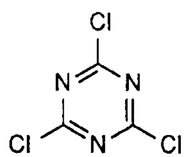 | " | rotstichig blau | 592, 618 |

Beispiel 204

32 g Kupferphthalocyaninkomponente der Formel

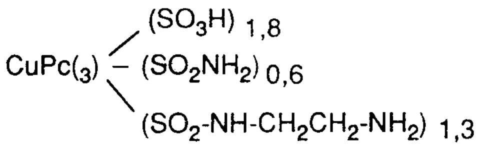

hergestellt nach den Angaben der Europäischen Patentschrift 0 073 267 wurden in 300 ml Wasser bei pH = 7,0 bis 7,5 gelöst und analog Beispiel 195 mit 2,4,6-Trichlor-1,3,5-triazin und der Verbindung der Formel (Va) umgesetzt.

Nach beendeter Umsetzung wurde das erhaltene Produkt aus der Lösung ausgesalzen, abgesaugt, mit etwas Phosphatlösung bei pH 6,0 gepuffert und im Vakuum bei 45°C getrocknet. Der Farbstoff entspricht der Formel

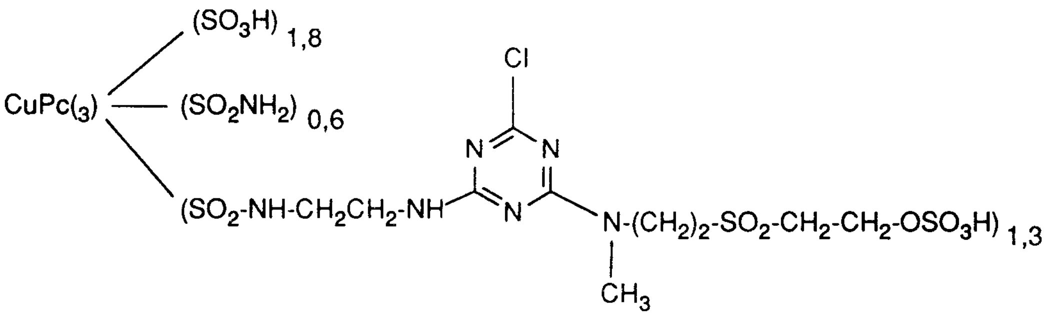

und färbt und druckt Baumwolle nach den für Reaktivfarbstoffe bekannten Verfahren echt und ergiebig in türkisblauen Tönen. $\lambda_{max}$ = 663, 622 nm

Weitere Phthalocyanin-Reaktivfarbstoffe wurden erhalten, indem man die in nachfolgender Zusammenstellung angegebenen Phthalocyaninkomponenten, Trihalogentriazine und die Komponente der Formel (Va) nach den in den Beispielen 183 und 195 beschriebenen Methoden miteinander kondensierte.

| Nr. | Phthalocyaninkomponente | Trihalogen-triazin | Komponente der Formel (V) | Farbton | $\lambda_{max}$(nm) |
|---|---|---|---|---|---|
| 205 | 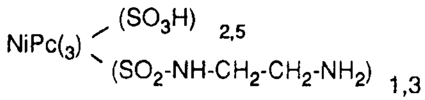 | 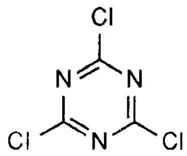 | 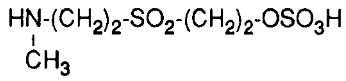 | blaustichig grün | 657, 623 |
| 206 | 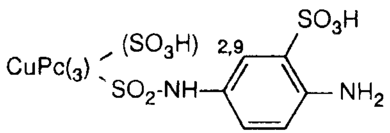 | 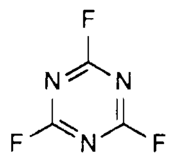 | " | türkis | |
| 207 | 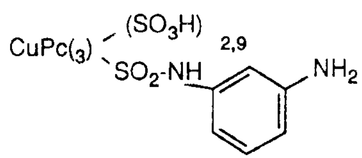 | 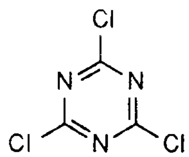 | " | türkis | 667, 623 |
| 208 | 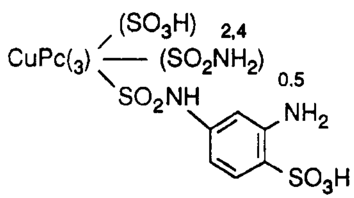 | 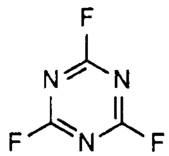 | " | türkis | |

Beispiel 209

22,1 g Triphendioxazinverbindung der Formel

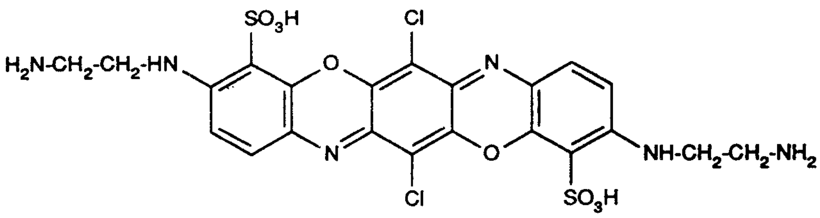

wurden in 300 ml Wasser durch Zugabe von Natronlauge bei pH 11,5 bis 12 gelöst und analog Beispiel 195 mit 2,4,6-Trichlor-1,3,5-triazin und der Verbindung der Formel (Va) umgesetzt.

Der Farbstoff wurde ausgesalzen, abgesaugt und nach Pufferung auf pH 6,7 bei 45°C im Vakuum getrocknet. Er entspricht der Formel

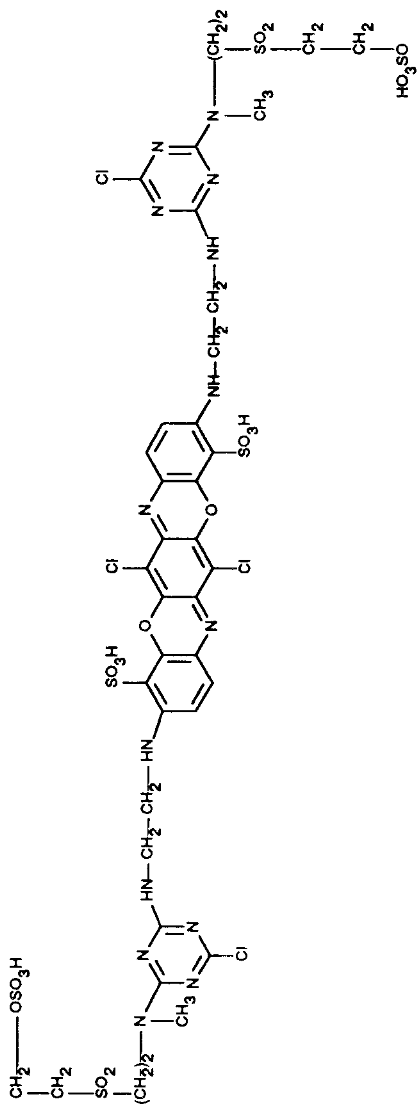

und färbt Baumwolle nach den üblichen Methoden mit guten Fixierausbeuten in kräftigen Blautönen. $\lambda_{max}$ = 622 nm

Beispiel 210

50 g Triphendioxazinverbindung der Formel

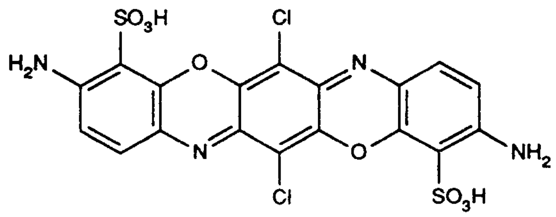

wurden in 2 000 ml Wasser mit 10 %iger Lithiumhydroxid durch Einstellen des pH-Wertes auf 7,0 gelöst und analog Beispiel 183 mit 2,4,6-Trifluor-1,3,5-triazin und der Verbindung der Formel (Va) umgesetzt.

Das Kondensationsprodukt wurde ausgesalzen, isoliert und getrocknet.

Der erhaltene Farbstoff entspricht der Formel

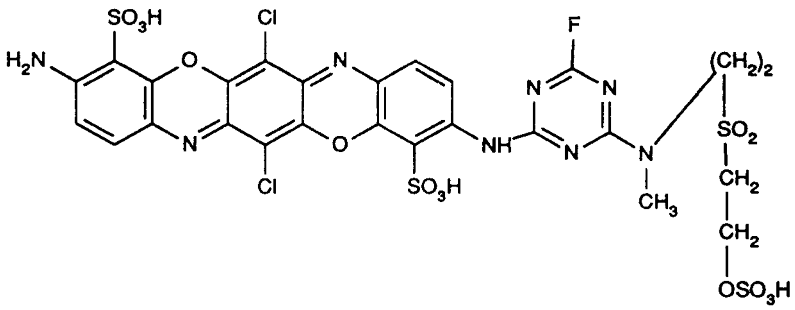

und färbt Baumwolle aus langer Flotte in stark rotstichig blauen Tönen.

$\lambda_{max}$= 580 nm

Weitere Triphendioxazin-Reaktivfarbstoffe wurden in analoger Verfahrensweise zu den Beispielen 183 und 195 durch Kondensation folgender Komponenten hergestellt:

| Nr. | Triphendioxazin | Trihalogen-triazin | Komponente der Formel (V) | | Farbton | $\lambda_{max}$(nm) |
|---|---|---|---|---|---|---|
| 211 | 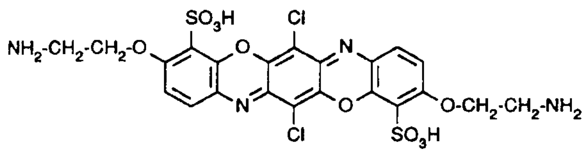 | 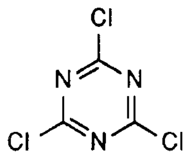 | " | Produkt-Typ entspricht dem aus Beispiel 209 | rot | |
| 212 | 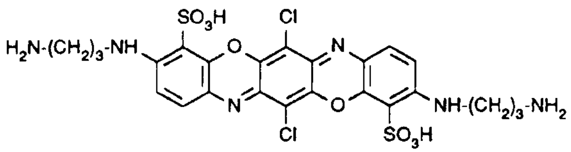 | 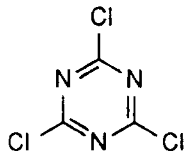 | " | | blau | 624 |
| 213 | 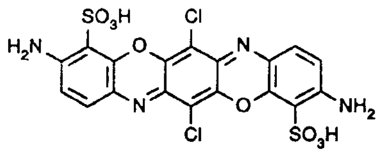 | 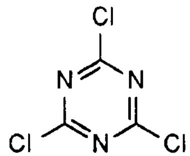 | " | Produkt-Typ entspricht dem aus Beispiel 210 | blau | 580 |

**Patentansprüche**

**1.** Reaktivfarbstoffe der Formel

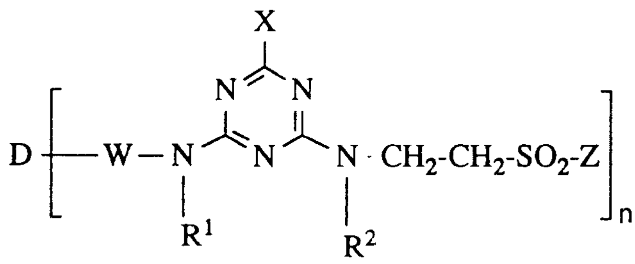

(I),

worin

| | |
|---|---|
| D | Rest eines organischen Farbstoffes aus der Monoazo-, Polyazo-, Metallkomplexazo-, Anthrachinon-, Phthalocyanin-, Formazan-, Azomethin-, Dioxazin-, Phenazin-, Stilben-, Triphenylmethan-, Xanthen-, Thioxanthon- oder der Nitroarylreihe |
| W | direkte Bindung oder Brückenglied, |
| $R^1$ | H oder gegebenenfalls substituiertes $C_1$-$C_4$-Alkyl, |
| $R^2$ | $C_1$-$C_4$-Alkyl, insbesondere Methyl, |
| X | F, Cl oder Br, |
| Z | $-CH=CH_2$ oder $-CH_2-CH_2-OSO_3H$ und |
| n | 1 oder 2 bedeutet. |

**2.** Reaktivfarbstoffe gemäß Anspruch 1, dadurch gekennzeichnet, daß $R^2$ für Methyl und Z für $CH_2CH_2OSO_3H$ steht.

**3.** Reaktivfarbstoffe gemäß Anspruch 1, dadurch gekennzeichnet, daß

D Rest eines organischen Farbstoffes aus der Anthrachinon-, Phthalocyanin-, Formazan-, Azomethin-, Dioxazin-, Phenazin-, Stilben-, Triphenylmethan-, Xanthen-, Thioxanthon- oder der Nitroarytreihe ist.

**4.** Reaktivfarbstoffe gemäß wenigstens einem der vorhergehenden Ansprüche, dadurch gekennzeichnet, daß sie einer der folgenden Formeln entsprechen, wobei

B' einen Rest der Formel

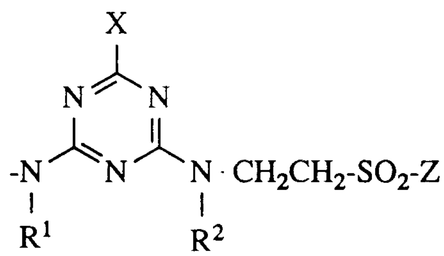

(VIb)

bedeutet, in welchem die Substituenten die in Anspruch 1 angegebene Bedeutung haben,

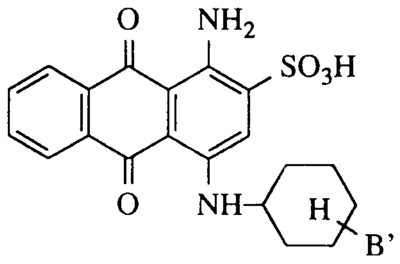

(1)

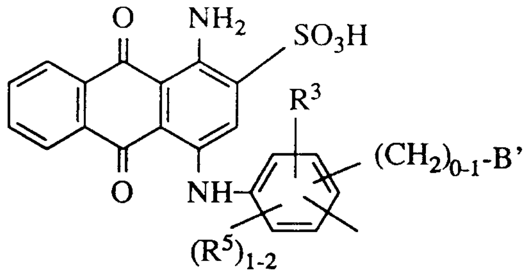

(2)

worin

$R^3$ = H, Methyl, Methoxy, Chlor
$R^4$ = H, $SO_3H$ und
$R^5$ = H, Methyl oder Ethyl

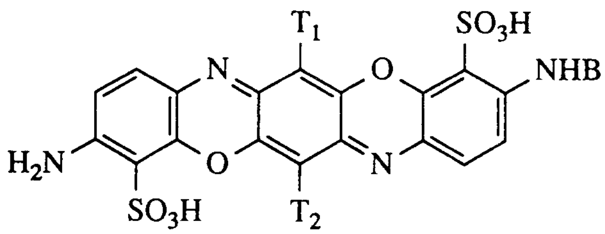

(3a)

worin

B einen Rest der Formel

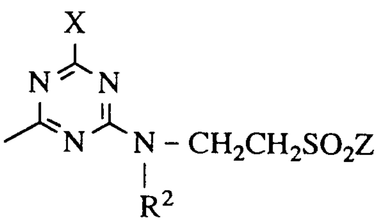

(VIa)

bedeutet, in welchem die Substituenten X, $R^2$ und Z die oben angegebene Bedeutung haben, und

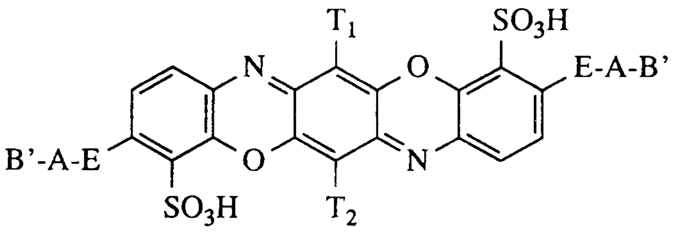

(3b)

worin

A für ein gegebenenfalls substituiertes Phenylen oder ein gegebenenfalls substituiertes aromatisch-aliphatisches Brückenglied oder für ein gegebenenenfalls durch Heteroatome wie $NR^6$, O oder S enthaltende Gruppierungen unterbrochenes, geradkettiges oder verzweigtes $C_1$-$C_6$-Alkylen, das mit $C_1$-$C_6$-Alkoxy, $OSO_3H$, $SO_3H$, COOR oder Halogen substituiert sein kann, steht und wobei innerhalb eines Brückengliedes A die Gruppe $NR^6$ mit der Gruppe $NR^1$ auch einen heterocyclischen aliphatischen Ring bilden kann, insbesondere

E =

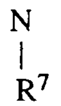
,

O und

$T_1$, $T_2$ = unabhängig voneinander H, Cl, Br, $C_1$-$C_2$-Alkyl, $OCH_3$, $OC_2H_5$, Acylamino, $C_1$-$C_2$-Alkoxycarbonyl ist,

$R^6$, $R^7$ = unabhängig voneinander H, $C_1$-$C_4$-Alkyl, das durch OR, $OSO_3H$, $SO_3H$, COOR oder Halogen substituiert sein kann,

R = H, $CH_3$ oder $C_2H_5$

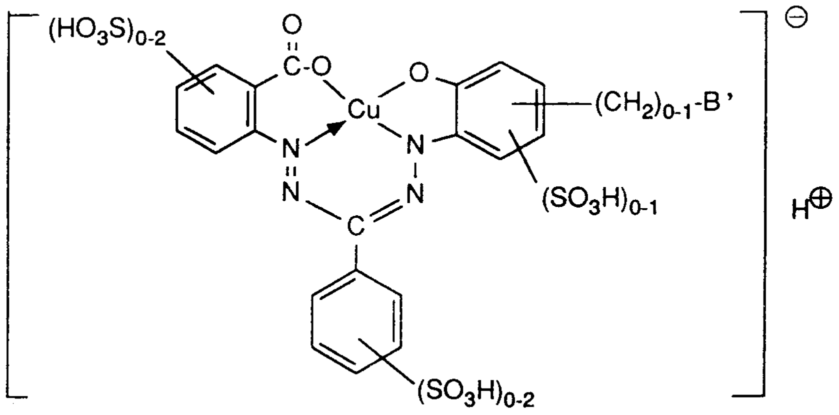

(4)

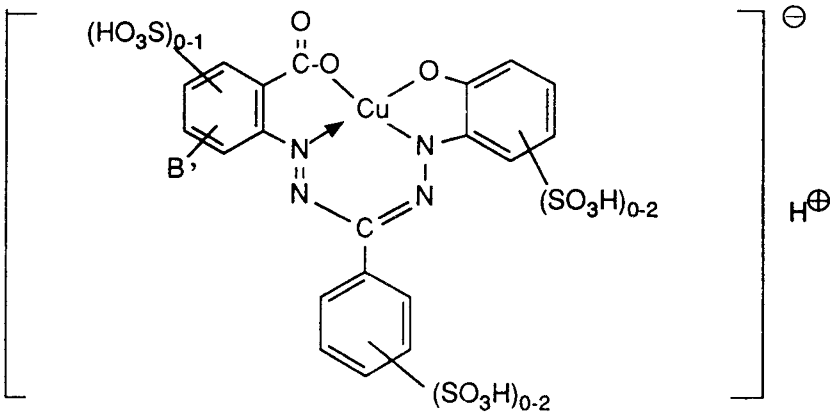

(5)

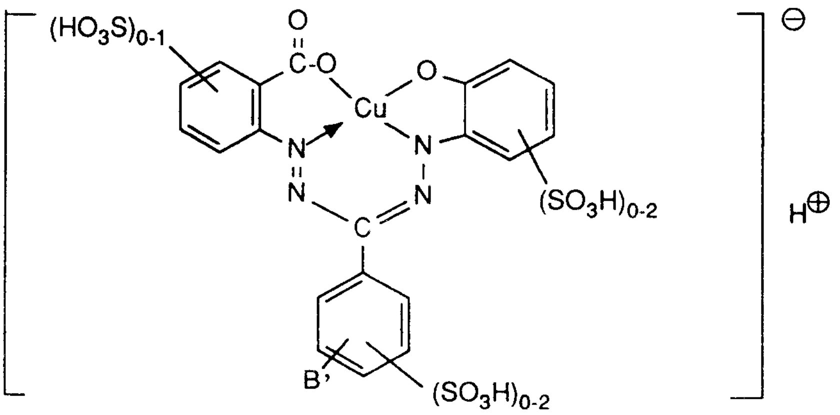

(6)

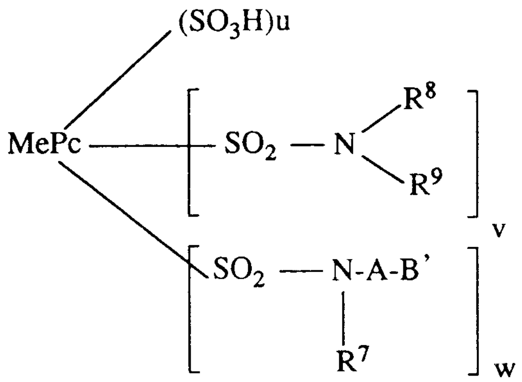

(7)

worin

Me = Cu, Ni,
Pc = Rest eines Phthalocyanins,
u+v+w = 3,4 - 4,0 , mit der Maßgabe, daß
u = 0,8 - 2,0,
v = 0 - 1,0,
w = 1,0 - 3,0 ist und

A die oben angegebene Bedeutung hat,
$R^7$ die oben angegebene Bedeutung hat,
$R^8$ und $R^9$ = H, $C_1$-$C_2$-Alkyl, das gegebenenfalls substituiert durch OH, $OSO_3H$, $SO_3H$ oder COOH ist,

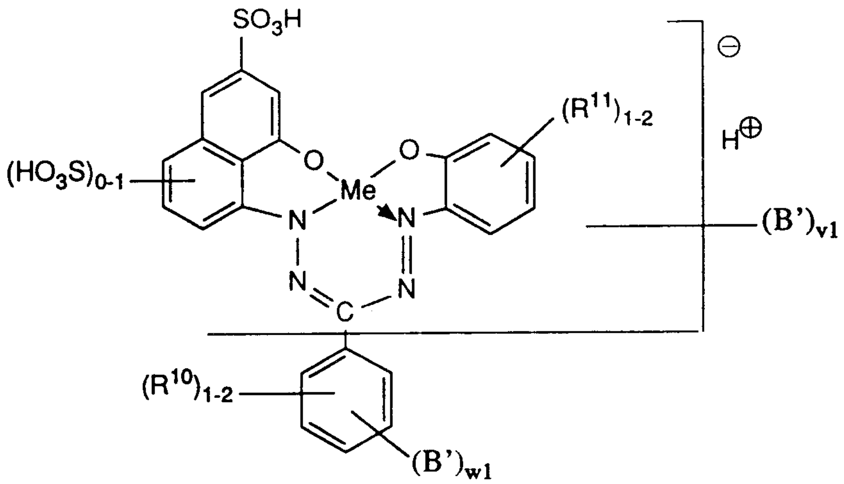

(8)

worin
v1, w1 = 0 oder 1, wobei w1 ungleich v1 ist,
$R^{10}$, $R^{11}$ = unabhängig voneinander H, $C_1$-$C_4$-Alkyl, $C_1$-$C_4$-Alkoxy, OH, Halogen, COOH, $NO_2$, $SO_3H$, Sulfonamido, $C_1$-$C_4$-Allkylcarbonylamino, gegebenenfalls substituiertes Phenylcarbonylamino, $C_1$-$C_4$-Alkylsulfonylamino, gegebenenfalls substituiertes Phenylsulfonylamino.

**5.** Reaktivfarbstoffe gemäß Anspruch 1, dadurch gekennzeichnet, daß

D ein Rest eines organischen Farbstoffes bedeutet, aus der Monoazo-, Polyazo-, Metallkomplexazoreihe ist, der keine weiteren faserreaktiven Gruppen enthält.

**6.** Reaktivfarbstoffe gemäß Anspruch 5, dadurch gekennzeichnet, daß sie einer der folgenden Formeln entsprechen, worin

B einen Rest der Formel

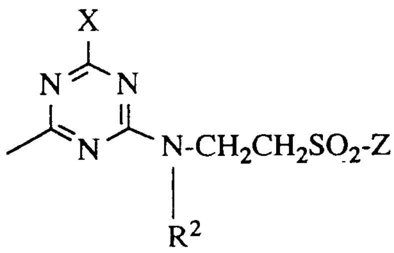

(VIa)

beinhaltet und X, $R^2$ und Z die in Anspruch 1 angegebene Bedeutung haben:

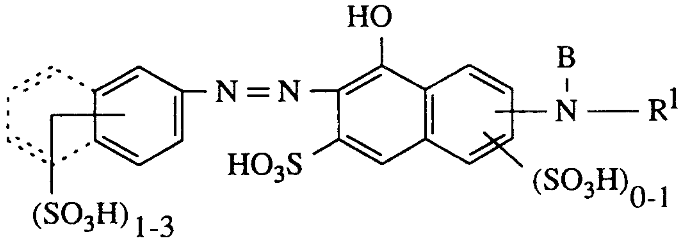

(9)

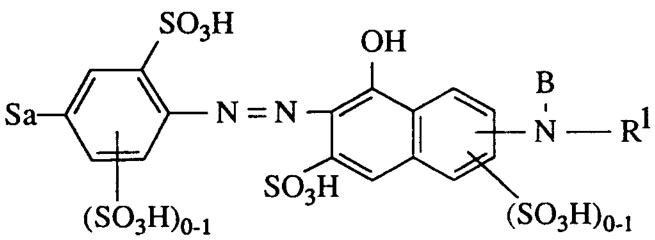

(10)

mit Sa = $OCH_3$ oder $OC_2H_5$

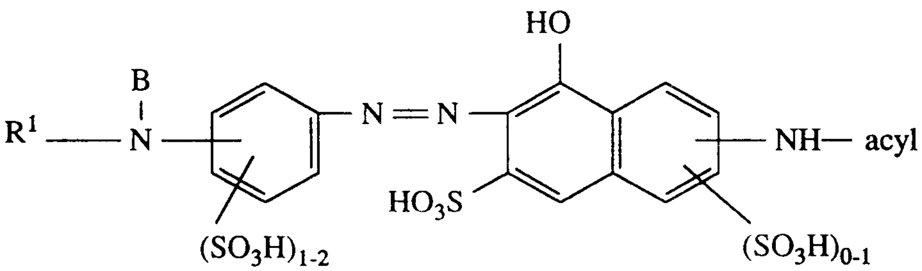

(11)

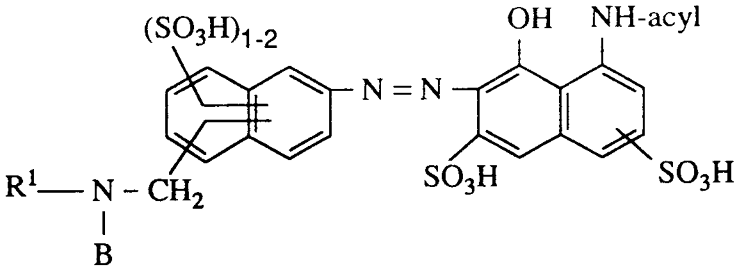

(12)

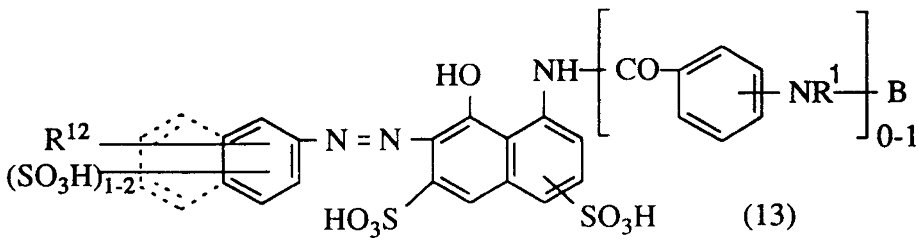

(13)

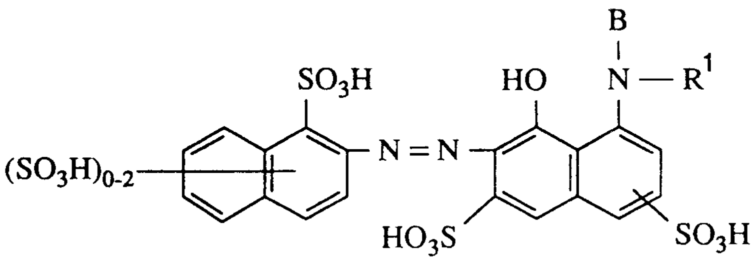

(14)

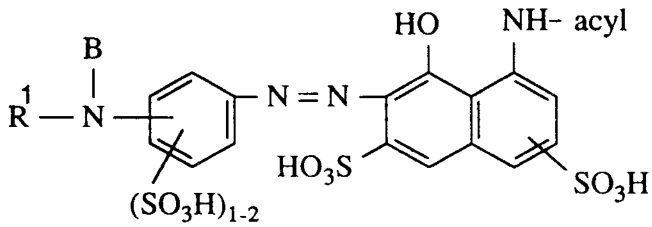

(15)

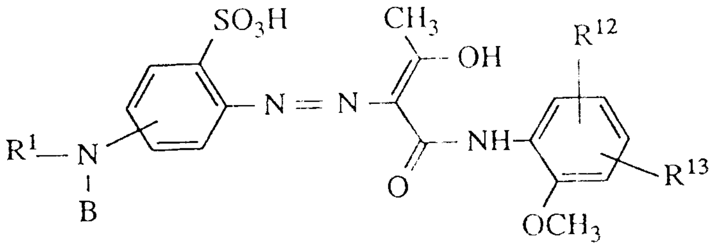

(16)

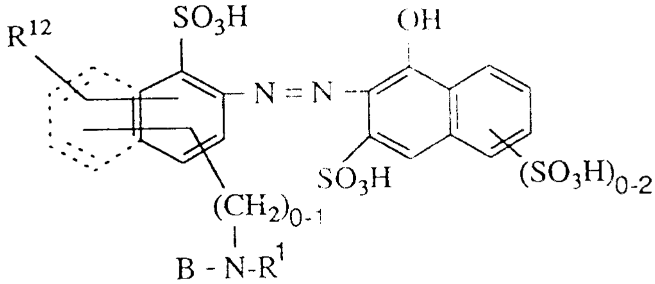

(17)

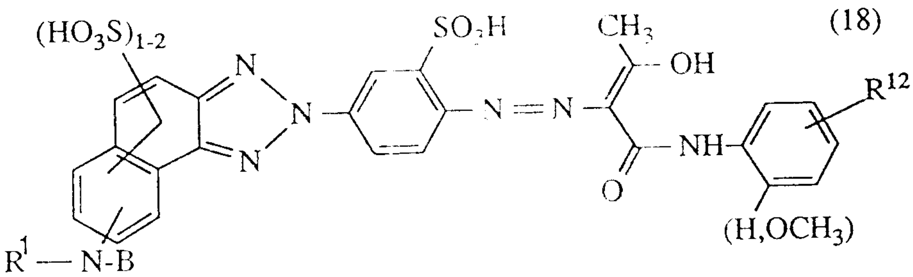

(18)

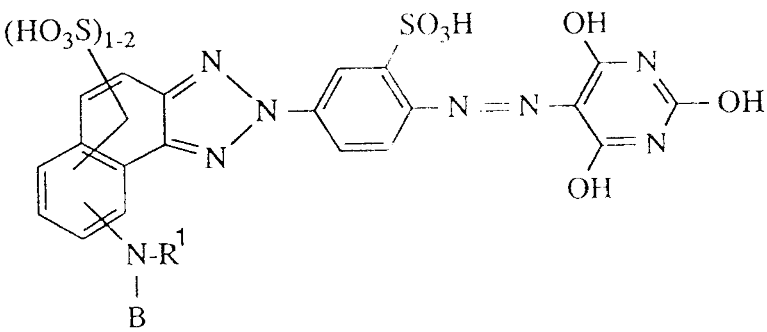

(19)

Metallkomplexe und Farbstoffe der Formeln (20) bis (50):

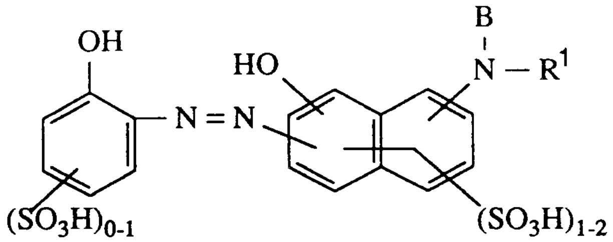

(20)

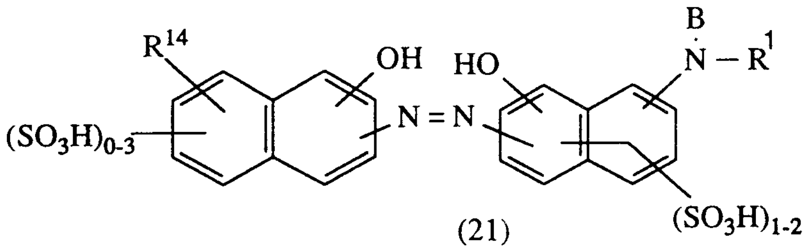

(21)

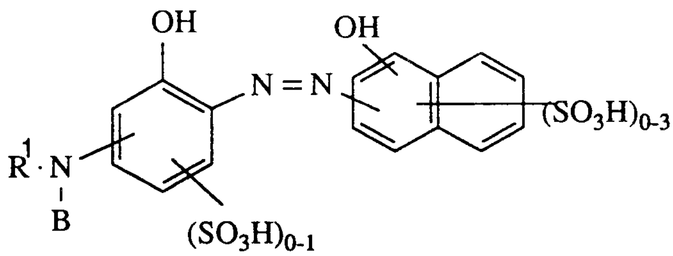

(22)

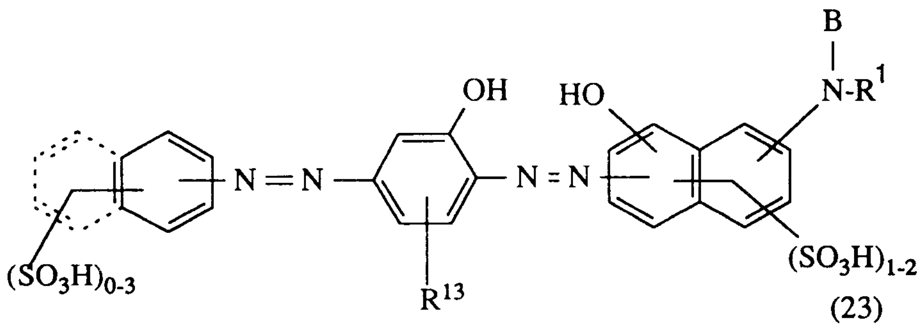

(23)

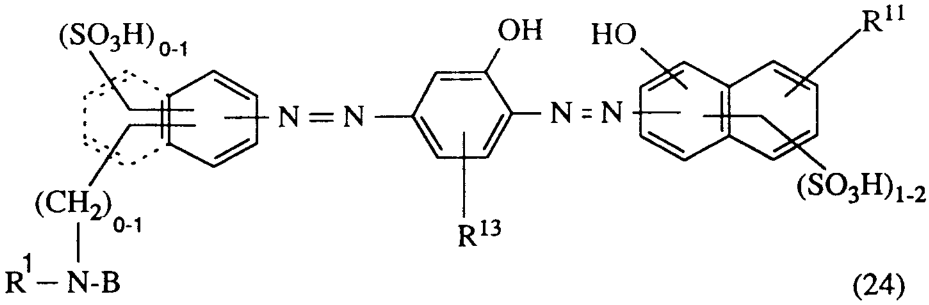

(24)

(25)

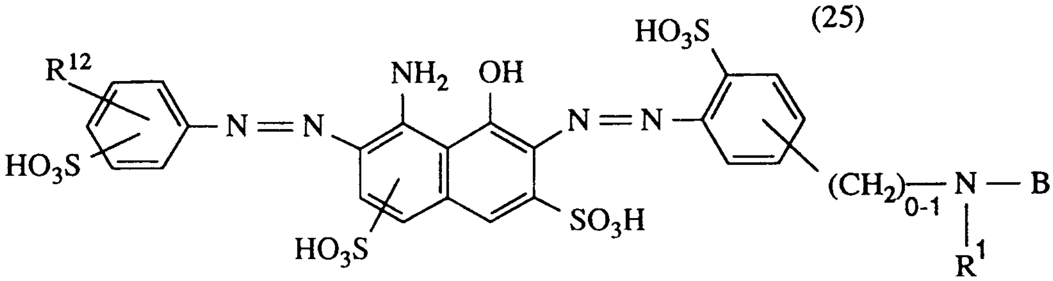

(26)

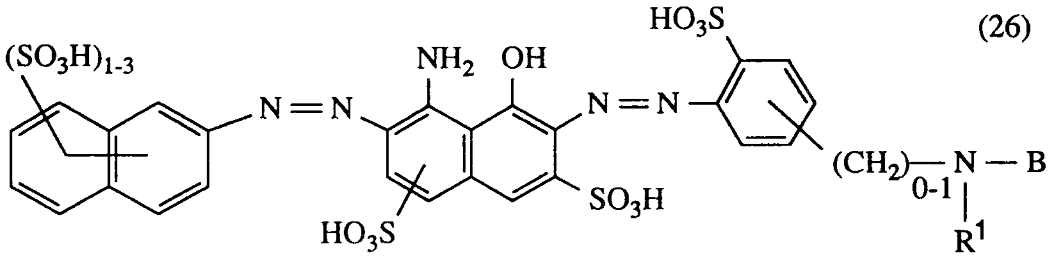

(27)

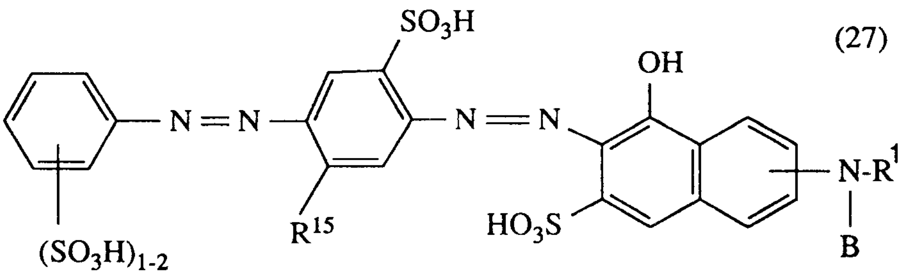

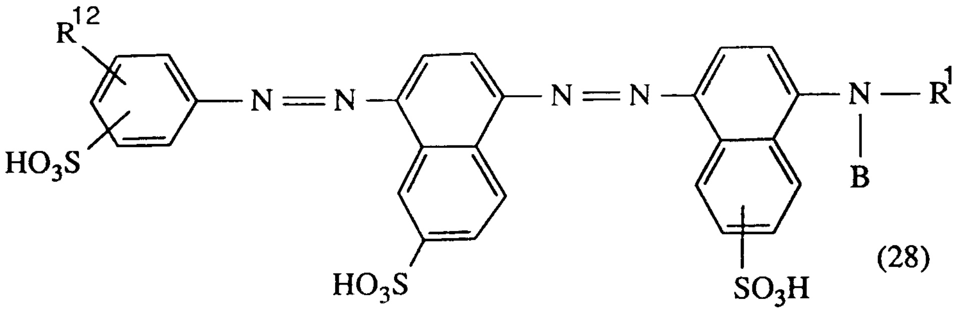

(28)

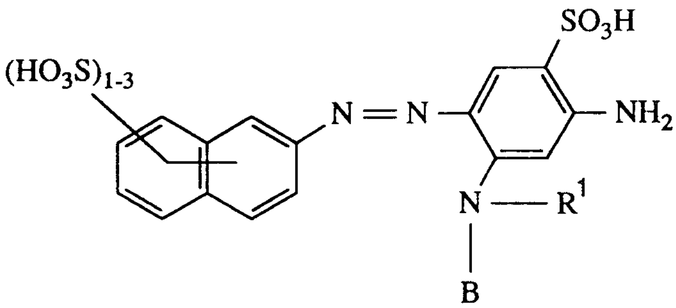

(29)

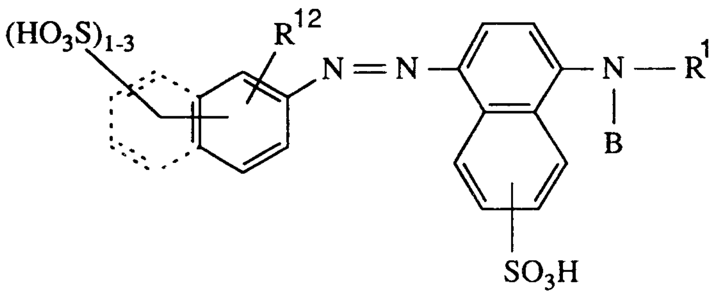

(30)

(31)

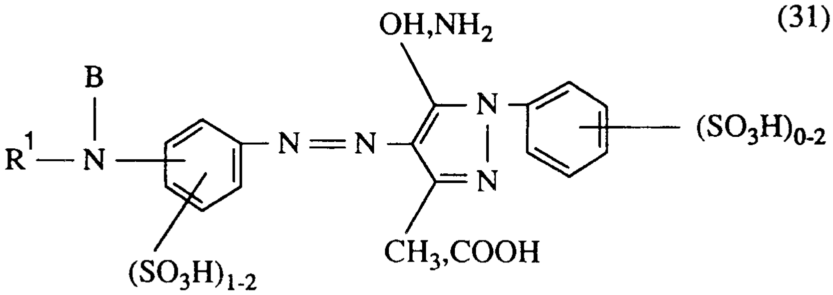

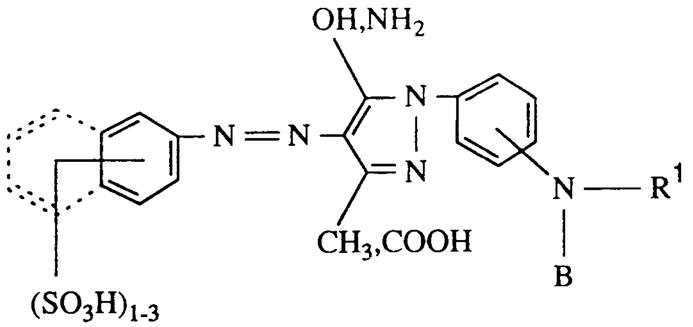

(32)

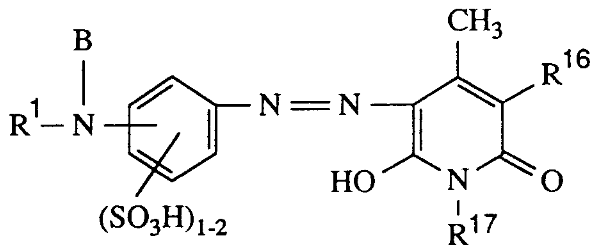

(33)

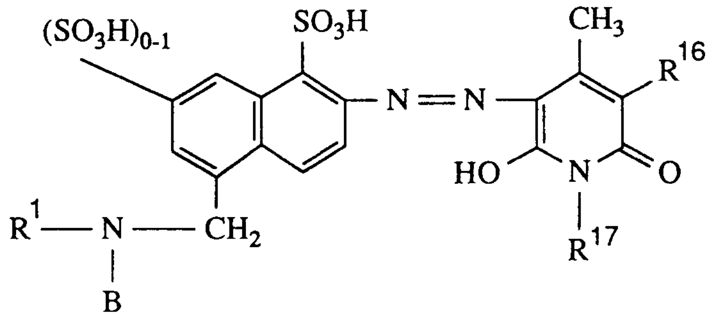

(34)

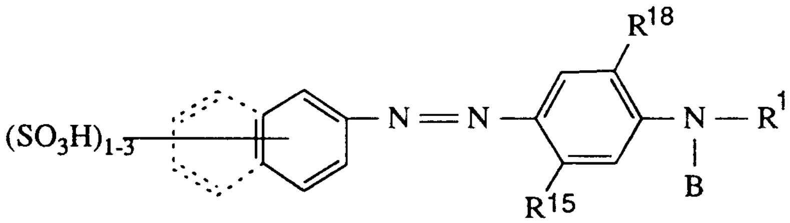

(35)

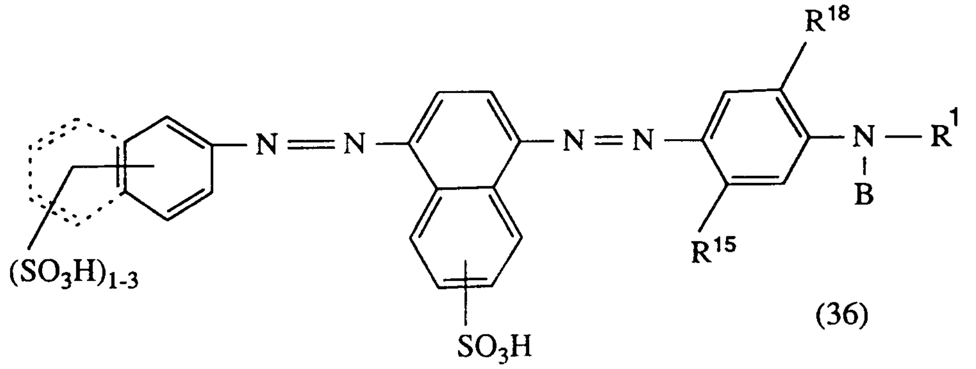

(36)

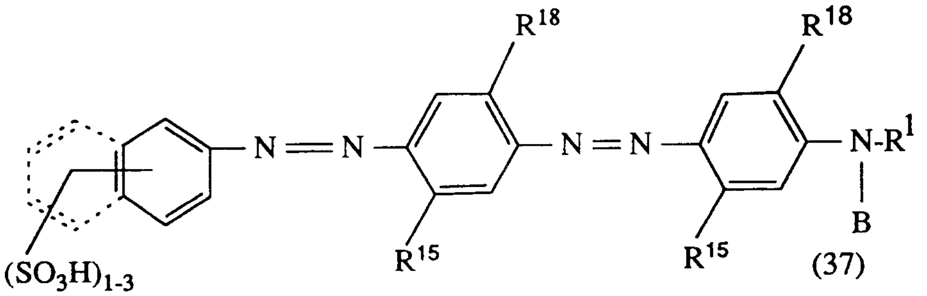

(37)

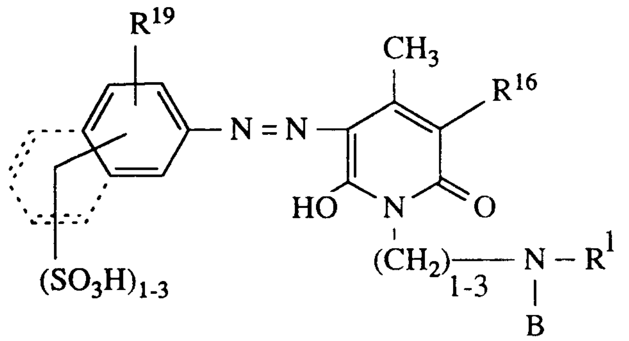

(38)

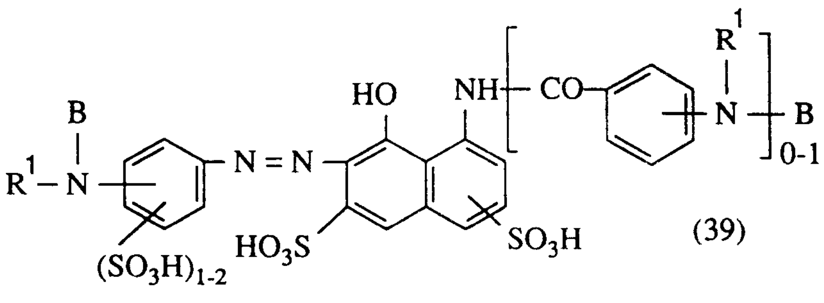

(39)

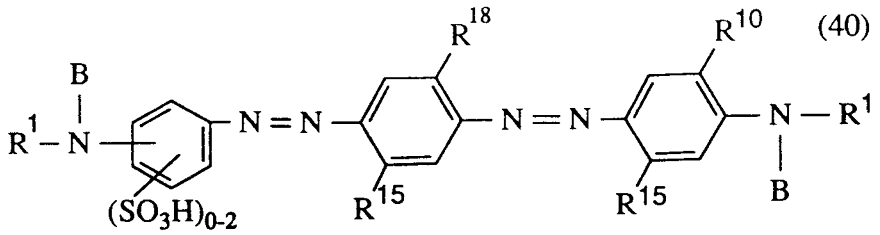

(40)

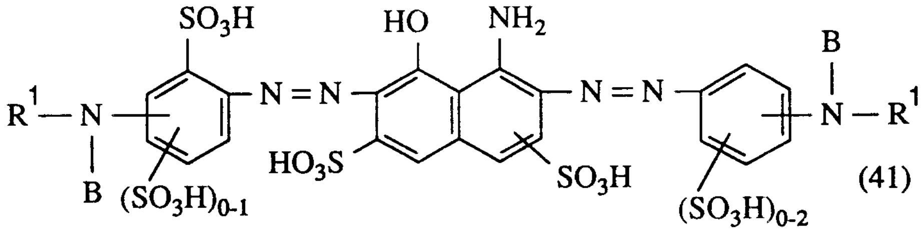

(41)

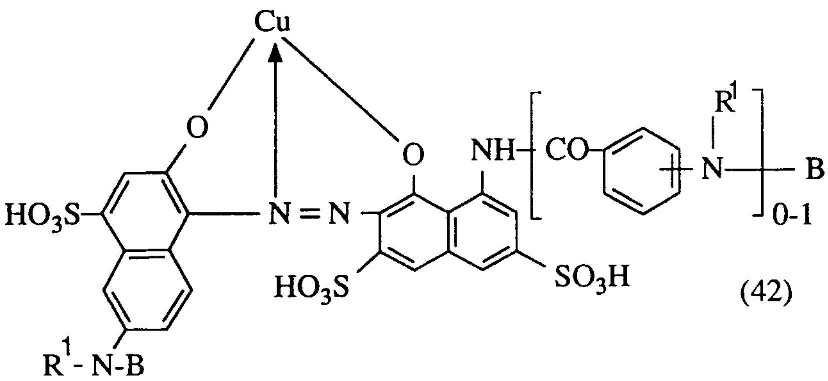

(42)

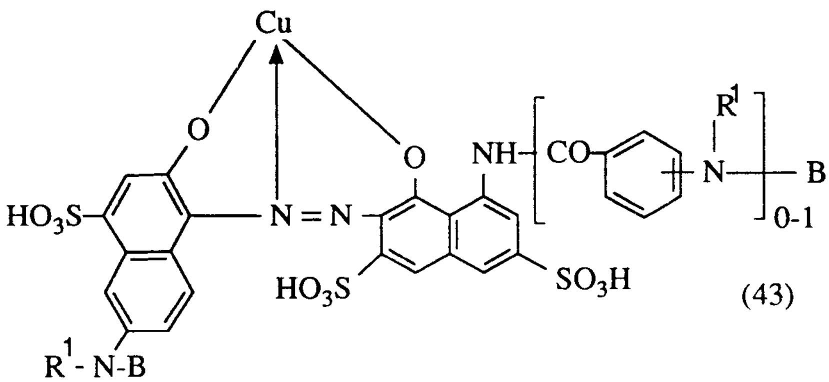

(43)

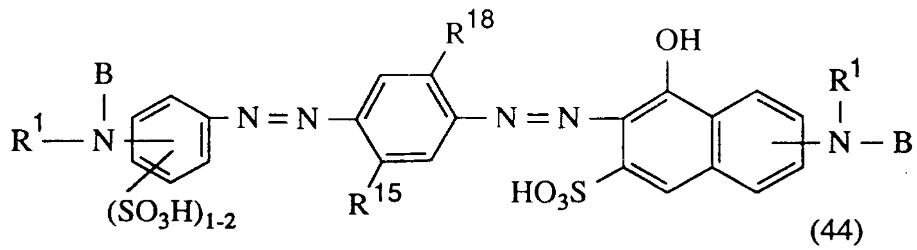

(44)

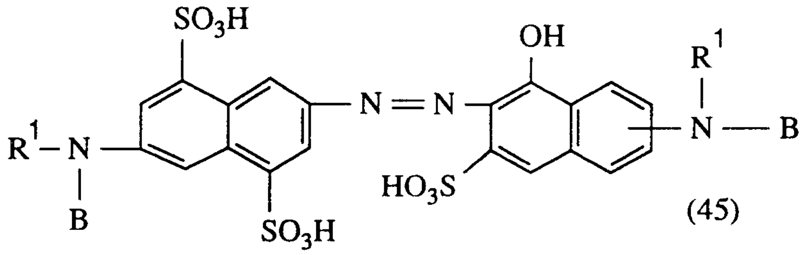

(45)

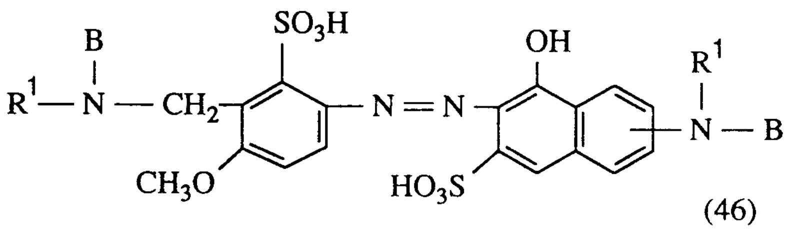

(46)

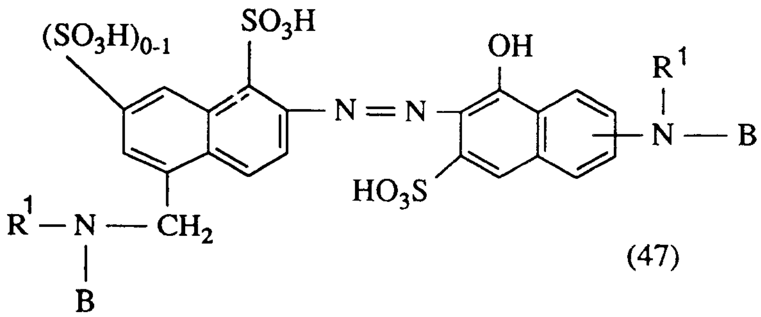

(47)

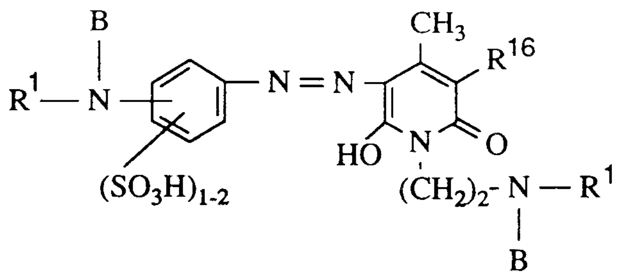

(48)

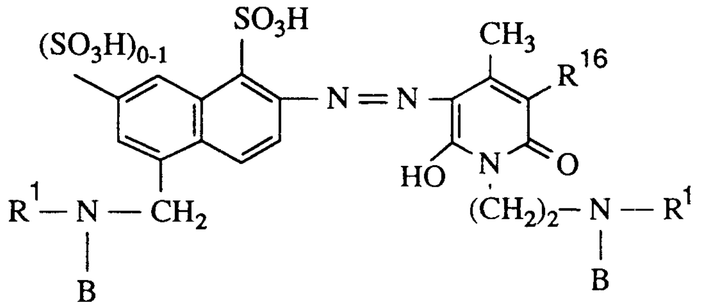

(49)

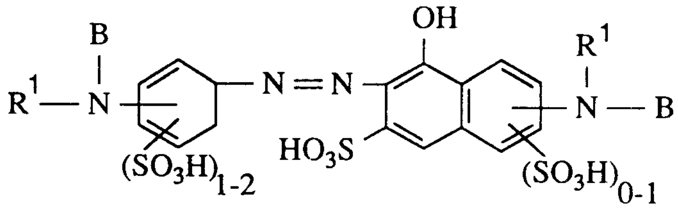

(50),

worin

Acyl z.B. Acetyl oder gegebenenfalls substituiertes Benzoyl ist,

$R^{17}$ = H, $C_1$-$C_2$-Alkyl, gegebenenfalls substituiert durch $SO_3H$, $NH_2$,

$R^1$ = H, $CH_3$ oder $C_2H_5$,

$R^{19}$ = H oder Sulfo,

$R^{13}$ = H, $CH_3$, $OCH_3$ oder Cl,

$R^{12}$ = H, $C_1$-$C_4$-Alkyl, $C_1$-$C_4$-Alkoxy, Cl, Br, COOH, $SO_3H$,

$R^{14}$ = H, OH, $NH_2$, $NHCOCH_3$, NHCOPh, Cl, $C_1$-$C_4$-Alkoxy oder $C_1$-$C_4$-Alkyl ist,

$R^{16}$ = H, $SO_3H$, $CH_2SO_3H$, Cl, $C_1$-$C_4$-Alkylsulfonyl, CN, Carbonamid, insbesondere $CONH_2$,

$R^{15}$ = H, $C_1$-$C_4$-Alkyl, $C_1$-$C_4$-Alkoxy, Cl, Br, Acylamino, insbesondere $C_1$-$C_4$-Alkylcarbonylamino oder Arylcarbonylamino wie gegebenenfalls substituiertes Phenylcarbonylamino, $C_1$-$C_4$-Alkylsulfonylamino, Aminocarbonylamino, $C_1$-$C_4$-Alkylsulfonylamino, Arylsulfonylamino,

$R^{18}$ = H, $C_1$-$C_4$-Alkyl, $C_1$-$C_4$-Alkoxy, OH, $SO_3H$,

und wobei die durch Strichelungen angedeuteten kondensierten Ringe für alternativ mögliche Naphthalinsysteme stehen.

**7.** Reaktivfarbstoffe gemäß Anspruch 1, dadurch gekennzeichnet, daß der Rest D, vorzugsweise ein Rest eines organischen Farbstoffs aus der Monoazo-oder Polyazoreihe, ein- oder zweimal mit der Gruppe

$$Z'\text{-}O_2S\text{-}(CH_2)_{0\text{-}1}\text{—}$$

substituiert ist, worin

Z' $-CH=CH_2$, $-CH_2-CH_2-OSO_3H$, $-CH_2-CH_2-Cl$, $-CH_2-CH_2-Br$, $-CH_2-CH_2-S_2O_3H$, $-CH_2-CH_2-O-CO-CH_3$, $-CH_2-CH_2-OPO_3H_2$ oder $-CH_2-CH_2-OH$ bedeutet.

**8.** Reaktivfarbstoffe gemäß Anspruch 7, dadurch gekennzeichnet, daß

n = 1,
W = direkte Bindung und
D ein Rest der allgemeinen Formel (IX) ist,

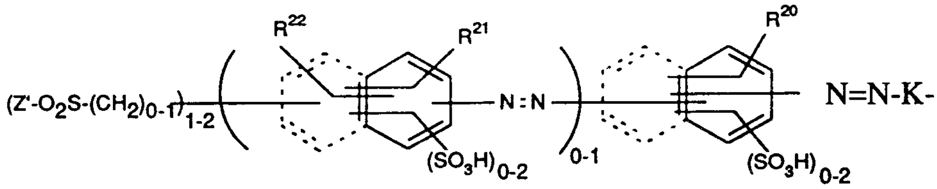

(IX)

worin
$R^{20}$ H, $C_1$-$C_4$-Alkyl, $C_1$-$C_4$-Alkoxy, Cl, Br oder Acylamino,
$R^{21}$ H, $C_1$-$C_4$-Alkyl, Cl, Br, $C_1$-$C_4$-Alkoxy oder COOH,
$R^{22}$ H, $C_1$-$C_4$-Alkyl, $C_1$-$C_4$-Alkoxy, $SO_3H$, Cl oder Br,
K eine bivalenter Rest der allgemeinen Formeln (Xa) - (Xd)

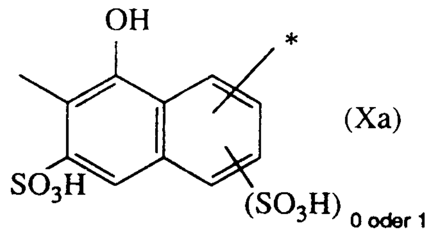

(Xa) ,

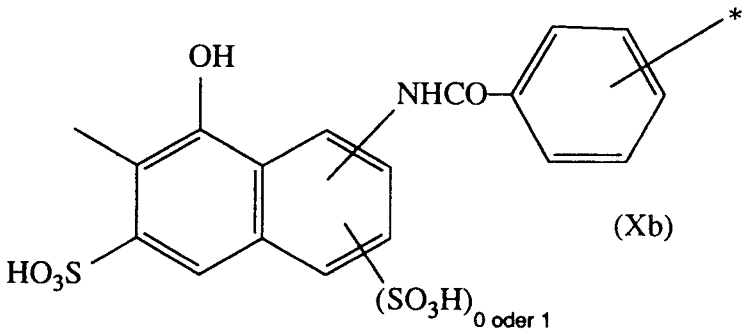

(Xb)

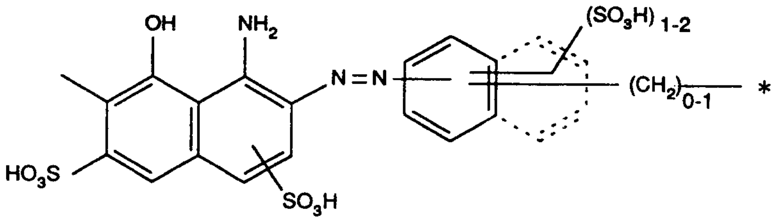

(Xc) ,

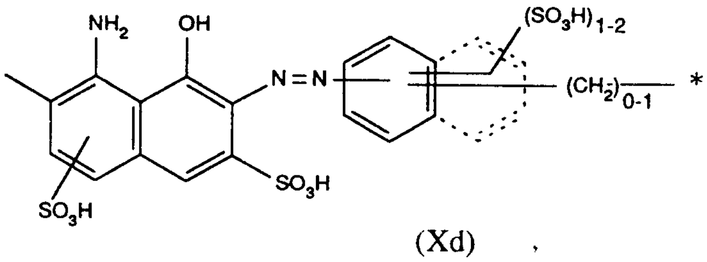

(Xd) ,

bedeutet, wobei die mit * gekennzeichneten Bindungen an die Gruppe $-NR^1-B$ gebunden sind und B die in Anspruch 4 genannte Bedeutung hat.

**9.** Reaktivfarbstoffe gemäß Anspruch 7, dadurch gekennzeichnet, daß sie einer der folgenden Formeln entsprechen, worin

B ein Rest der Formel

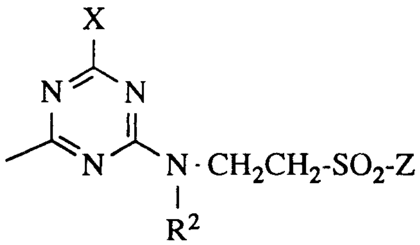

(VIa)

beinhaltet:

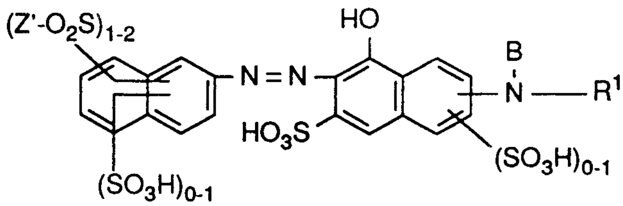

(51),

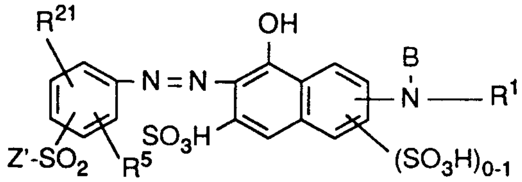

(52),

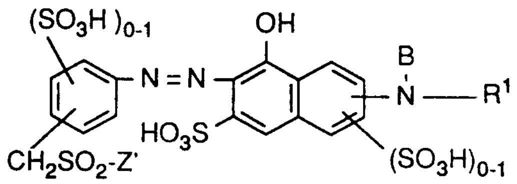

(53),

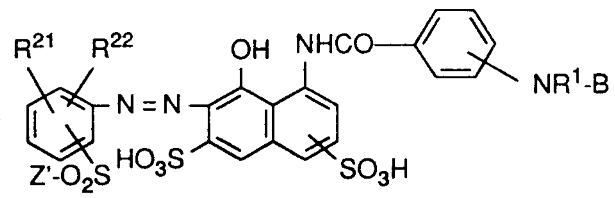

(54),

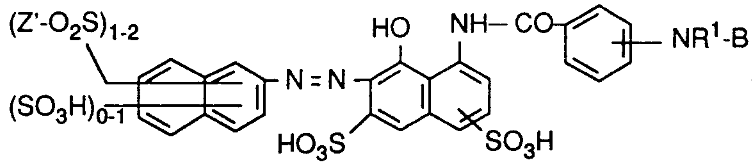

(55),

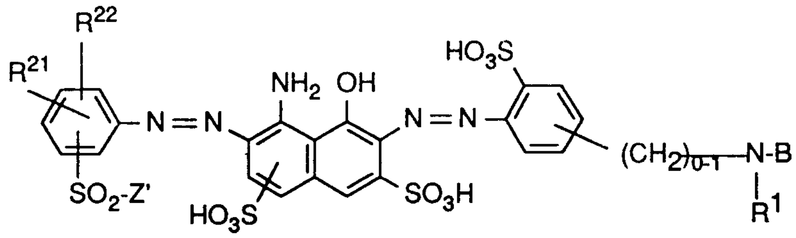

(56),

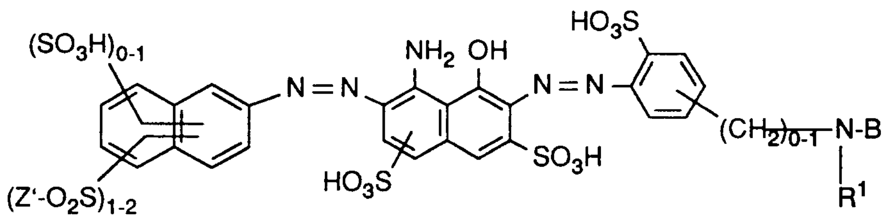

(57),

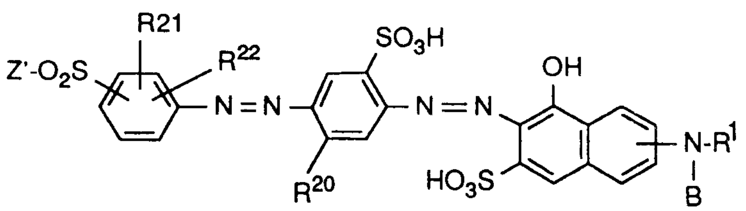

(58),

worin

$R^1$ = H, $CH_3$ oder $C_2H_5$,

$R^{20}$= H, $C_1$-$C_4$-Alkyl, $C_1$-$C_4$-Alkoxy, Acylamino, Cl oder Br,

$R^{21}$= H, $C_1$-$C_4$-Alkyl, $C_1$-$C_4$-Alkoxy, Cl, Br oder COOH,

$R^{22}$= H, $C_1$-$C_4$-Alkyl, $C_1$-$C_4$-Alkoxy, Cl, Br, $SO_3H$.

**10.** Reaktivfarbstoffe gemäß Anspruch 1 der Formel

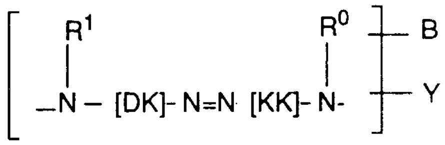

(XI)

worin

DK = Rest einer Diazokomponente der Benzol- oder Naphthalinreihe,
KK = Rest einer Kupplungskomponente der Formel

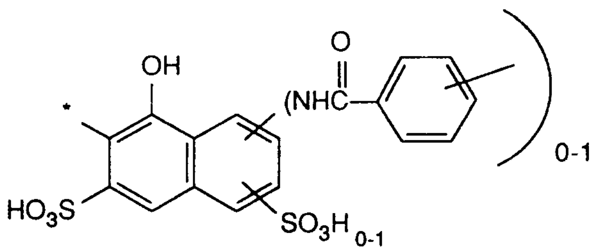

(XII)

wobei der Rest der Formel (XII) und die Azogruppe über die mit * markierte Bindung miteinander verknüpft sind,
B = Rest der Formel

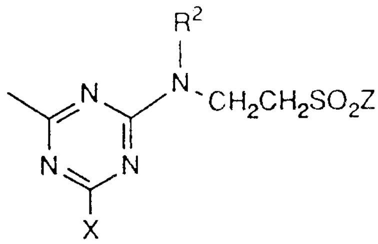

(VIa)

Y = heterocyclischer faserreaktiver Rest, der von B verschieden ist,
$R^0$, $R^1$ = unabhängig voneinander H, gegebenenfalls substituiertes $C_1$-$C_6$-Alkyl, und
$R^2$, X und Z die in Anspruch 1 augegebene Bedeutung haben.

**11.** Reaktivfarbstoffe gemäß Anspruch 10 der Formeln (61) bis (72)

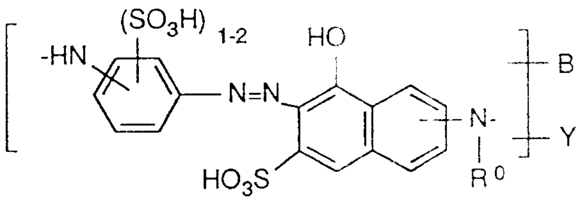

(61)

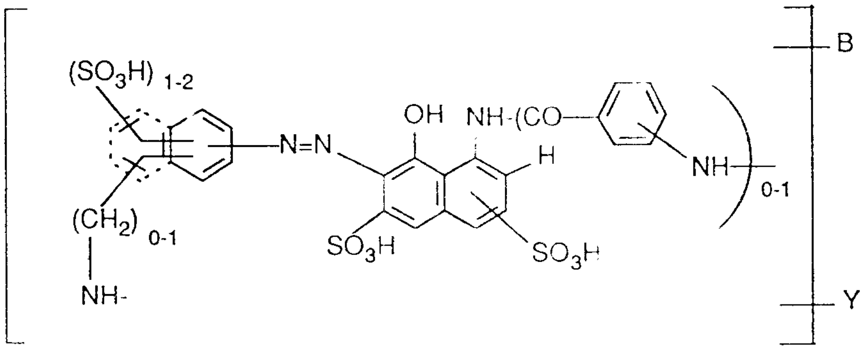

(62)

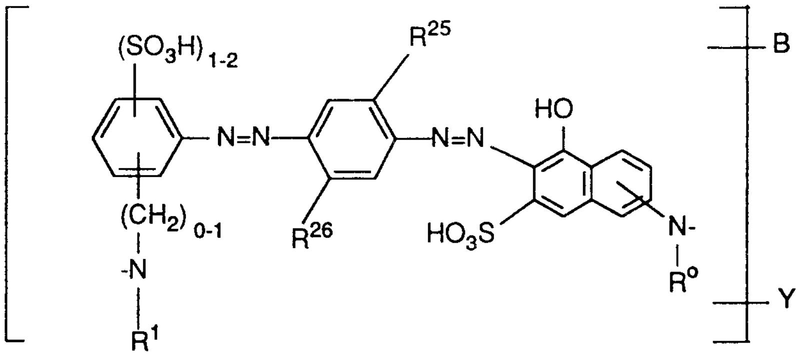

(63)

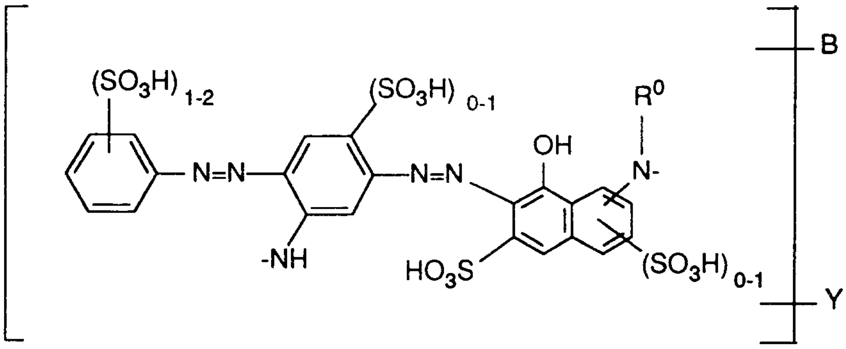

(64)

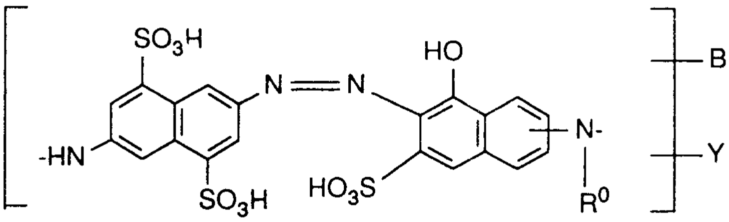

(65)

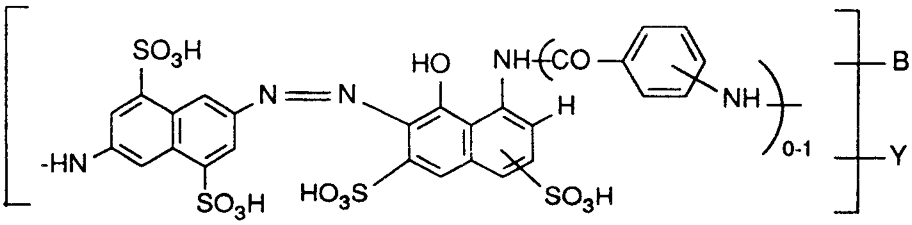

(66)

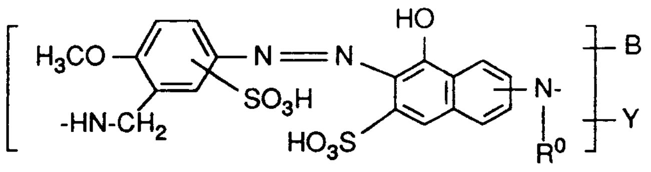
(67)

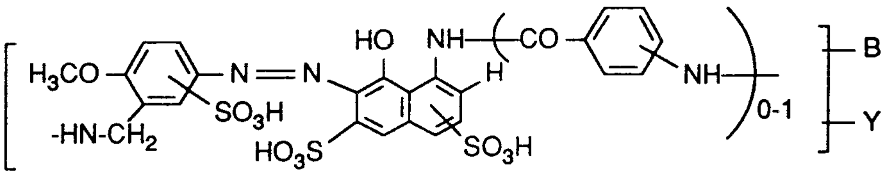
(68)

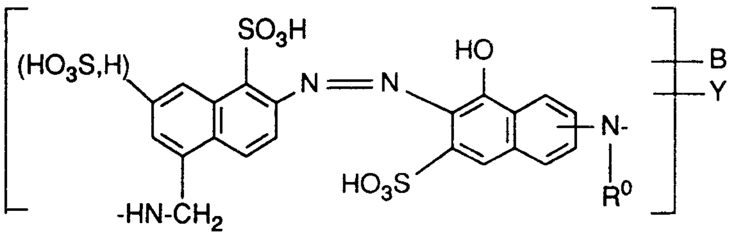
(69)

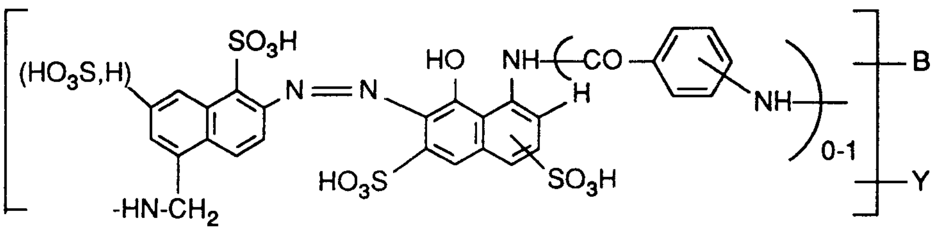
(70)

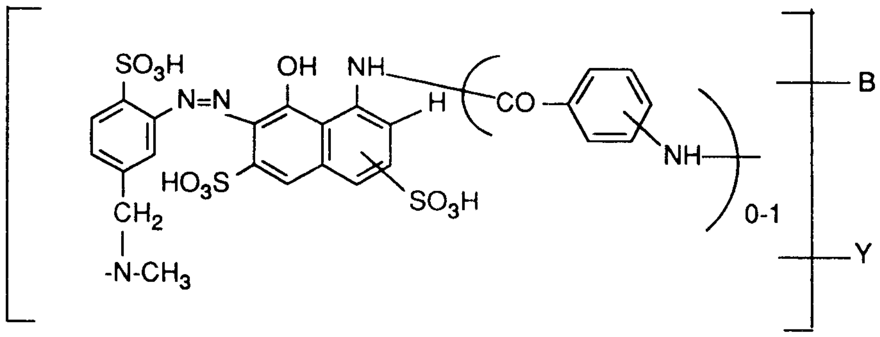
(71)

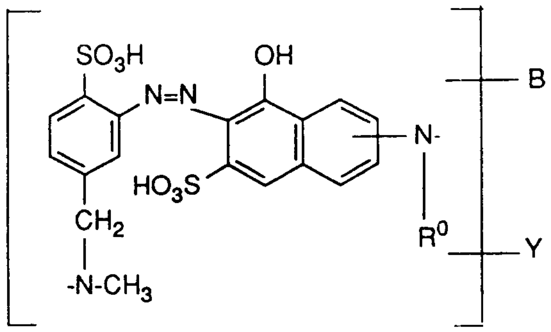

(72)

worin

$R^0$ = H, $CH_3$ oder $C_2H_5$,
$R^{25}$ = H, $C_1$-$C_4$-Alkyl, $C_1$-$C_4$Alkoxy, OH oder $SO_3H$,
$R^{26}$ = H, $C_1$-$C_4$-Alkyl, $C_1$-$C_4$Alkoxy oder Acylamino bedeutet und X und Z die in Anspruch 1 angegebenen Bedeutungen besitzen.

**12.** Verfahren zur Herstellung von Reaktivfarbstoffen der Formel

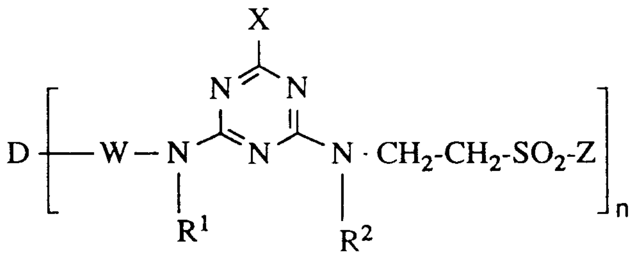

(I),

worin

D Rest eines organischen Farbstoffes aus der Monoazo-, Polyazo-, Metallkomplexazo-, Anthrachinon-, Phthalocyanin-, Formazan-, Azomethin-, Dioxazin-, Phenazin-, Stilben-, Triphenylmethan-, Xanthen-, Thioxanthon- oder der Nitroarylreihe
W direkte Bindung oder Brückenglied,
$R^1$ H oder gegebenenfalls substituiertes $C_1$-$C_4$-Alkyl,
$R^2$ $C_1$-$C_4$-Alkyl, insbesondere Methyl,
X für F, Cl oder Br,
Z für $-CH=CH_2$ oder $-CH_2-CH_2-OSO_3H$ und
n 1 oder 2 bedeutet,

a) entweder durch Kondensation von Farbstoffen der Formel

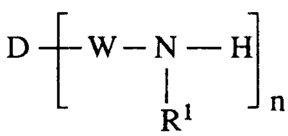

**(II),**

worin

D, W, $R^1$ und n die oben angegebene Bedeutung haben,

mit n-Molen Trihalogentriazinen der Formel

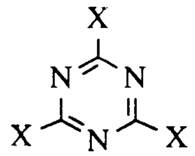

(III)

zu Verbindungen der Formel

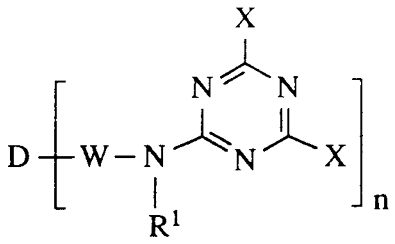

(IV)

und weitere Kondensation der Verbindungen der Formel (IV) mit n-Molen der Komponenten der Formel

$$R^2\text{-NH-CH}_2\text{CH}_2\text{-SO}_2\text{-Z} \qquad (V),$$

worin
$R^2$ und Z die oben angegebene Bedeutung haben,

oder
b) in umgekehrter Reihenfolge durch Kondensation von Trihalogentriazinen der Formel (III) mit den Komponenten der Formel (V) zu den Primärkondensationsprodukten

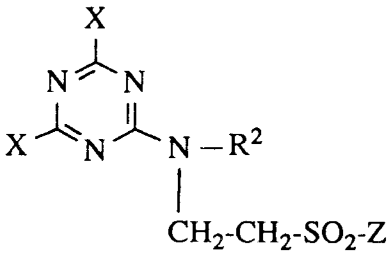

(VI),

worin

$R^2$ und Z die oben angegebene Bedeutung haben,
und weitere Kondensation von n-Molen der Verbindungen der Formel (VI) mit den Farbstoffen der Formel (II),

oder
c) durch Kondensation geeigneter Vorprodukte mit den Trihalogentriazinen (III) und den Komponenten der Formel (V) bzw. durch Kondensation geeigneter Vorprodukte mit den Primärkondensationsprodukten der Formel (VI) und anschließender Farbstoffsynthese.

**13.** Verfahren zur Herstellung von Verbindungen der Formel (V)

$$R^2\text{-NH-}CH_2CH_2\text{-}SO_2\text{-Z}$$

worin

$R^2$ für $C_1$-$C_4$-Alkyl, insbesondere Methyl und
Z für $-CH_2CH_2-OSO_3H$ oder $-CH=CH_2$

steht,
dadurch gekennzeichnet, daß Verbindungen der Formel

$$R^2\text{-NH-}C_2H_4\text{-S-}C_2H_4\text{-OH},$$

die durch Umsetzung von 3-Alkyl-2-oxo-oxazolidinonen der allgemeinen Formel

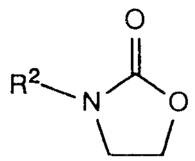

mit 2-Mercaptoethanol erhalten werden durch Oxidation, insbesondere mit $H_2O_2$, in Verbindungen der Formel

$$R^2\text{-NH-}CH_2CH_2SO_2\text{-}CH_2CH_2OH$$

überführt werden und in üblicher Weise zu Verbindungen der allgemeinen Formel (V) umgesetzt werden.

**14.** Verwendung der Reaktivfarbstoffe gemäß Anspruch 1 zum Färben oder Bedrucken von natürlichen oder synthetischen hydroxyl- oder amidgruppenhaltigen Materialien.

**15.** Textilprodukte enthaltend mit Farbstoffen gemäß Anspruch 1 gefärbte hydroxyl- oder amidgruppenhaltige Materialien.

## Claims

**1.** Reactive dyestuffs of the formula

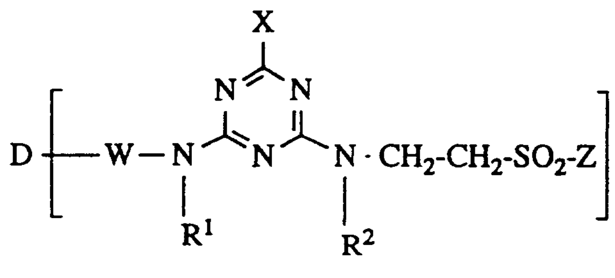

(I),

wherein

D is the radical of an organic dyestuff from the monoazo, polyazo, metal complex azo, anthraquinone, phthalocyanine, formazan, azomethine, dioxazine, phenazine, stilbene, triphenylmethane, xanthene, thioxanthone or the nitroaryl series,

W denotes a direct bond or bridge member,

$R^1$ denotes H or optionally substituted $C_1$-$C_4$-alkyl,

$R^2$ denotes $C_1$-$C_4$-alkyl, in particular methyl,

X denotes F, Cl or Br,

Z denotes $-CH{=}CH_2$ or $-CH_2$-$CH_2$-$OSO_3H$ and

n denotes 1 or 2.

**2.** Reactive dyestuffs according to Claim 1, characterized in that $R^2$ represents methyl and Z represents $CH_2CH_2OSO_3H$.

**3.** Reactive dyestuffs according to Claim 1, characterized in that

D is the radical of an organic dyestuff from the anthraquinone, phthalocyanine, formazan, azomethine, dioxazine, phenazine, stilbene, triphenylmethane, xanthene, thioxanthone or the nitroaryl series.

**4.** Reactive dyestuffs according to at least one of the preceding claims, characterized in that they correspond to one of the following formulae wherein

B' denotes a radical of the formula

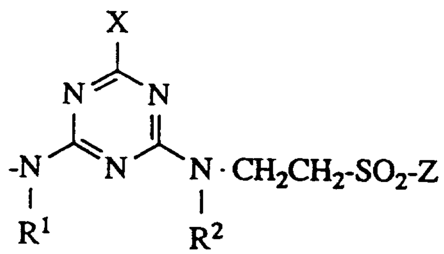

(VIb)

in which the substituents have the meaning given in Claim 1,

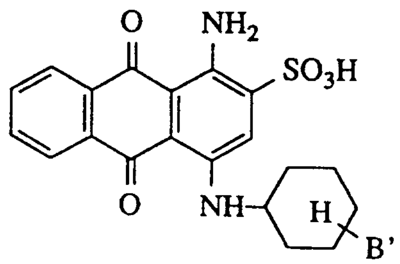

(1)

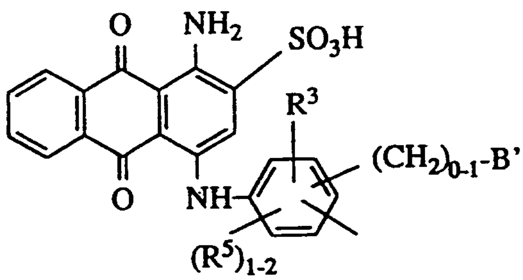

(2)

wherein

$R^3$ = H, methyl, methoxy or chlorine

$R^4$ = H or $SO_3H$ and

$R^5$ = H, methyl or ethyl

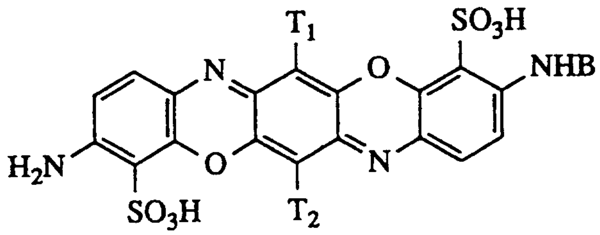

(3a)

wherein

B denotes a radical of the formula

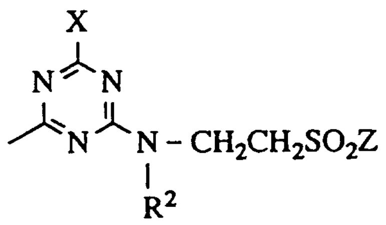

(VIa)

in which the substituents X, $R^2$ and Z have the abovementioned meaning, and

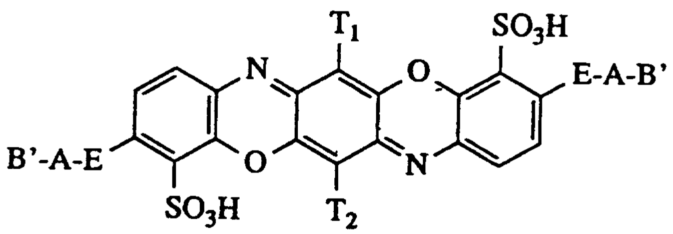

(3b)

wherein

A represents an optionally substituted phenylene or an optionally substituted aromatic-aliphatic bridge member, or represents a straight-chain or branched $C_1$-$C_6$-alkylene which is optionally interrupted by groupings containing heteroatoms, such as $NR^6$, O or S, and can be substituted by $C_1$-$C_6$-alkoxy, $OSO_3H$, $SO_3H$, COOR or halogen, and wherein, within a bridge member A, the group $NR^6$ can also form a heterocyclic aliphatic ring with the group $NR^1$, in particular

E =

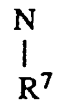

or O and

| | |
|---|---|
| $T_1$ and $T_2$ = | independently of one another H, Cl, Br, $C_1$-$C_2$-alkyl, $OCH_3$, $OC_2H_5$, acylamino, $C_1$-$C_2$-alkoxycarbonyl, |
| $R_6$ and $R_7$ = | independently of one another H or $C_1$-$C_4$-alkyl, which can be substituted by OR, $OSO_3H$, $SO_3H$, COOR or halogen, |
| R = | H, $CH_3$ or $C_2H_5$ |

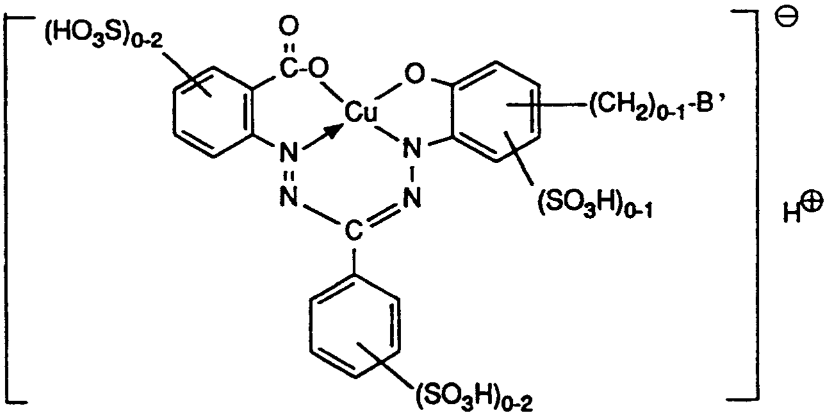

(4)

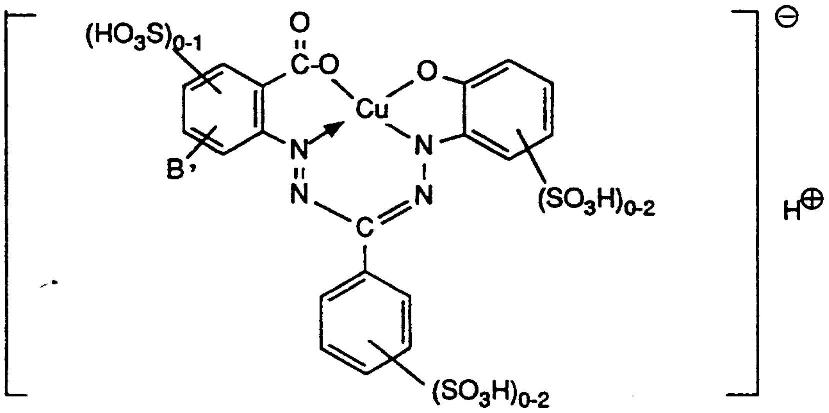

(5)

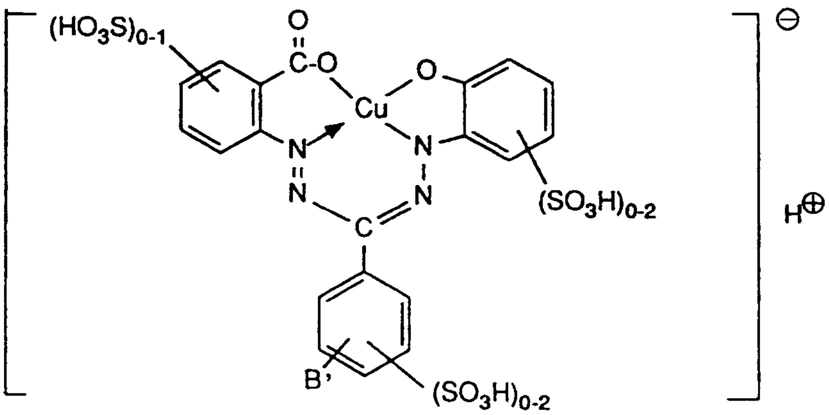

(6)

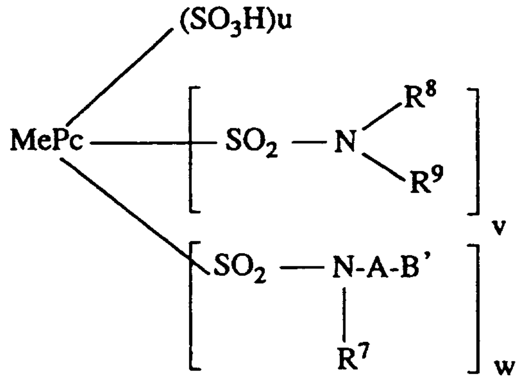

(7)

wherein

Me = Cu or Ni,
Pc = the radical of a phthalocyanine,
u+v+w = 3.4-4.0 , with the proviso that
u = 0.8-2.0,
v = 0-1.0 and
w = 1.0-3.0, and

A has the abovementioned meaning,

$R^7$ has the abovementioned meaning,

$R^8$ and $R^9$ = H or $C_1$-$C_2$-alkyl, which is optionally substituted by OH, $OSO_3H$, $SO_3H$ or COOH,

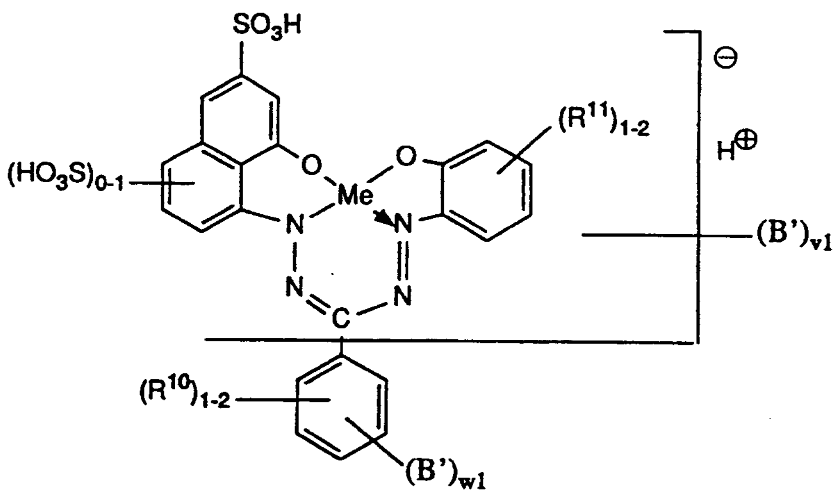

(8)

wherein

v1 and w1 = 0 or 1, where w1 is not identical to v1,

$R^{10}$ and $R^{11}$ = independently of one another H, $C_1$-$C_4$-alkyl, $C_1$-$C_4$-alkoxy, OH, halogen, COOH, $NO_2$, $SO_3H$, sulphonamido, $C_1$-$C_4$-alkylcarbonylamino, optionally substituted phenylcarbonylamino, $C_1$-$C_4$-alkylsulphonylamino or optionally substituted phenylsulphonylamino.

**5.** Reactive dyestuffs according to Claim 1, characterized in that

D is a radical of an organic dyestuff from the monoazo, polyazo or metal complex azo series, which radical contains no further fibre-reactive groups.

6. Reactive dyestuffs according to Claim 5, characterized in that they correspond to one of the following formulae, wherein

B comprises a radical of the formula

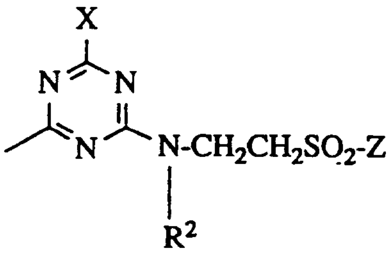

(VIa)

and X, $R^2$ and Z have the meaning given in Claim 1:

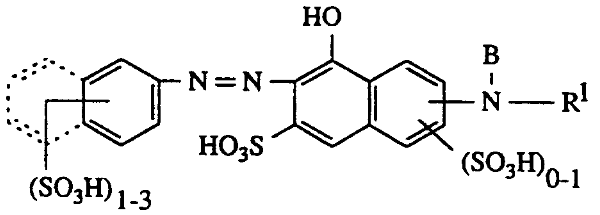

(9)

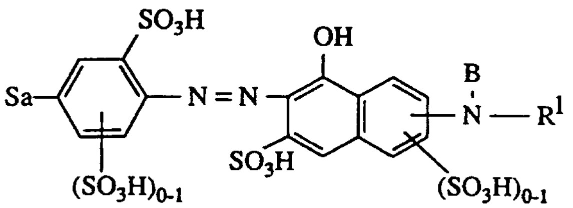

(10)

with Sa = $OCH_3$ or $OC_2H_5$

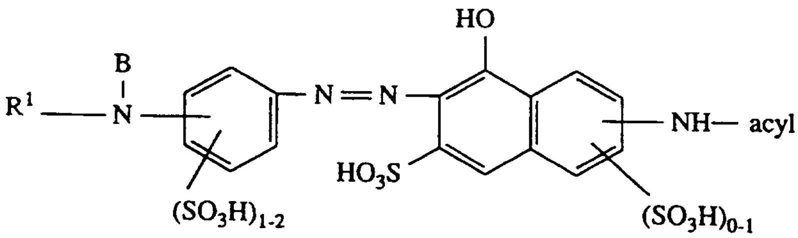

(11)

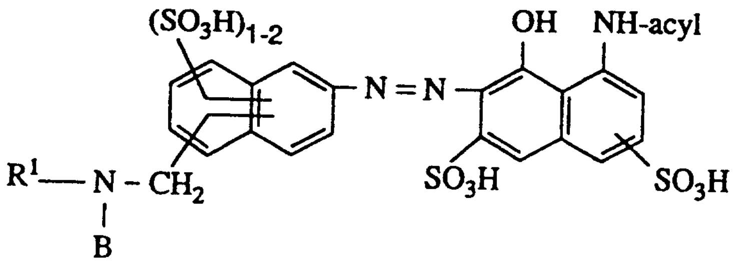

(12)

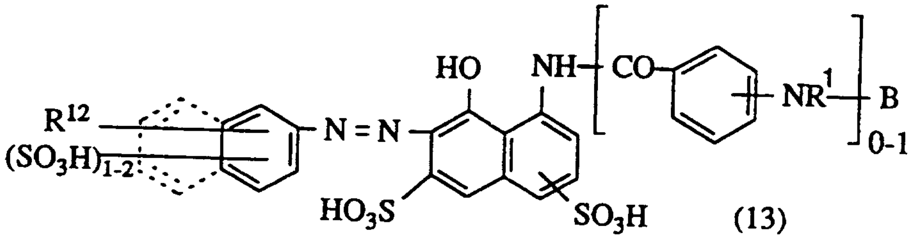

(13)

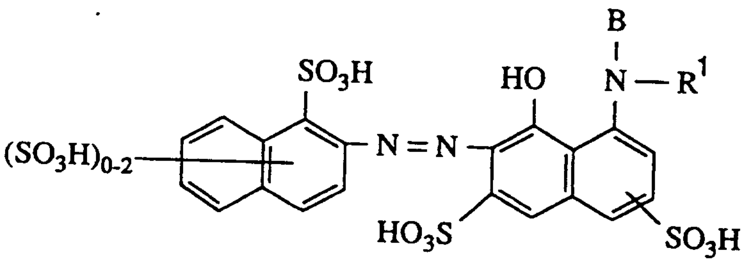

(14)

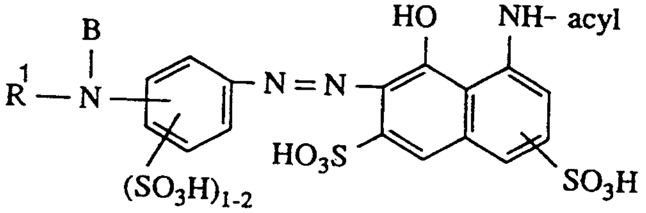

(15)

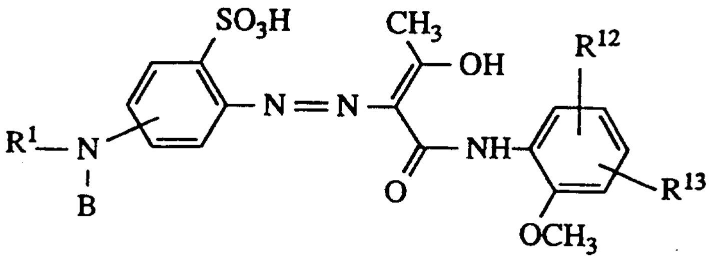

(16)

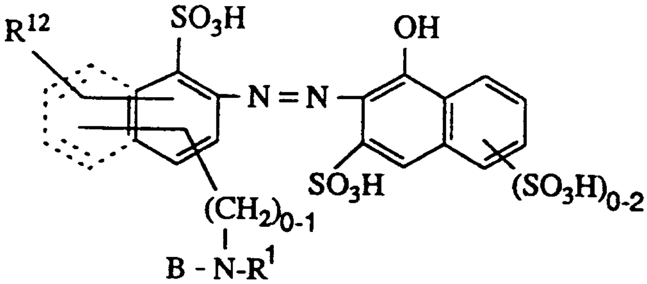

(17)

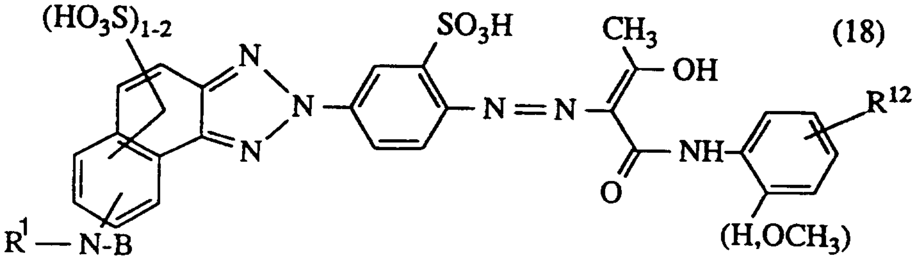

(18)

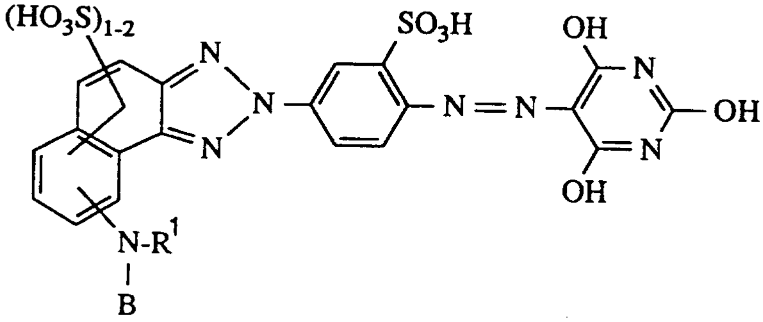

(19)

metal complexes and dyestuffs of the formulae (20) to (50):

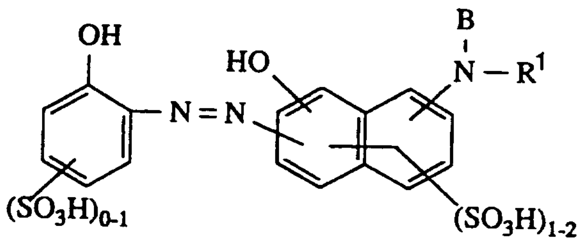

(20)

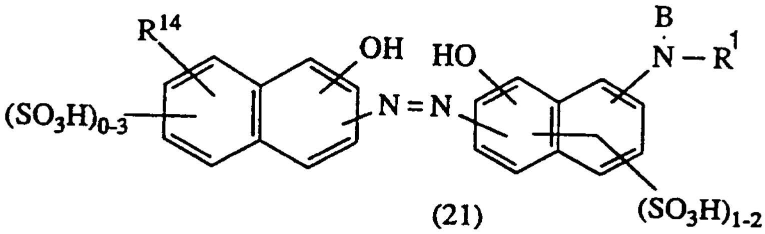

(21)

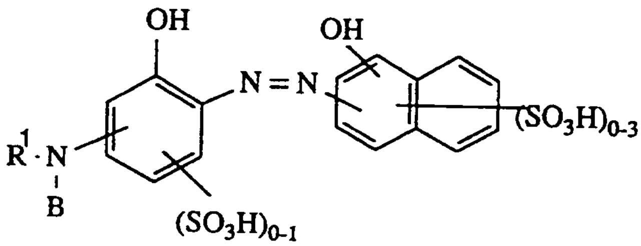

(22)

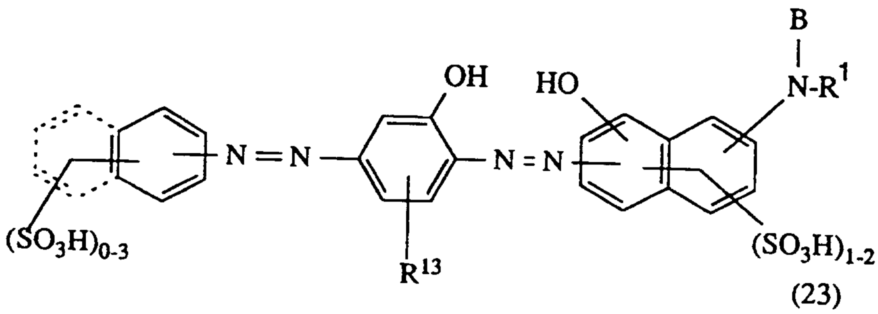

(23)

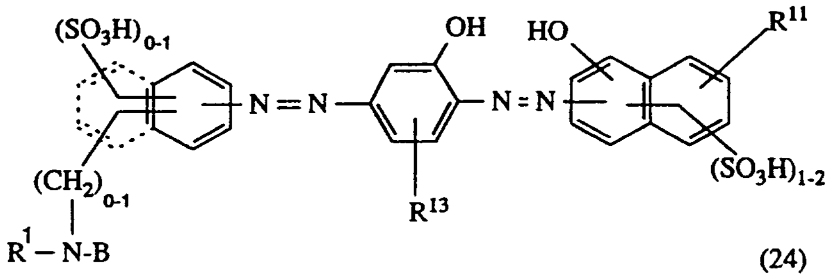

(24)

(25)

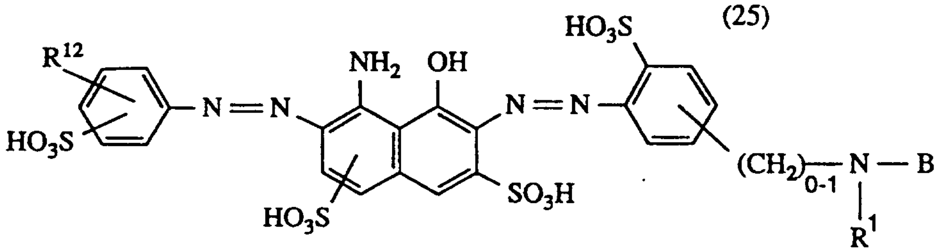

(26)

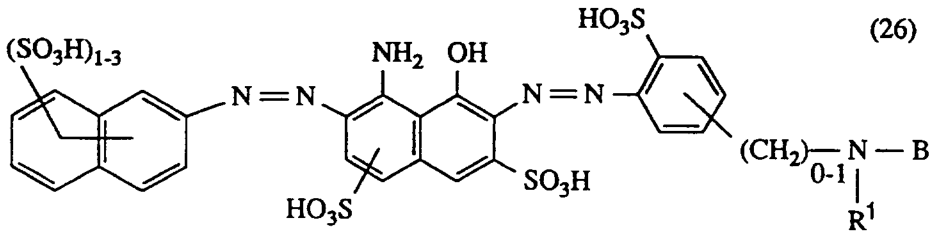

(27)

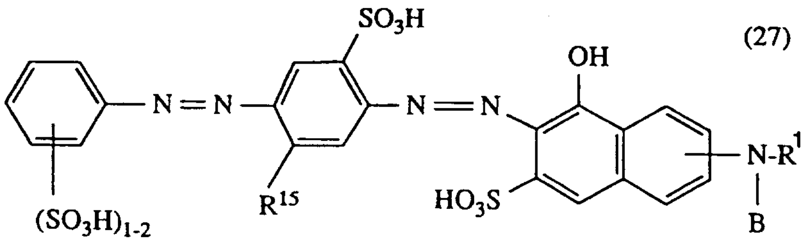

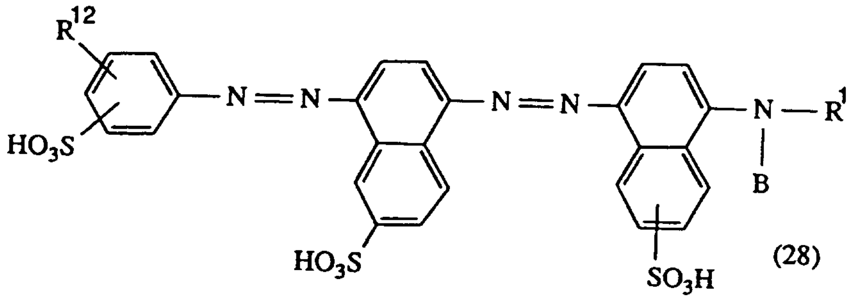

(28)

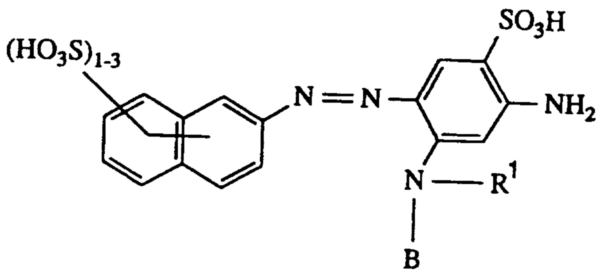

(29)

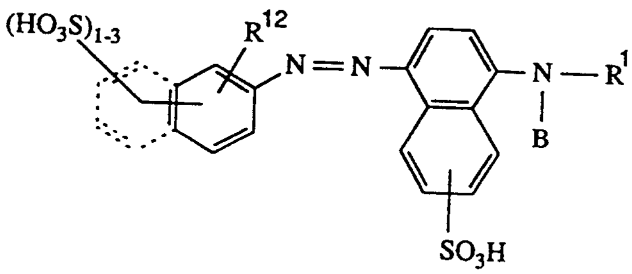

(30)

(31)

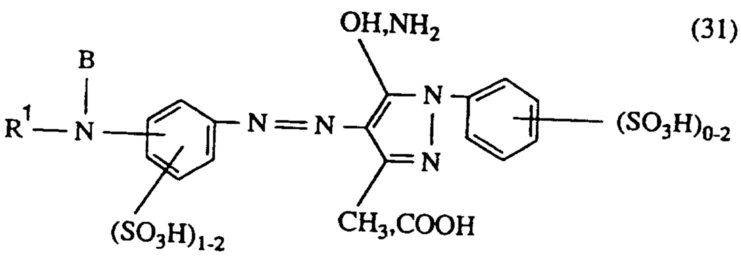

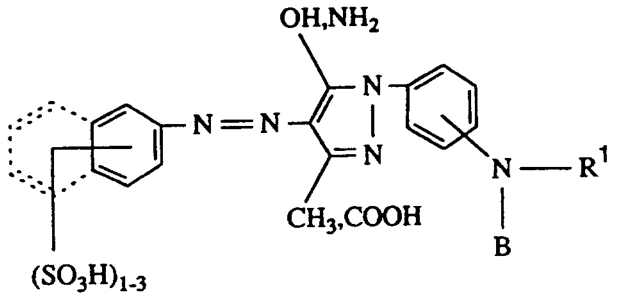

(32)

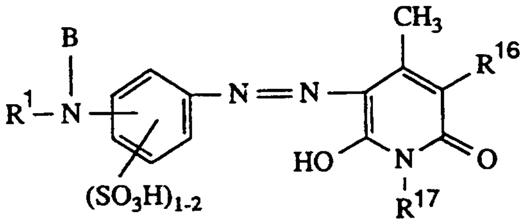

(33)

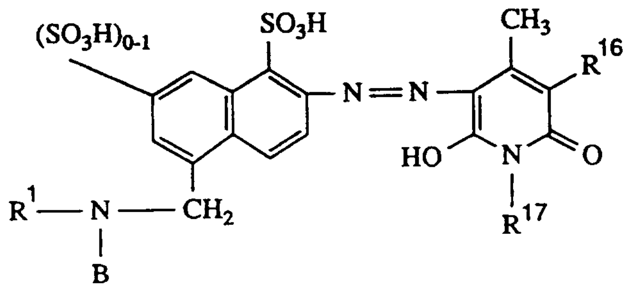

(34)

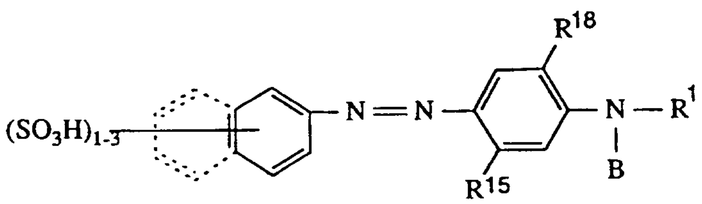

(35)

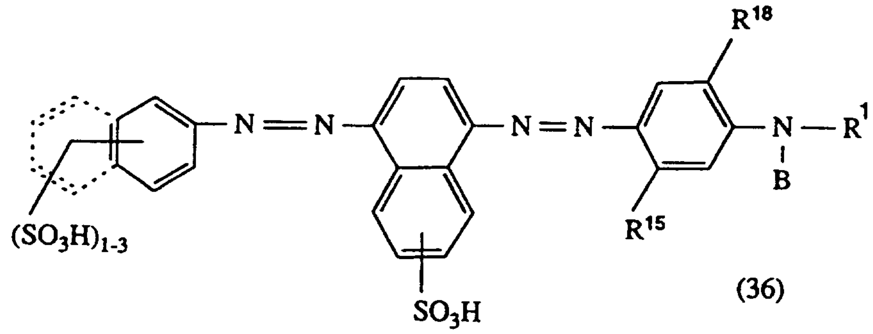

(36)

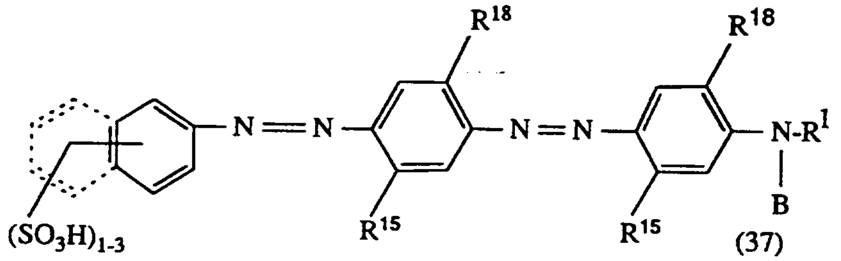

(37)

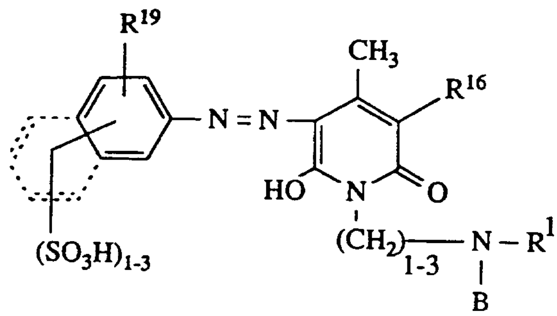

(38)

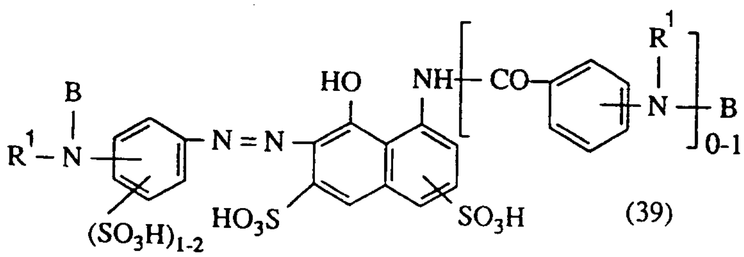

(39)

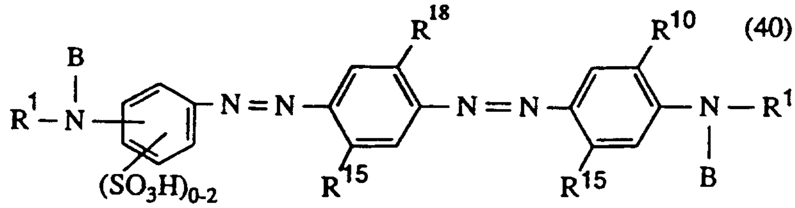

(40)

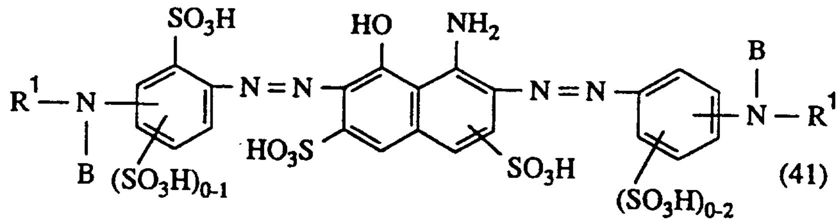

(41)

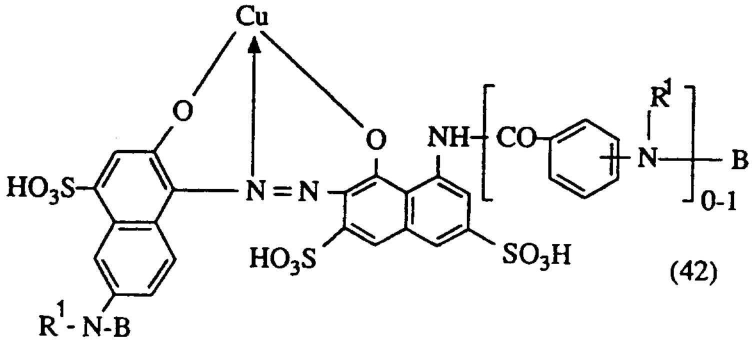

(42)

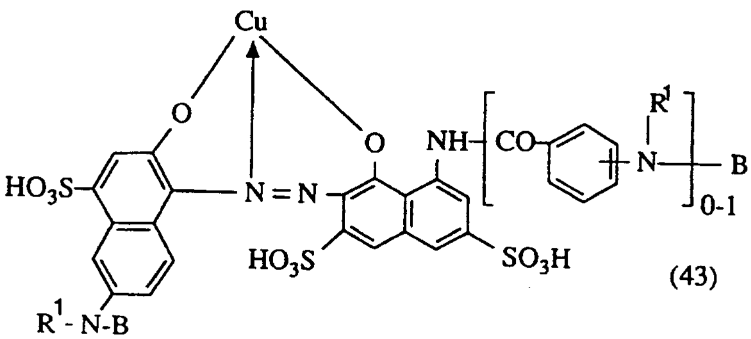

(43)

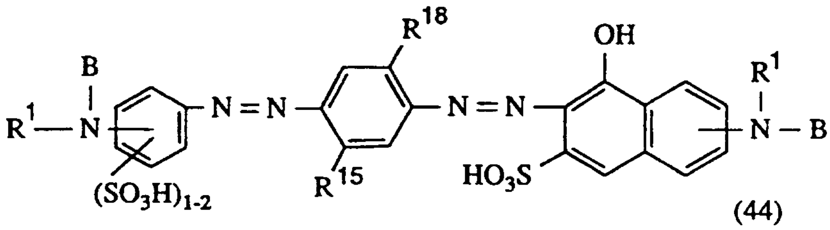

(44)

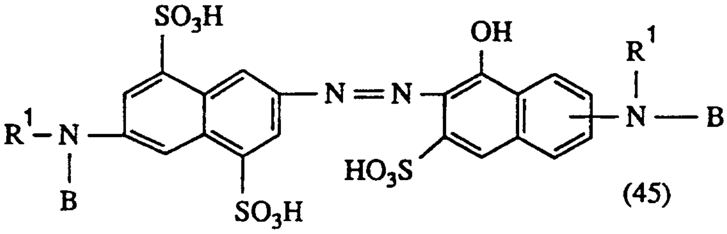

(45)

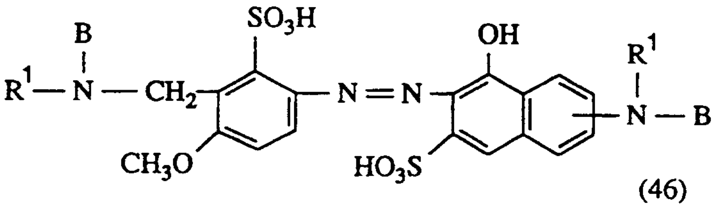

(46)

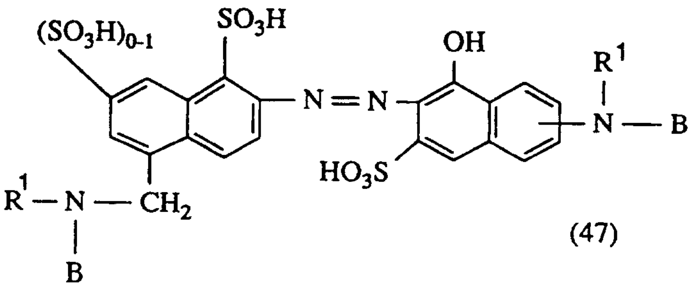

(47)

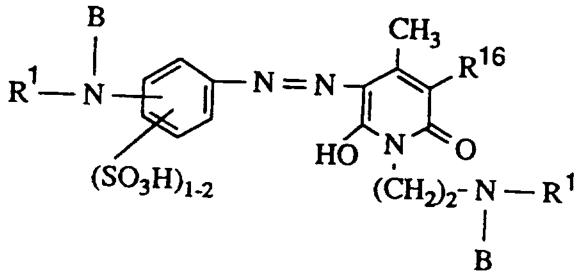

(48)

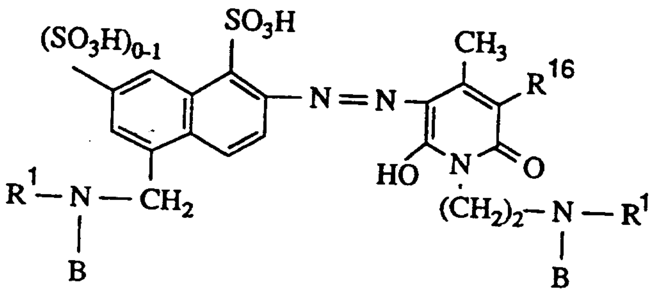

(49)

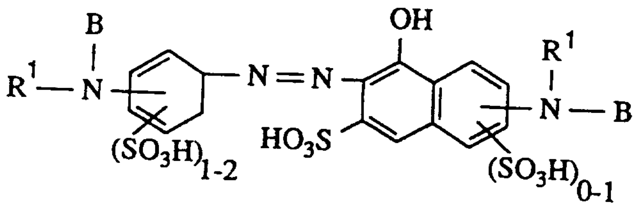

(50),

wherein
acyl is, for example, acetyl or optionally substituted benzoyl,

$R^{17}$ = H or $C_1$-$C_2$-alkyl, optionally substituted by $SO_3H$ or $NH_2$,

$R^1$ = H, $CH_3$ or $C_2H_5$,

$R^{19}$ = H or sulpho,

$R^{13}$ = H, $CH_3$, $OCH_3$ or Cl,

$R^{12}$ = H, $C_1$-$C_4$-alkyl, $C_1$-$C_4$-alkoxy, Cl, Br, COOH or $SO_3H$,

$R^{14}$ = H, OH, $NH_2$, $NHCOCH_3$, NHCOPh, Cl, $C_1$-$C_4$-alkoxy or $C_1$-$C_4$-alkyl,

$R^{16}$ = H, $SO_3H$, $CH_2SO_3H$, Cl, $C_1$-$C_4$-alkylsulphonyl, CN or carboxamide, in particular $CONH_2$,

$R^{15}$ = H, $C_1$-$C_4$-alkyl, $C_1$-$C_4$-alkoxy, Cl, Br or acylamino, in particular $C_1$-$C_4$-alkylcarbonylamino or arylcarbonylamino, such as optionally substituted phenylcarbonylamino, $C_1$-$C_4$-alkylsulphonylamino, aminocarbonylamino, $C_1$-$C_4$-alkylsulphonylamino or arylsulphonylamino,

$R^{18}$ = H, $C_1$-$C_4$-alkyl, $C_1$-$C_4$-alkoxy, OH or $SO_3H$,

and wherein the fused rings indicated by broken lines represent alternatively possible naphthalene systems.

**7.** Reactive dyestuffs according to Claim 1, characterized in that the radical D, preferably a radical of an organic dyestuff from the monoazo or polyazo series, is substituted once or twice by the group

$$Z'\text{-}O_2S\text{-}(CH_2)_{0\text{-}1}\text{-}$$

wherein

Z' denotes $-CH{=}CH_2$, $-CH_2\text{-}CH_2\text{-}OSO_3H$, $-CH_2\text{-}CH_2\text{-}Cl$, $-CH_2\text{-}CH_2\text{-}Br$, $-CH_2\text{-}CH_2\text{-}S_2O_3H$, $-CH_2\text{-}CH_2\text{-}O\text{-}CO\text{-}CH_3$, $-CH_2\text{-}CH_2\text{-}OPO_3H_2$ or $-CH_2\text{-}CH_2\text{-}OH$.

**8.** Reactive dyestuffs according to Claim 7, characterized in that

n = 1,

W = a direct bond and

D is a radical of the general formula (IX)

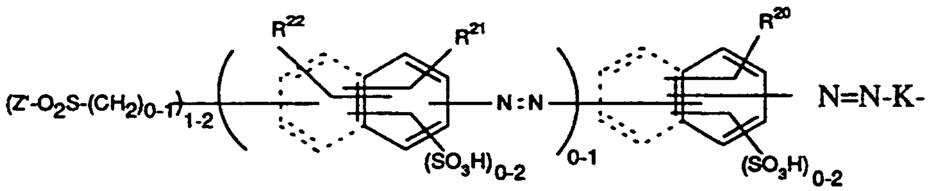

(IX)

wherein

$R^{20}$ denotes H, $C_1$-$C_4$-alkyl, $C_1$-$C_4$-alkoxy, Cl, Br or acylamino,

$R^{21}$ denotes H, $C_1$-$C_4$-alkyl, Cl, Br, $C_1$-$C_4$-alkoxy or COOH,

$R^{22}$ denotes H, $C_1$-$C_4$-alkyl, $C_1$-$C_4$-alkoxy, $SO_3H$, Cl or Br,

K denotes a bivalent radical of the general formulae (Xa) - (Xd)

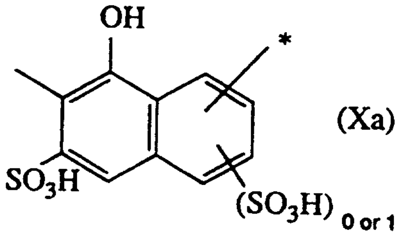

(Xa) ,

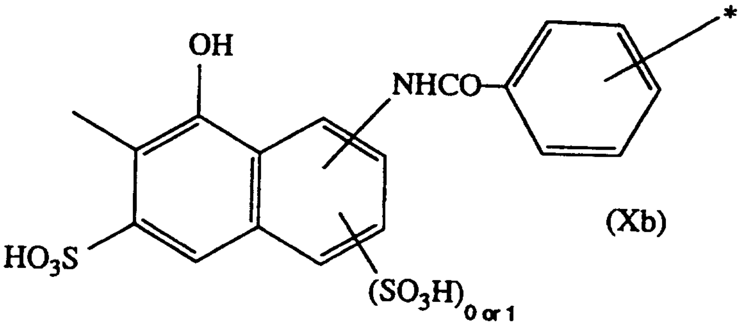

(Xb)

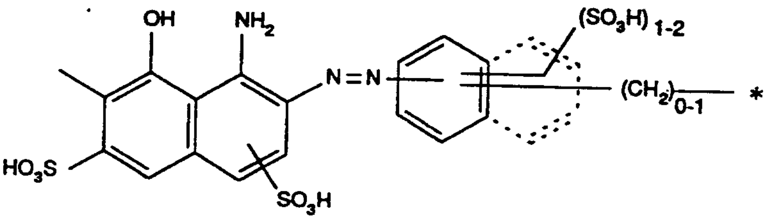

(Xc) ,

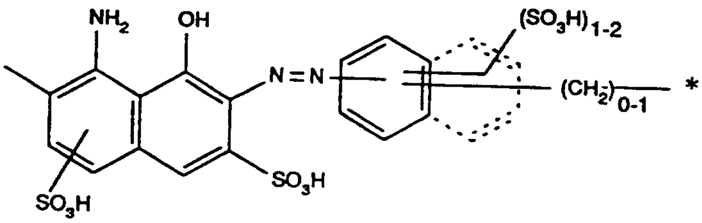

(Xd) ,

and where the bonds identified with * are bonded to the group $-NR^1$-B and B has the meaning given in Claim 4.

**9.** Reactive dyestuffs according to Claim 7, characterized in that they correspond to one of the following formulae, wherein

B comprises a radical of the formula

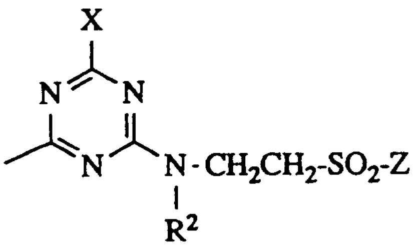

(VIa)

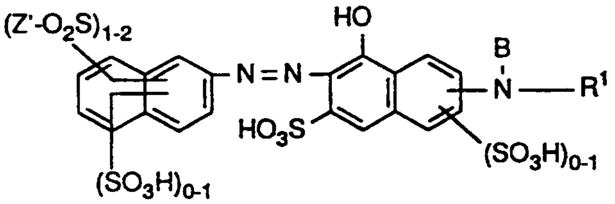

(51),

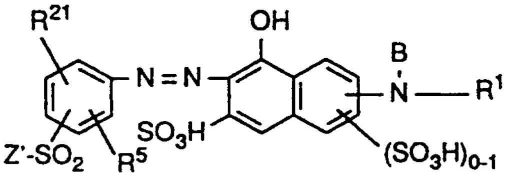

(52),

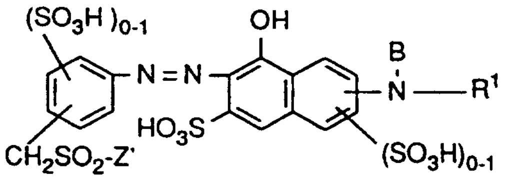

(53),

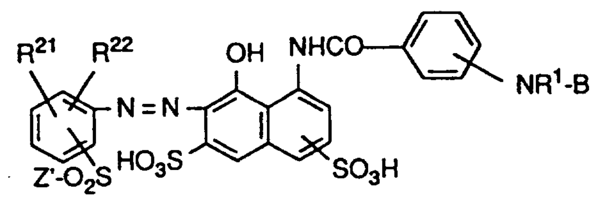

(54),

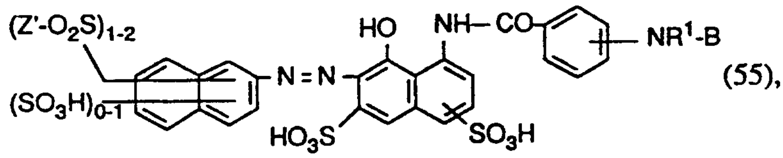

(55),

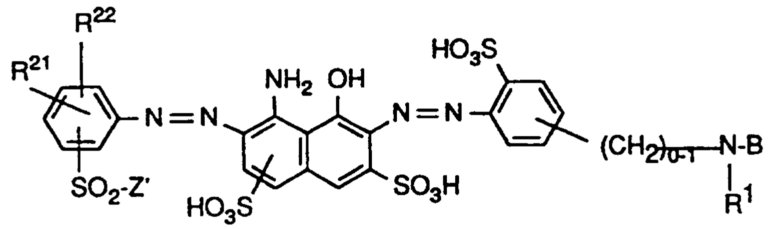

(56),

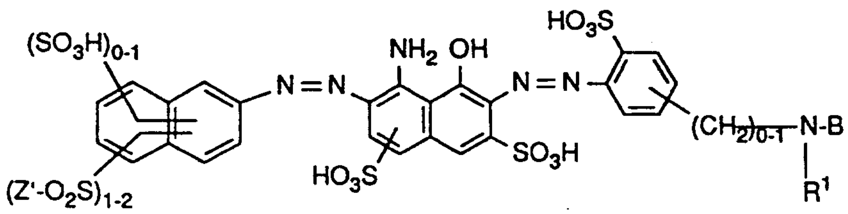

(57),

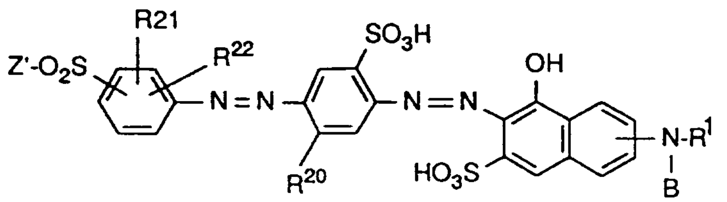

(58),

wherein

$R^1$ = H, $CH_3$ or $C_2H_5$,

$R^{20}$ = H, $C_1$-$C_4$-alkyl, $C_1$-$C_4$-alkoxy, acylamino, Cl or Br,

$R^{21}$ = H, $C_1$-$C_4$-alkyl, $C_1$-$C_4$-alkoxy, Cl, Br or COOH,

$R^{22}$ = H, $C_1$-$C_4$-alkyl, $C_1$-$C_4$-alkoxy, Cl, Br or $SO_3H$.

**10.** Reactive dyestuffs according to Claim 1, of the formula

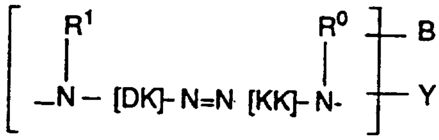

(XI)

wherein

DK = the radical of a diazo component of the benzene or naphthalene series,

KK = the radical of a coupling component of the formula

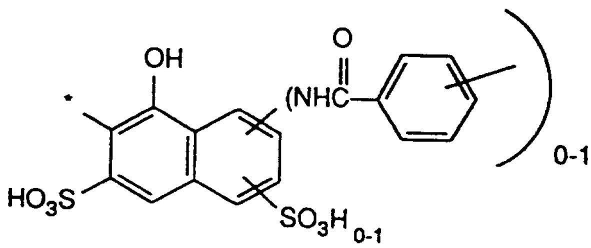

(XII)

wherein the radical of the formula (XII) and the azo group are linked to one another via the bond marked with *,

B = the radical of the formula

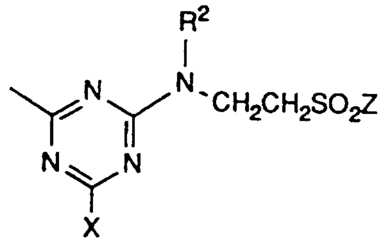

(VIa)

Y = a heterocyclic fibre-reactive radical which differs from B,

$R^0$ and $R^1$ = independently of one another H or optionally substituted $C_1$-$C_6$-alkyl, and $R^2$, X and Z have the meaning given in Claim 1.

**11.** Reactive dyestuffs according to Claim 10 of the formulae (61) to (72)

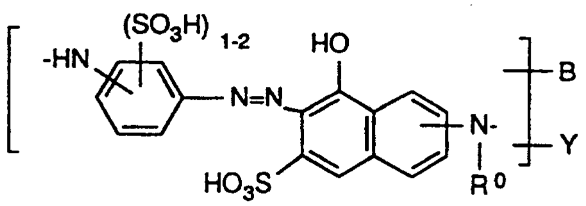

(61)

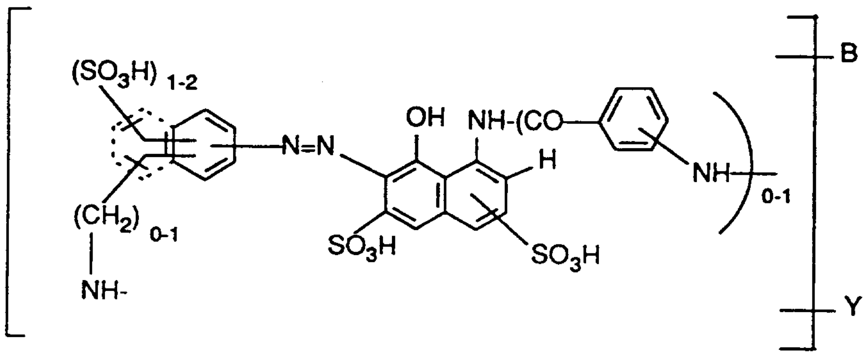

(62)

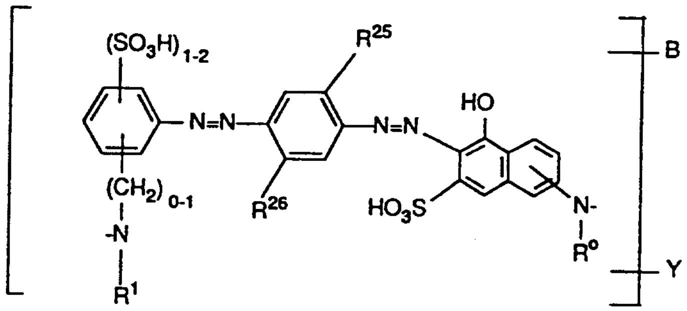

(63)

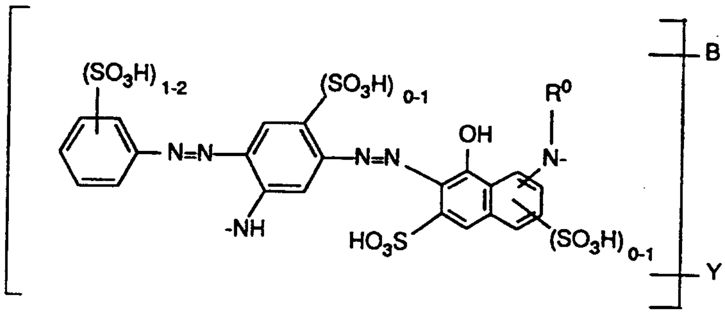

(64)

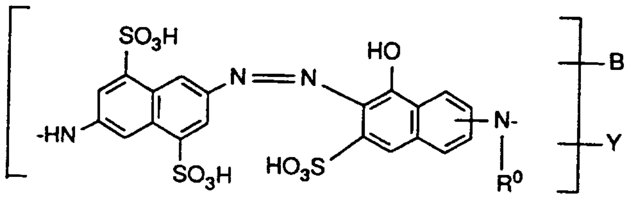

(65)

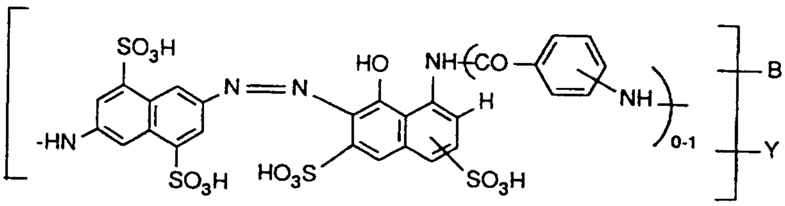

(66)

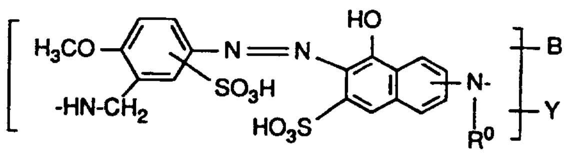
(67)

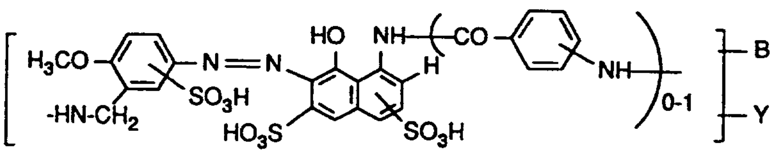
(68)

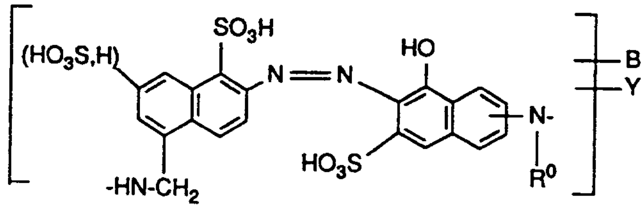
(69)

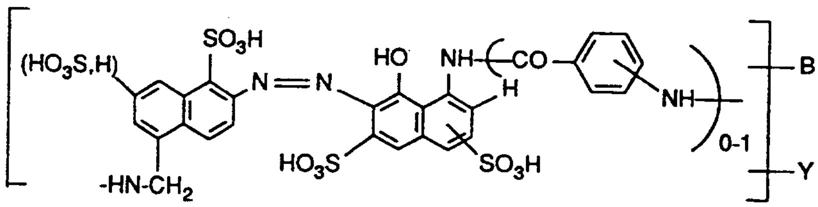
(70)

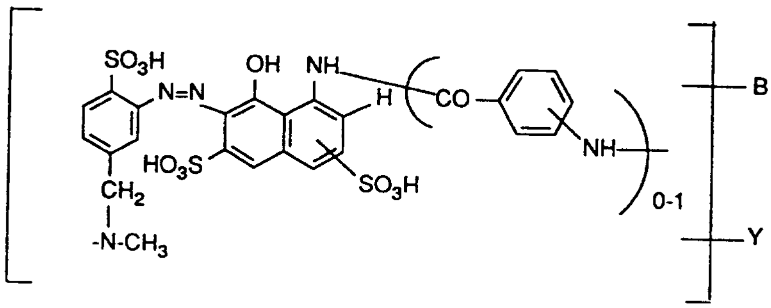
(71)

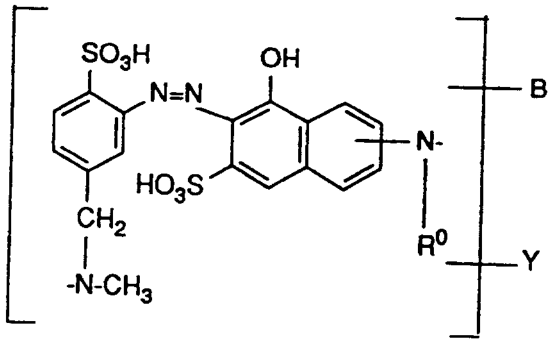

(72)

wherein

$R^0$ = H, $CR_3$ or $C_2H_5$,

$R^{25}$ = H, $C_1$-$C_4$-alkyl, $C_1$-$C_4$-alkoxy, OH or $SO_3H$,

$R^{26}$ denotes H, $C_1$-$C_4$-alkyl, $C_1$-$C_4$-alkoxy or acylamino and X and Z have the meanings given in Claim 1.

**12.** Process for the preparation of reactive dyestuffs of the formula

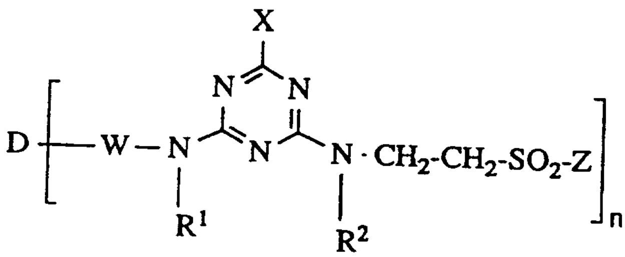

(I),

wherein

D is the radical of an organic dyestuff from the monoazo, polyazo, metal complex azo, anthraquinone, phthalocyanine, formazan, azomethine, dioxazine, phenazine, stilbene, triphenylmethane, xanthene, thioxanthone or the nitroaryl series,

W denotes a direct bond or bridge member,

$R^1$ denotes H or optionally substituted $C_1$-$C_4$-alkyl,

$R^2$ denotes $C_1$-$C_4$-alkyl, in particular methyl,

X denotes F, Cl or Br,

Z denotes $-CH=CH_2$ or $-CH_2-CH_2-OSO_3H$ and

n denotes 1 or 2,

a) either by condensation of dyestuffs of the formula

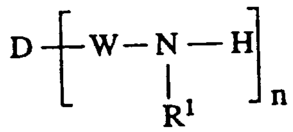

(II),

wherein

D, W, $R^1$ and n have the abovementioned meaning,

with n moles of trihalogenotriazines of the formula

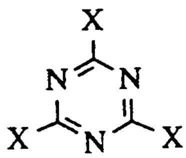

(III)

to give compounds of the formula

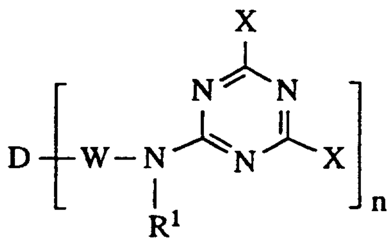

(IV)

and further condensation of the compounds of the formula (IV) with n moles of the components of the formula

$$R^2\text{-NH-CH}_2\text{CH}_2\text{-SO}_2\text{-Z} \quad (V),$$

wherein

$R^2$ and Z have the abovementioned meaning,

or

b) in the reverse sequence, by condensation of trihalogenotriazines of the formula (III) with the components of the formula (V) to give the primary condensation products

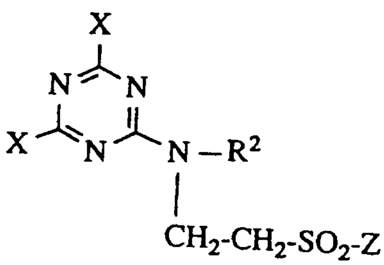

(VI),

wherein

$R^2$ and Z have the abovementioned meaning,
and further condensation of n moles of the compounds of the formula (VI) with the dyestuffs of the formula (II)

or

c) by condensation of suitable precursors with the trihalogenotriazines (III) and the components of the formula (V) or by condensation of suitable precursors with the primary condensation products of the formula (VI) and subsequent dyestuff synthesis.

**13.** Process for the preparation of compounds of the formula (V)

$$R^2\text{-NH-}CH_2CH_2\text{-}SO_2\text{-Z}$$

wherein

$R^2$ represents $C_1$-$C_4$-alkyl, in particular methyl, and

Z represents $-CH_2CH_2-OSO_3H$ or $-CH=CH_2$,

characterized in that compounds of the formula

$$R^2\text{-NH-}C_2H_4\text{-S-}C_2H_4\text{-OH,}$$

which are obtained by reaction of 3-alkyl-2-oxo-oxazolidinones of the general formula

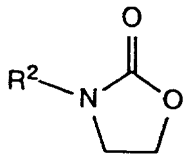

with 2-mercaptoethanol, are converted by oxidation, in particular with $H_2O_2$, into compounds of the formula

$$R^2\text{-NH-}CH_2CH_2SO_2\text{-}CH_2CH_2OH$$

and these are then converted into compounds of the general formula (V) in the customary manner.

**14.** Use of the reactive dyestuffs according to Claim 1 for dyeing or printing naturally occurring or synthetic materials containing hydroxyl groups or amide groups.

**15.** Textile products comprising materials containing hydroxyl groups or amide groups which have been dyed with dyestuffs according to Claim 1.

**Revendications**

**1.** Colorants réactifs de formule

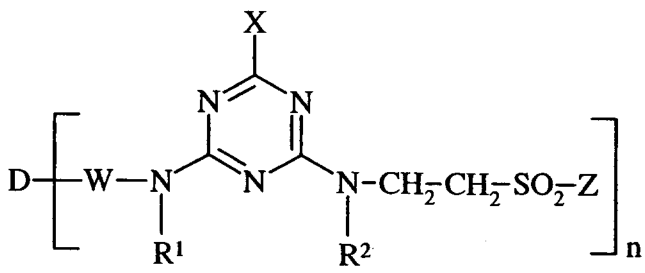

(I),

dans laquelle

D représente le radical d'un colorant organique des classes des colorants monoazoïques, polyazoïques, azoïques-complexes métalliques, anthraquinoniques, des colorants de phtalocyanine, des colorants de formazane, des colorants d'azométhine, de dioxazine, de phénazine, des colorants stilbéniques, des colorants de triphénylméthane, de xanthène, de thioxanthone ou des colorants nitroaryliques,

W représente une liaison directe ou un pont,

$R^1$ représente H ou un groupe alkyle en $C_1$-$C_4$ éventuellement substitué,

$R^2$ représente un groupe alkyle en $C_1$-$C_4$, plus spécialement un groupe méthyle,

X représente F, Cl ou Br,

Z représente $-CH=CH_2$ ou $-CH_2-CH_2-OSO_3H$ et

n est égal à 1 ou 2.

**2.** Colorants réactifs selon la revendication 1, caractérisés en ce que $R^2$ représente un groupe méthyle et Z représente $CH_2CH_2OSO_3H$.

**3.** Colorants réactifs selon la revendication 1, caractérisés en ce que

D représente le radical d'un colorant organique des classes des colorants anthraquinoniques, de phtalocyanines, de formazane, d'azométhine, de dioxazine, de phénazine, des colorants stilbéniques, des colorants du triphénylméthane, de xanthène, de thioxanthone ou des colorants nitroaryliques.

**4.** Colorants réactifs selon au moins une des revendications qui précèdent, caractérisés en ce qu'ils répondent à l'une des formules ci-après dans lesquelles

B' représente un groupe de formule

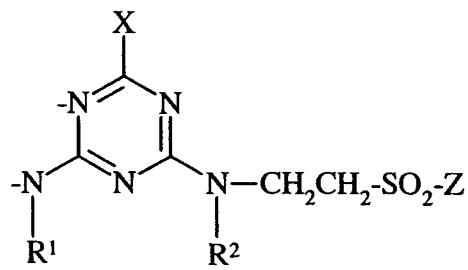

(VIb)

dans laquelle les symboles ont les significations indiquées dans la revendication 1,

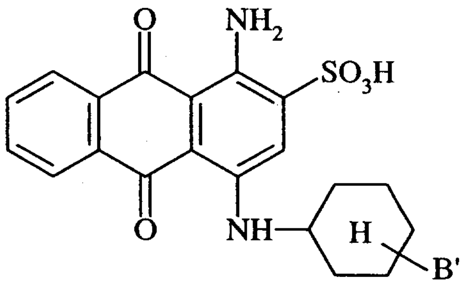

(1)

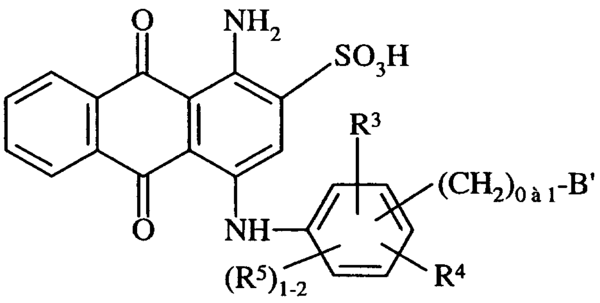

(2)

dans laquelle

$R^3$ = H, méthyle, méthoxy, chlore

$R^4$ = H, $SO_3H$ et

$R^5$ = H, méthyle ou éthyle

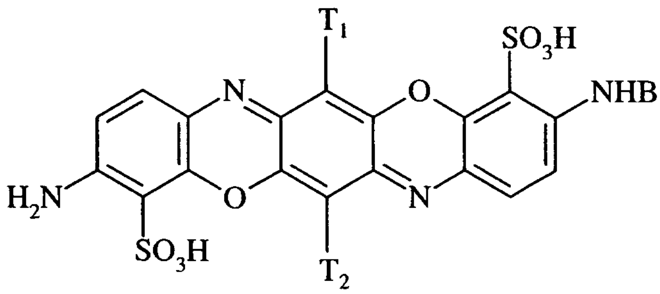

(3a)

dans laquelle

B représente un groupe de formule

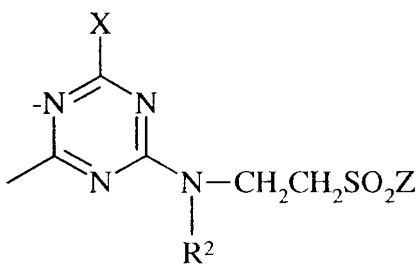

(VIa)

dans laquelle les symboles X, $R^2$ et Z ont les significations indiquées ci-dessus, et

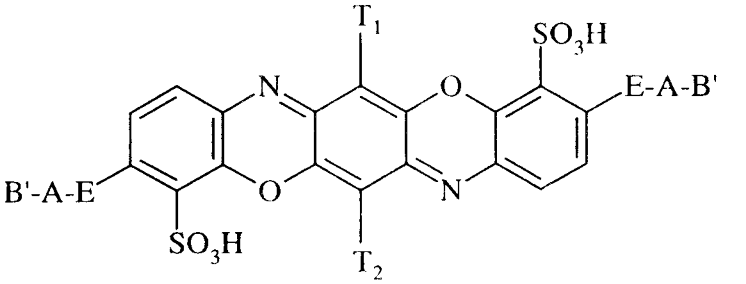

(3b)

dans lesquelles

A représente un groupe phénylène éventuellement substitué ou un pont aromatique-aliphatique éventuellement substitué ou un groupe alkylène à chaîne droite ou ramifiée en $C_1$-$C_6$, éventuellement interrompu par des groupements contenant des hétéroatomes, tels que $NR^6$, O ou S, et qui peut porter des substituants alcoxy en $C_1$-$C_6$, $OSO_3H$, $SO_3H$, COOR ou halogéno, le groupe $NR^6$ pouvant également former avec le groupe $NR^1$, à l'intérieur d'un pont A, un cycle aliphatique hétérogène, en particulier

E =

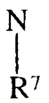

,

O et

$T_1$, $T_2$ = indépendamment l'un de l'autre, H, Cl, Br, alkyle en $C_1$-$C_2$, $OCH_3$, $OC_2H_5$, acylamino, (alcoxy en $C_1$-$C_2$)carbonyle,

$R^6$, $R^7$ = indépendamment l'un de l'autre, H, alkyle en $C_1$-$C_4$, lequel peut être substitué par OR, $OSO_3H$, $SO_3H$, COOR ou des halogènes,

R = H, $CH_3$ ou $C_2H_5$

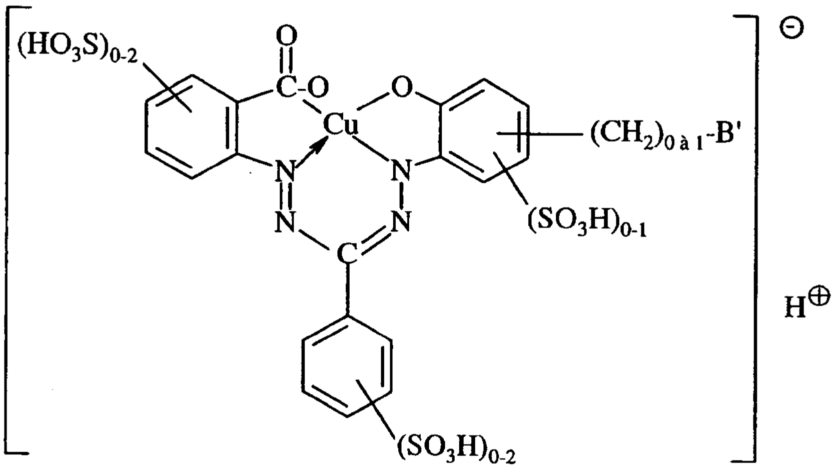

(4)

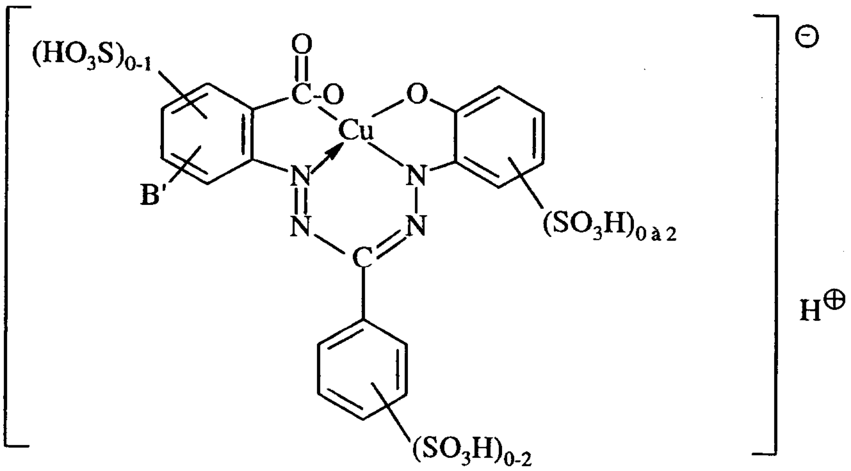

(5)

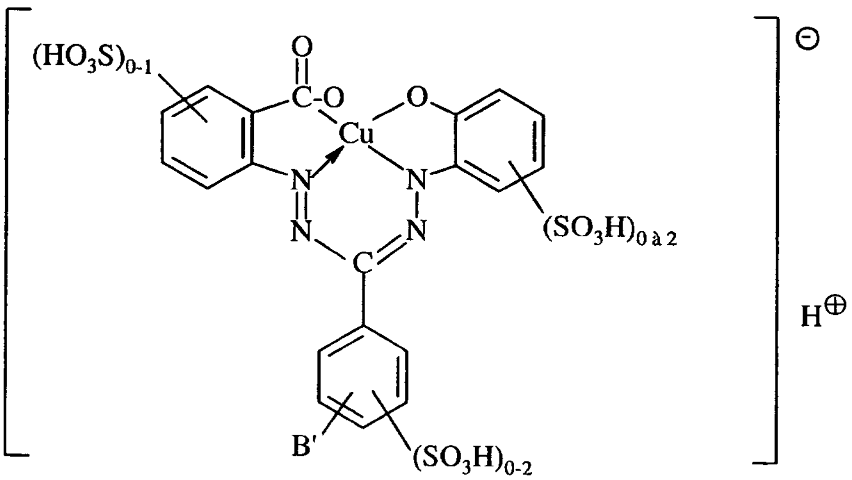

(6)

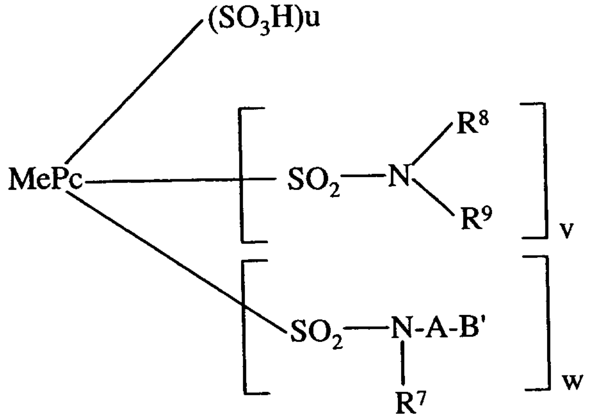

(7)

dans lesquelles

Me = Cu, Ni,
Pc = radical d'une phtalocyanine
u+v+w = 3,4 à 4,0 , sous réserve que
u = 0,8 à 2,0,
v = 0 à 1,0,
w = 1,0 à 3,0 et

A a les significations indiquées ci-dessus,
$R^7$ a les significations indiquées ci-dessus,
$R^8$ et $R^9$ = H, alkyle en $C_1$-$C_2$ éventuellement substitué par OH, $OSO_3H$, $SO_3H$ ou COOH.

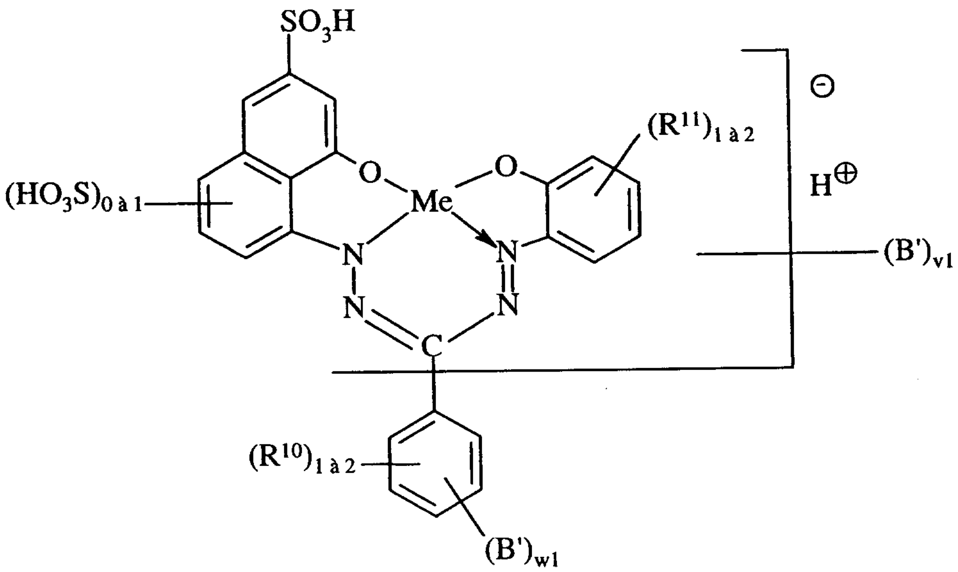

(8)

dans laquelle

v1, w1 = 0 ou 1, w1 étant différent de v1,
$R^{10}$, $R^{11}$ = indépendamment l'un de l'autre, H, alkyle en $C_1$-$C_4$, alcoxy en $C_1$-$C_4$, OH, halogène, COOH, $NO_2$, $SO_3H$, sulfonamido, (alkyle en $C_1$-$C_4$)carbonylamino, phénylcarbonylamino éventuellement substitué, alkylsulfonylamino en $C_1$-$C_4$, phénylsulfonylamino éventuellement substitué.

**5.** Colorants réactifs selon la revendication 1, caractérisés en ce que

D est le radical d'un colorant organique appartenant aux classes des colorants monoazoïques, polyazoïques, azoïques-complexes métalliques et qui ne contient pas d'autres groupes réactifs avec les fibres.

**6.** Colorants réactifs selon la revendication 5, caractérisés en ce qu'ils répondent à l'une des formules suivantes dans lesquelles

B représente un groupe de formule

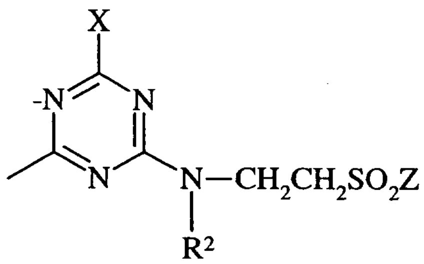

(VIa)

et X, $R^2$ et Z ont les significations indiquées dans la revendication 1 :

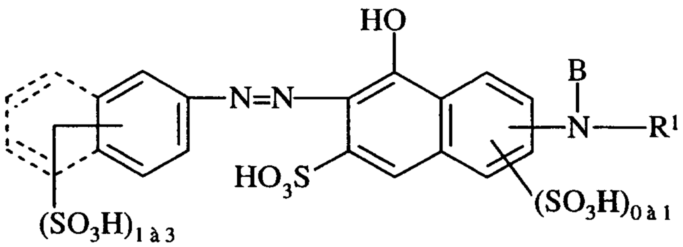

(9)

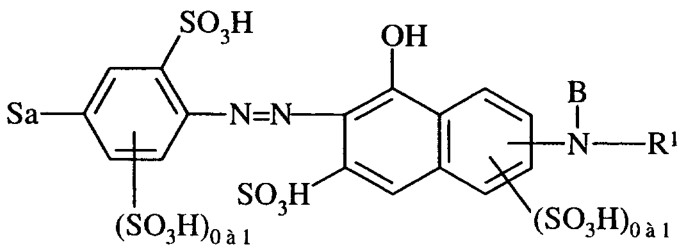

(10)

avec Sa = $OCH_3$ ou $OC_2H_5$

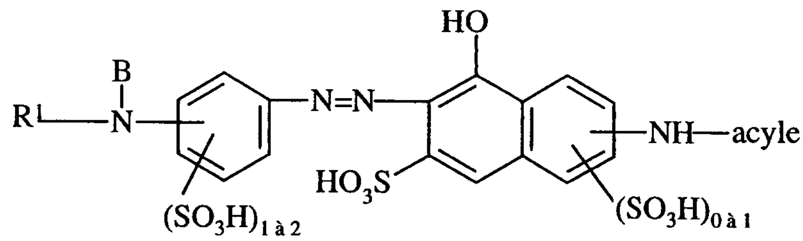

(11)

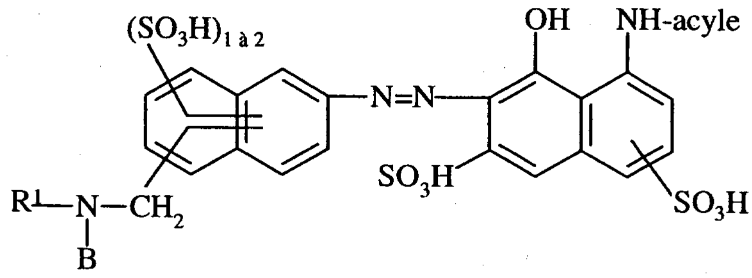

(12)

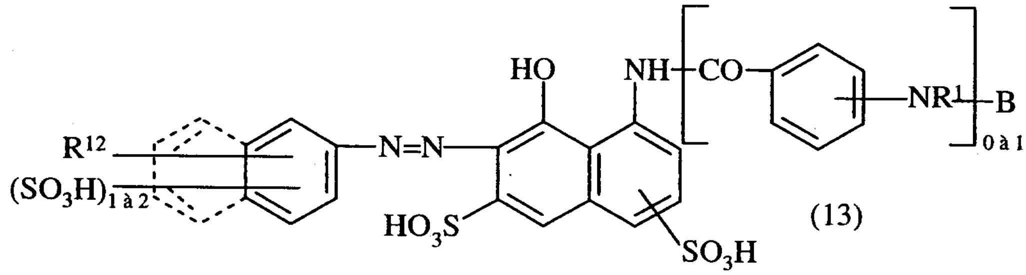

(13)

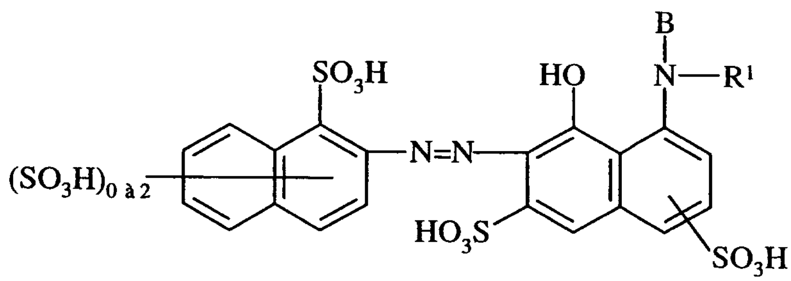

(14)

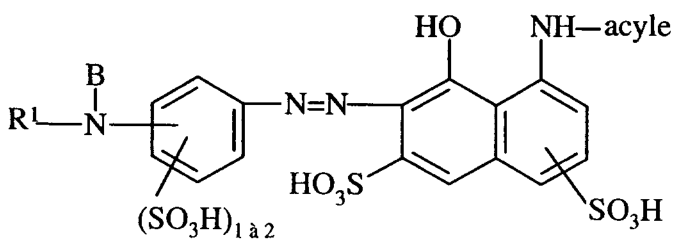

(15)

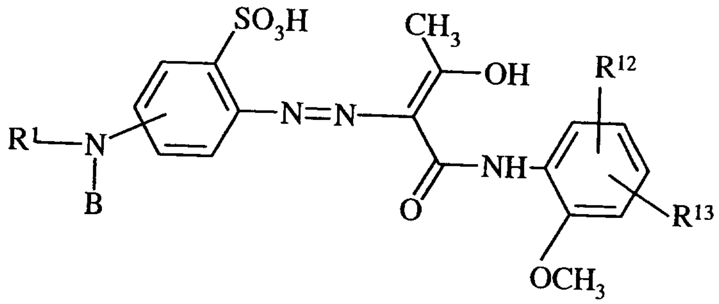

(16)

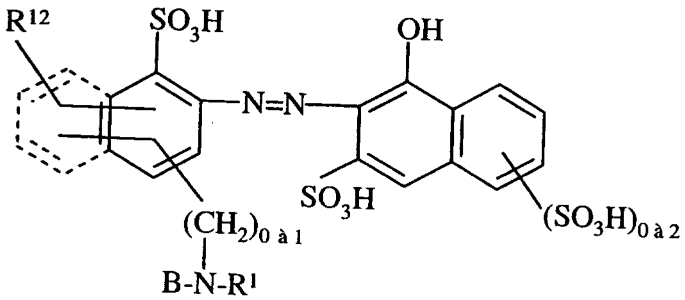

(17)

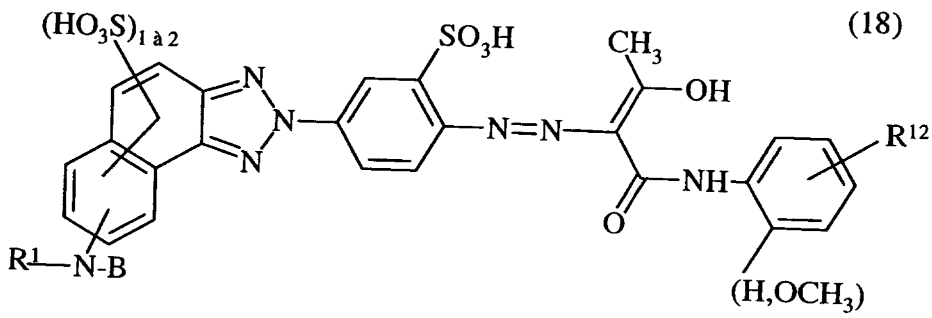

(18)

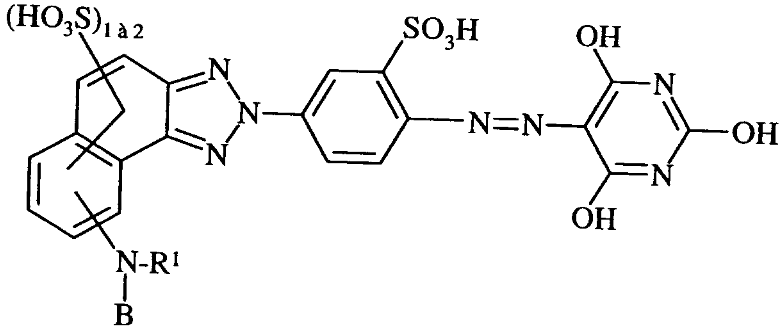

(19),

complexes métalliques et colorants de formules (20) à (50) :

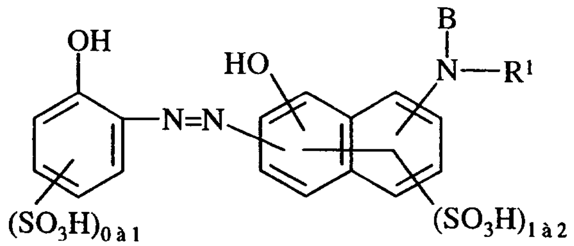

(20)

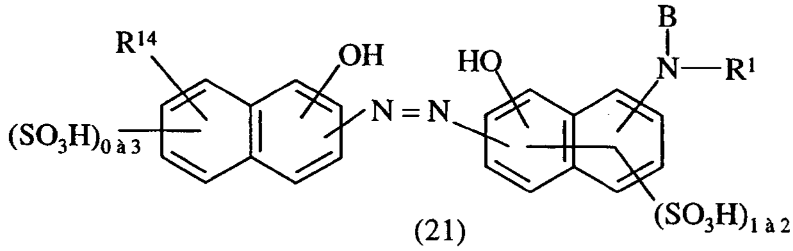

(21)

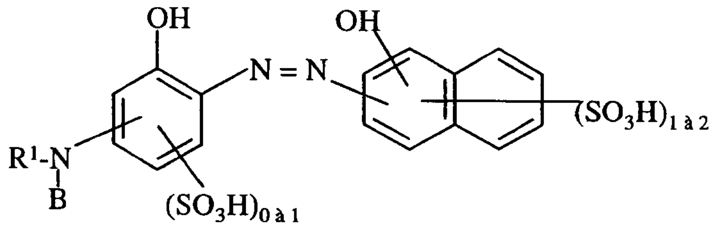

(22)

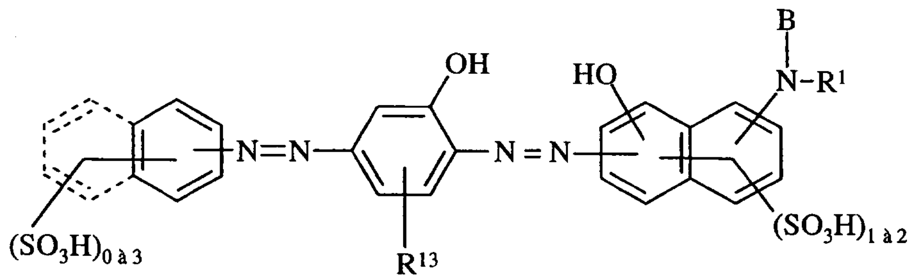

(23)

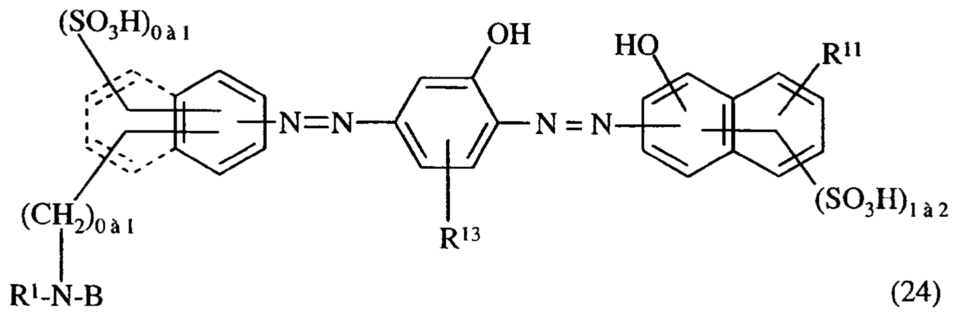

(24)

(25)

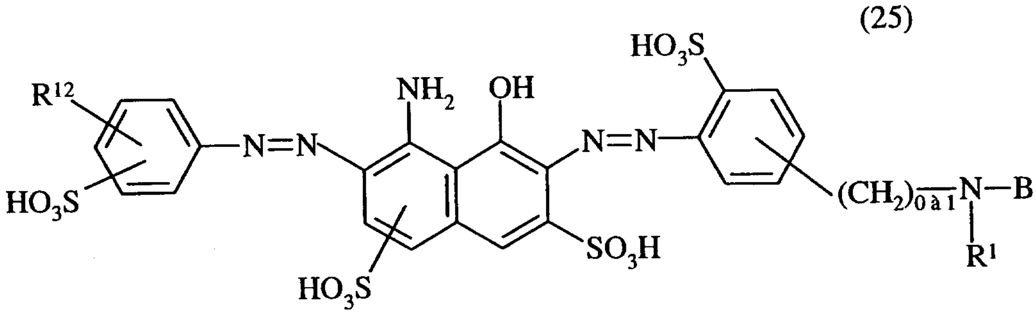

(26)

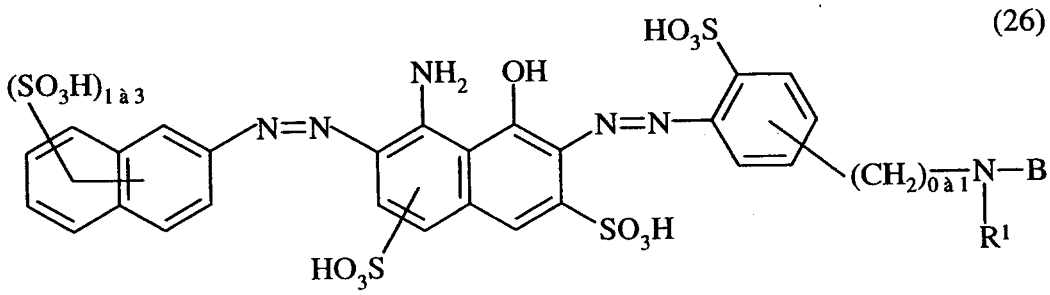

(27)

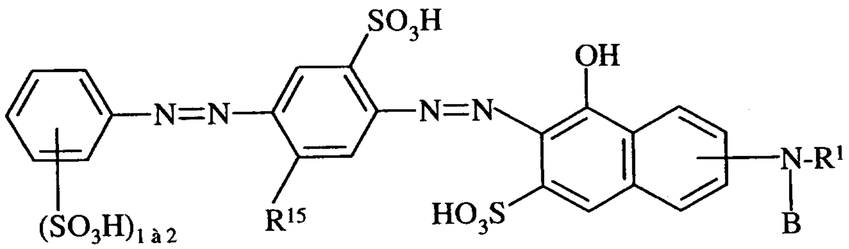

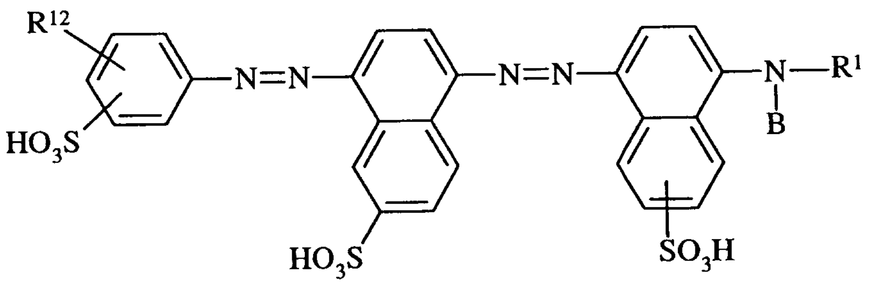

(28)

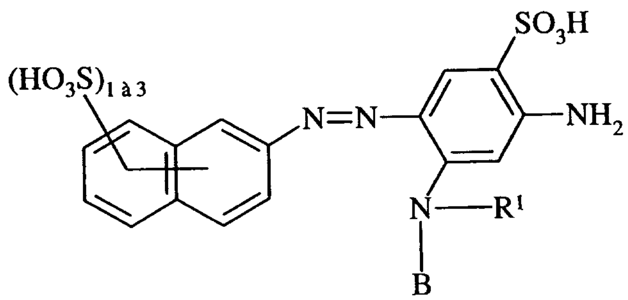

(29)

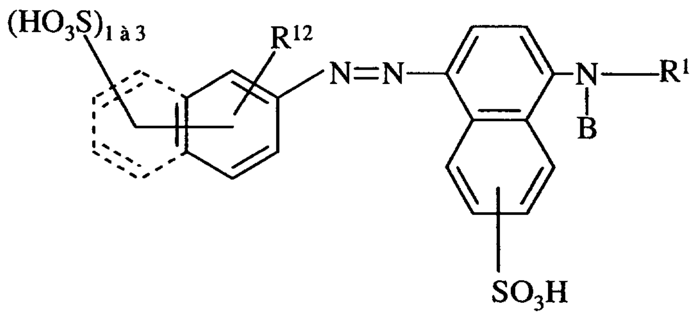

(30)

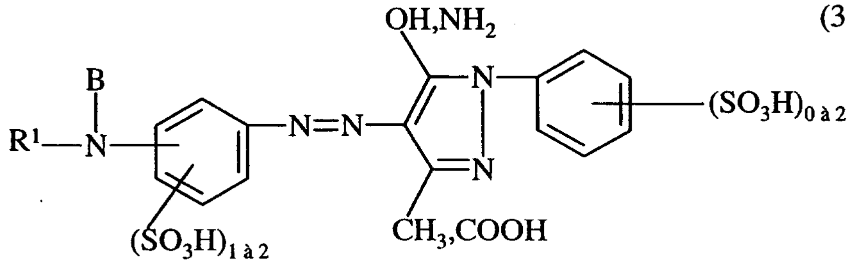

(31)

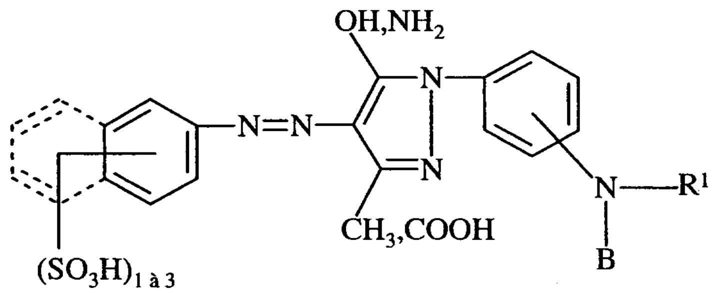

(32)

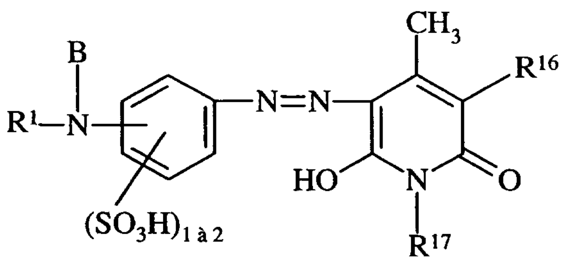

(33)

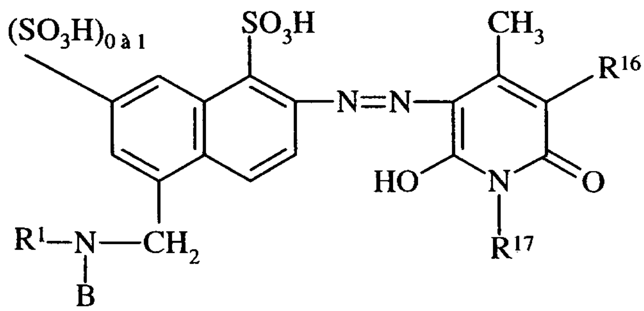

(34)

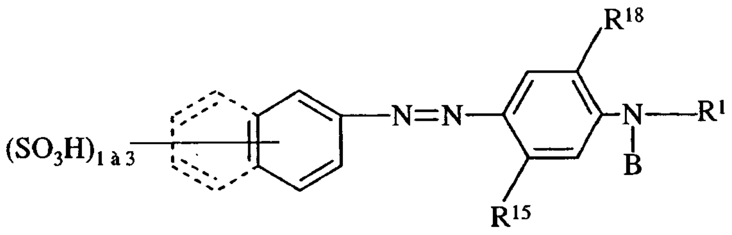

(35)

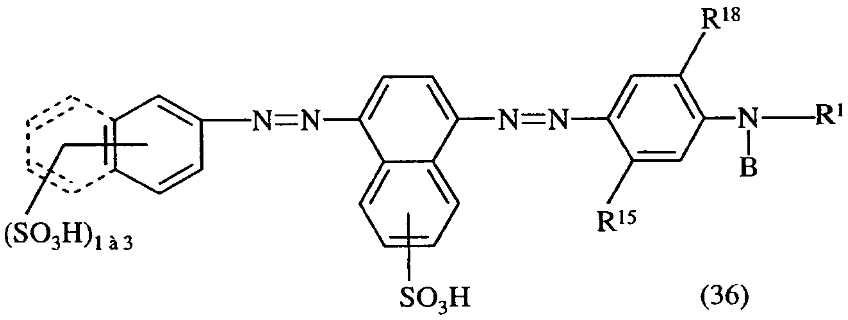

(36)

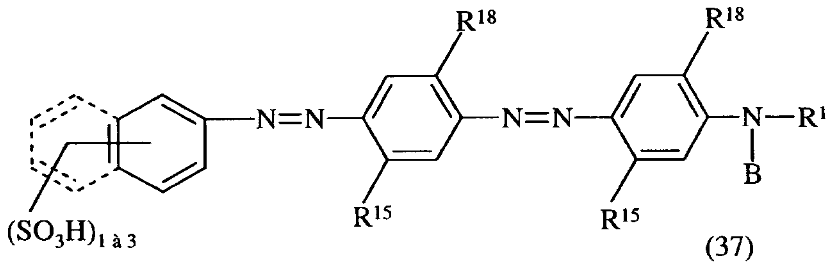

(37)

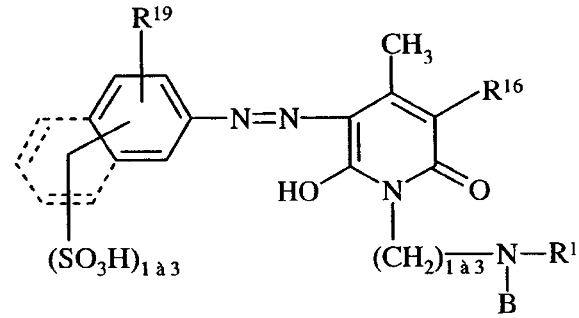

(38)

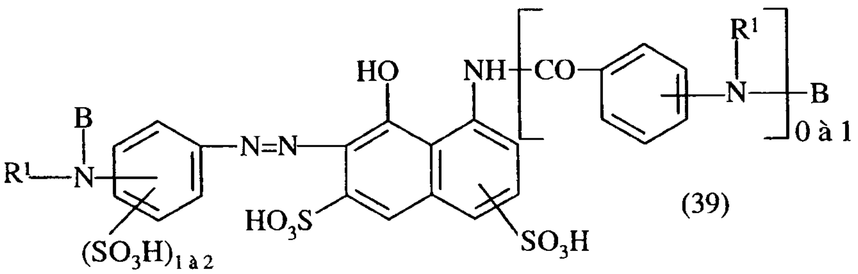

(39)

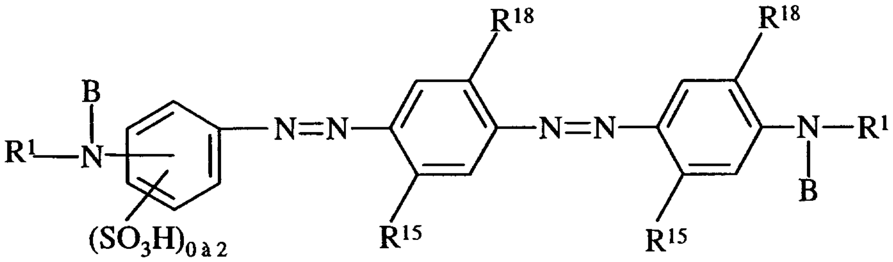

(40)

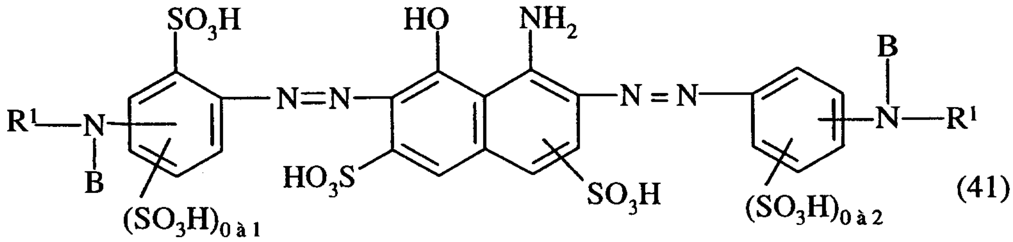

(41)

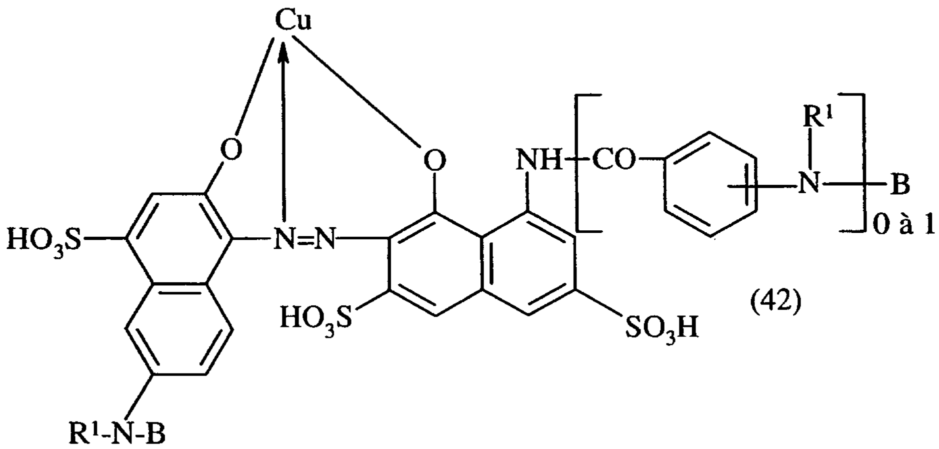

(42)

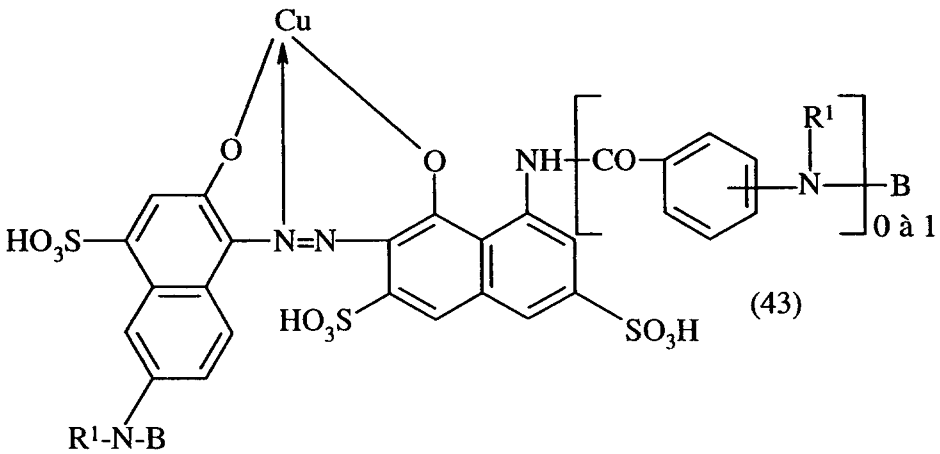

(43)

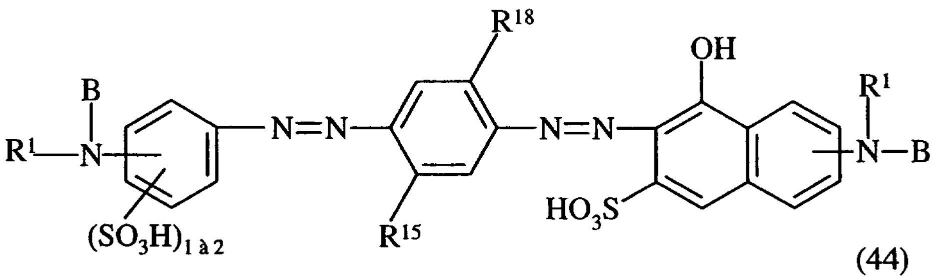

(44)

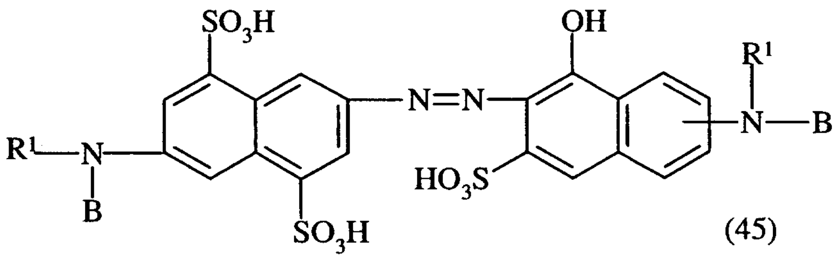

(45)

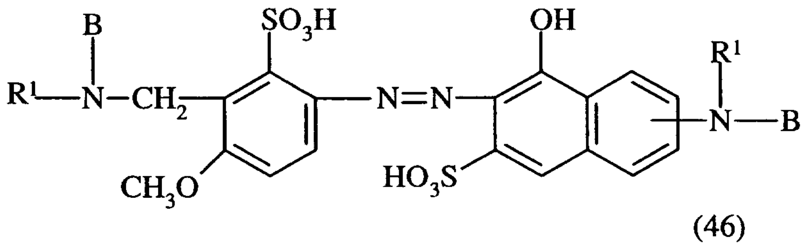

(46)

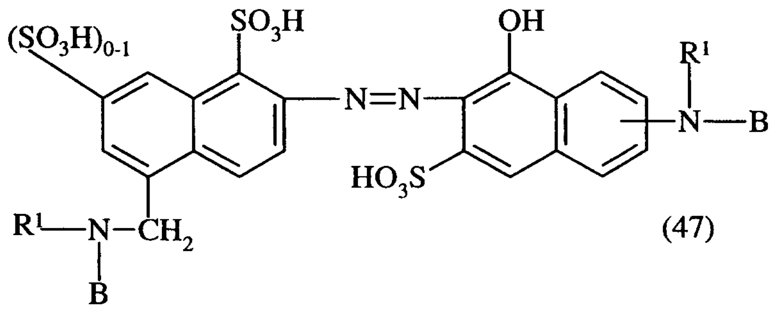

(47)

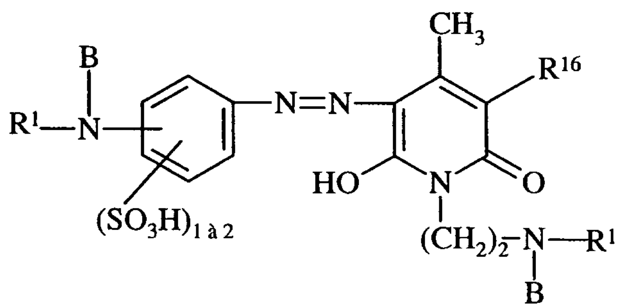

(48)

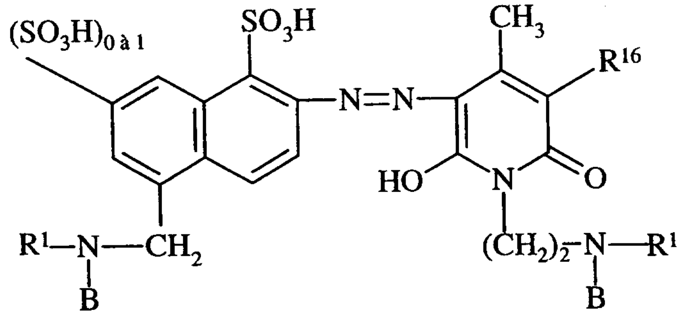

(49)

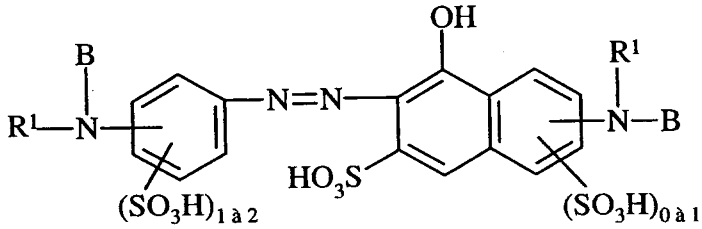

(50),

dans lesquelles

le groupe "acyle" est par exemple un groupe acétyle ou un groupe benzoyle éventuellement substitué,

$R^{17}$ = H, alkyle en $C_1$-$C_2$ éventuellement substitué par $SO_3H$, $NH_2$,

$R^1$ = H, $CH_3$ ou $C_2H_5$,

$R^{19}$ = H ou sulfo,

$R^{13}$ = H, $CH_3$, $OCH_3$ ou Cl,

$R^{12}$ = H, alkyle en $C_1$-$C_4$, alcoxy en $C_1$-$C_4$, Cl, Br, COOH, $SO_3H$,

$R^{14}$ = H, OH, $NH_2$, $NHCOCH_3$, NHCOPh, Cl, alcoxy en $C_1$-$C_4$ ou alkyle en $C_1$-$C_4$,

$R^{16}$ = H, $SO_3H$, $CH_2SO_3H$, Cl, alkylsulfonyle en $C_1$-$C_4$, CN, carboxamide, en particulier $CONH_2$,

$R^{15}$ = H, alkyle en $C_1$-$C_4$, alcoxy en $C_1$-$C_4$, Cl, Br, acylamino, plus spécialement (alkyle en $C_1$-$C_4$)carbonylamino ou arylcarbonylamino, tel que phénylcarbonylamino éventuellement substitué, alkylsulfonylamino en $C_1$-$C_4$, aminocarbonylamino, alkylsulfonylamino en $C_1$-$C_4$, arylsulfonylamino,

$R^{18}$ = H, alkyle en $C_1$-$C_4$, alcoxy en $C_1$-$C_4$, OH, $SO_3H$,

et les cycles condensés représentés en traits interrompus illustrent des variantes naphtaléniques possibles.

**7.** Colorants réactifs selon la revendication 1, caractérisés en ce que le radical D, de préférence le radical d'un colorant organique de la série des colorants monoazoïques ou polyazoïques, est substitué une ou deux fois par le groupe

$$Z'\text{-}O_2S\text{-}(CH_2)_{0\,à\,1}\text{—}$$

dans lequel

Z' représente $-CH=CH_2$, $-CH_2-CH_2-OSO_3H$, $-CH_2-CH_2-Cl$, $-CH_2-CH_2-Br$, $-CH_2-CH_2-S_2O_3H$, $-CH_2-CH_2-O-CO-CH_3$, $-CH_2-CH_2-OPO_3H_2$ ou $-CH_2-CH_2-OH$.

**8.** Colorants réactifs selon la revendication 7, caractérisés en ce que

n = 1,

W = liaison directe et

D : radical de formule générale (IX)

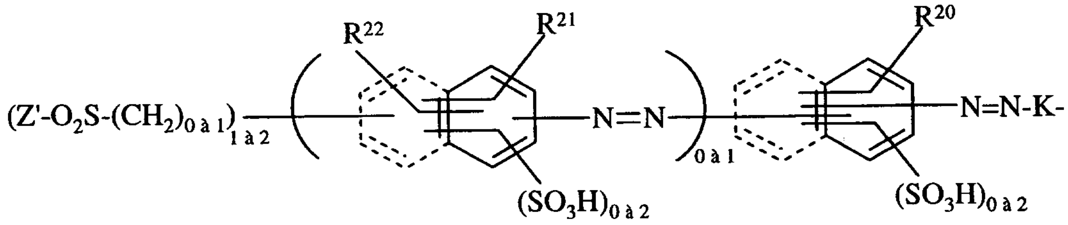

(IX)

dans laquelle

$R^{20}$ = H, alkyle on $C_1$-$C_4$, alcoxy en $C_1$-$C_4$, Cl, Br ou acylamino,

$R^{21}$ = H, alkyle on $C_1$-$C_4$, Cl, Br, alcoxy en $C_1$-$C_4$ ou COOH,

$R^{22}$ = H, alkyle on $C_1$-$C_4$, alcoxy en $C_1$-$C_4$, $SO_3H$, Cl ou Br,

K = radical bivalent de formule générale (Xa) à (Xd)

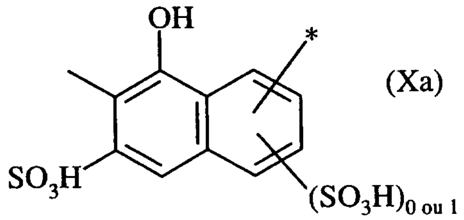

(Xa)

ou

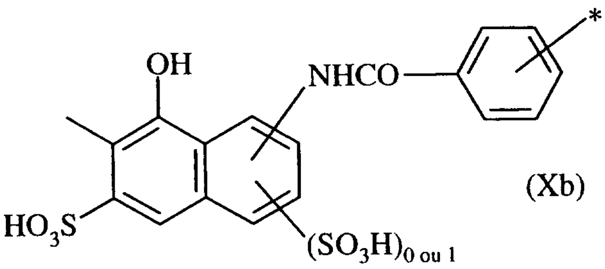

(Xb)

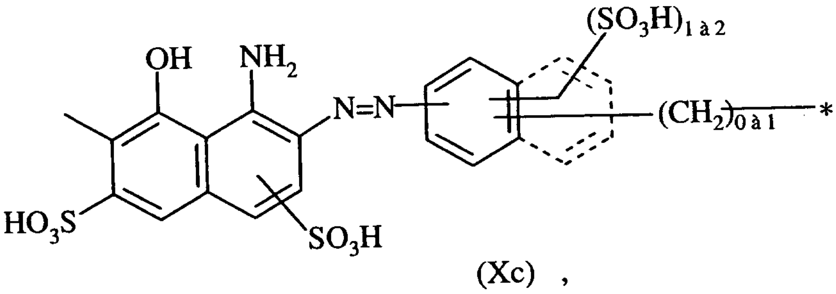

(Xc) ,

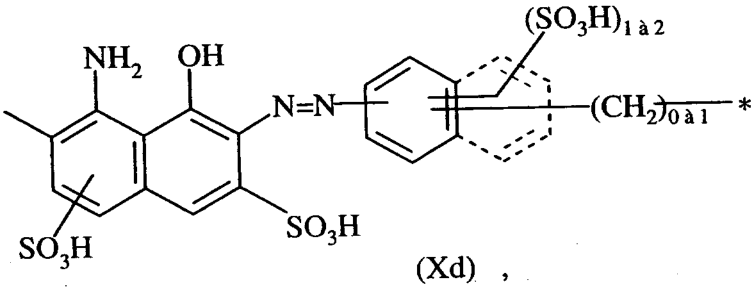

(Xd) ,

dans lesquelles les liaisons signalées par * sont reliées au groupe -NR[1]-B, B ayant les significations indiquées dans la revendication 4.

**9.** Colorants réactifs selon la revendication 7, caractérisés en ce qu'ils répondent à l'une des formules suivantes, dans lesquelles

B représente un groupe de formule

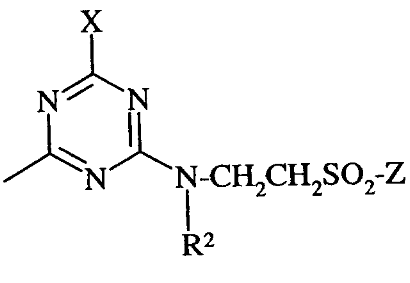

(VIa)

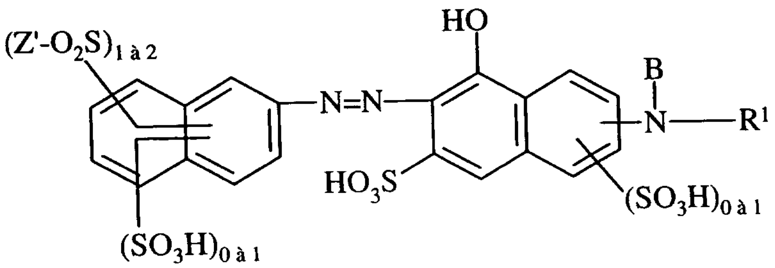

(51),

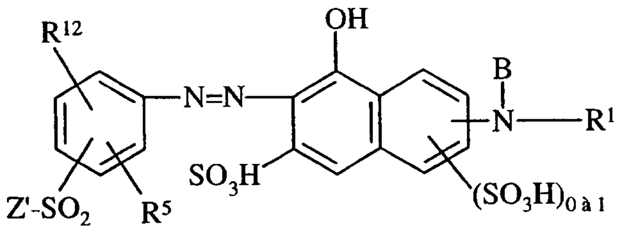

(52),

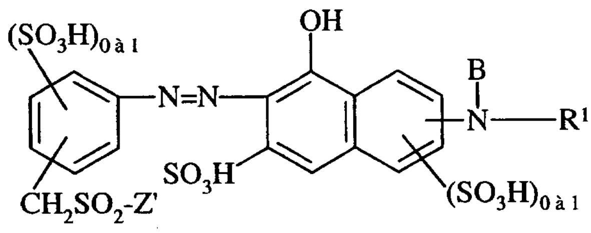

(53),

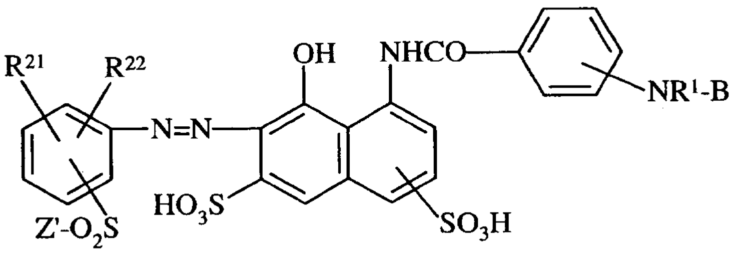

(54),

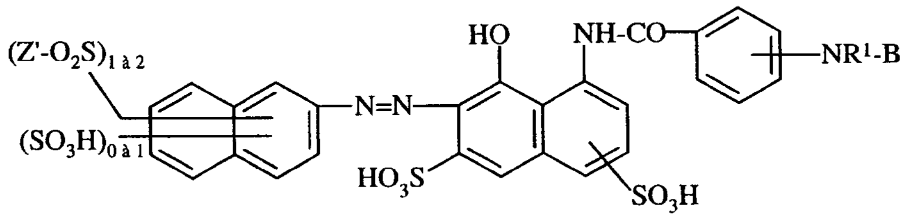

(55),

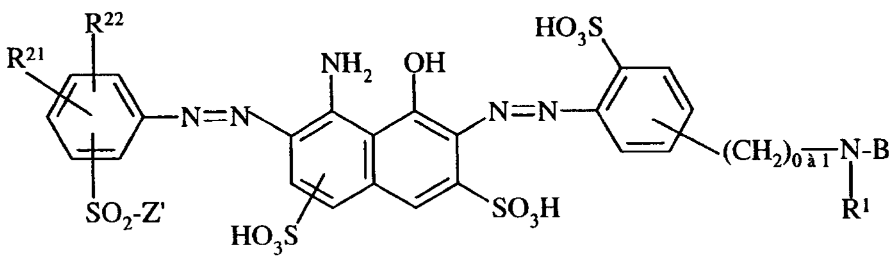

(56),

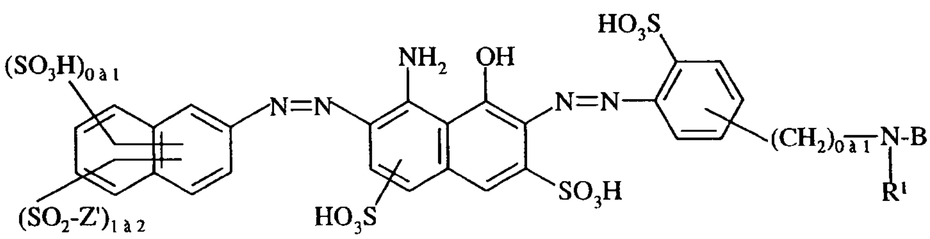

(57),

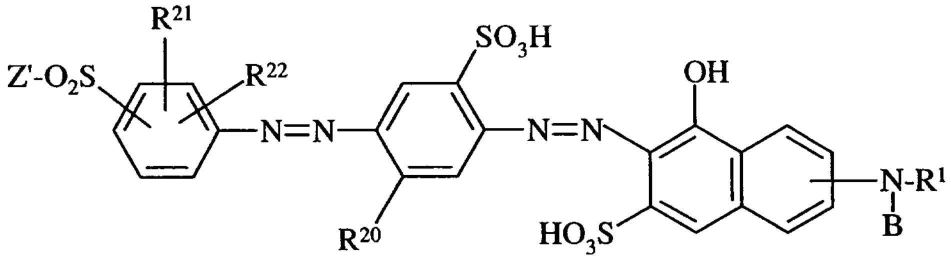

(58),

dans lesquelles

$R^1$ = H, $CH_3$ ou $C_2H_5$,

$R^{20}$= H, alkyle en $C_1$-$C_4$, alcoxy en $C_1$-$C_4$, acylamino, Cl ou Br,

$R^{21}$ = H, alkyle en $C_1$-$C_4$, alcoxy en $C_1$-$C_4$, Cl, Br ou COOH,

$R^{22}$ = H, alkyle en $C_1$-$C_4$, alcoxy en $C_1$-$C_4$, Cl, Br, $SO_3H$.

**10.** Colorants réactifs selon la revendication 1, de formule

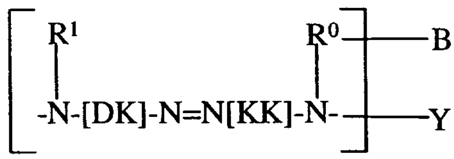

(XI)

dans laquelle

DK est le radical d'un composant diazotable de la série benzénique ou naphtalénique,

KK est le radical d'un copulant de formule

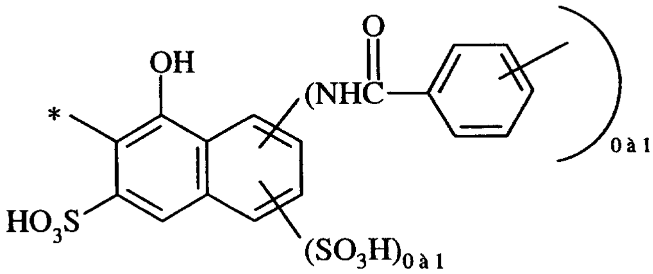

(XII)

dans laquelle le groupe de formule (XII) et le groupe azo sont reliés entre eux par la liaison signalée par *,

B = groupe de formule

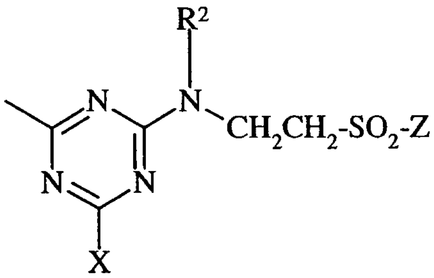

(VIa)

| | |
|---|---|
| Y = | groupe hétérocyclyclique réactif avec les fibres, autre que B, |
| $R^0$, $R^1$ = | indépendamment l'un de l'autre H, alkyle en $C_1$-$C_6$ éventuellement substitué, et |
| $R^2$, X et Z | ont les significations indiquées dans la revendication 1. |

**11.** Colorants réactifs selon la revendication 10, de formules (61) à (72)

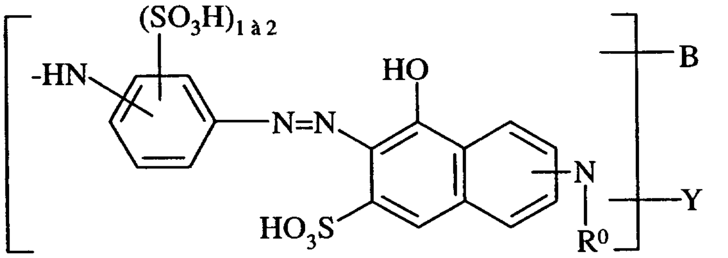

(61)

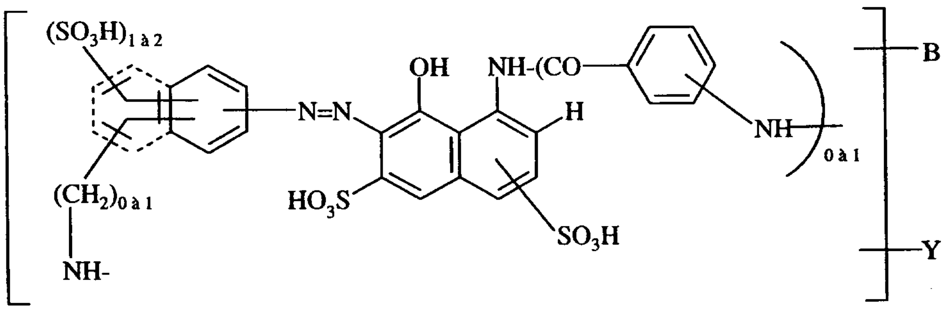

(62)

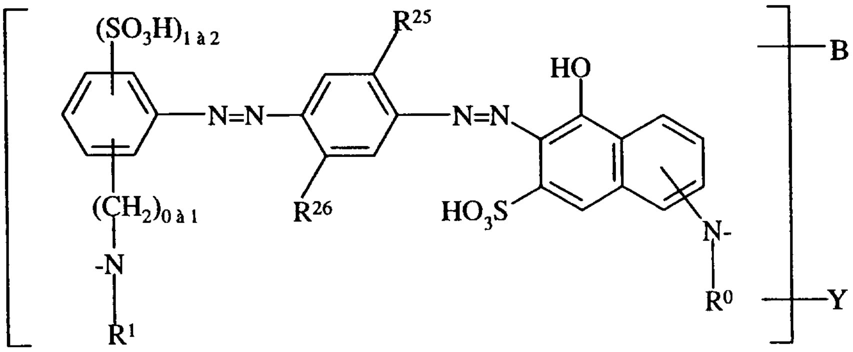

(63)

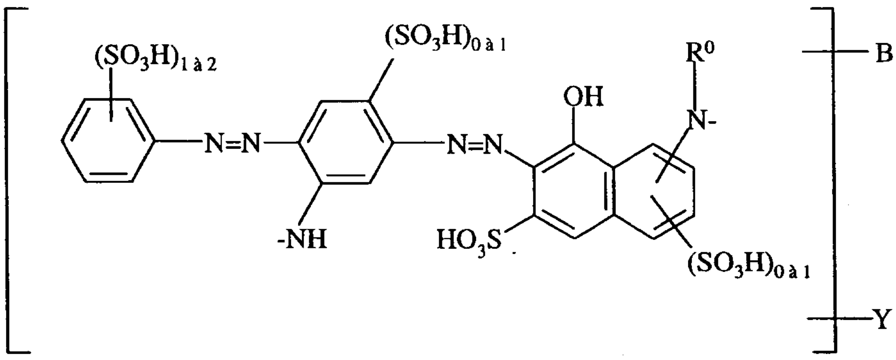
(64)

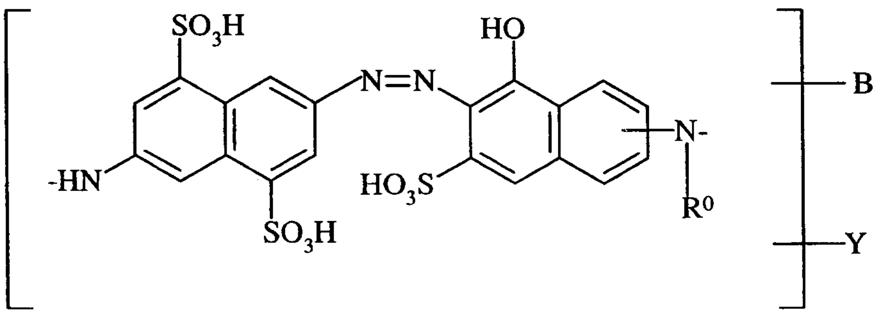
(65)

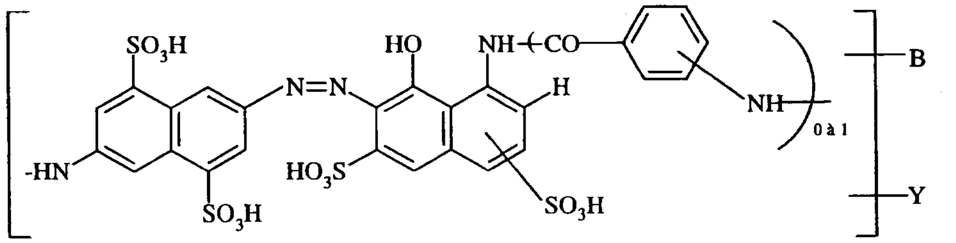
(66)

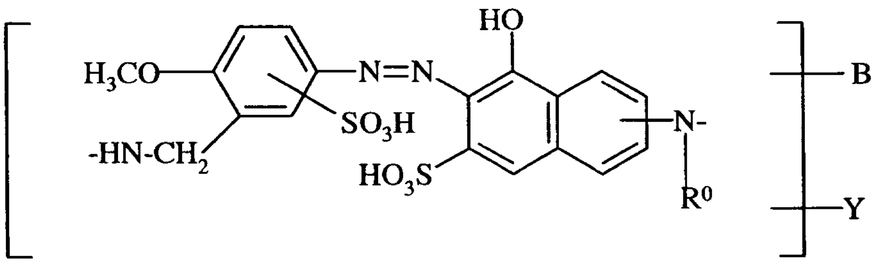
(67)

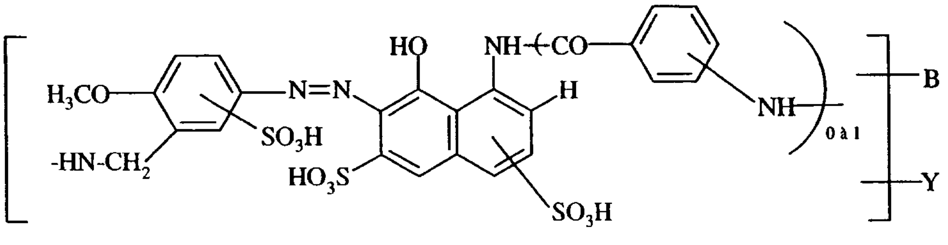
(68)

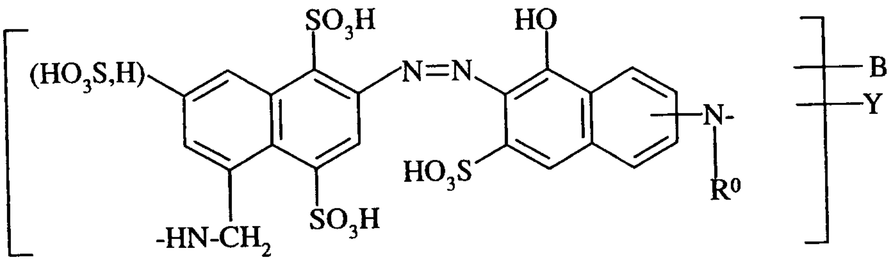

(69)

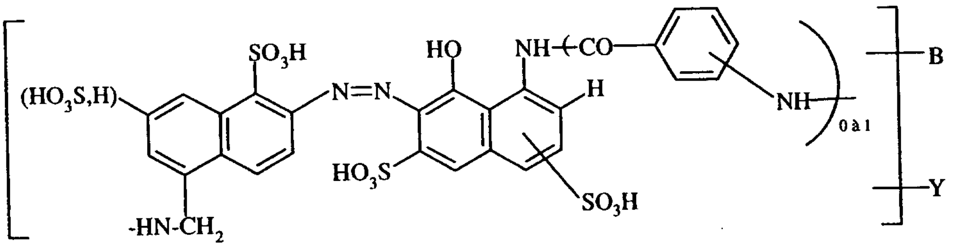

(70)

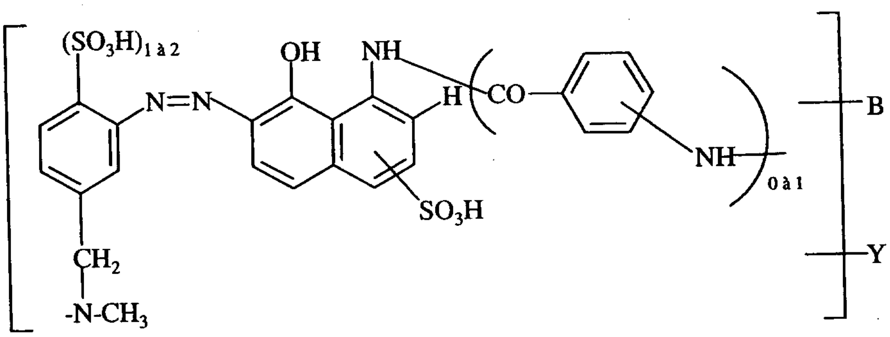

(71)

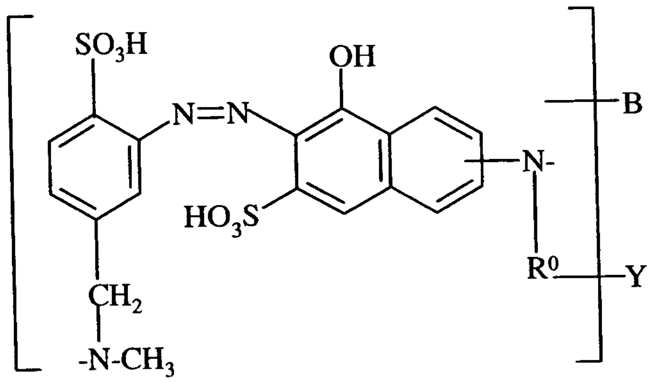

(72)

dans lesquelles

$R^0$ = H, $CH_3$ ou $C_2H_5$, et
$R^{25}$ = H, alkyle en $C_1$-$C_4$, alcoxy en $C_1$-$C_4$, OH ou $SO_3H$,
$R^{26}$ = H, alkyle en $C_1$-$C_4$, alcoxy en $C_1$-$C_4$ ou acylamino, X et Z ayant les significations indiquées dans la revendication 1.

**12.** Procédé de préparation des colorants réactifs de formule

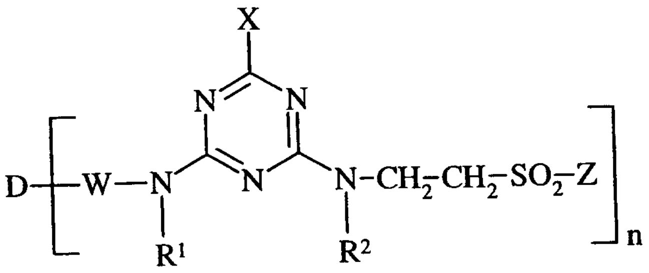

(I),

dans laquelle

D représente le radical d'un colorant organique des classes des colorants monoazoïques, polyazoïques, azoïques-complexes métalliques, anthraquinoniques, des phtalocyanines, des colorants de formazane, d'azométhine, de dioxazine, de phénazine, des colorants stilbéniques, des colorants de triphénylméthane, de xanthène, de thioxanthone ou des colorants nitroaryliques,

W représente une liaison directe ou un pont,

$R^1$ représente H ou un groupe alkyle en $C_1$-$C_4$ éventuellement substitué,

$R^2$ représente un groupe alkyle en $C_1$-$C_4$, plus spécialement un groupe méthyle,

X représente F, Cl ou Br,

Z représente $-CH=CH_2$ ou $-CH_2-CH_2-OSO_3H$ et

n est égal à 1 ou 2,

a) soit par condensation de colorants de formule

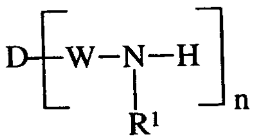

(II),

dans laquelle

D, W, $R^1$ et n ont les significations indiquées ci-dessus,
avec n mol de trihalogénotriazines de formule

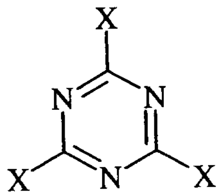

(III)

donnant des composés de formule

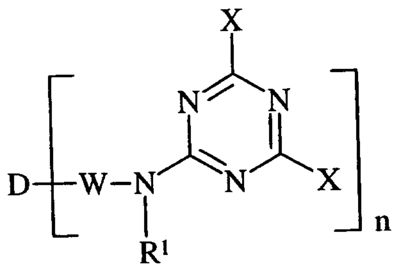

(IV)

qu'on condense ensuite avec n mol des composants de formule

$$R^2\text{-NH-}CH_2CH_2\text{-}SO_2\text{-Z} \quad (V),$$

dans laquelle
$R^2$ et Z ont les significations indiquées ci-dessus,

b) soit, dans l'ordre inverse, par condensation des trihalogénotriazines de formule (III) avec les composants de formule (V), donnant les produits de condensation primaires de formule

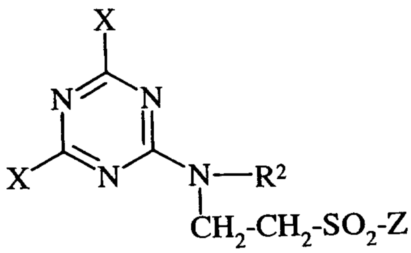

(VI),

dans laquelle

$R^2$ et Z ayant les significations indiquées ci-dessus,

qu'on condense ensuite en quantité de n mol avec les colorants de formule (II),
c) soit par condensation de produits intermédiaires appropriés avec les trihalogénotriazines (III) et les composants de formule (V) ou par condensation de produits intermédiaires appropriés avec les produits de condensation primaire de formule (VI), en faisant suivre de la synthèse des colorants.

**13.** Procédé de préparation des composés de formule (V)

$$R^2\text{-NH-}CH_2CH_2\text{-}SO_2\text{-Z}$$

dans laquelle

$R^2$ représente un groupe alkyle en $C_1$-$C_4$, plus spécialement méthyle et
Z représente $-CH_2CH_2-OSO_3H$ ou $-CH=CH_2$,

caractérisé en ce que l'on convertit des composés de formule

$$R^2\text{-NH-}C_2H_4\text{-S-}C_2H_4\text{-OH},$$

eux-mêmes obtenus par réaction de 3-alkyl-2-oxo-oxazolidinones de formule générale

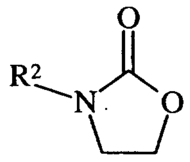

avec le 2-mercaptoéthanol, en composés de formule

$$R^2\text{-NH-}CH_2CH_2SO_2\text{-}CH_2CH_2OH$$

par oxydation, en particulier à l'aide de $H_2O_2$, après quoi on convertit de la manière habituelle en composés de formule générale (V).

**14.** Utilisation des colorants réactifs selon la revendication 1 pour la teinture ou l'impression de matières naturelles ou synthétiques contenant des groupes hydroxy ou amide.

**15.** Produits textiles contenant des matières à groupes hydroxy ou amide teintes par des colorants selon la revendication